# EUROPEAN PATENT SPECIFICATION

(11) **EP 4 509 578 B1**
(45) Date of publication and mention of the grant of the patent: **09.07.2025**
(21) Application number: 24170932.8
(22) Date of filing: 18.04.2024
(51) Int. Cl.: C09K 11/06, C07D 405/14, H10K 50/00, H10K 85/60, C07D 495/04, H10K 50/11, H10K 50/16, H10K 50/18, H10K 85/40, H10K 99/00

(54) **HETEROCYCLIC COMPOUND AND ORGANIC ELECTROLUMINESCENT DEVICE THEREOF**
HETEROCYCLISCHE VERBINDUNG UND ORGANISCHE ELEKTROLUMINESZENZVORRICHTUNG DAFÜR
COMPOSÉ HÉTÉROCYCLIQUE ET DISPOSITIF ÉLECTROLUMINESCENT ORGANIQUE ASSOCIÉ

(30) Priority: 16.08.2023 CN 202311034007
(43) Date of publication of application: 19.02.2025
(73) Proprietor: Changchun Hyperions Technology Co., Ltd., Bayhood Science and Technology Development Zone Changchun City Jilin 130000 (CN)
(72) Inventor: GUO, Jianhua, Changchun City, 130000 (CN); MIAO, Yuhe, Changchun City, 130000 (CN); DU, Mingzhu, Changchun City, 130000 (CN)
(74) Representative: Plougmann Vingtoft a/s

(56) References cited:
- WO-A1-2022/060042
- CN-A- 112 480 069
- CN-B- 108 409 773

## Description

### TECHNICAL FIELD

The present disclosure relates to the technical field of organic electroluminescent materials and specifically, relates to a heterocyclic compound and an organic electroluminescent device thereof.

### BACKGROUND

An organic light-emitting diode (OLED) has features such as high brightness, a wide selection range of materials, a low drive voltage and full-curing active light emission and has advantages such as high clarity, a wide angle of view and a high-speed response that can smoothly display an animation. The OLED is a hot research field in more than a recent decade and widely applied to high-end products in fields such as flat-panel display, lamp lighting and micro-display.

A light-emitting principle of an organic electroluminescent device is as follows: under an action of an applied electric field, holes and electrons are injected from an anode and a cathode, respectively, and recombined in a light-emitting layer to form excitons, the excitons transfer energy to organic light-emitting molecules so that the organic light-emitting molecules transit from a ground state to an excited state, excited molecules are in an unstable state, and when the excited molecules return from the excited state to the ground state, energy is released in a light form so that a light emission phenomenon occurs. At present, a device structure of the OLED is mostly sandwich-shaped and includes a cathode, an anode and organic layers disposed between the cathode and the anode. The organic layers are also divided into a hole injection layer, a hole transport layer, a hole blocking layer, an electron blocking layer, an electron injection layer, an electron transport layer, a light-emitting layer and a light extraction layer according to different functions of the organic layers.

With the continuous development of the organic electroluminescent device, the development of various functional materials has been far from meeting market requirements so that an organic electroluminescent material has also become a research hotspot in the field. At present, the organic electroluminescent device has the problems such as a high drive voltage, low luminescence efficiency and high power consumption. This is because an electron transport material has low electron transport efficiency, transport of electrons and holes is imbalanced and the electrons and the holes cannot be effectively transported to the light-emitting layer. Moreover, energy levels between functional layers are mismatched, which causes some electrons and holes to escape from the light-emitting layer, resulting in the decrease of the luminescence efficiency and the increase of the drive voltage. The light-emitting layer also has the problems such as imbalanced migrations of electrons and holes and a mismatch between triplet energy levels of host and guest materials so that efficiency of forming excitons through the recombination of the electrons and the holes is also low, thereby affecting the luminescence efficiency of the organic electroluminescent device.

The optimization and performance improvement of the OLED device can be achieved by improving materials of different functional layers in the device. Therefore, for the problems in the electron transport material and a material of the light-emitting layer at present, an electron transport material, a hole blocking material and a host material of the light-emitting layer with more excellent performance need to be developed.

### SUMMARY

For the problems such as a high drive voltage, low luminescence efficiency and a short lifetime of an organic electroluminescent device in the related art, the present disclosure provides a heterocyclic compound, which can significantly improve the above problems, so that the organic electroluminescent device has a low drive voltage, high luminescence efficiency and a long service life.

Specifically, the present disclosure provides a heterocyclic compound, wherein the heterocyclic compound has a structure represented by Formula I: 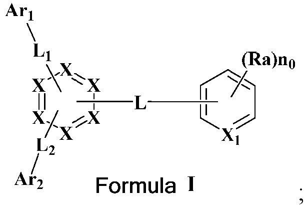
wherein in Formula I, X is independently selected from C(R₂) or an N atom, at least one X is selected from an N atom, and X bonded to Ar₁, Ar₂ or L is selected from a C atom;
X₁ is independently selected from a C atom or an N atom;
R₂ is independently selected from any one of hydrogen, deuterium, tritium, halogen, cyano, nitro, substituted or unsubstituted C1 to C12 alkyl, substituted or unsubstituted silyl, substituted or unsubstituted C1 to C12 alkoxy, substituted or unsubstituted C3 to C12 cycloalkyl, substituted or unsubstituted C2 to C12 heterocycloalkyl, substituted or unsubstituted C6 to C30 aryl or substituted or unsubstituted C2 to C30 heteroaryl;
Ar₁ and Ar₂ are independently selected from any one of substituted or unsubstituted C6 to C30 aryl or substituted or unsubstituted C2 to C30 heteroaryl;
L₁ and L₂ are independently selected from any one of a single bond, substituted or unsubstituted C6 to C30 arylene or substituted or unsubstituted C2 to C30 heteroarylene;
Ra is independently selected from any one of hydrogen, deuterium, halogen, cyano, nitro, substituted or unsubstituted C1 to C12 alkyl, substituted or unsubstituted silyl, substituted or unsubstituted C1 to C12 alkoxy, substituted or unsubstituted C3 to C12 cycloalkyl or substituted or unsubstituted C2 to C12 heterocycloalkyl;
n₀ is independently selected from 0, 1, 2, 3, 4 or 5, when n₀ is greater than 1, two or more Ra are the same as or different from each other, or two adjacent Ra are joined to form a substituted or unsubstituted benzene ring;
L is independently selected from any one of the following groups: 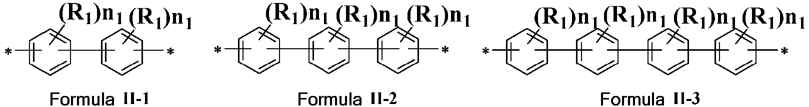 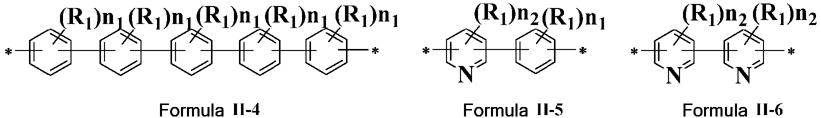 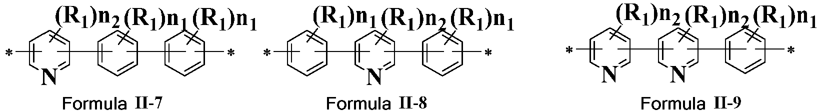 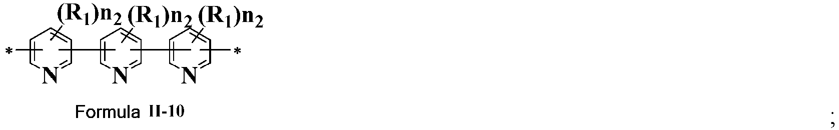
wherein R₁ is independently selected from any one of hydrogen, deuterium, halogen, cyano, nitro, substituted or unsubstituted C1 to C12 alkyl, substituted or unsubstituted silyl, substituted or unsubstituted C1 to C12 alkoxy, substituted or unsubstituted C3 to C12 cycloalkyl or substituted or unsubstituted C2 to C12 heterocycloalkyl;
n₁ is independently selected from 0, 1, 2, 3 or 4; n₂ is independently selected from 0, 1, 2 or 3; when n₁ is greater than 1, two or more R₁ are the same as or different from each other, or two adjacent R₁ are joined to form a substituted or unsubstituted benzene ring;
with the proviso that one or more hydrogens in at least one group of Ar₁, Ar₂, L, L₁, L₂, R₂ or Ra are substituted with a group represented by Formula III: 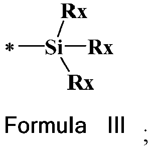 Rx is independently selected from any one of hydrogen, deuterium, cyano, nitro, halogen, substituted or unsubstituted C1 to C12 alkyl, substituted or unsubstituted C2 to C12 alkenyl , substituted or unsubstituted C3 to C12 cycloalkyl or substituted or unsubstituted C2 to C12 heterocycloalkyl; and
the present disclosure does not include the following compound: 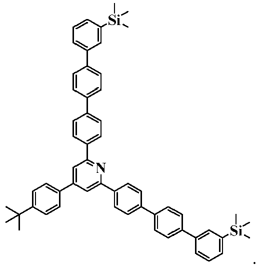
WO2022/060042A1 and CN112480069A disclose heterocyclic compounds similar to Formula I of the present invention.

The present disclosure further provides an organic electroluminescent device, wherein the organic electroluminescent device includes an anode, a cathode facing the anode and an organic layer located between the anode and the cathode or outside at least one electrode of the anode or the cathode, wherein the organic layer includes any one or more of the heterocyclic compounds of the present disclosure.

The heterocyclic compound provided in the present disclosure has a relatively large conjugate so that electron migration efficiency can be improved, thereby balancing transport of electrons and holes. Moreover, the heterocyclic compound further has proper HOMO and LUMO energy levels, thereby reducing a transport barrier in an electron migration process and reducing the drive voltage. Moreover, the holes can also be blocked to escape to an interface of a light-emitting layer to avoid the recombination of the holes and the electrons at the interface of the light-emitting layer, thereby reducing a loss of the lifetime of the device and improving the luminescence efficiency of the device. Moreover, the heterocyclic compound has the relatively large conjugate and a relatively large steric hindrance so that a glass transition temperature is further improved and the heterocyclic compound is not easy to decompose at a high temperature and has good chemical stability and thermal stability, thereby improving a form of evaporation film formation and prolonging the lifetime of the device.

### DETAILED DESCRIPTION

The present disclosure is further described below in conjunction with embodiments. It is to be understood that the embodiments described below are intended to describe the present disclosure and not to limit the scope of the present disclosure.

In a compound of the present disclosure, any atom not specified as a particular isotope is included as any stable isotope of the atom and includes atoms between its natural isotope abundance and its unnatural abundance.

A halogen atom in the present disclosure includes fluorine, chlorine, bromine and iodine.

In the present disclosure, when a position of a substituent on an aromatic ring is unfixed, it indicates that the substituent may be linked to any of the corresponding optional sites of the aromatic ring. For example, 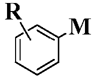 may represent 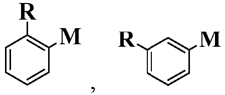 or 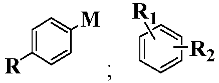 may represent 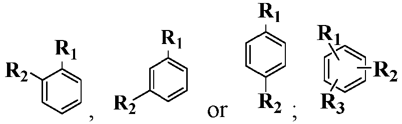 may represent 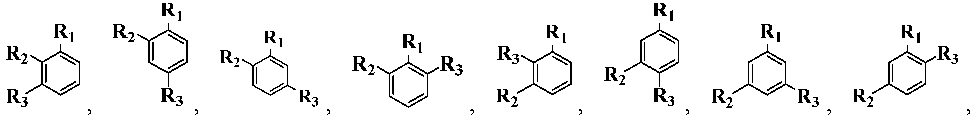 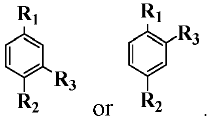 The same is true in other cases.

In the present disclosure, an expression that "two adjacent groups are joined to form a ring" means that adjacent groups are bound to each other and optionally aromatized to form a substituted or unsubstituted aromatic ring, heteroaromatic ring, aliphatic ring or aliphatic heterocyclic ring. The "adjacent groups" refer to two substituents on two directly joined atoms, a substituent disposed closest to a corresponding substituent in space, or another substituent on an atom with a corresponding substituent. For example, two substituents substituting at ortho positions of a benzene ring or two substituents on the same carbon atom in an alicyclic ring may be considered "adjacent" to each other.

The aliphatic ring or the aliphatic heterocyclic ring may be a saturated ring or an unsaturated ring. Specifically, the ring formed through the joining may be a three-membered ring, a four-membered ring, a five-membered ring, a six-membered ring, a seven-membered ring, a spirocyclic ring or a fused ring. The number of ring-forming carbon atoms of the formed aromatic ring is preferably 6 to 30 carbon atoms, particularly preferably 6 to 18 carbon atoms and most preferably 6 to 12 carbon atoms. The number of ring-forming carbon atoms of the formed heteroaromatic ring is preferably 2 to 30 carbon atoms, particularly preferably 2 to 18 carbon atoms and most preferably 2 to 12 carbon atoms. The number of ring-forming carbon atoms of the formed aliphatic ring is preferably 3 to 30 carbon atoms, particularly preferably 3 to 18 carbon atoms, more preferably 3 to 12 carbon atoms and most preferably 3 to 8 carbon atoms. The number of ring-forming carbon atoms of the formed aliphatic heterocyclic ring is preferably 3 to 30 carbon atoms, particularly preferably 2 to 18 carbon atoms, more preferably 2 to 12 carbon atoms and most preferably 2 to 8 carbon atoms. Further, the ring formed through the joining may have, but is not limited to, the following cases: for example, benzene, naphthalene, indene, cyclopentene, cyclopentane, cyclopentanobenzene, cyclohexene, cyclohexane, cyclohexanobenzene, pyridine, quinoline, isoquinoline, benzofuran, benzothiophene, dibenzofuran, dibenzothiophene, phenanthrene or pyrene.

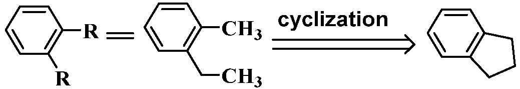

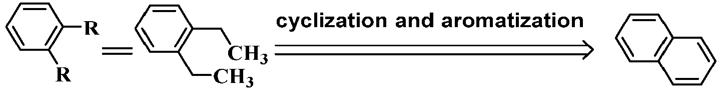

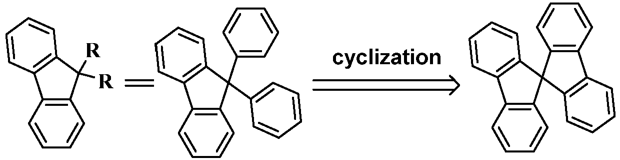

For "substituted or unsubstituted" in the present disclosure, for example, "substituted" in "substituted or unsubstituted alkyl, substituted or unsubstituted silyl, substituted or unsubstituted alkoxy, substituted or unsubstituted cycloalkyl, substituted or unsubstituted aryl, substituted or unsubstituted heteroaryl, substituted or unsubstituted arylene or substituted or unsubstituted heteroarylene" indicates that at least one hydrogen atom on a group is substituted with a substituent. When multiple hydrogens are substituted with multiple substituents, the multiple substituents may be the same or different. Substituents represented by "substituted" in the above "substituted or unsubstituted" include, but are not limited to, the following groups: deuterium, tritium, cyano, nitro, hydroxyl, a halogen atom, substituted or unsubstituted C1 to C12 alkyl, substituted or unsubstituted C2 to C12 alkenyl, substituted or unsubstituted C3 to C12 cycloalkyl, substituted or unsubstituted C6 to C30 aryl, substituted or unsubstituted C2 to C30 heteroaryl, substituted or unsubstituted C1 to C12 alkoxy, substituted or unsubstituted C1 to C12 alkylthio, substituted or unsubstituted C1 to C12 alkylamino, substituted or unsubstituted C6 to C30 aryloxy and substituted or unsubstituted C6 to C30 arylamine. The substituents are preferably the following groups: deuterium, tritium, cyano, fluorine, chlorine, bromine, iodine, nitro, methyl, ethyl, propyl, butyl, pentyl, hexyl, heptyl, octyl, cyclopropyl, cyclobutyl, cyclopentyl, cyclohexyl, cyclobutenyl, cyclopentenyl, cyclohexenyl, cyclopentadienyl, cyclohexadienyl, adamantyl, norbornyl, trifluoromethyl, trifluoroethyl, trimethylsilyl, triethylsilyl, tri-*tert*-butylsilyl, triphenylsilyl, trideuteromethyl, methoxy, ethoxy, phenyl, biphenyl, terphenyl, naphthyl, phenanthryl, triphenylenyl, anthryl, pyrenyl, chrysenyl, fluoranthenyl, benzocyclopropyl, benzocyclobutyl, benzocyclopentyl, benzocyclohexyl, benzocycloheptyl, benzocyclobutenyl, benzcyclopentenyl, benzocyclohexenyl, 9,9-dimethylfluorenyl, 9,9-diphenylfluorenyl, 9-methyl-9-phenylfluorenyl, 9,9'-spirobifluorenyl, diphenylamino, pyridyl, pyrimidinyl, pyrazinyl, pyridazinyl, triazinyl, quinolyl, isoquinolyl, quinazolinyl, quinoxalinyl, benzoquinolyl, benzisoquinolyl, phenanthrolinyl, oxazolyl, benzoxazolyl, thiazolyl, benzothiazolyl, imidazolyl, benzimidazolyl, benzofuryl, dibenzofuryl, benzothienyl, dibenzothienyl, indolyl, carbazolyl, and the like. In addition, each of the preceding substituents may be substituted or unsubstituted, and two adjacent substituents may be linked to form a ring.

Alkyl in the present disclosure refers to a monovalent group obtained by removing one hydrogen atom from an alkane molecule. Alkyl may be linear alkyl or branched alkyl, preferably has 1 to 12 carbon atoms, more preferably has 1 to 8 carbon atoms and particularly preferably has 1 to 6 carbon atoms. Examples of alkyl may include, but are not limited to, methyl, ethyl, *n*-propyl, isopropyl, *n*-butyl, *tert*-butyl, isobutyl, *sec*-butyl, *n*-pentyl, isopentyl, *n*-hexyl, and the like.

Cycloalkyl in the present disclosure refers to a monovalent group obtained by removing one hydrogen atom from a cycloalkane molecule. Cycloalkyl preferably has 3 to 12 carbon atoms, more preferably has 3 to 10 carbon atoms and particularly preferably has 3 to 6 carbon atoms. Examples of cycloalkyl may include, but are not limited to, cyclopropyl, cyclobutyl, cyclopentyl, cyclohexyl, cycloheptyl, adamantyl, norbornyl, and the like..

Alkoxy in the present disclosure is represented by -O-alkyl. Alkoxy may be linear alkoxy or branched alkoxy, preferably has 1 to 12 carbon atoms, more preferably has 1 to 8 carbon atoms and particularly preferably has 1 to 6 carbon atoms. Examples may include, but are not limted to, methoxy, ethoxy, *n*-propoxy, isopropoxy, *n*-butoxy, isobutoxy, *tert-*butoxy, *n*-pentoxy, *n*-hexoxy, and the like.

Aryl in the present disclosure refers to the generic term of monovalent groups obtained by removing one hydrogen atom from the aromatic nucleus carbon of an aromatic compound molecule.. Aryl includes monocyclic aryl, polycyclic aryl, fusedcyclic aryl, or a combination thereof. Aryl preferably has 6 to 30 carbon atoms, particularly preferably has 6 to 18 carbon atoms and most preferably has 6 to 12 carbon atoms. Examples include, but are not limited to, the following groups: phenyl, biphenyl, terphenyl, tetraphenyl, naphthyl, phenanthryl, anthryl, triphenylenyl, fluorenyl, benzofluorenyl, spirobifluorenyl, benzospirobifluorenyl, pyrenyl, fluoranthenyl, chrysenyl, and the like.

Heteroaryl in the present disclosure refers to a monovalent group obtained by substituting one or more aromatic nucleus carbon atoms in an aromatic hydrocarbon molecule by heteroatoms, where the heteroatom includes, but is not limited to, an oxygen, sulfur, nitrogen, silicon or phosphorus atom. Heteroaryl preferably has 2 to 30 carbon atoms, particularly preferably has 2 to 18 carbon atoms and most preferably has 2 to 12 carbon atoms. Examples of heteroaryl include, but are not limited to, oxazolyl, benzoxazolyl, naphthoxazolyl, phenanthroxazolyl, anthroxazolyl, triphenylenoxazolyl, pyridoxazoly, thiazolyl, benzothiazolyl, naphthothiazolyl, phenanthrothiazolyl, anthrothiazolyl, triphenylenothiazolyl, pyridothiazolyl, imidazolyl, benzimidazolyl, naphthoimidazolyl, phenanthroimidazolyl, anthroimidazoly, triphenylenoimidazoly, pyridoimidazoly, spirofluorenexanthyl, spirofluorenethioxanthyl, furyl, benzofuryl, pyridofuryl, naphthofuryl, phenanthrofuryl, anthrofuryl, triphenylenofuryl, dibenzofuryl, benzodibenzofuryl, thienyl, benzothienyl, pyridothienyl, naphthothienyl, phenanthrothienyl, anthrothienyl, triphenylenothienyl, dibenzothienyl, benzodibenzothienyl, indolyl, naphthoindolyl, phenanthroindolyl, anthroindolyl, triphenylenoindolyl, carbazolyl, pyridyl, pyrimidinyl, bipyridyl, bipyrimidinyl, phenylpyridyl, phenylpyrimidinyl, pyrazinyl, pyridazinyl, triazinyl, quinolyl, isoquinolyl, quinazolinyl, quinoxalinyl, naphthyridinyl, ortho-phenanthrolinyl, benzoquinolyl, benzisoquinolyl, benzoquinazolinyl, benzoquinoxalinyl, and the like..

Silyl in the present disclosure refers to a monovalent group obtained by removing one hydrogen atom from a silane molecule and may be represented by a group shown in -Si(Rs)(Rs)(Rs), where Rs is selected from hydrogen, deuterium, cyano or halogen or selected from any one or more of the above alkyl, alkenyl, alkoxy or cycloalkyl. Silyl preferably has 1 to 30 carbon atoms, preferably has 1 to 25 carbon atoms, more preferably has 1 to 22 carbon atoms and most preferably has 1 to 18 carbon atoms. Examples of silyl may include, but are not limited to, trimethylsilyl, triethylsilyl, triisopropylsilyl, tri-*tert*-butylsilyl, dimethylethylsilyl, dimethyl-*tert*-butylsilyl, diethylmethyllsilyl, tricyclopropylsilyl, tricyclobutylsilyl, and the like..

The aliphatic ring in the present disclosure may be a saturated ring or an unsaturated ring, may include cycloalkane, cycloolefin, cycloalkyne, and the like. The aliphatic ring preferably has 3 to 25 carbon atoms, more preferably 3 to 20 carbon atoms, particularly preferably 3 to 15 carbon atoms, preferably 5 to 10 carbon atoms, and most preferably 5 to 7 carbon atoms. Examples of the aliphatic ring may include, but are not limited to, cyclopropane, cyclobutane, cyclopentane, cyclohexane, cycloheptane, adamantane, norbornene alkanes, cyclopropene, cyclobutene, cyclopentene, cyclohexene, cycloheptene, and the like.

Arylene in the present disclosure refers to aryl having two binding positions, that is, a divalent group. In addition to being the divalent group, arylene may be applicable to the above description of aryl.

Heteroarylene in the present disclosure refers to heteroaryl having two binding positions, that is, a divalent group. In addition to being the divalent group, heteroarylene may be applicable to the above description of heteroaryl.

"At least one" or "one or more" in the present disclosure include, if permitted, one, two, three, four, five, six, seven, eight or more.

The present disclosure provides a heterocyclic compound, wherein the heterocyclic compound has a structure represented by Formula I: 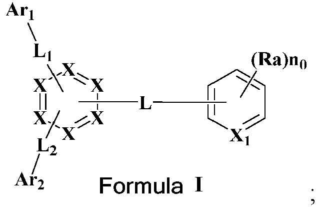
wherein in Formula I, X is independently selected from C(R₂) or an N atom, at least one X is selected from an N atom, and X bonded to Ar₁, Ar₂ or L is selected from a C atom;
X₁ is independently selected from a C atom or an N atom;
R₂ is independently selected from any one of hydrogen, deuterium, tritium, halogen, cyano, nitro, substituted or unsubstituted C1 to C12 alkyl, substituted or unsubstituted silyl, substituted or unsubstituted C1 to C12 alkoxy, substituted or unsubstituted C3 to C12 cycloalkyl, substituted or unsubstituted C2 to C12 heterocycloalkyl, substituted or unsubstituted C6 to C30 aryl or substituted or unsubstituted C2 to C30 heteroaryl;
Ar₁ and Ar₂ are independently selected from any one of substituted or unsubstituted C6 to C30 aryl or substituted or unsubstituted C2 to C30 heteroaryl;
L₁ and L₂ are independently selected from any one of a single bond, substituted or unsubstituted C6 to C30 arylene or substituted or unsubstituted C2 to C30 heteroarylene;
Ra is independently selected from any one of hydrogen, deuterium, halogen, cyano, nitro, substituted or unsubstituted C1 to C12 alkyl, substituted or unsubstituted silyl, substituted or unsubstituted C1 to C12 alkoxy, substituted or unsubstituted C3 to C12 cycloalkyl or substituted or unsubstituted C2 to C12 heterocycloalkyl;
n₀ is independently selected from 0, 1, 2, 3, 4 or 5, when n₀ is greater than 1, two or more Ra are the same as or different from each other, or two adjacent Ra are joined to form a substituted or unsubstituted benzene ring;
L is independently selected from any one of the following groups: 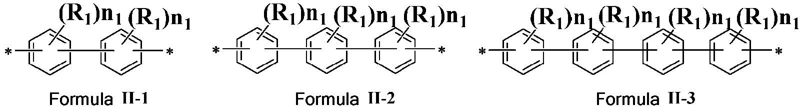 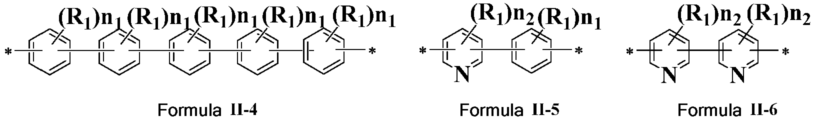 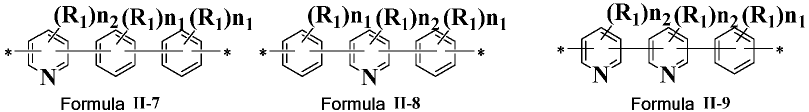 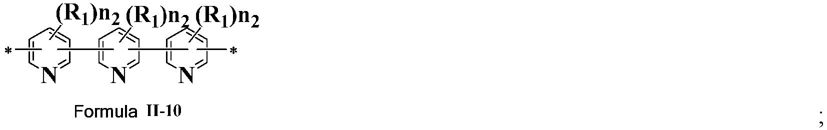
wherein R₁ is independently selected from any one of hydrogen, deuterium, halogen, cyano, nitro, substituted or unsubstituted C1 to C12 alkyl, substituted or unsubstituted silyl, substituted or unsubstituted C1 to C12 alkoxy, substituted or unsubstituted C3 to C12 cycloalkyl or substituted or unsubstituted C2 to C12 heterocycloalkyl;
n₁ is independently selected from 0, 1, 2, 3 or 4; n₂ is independently selected from 0, 1, 2 or 3; when n₁ is greater than 1, two or more R₁ are the same as or different from each other, or two adjacent R₁ are joined to form a substituted or unsubstituted benzene ring;
with the proviso that one or more hydrogens in at least one group of Ar₁, Ar₂, L, L₁, L₂, R₂ or Ra are substituted with a group represented by Formula III: 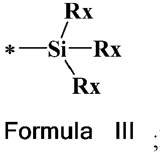 Rx is independently selected from any one of hydrogen, deuterium, cyano, nitro, halogen, substituted or unsubstituted C1 to C12 alkyl, substituted or unsubstituted C2 to C12 alkenyl , substituted or unsubstituted C3 to C12 cycloalkyl or substituted or unsubstituted C2 to C12 heterocycloalkyl; and
the present disclosure does not include the following compound:

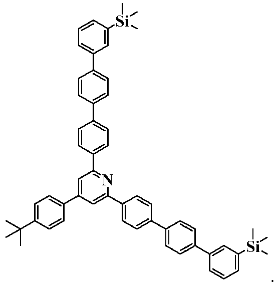

Preferably, one X in 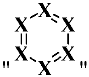 is selected from an N atom; more preferably, two X in " 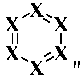 are selected from an N atom; more preferably, three X in 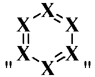 are selected from an N atom.

Preferably, 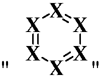 is selected from any one of the following groups: 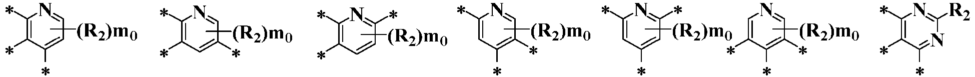 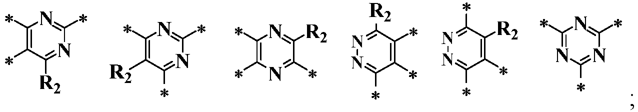
wherein a definition of R₂ is the same as that described above, and m₀ is independently selected from 0, 1 or 2; and
"*" is a linkage site where L₁, L₂ or L is joined.

Preferably, the heterocyclic compound is selected from at least one of the following structures: 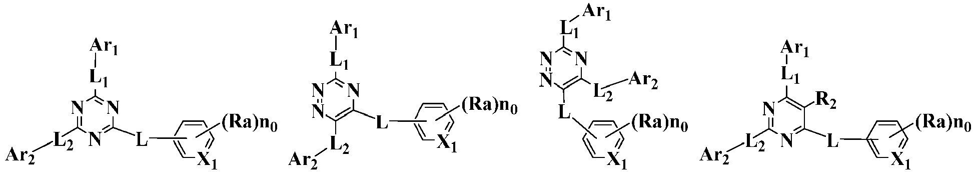 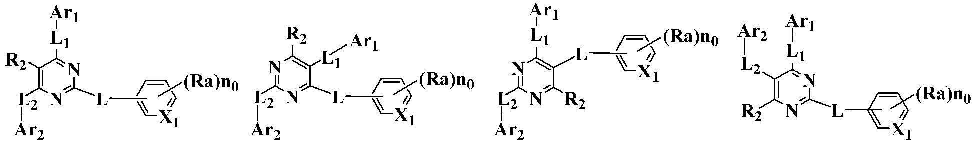 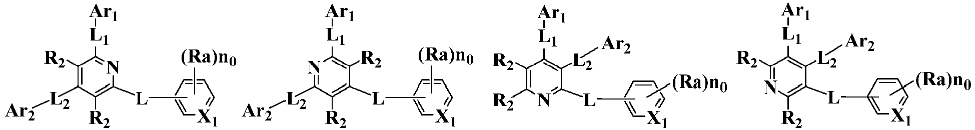 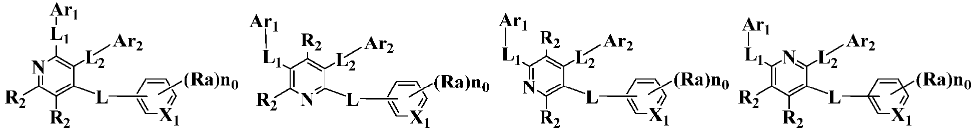 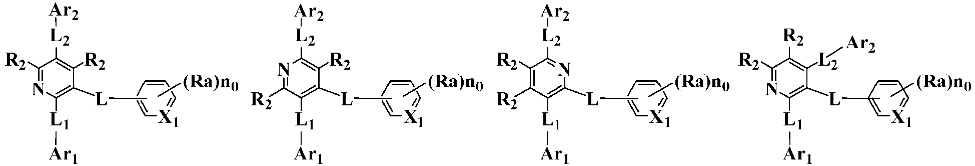 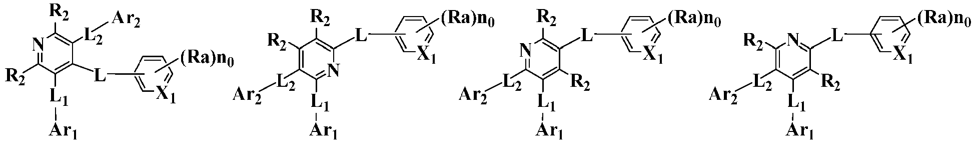 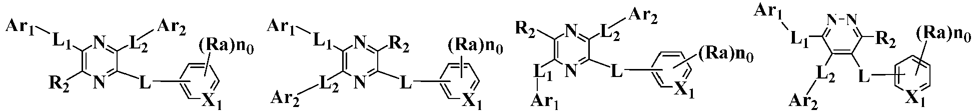 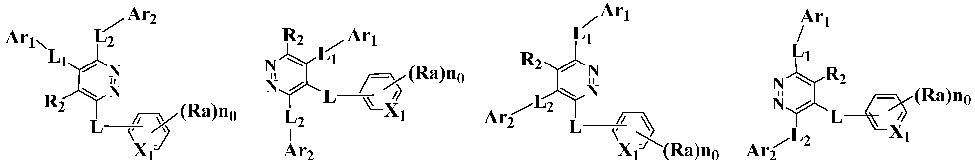 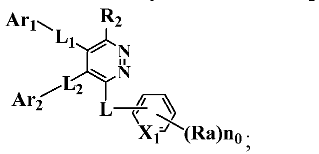 wherein definitions of Ar₁, Ar₂, R₂, L, L₁, L₂, X₁, Ra and n₀ are the same as those described above.

More preferably, the heterocyclic compound represented by Formula I is selected from any one of the following structures represented by Formula III-1 to Formula III-5: 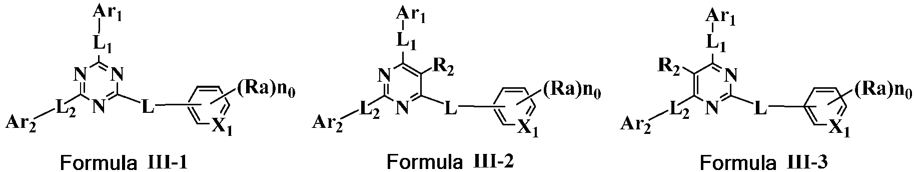 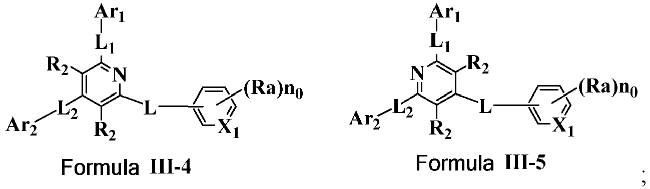 wherein definitions of Ar₁, Ar₂, R₂, L, L₁, L₂, X₁, Ra and n₀ are the same as those described above.

Preferably, R₂ is independently selected from any one of hydrogen, deuterium, tritium, cyano, nitro, fluorine, chloride, bromine, iodine or the following substituted or unsubstituted groups: methyl, ethyl, *n*-propyl, isopropyl, *n*-butyl, isobutyl, *tert*-butyl, methoxy, ethoxy, trimethylsilyl, triethylsilyl, cyclopropyl, cyclobutyl, cyclopentyl, cyclohexyl, cycloheptyl, adamantyl, norbornyl, benzocyclopropyl, benzocyclobutyl, benzocyclopentyl, benzocyclohexyl, phenyl, biphenyl, terphenyl, naphthyl, pyridyl, pyrimidinyl, pyrazinyl, pyridazinyl, quinolyl, isoquinolyl, quinazolinyl or quinoxalinyl, or selected from the group represented by Formula III.

Preferably, R₂ is independently selected from the group represented by Formula III.

Preferably, Ar₁ and Ar₂ are independently selected from any one of the following groups: 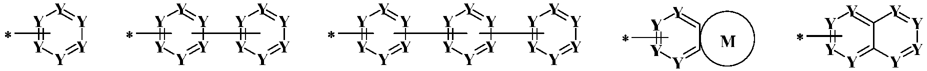 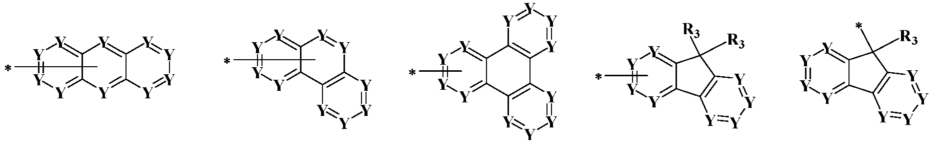 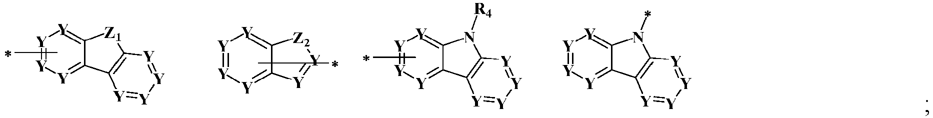
wherein Y is independently selected from C(R₅) or an N atom, and Y at a linkage site is selected from a C atom;
the ring M is selected from an unsubstituted C3 to C7 aliphatic ring or a C3 to C7 aliphatic ring substituted with one or more R₆;
Z₁ is selected from an O atom or an S atom, and Z₂ is selected from O, S, C(R₇)₂ or N(R₈);
R₃ and R₇ are independently selected from any one of hydrogen, deuterium, substituted or unsubstituted C1 to C12 alkyl, substituted or unsubstituted C1 to C12 alkoxy, substituted or unsubstituted C3 to C12 cycloalkyl, substituted or unsubstituted silyl, substituted or unsubstituted C2 to C12 heterocycloalkyl, substituted or unsubstituted C6 to C30 aryl or substituted or unsubstituted C2 to C30 heteroaryl, or two adjacent R₃ are joined to form a substituted or unsubstituted spirocyclic structure;
R₄ and R₈ are independently selected from any one of substituted or unsubstituted C1 to C12 alkyl, substituted or unsubstituted C3 to C12 cycloalkyl, substituted or unsubstituted silyl, substituted or unsubstituted C2 to C12 heterocycloalkyl, substituted or unsubstituted C6 to C30 aryl or substituted or unsubstituted C2 to C30 heteroaryl; and
R₅ and R₆ are independently selected from any one of hydrogen, deuterium, tritium, cyano, nitro, halogen, substituted or unsubstituted C1 to C12 alkyl, substituted or unsubstituted C1 to C12 alkoxy, substituted or unsubstituted silyl, substituted or unsubstituted C3 to C12 cycloalkyl, substituted or unsubstituted C2 to C12 heterocycloalkyl, substituted or unsubstituted C6 to C30 aryl or substituted or unsubstituted C2 to C30 heteroaryl, or two adjacent R₅ are joined to form a substituted or unsubstituted ring.

Preferably, at most three Y in each of the above groups are selected from an N atom; further preferably, three Y in each of the above groups are selected from an N atom; more preferably, two Y in each of the above groups are selected from an N atom; more preferably, one Y in each of the above groups is selected from an N atom; particularly preferably, each Y in each of the above groups is independently selected from C(R₅).

Preferably, the ring M is selected from any one of the following groups: 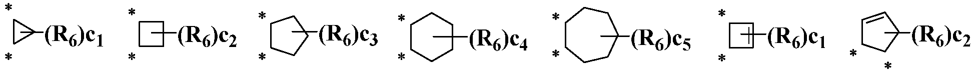 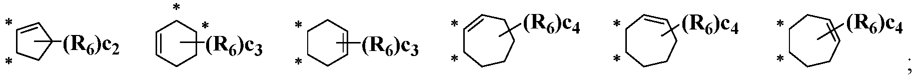
wherein "*" represents a bonding site; and
R₆ is independently selected from any one of hydrogen, deuterium, cyano, nitro, halogen, substituted or unsubstituted C1 to C12 alkyl, substituted or unsubstituted C1 to C12 alkoxy, substituted or unsubstituted silyl, substituted or unsubstituted C3 to C12 cycloalkyl, substituted or unsubstituted C2 to C12 heterocycloalkyl, substituted or unsubstituted C6 to C30 aryl or substituted or unsubstituted C2 to C30 heteroaryl.

c₁ is independently selected from 0, 1 or 2, c₂ is independently selected from 0, 1, 2, 3 or 4, c₃ is independently selected from 0, 1, 2, 3, 4, 5 or 6, c₄ is independently selected from 0, 1, 2, 3, 4, 5, 6, 7 or 8, and c₅ is independently selected from 0, 1, 2, 3, 4, 5, 6, 7, 8, 9 or 10.

More preferably, Ar₁ and Ar₂ are independently selected from any one of the following groups: 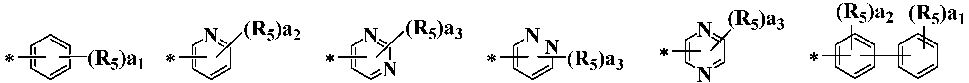 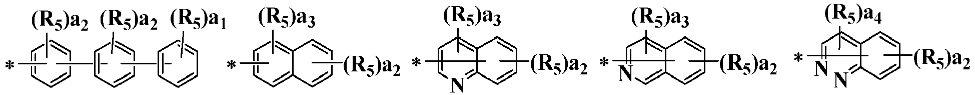 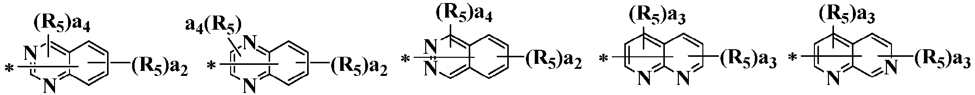 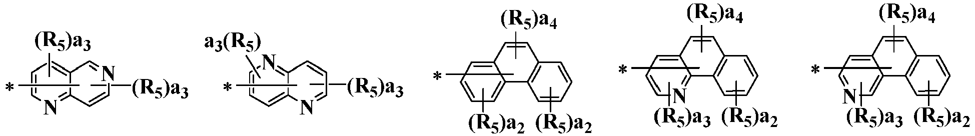 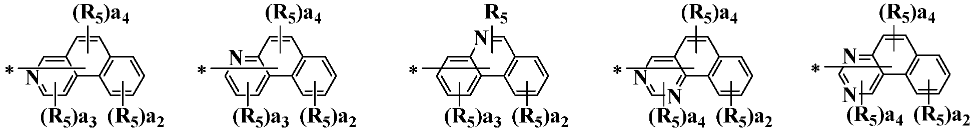 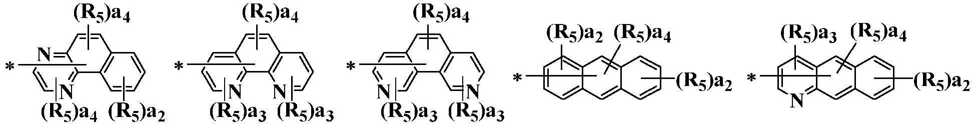 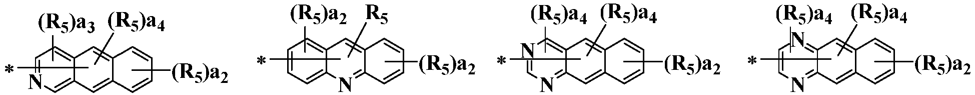 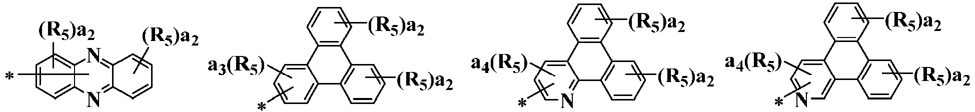 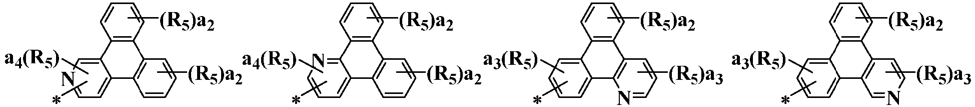 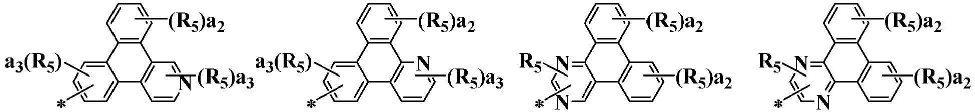 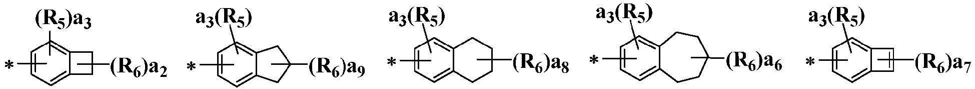 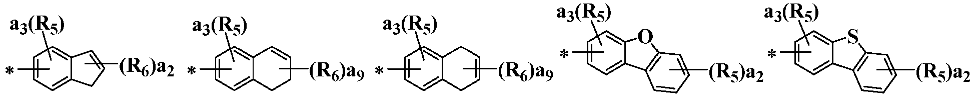 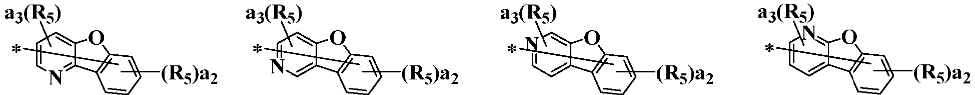 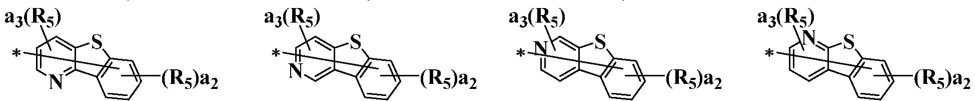 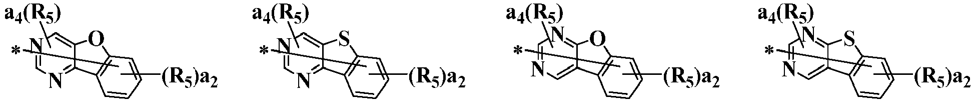 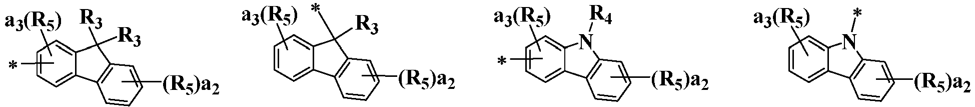 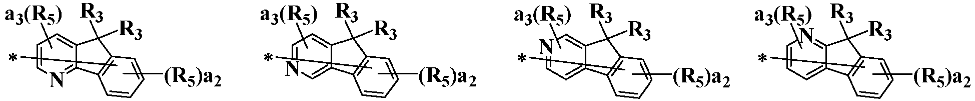 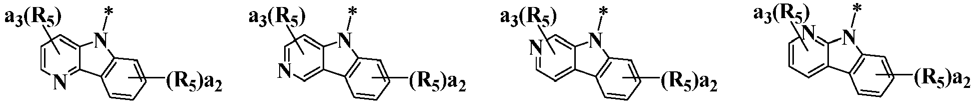 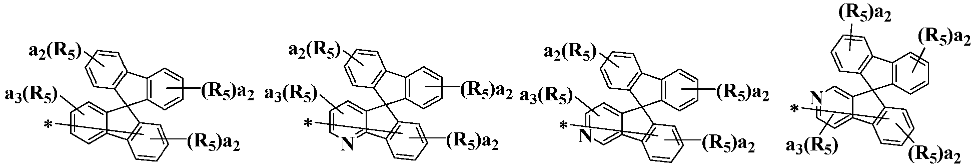 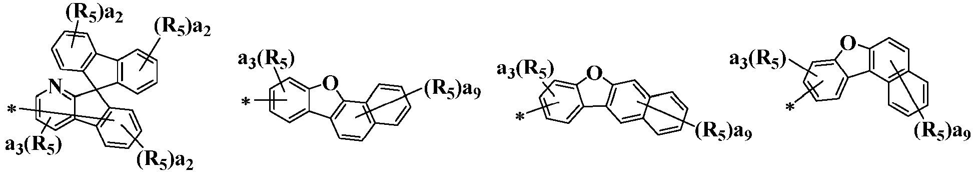 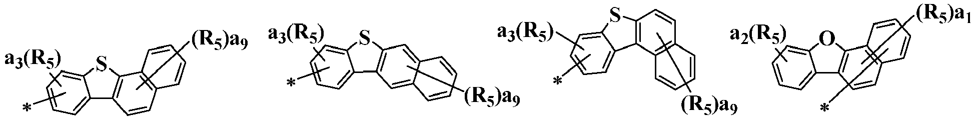 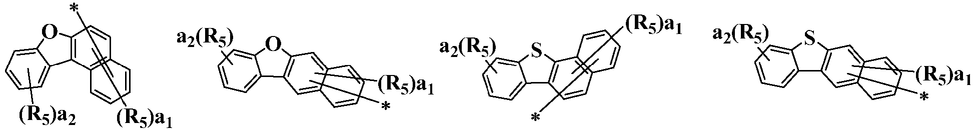 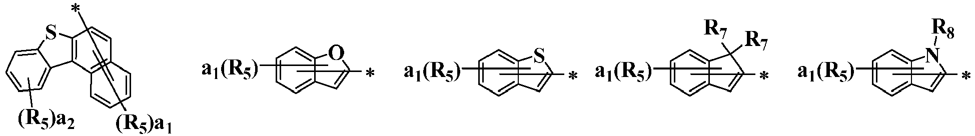 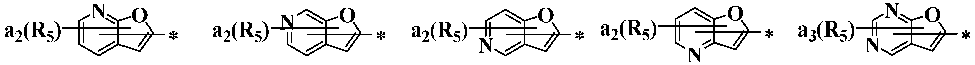 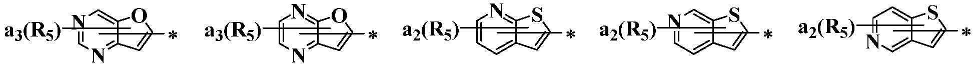 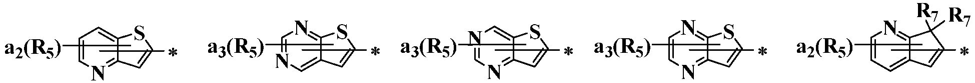 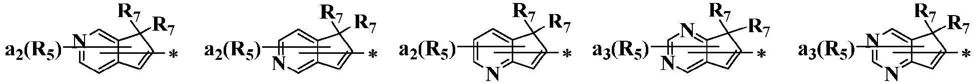 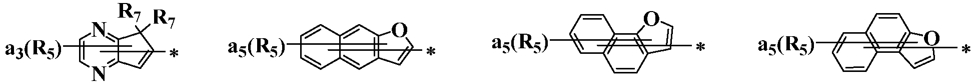 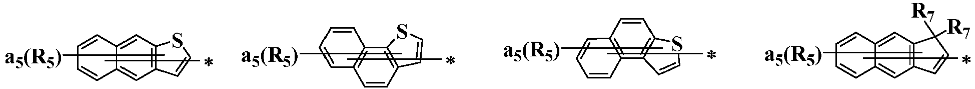 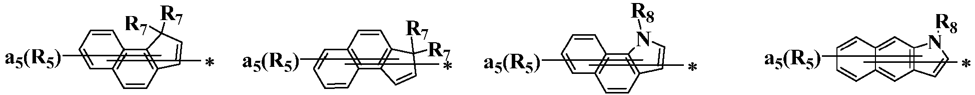 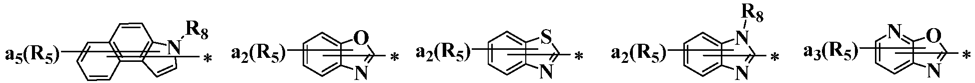 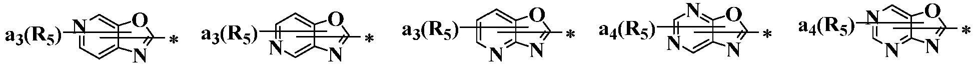 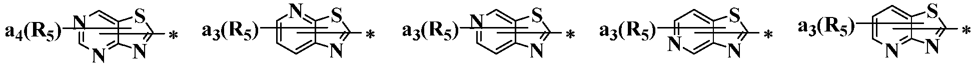 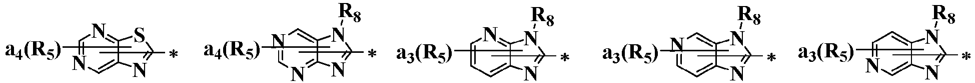 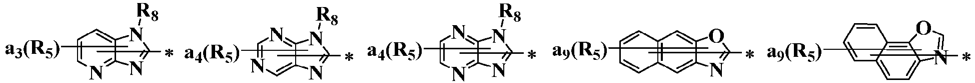 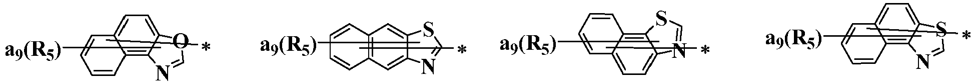 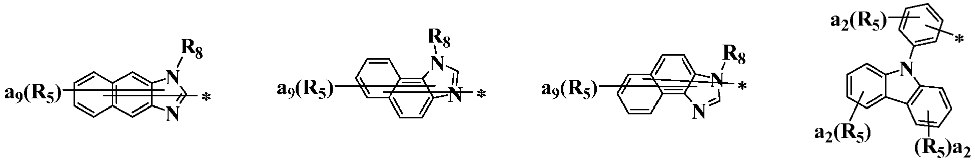 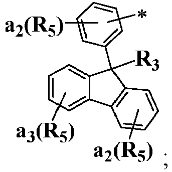
wherein R₃ and R₇ are independently selected from any one of hydrogen, deuterium, substituted or unsubstituted C1 to C12 alkyl, substituted or unsubstituted C1 to C12 alkoxy, substituted or unsubstituted C3 to C12 cycloalkyl, substituted or unsubstituted silyl, substituted or unsubstituted C2 to C12 heterocycloalkyl, substituted or unsubstituted C6 to C30 aryl or substituted or unsubstituted C2 to C30 heteroaryl, or two adjacent R₃ are joined to form the following spirocyclic structure: 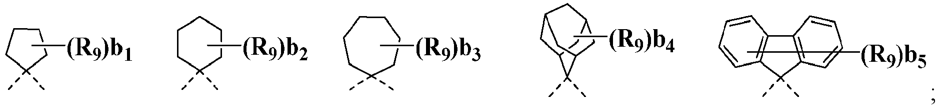
wherein R₉ is independently selected from any one of hydrogen, deuterium, tritium, cyano, nitro, halogen, substituted or unsubstituted C1 to C12 alkyl, substituted or unsubstituted C1 to C12 alkoxy, substituted or unsubstituted silyl, substituted or unsubstituted C3 to C12 cycloalkyl, substituted or unsubstituted C2 to C12 heterocycloalkyl, substituted or unsubstituted C6 to C30 aryl or substituted or unsubstituted C2 to C30 heteroaryl;
b₁ is selected from 0, 1, 2, 3, 4, 5, 6, 7 or 8, b₂ is selected from 0, 1, 2, 3, 4, 5, 6, 7, 8, 9 or 10, b₃ is selected from 0, 1, 2, 3, 4, 5, 6, 7, 8, 9, 10, 11 or 12, and b₄ is selected from 0, 1, 2, 3, 4, 5, 6, 7, 8, 9, 10, 11, 12, 13 or 14;
R₄ and R₈ are independently selected from any one of substituted or unsubstituted C1 to C12 alkyl, substituted or unsubstituted C3 to C12 cycloalkyl, substituted or unsubstituted silyl, substituted or unsubstituted C2 to C12 heterocycloalkyl, substituted or unsubstituted C6 to C30 aryl or substituted or unsubstituted C2 to C30 heteroaryl;
R₅ and R₆ are independently selected from any one of hydrogen, deuterium, tritium, cyano, nitro, halogen, substituted or unsubstituted C1 to C12 alkyl, substituted or unsubstituted C1 to C12 alkoxy, substituted or unsubstituted silyl, substituted or unsubstituted C3 to C12 cycloalkyl, substituted or unsubstituted C2 to C12 heterocycloalkyl, substituted or unsubstituted C6 to C30 aryl or substituted or unsubstituted C2 to C30 heteroaryl; and
a₁ is independently selected from 0, 1, 2, 3, 4 or 5, a₂ is independently selected from 0, 1, 2, 3 or 4, a₃ is independently selected from 0, 1, 2 or 3, a₄ is independently selected from 0, 1 or 2, as is independently selected from 0, 1, 2, 3, 4, 5, 6 or 7, a₆ is independently selected from 0, 1, 2, 3, 4, 5, 6, 7, 8, 9 or 10, a₇ is independently selected from 0, 1 or 2, a₈ is independently selected from 0, 1, 2, 3, 4, 5, 6, 7 or 8, and a₉ is independently selected from 0, 1, 2, 3, 4, 5 or 6.

Preferably, R₅ and R₆ are independently selected from any one of hydrogen, deuterium, cyano, nitro, fluorine, chloride, bromine, iodine, methyl, ethyl, *n*-propyl, isopropyl, *n*-butyl, isobutyl, *tert*-butyl, methoxy, ethoxy, trimethylsilyl, triethylsilyl, tri-*tert*-butylsilyl, deuterated trimethylsilyl, fluorine-substituted methyl, fluorine-substituted ethyl, fluorine-substituted isopropyl, fluorine-substituted *tert*-butyl, cyclopropyl, cyclobutyl, cyclopentyl, cyclohexyl, cycloheptyl, adamantyl, norbornyl, phenyl, biphenyl, naphthyl, pyridyl, pyrimidinyl, deuterated methyl, deuterated ethyl, deuterated *n*-propyl, deuterated isopropyl, deuterated *tert*-butyl, deuterated *n*-butyl, deuterated isobutyl, deuterated adamantyl, deuterated phenyl, deuterated biphenyl, deuterated pyridyl, deuterated naphthyl or deuterated pyrimidinyl or selected from the group represented by Formula III.

Preferably, R₆ is selected from the group represented by Formula III; more preferably, R₅ is selected from the group represented by Formula III.

More preferably, one or two of R₅ in each group and, if permitted, three or more of R₅ in each group are selected from the group represented by Formula III.

Preferably, R₃ and R₇ are independently selected from any one of hydrogen, deuterium, cyano, nitro, fluorine, chloride, bromine, iodine, methyl, ethyl, *n*-propyl, isopropyl, *n*-butyl, isobutyl, *tert*-butyl, adamantyl, norbornyl, trimethylsilyl, triethylsilyl, tri-*tert*-butylsilyl, deuterated trimethylsilyl, cyclopropyl, cyclobutyl, cyclopentyl, cyclohexyl, phenyl, biphenyl, pyridyl, pyrimidinyl, naphthyl, quinolyl, isoquinolyl, deuterated methyl, deuterated ethyl, deuterated *n*-propyl, deuterated isopropyl, deuterated *n*-butyl, deuterated isobutyl, deuterated *tert*-butyl, deuterated adamantyl, deuterated phenyl, deuterated biphenyl, deuterated naphthyl, deuterated pyridyl, deuterated pyrimidinyl, deuterated pyrazinyl, deuterated pyridazinyl, deuterated quinolyl, deuterated isoquinolyl, methyl-substituted phenyl, ethyl-substituted phenyl, isopropyl-substituted phenyl, *tert*-butyl-substituted phenyl, cyano-substituted phenyl, fluorine-substituted phenyl, adamantyl-substituted phenyl, norbornyl-substituted phenyl, methyl-substituted biphenyl or ethyl-substituted biphenyl or selected from the group represented by Formula III.

Preferably, R₃ is selected from the group represented by Formula III; more preferably, R₇ is selected from the group represented by Formula III.

R₄ and R₈ are independently selected from any one of methyl, ethyl, *n*-propyl, isopropyl, *n*-butyl, isobutyl, *tert*-butyl, adamantyl, norbornyl, trimethylsilyl, triethylsilyl, tri-*tert*-butylsilyl, deuterated trimethylsilyl, cyclopropyl, cyclobutyl, cyclopentyl, cyclohexyl, phenyl, biphenyl, pyridyl, pyrimidinyl, naphthyl, quinolyl, isoquinolyl, deuterated methyl, deuterated ethyl, deuterated *n*-propyl, deuterated isopropyl, deuterated *n*-butyl, deuterated isobutyl, deuterated *tert*-butyl, deuterated adamantyl, deuterated phenyl, deuterated biphenyl, deuterated naphthyl, deuterated pyridyl, deuterated pyrimidinyl, deuterated pyrazinyl, deuterated pyridazinyl, deuterated quinolyl, deuterated isoquinolyl, methyl-substituted phenyl, ethyl-substituted phenyl, isopropyl-substituted phenyl, *tert*-butyl-substituted phenyl, cyano-substituted phenyl, fluorine-substituted phenyl, adamantyl-substituted phenyl, norbornyl-substituted phenyl, methyl-substituted biphenyl, ethyl-substituted biphenyl or naphthyl-substituted phenyl or selected from the group represented by Formula III.

Preferably, R₄ is selected from the group represented by Formula III; more preferably, R₈ is selected from the group represented by Formula III.

Preferably, Ra is independently selected from any one of hydrogen, deuterium, cyano, nitro, fluorine, chlorine, bromine, iodine, methyl, ethyl, *n*-propyl, isopropyl, *n*-butyl, isobutyl, *tert*-butyl, cyclopropyl, cyclobutyl, cyclopentyl, cyclohexyl, cycloheptyl, adamantyl, norbornyl, trimethylsilyl, triethylsilyl, tri-*tert*-butylsilyl, deuterated trimethylsilyl, deuterated triethylsilyl, deuterated tri-*tert*-butylsilyl, deuterated methyl, deuterated ethyl, deuterated *n*-propyl, deuterated isopropyl, deuterated *n*-butyl, deuterated isobutyl, deuterated *tert*-butyl, deuterated adamantyl, deuterated norbornyl, fluorine-substituted methyl, fluorine-substituted ethyl, fluorine-substituted isopropyl, fluorine-substituted *tert*-butyl, methyl-substituted adamantyl or methyl-substituted norbornyl or selected from the group represented by Formula III, and when two or more Ra are present, two adjacent Ra can be joined to form a substituted or unsubstituted benzene ring.

Preferably, Ra is selected from the group represented by Formula III.

Preferably, " 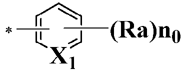 " is independently selected from any one of the following groups: 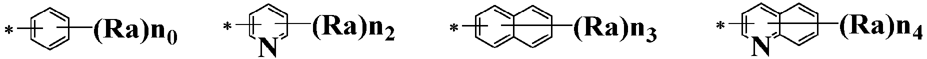 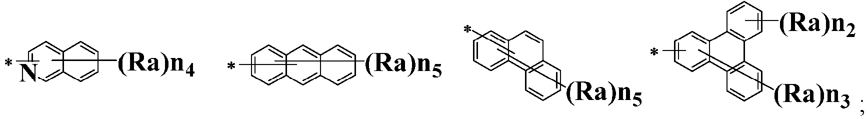 wherein a definition of Ra is described above; n₀ is independently selected from 0, 1, 2, 3, 4 or 5, n₂ is independently selected from 0, 1, 2, 3 or 4, n₃ is independently selected from 0, 1, 2, 3, 4, 5, 6 or 7, n₄ is independently selected from 0, 1, 2, 3, 4, 5 or 6, and n₅ is independently selected from 0, 1, 2, 3, 4, 5, 6, 7, 8 or 9.

Preferably, Rx is independently selected from any one of hydrogen, deuterium, methyl, ethyl, *n*-propyl, isopropyl, *n*-butyl, isobutyl, *tert*-butyl, adamantyl, norbornyl, cyclopropyl, cyclobutyl, cyclopentyl, cyclohexyl, cycloheptyl, vinyl, propenyl, deuterated methyl, deuterated ethyl, deuterated *n*-propyl, deuterated isopropyl, deuterated *tert*-butyl, deuterated *n*-butyl, deuterated isobutyl, deuterated adamantyl, deuterated norbornyl, deuterated cyclopropyl, deuterated cyclobutyl, deuterated cyclopentyl, deuterated cyclohexyl or deuterated cycloheptyl.

In the present disclosure, the expression that "one or more hydrogens in at least one group of Ar₁, Ar₂, L, L₁, L₂, R₂ or Ra are substituted with a group represented by Formula III" specifically means that one, two, three, four, five, six, seven, eight or more hydrogens in any one, any two, any three, any four or all groups of Ar₁, Ar₂, L, L₁, L₂, R₂ or Ra are substituted with the group represented by Formula III.

Preferably, one or more hydrogens in at least one group of Ar₁, Ar₂, L, L₁, L₂ or Ra are substituted with the group represented by Formula III; more preferably, one or more hydrogens in at least one group of Ar₁, Ar₂ or Ra are substituted with the group represented by Formula III; more preferably, one or more hydrogens in Ar₁ or Ar₂ are substituted with the group represented by Formula III; particularly preferably, one or more hydrogens in Ra are substituted with the group represented by Formula III.

Preferably, one, two, three or four of Ra or R₅ are selected from the group represented by Formula III.

More preferably, one or two of Ra are selected from the group represented by Formula III.

More preferably, one or two of R₅ are selected from the group represented by Formula III.

More preferably, one or two of Ra and one or two of R₅ are selected from the group represented by Formula III.

Preferably, Formula III is selected from any one of the following groups:

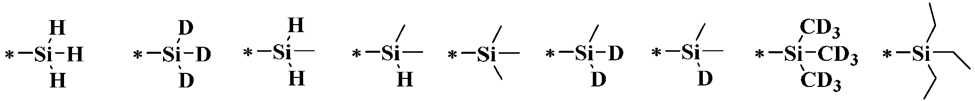

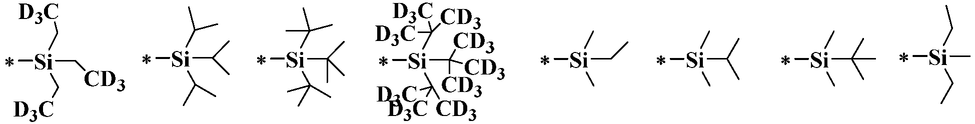

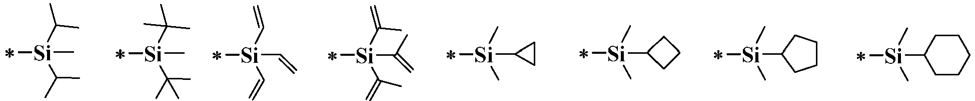

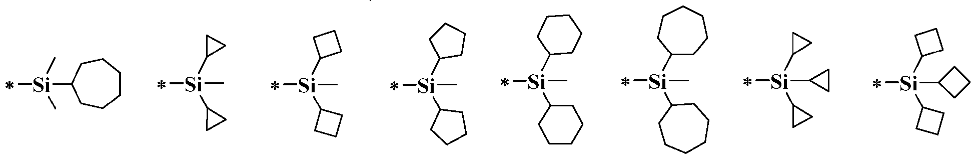

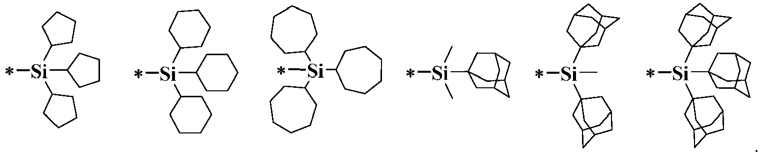

Preferably, R₁ is independently selected from any one of hydrogen, deuterium, cyano, nitro, fluorine, chloride, bromine, iodine, methyl, ethyl, n-propyl, isopropyl, n-butyl, isobutyl, tert-butyl, cyclopropyl, cyclobutyl, cyclopentyl, cyclohexyl, cycloheptyl, adamantyl, norbornyl, deuterated methyl, deuterated ethyl, deuterated n-propyl, deuterated isopropyl, deuterated n-butyl, deuterated isobutyl, deuterated tert-butyl, deuterated adamantyl, deuterated norbornyl, trifluoromethyl, methyl-substituted adamantyl or methyl-substituted norbornyl, or two adjacent R₁ are joined to form a substituted or unsubstituted benzene ring.

More preferably, L is independently selected from any one of the following groups:

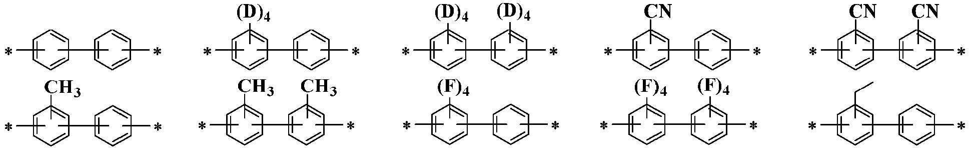

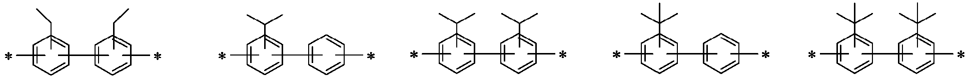

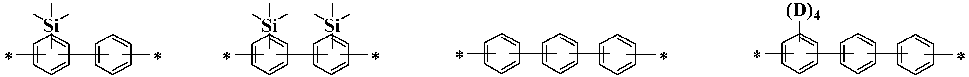

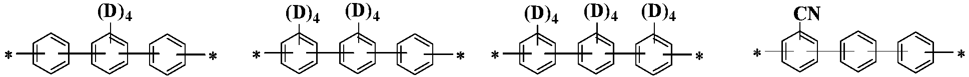

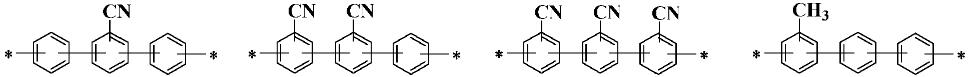

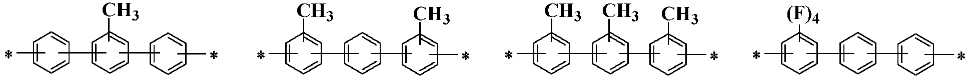

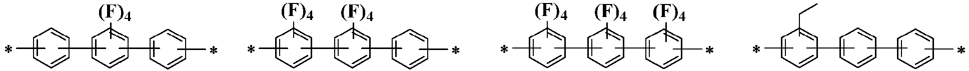

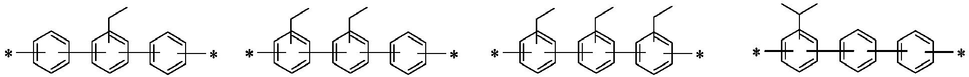

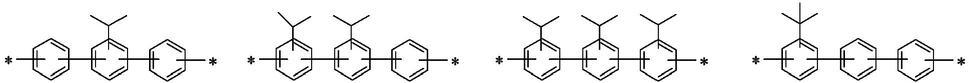

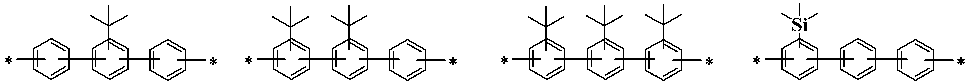

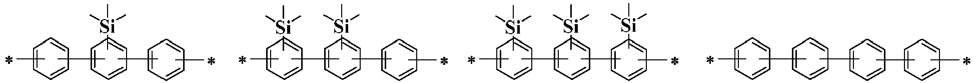

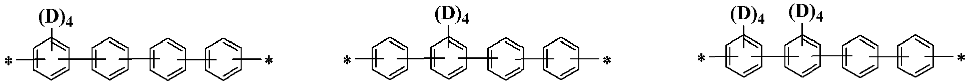

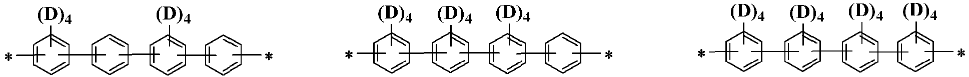

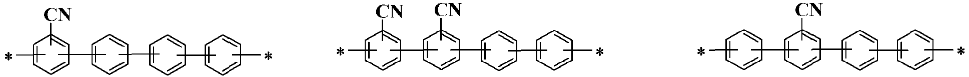

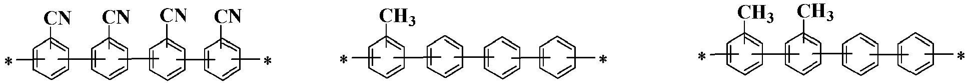

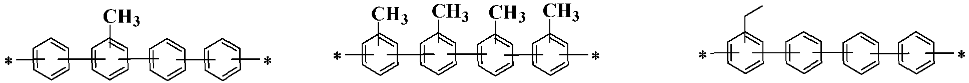

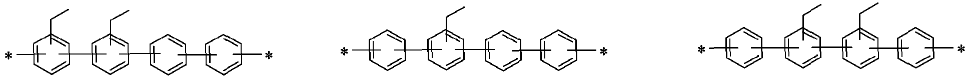

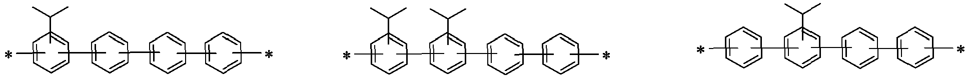

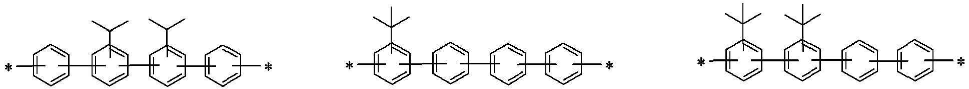

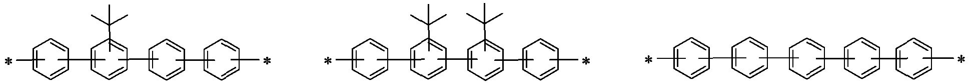

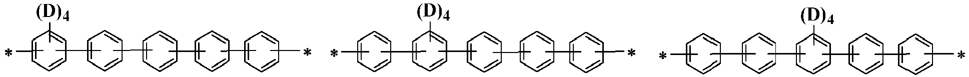

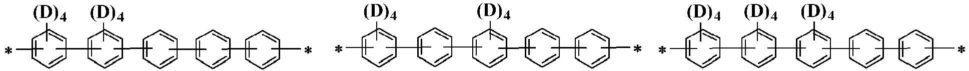

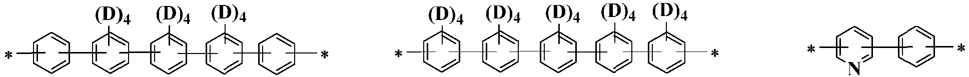

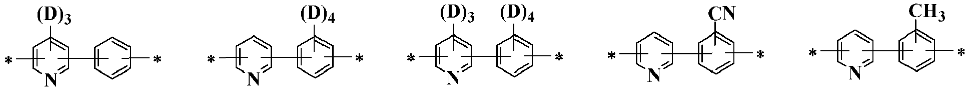

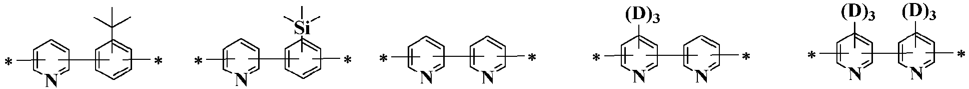

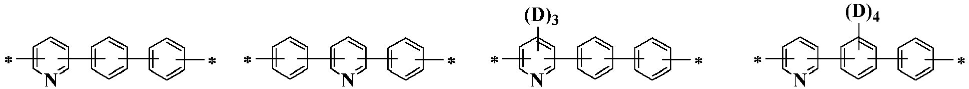

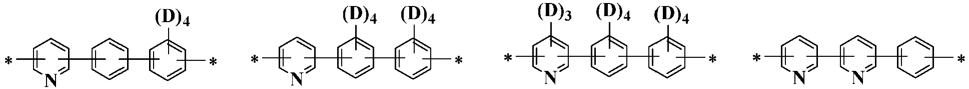

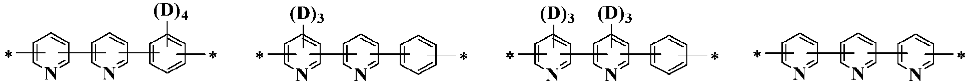

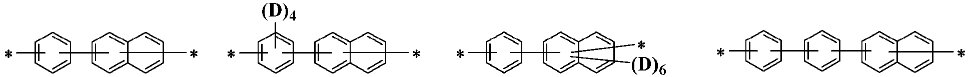

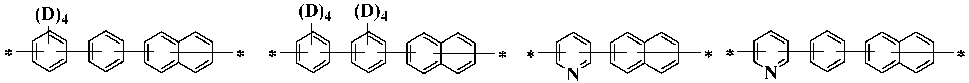

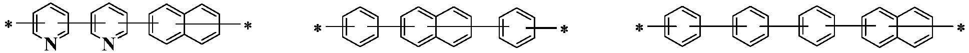

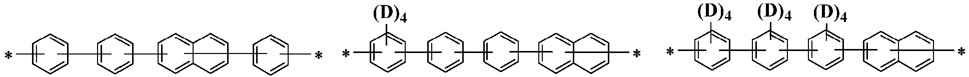

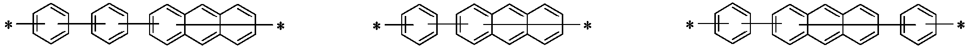

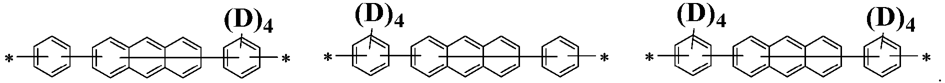

Preferably, L₁ and L₂ are independently selected from any one of a single bond or the following group: 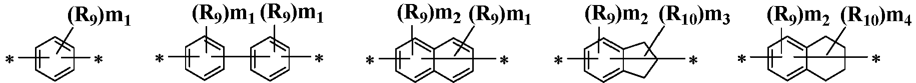 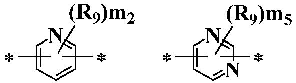
wherein R₉ is independently selected from any one of hydrogen, deuterium, cyano, nitro, halogen, substituted or unsubstituted C1 to C12 alkyl, substituted or unsubstituted C1 to C12 alkoxy, substituted or unsubstituted C3 to C12 cycloalkyl, substituted or unsubstituted silyl, substituted or unsubstituted C2 to C12 heterocycloalkyl, substituted or unsubstituted C6 to C30 aryl or substituted or unsubstituted C2 to C30 heteroaryl; and
m₁ is independently selected from 0, 1, 2, 3 or 4, m₂ is independently selected from 0, 1, 2 or 3, m₃ is independently selected from 0, 1, 2, 3, 4, 5 or 6, m₄ is independently selected from 0, 1, 2, 3, 4, 5, 6, 7 or 8, and m₅ is independently selected from 0, 1 or 2.

More preferably, R₉ is independently selected from any one of hydrogen, deuterium, tritium, cyano, nitro, fluorine, chloride, bromine, iodine, methyl, ethyl, *n*-propyl, isopropyl, *n*-butyl, isobutyl, *tert*-butyl, methoxy, ethoxy, trimethylsilyl, triethylsilyl, cyclopropyl, cyclobutyl, cyclopentyl, cyclohexyl, cycloheptyl, adamantyl, norbornyl, phenyl, biphenyl, naphthyl, pyridyl, pyrimidinyl, deuterated methyl, deuterated ethyl, deuterated *n*-propyl, deuterated isopropyl, deuterated *tert*-butyl, deuterated *n*-butyl, deuterated isobutyl, deuterated adamantyl, deuterated phenyl, deuterated biphenyl or deuterated pyridyl or selected from the group represented by Formula III.

Preferably, R₉ is selected from the group represented by Formula III.

Most preferably, the heterocyclic compound is selected from any one of the following structures:

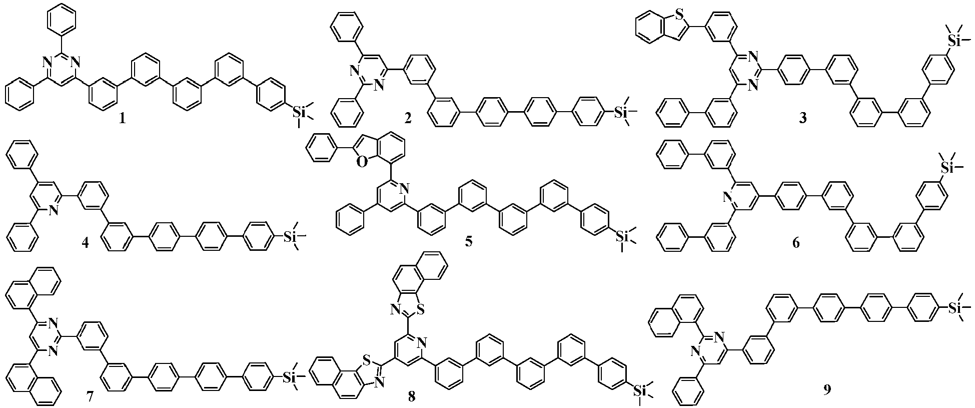

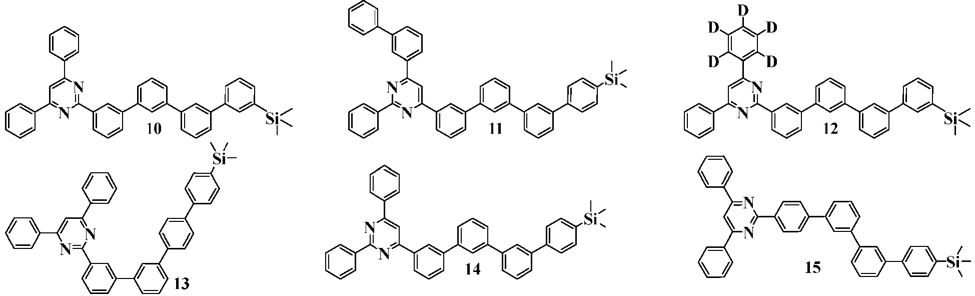

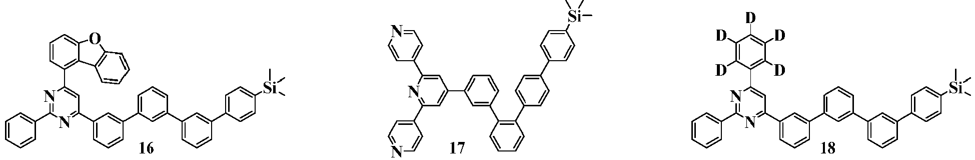

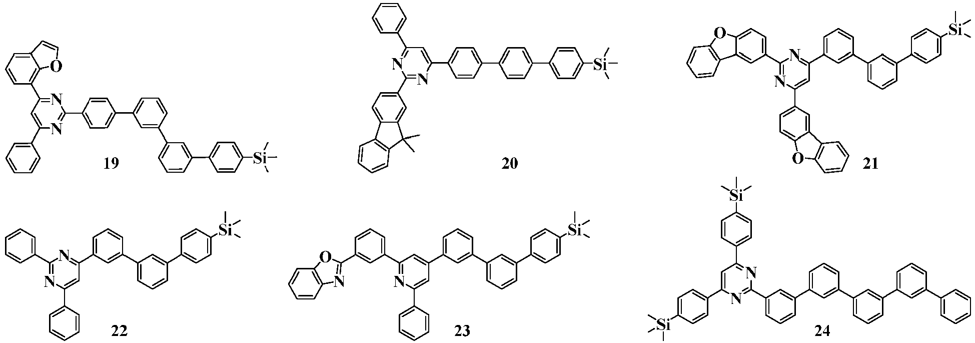

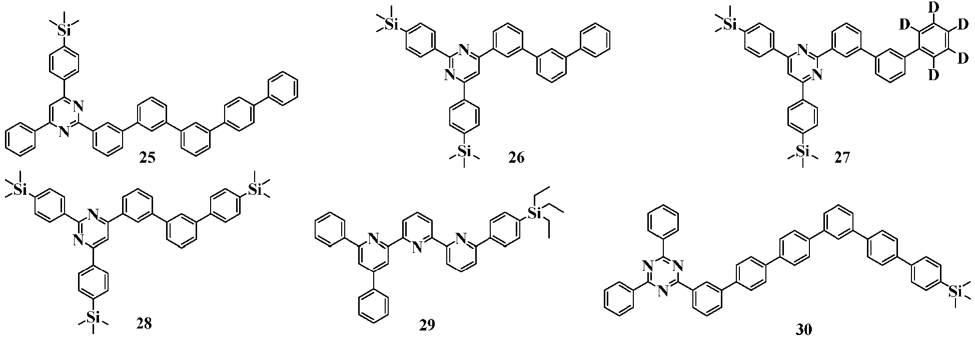

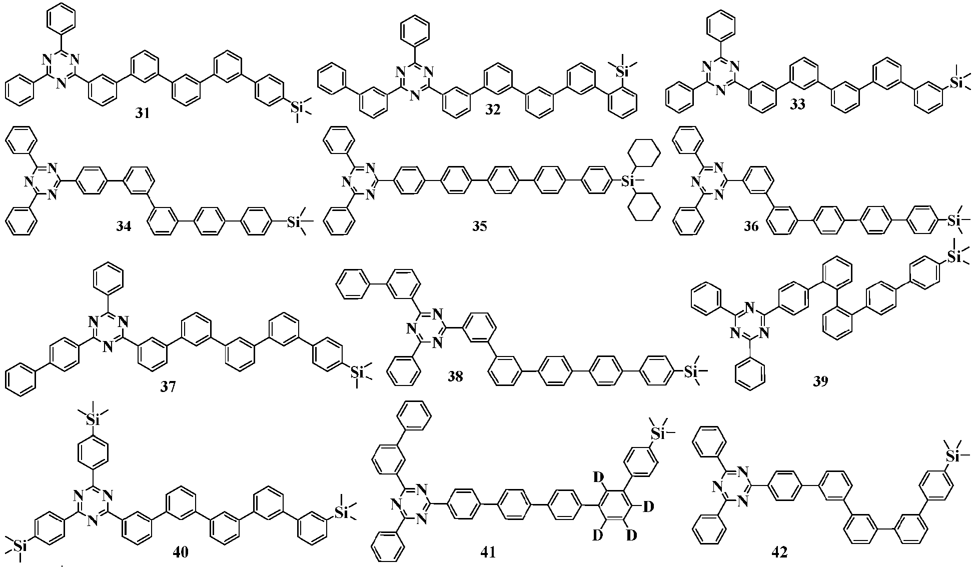

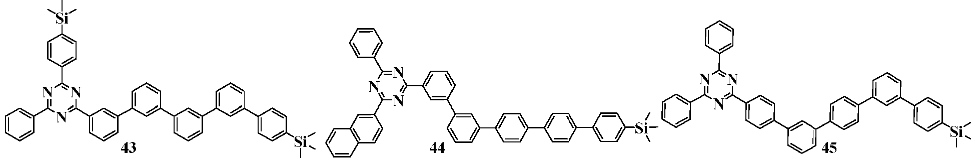

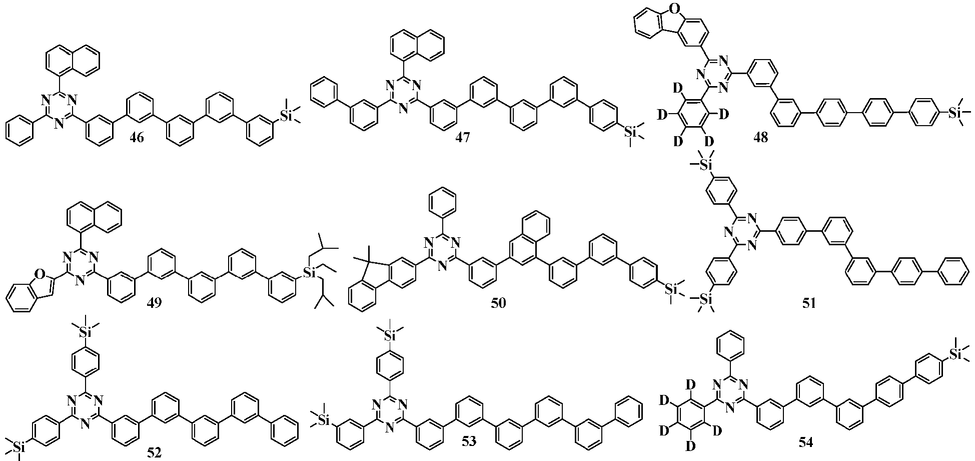

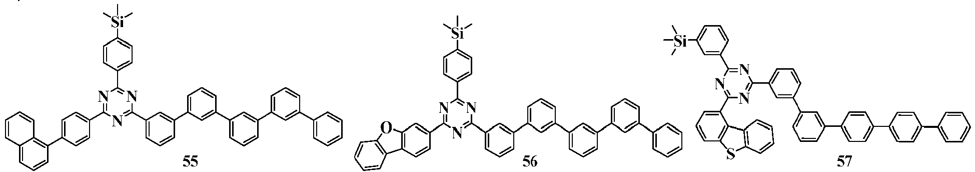

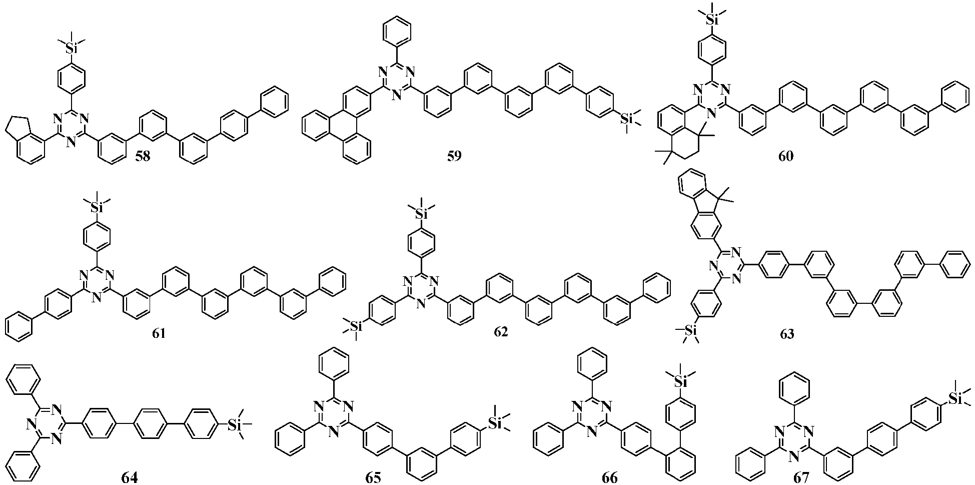

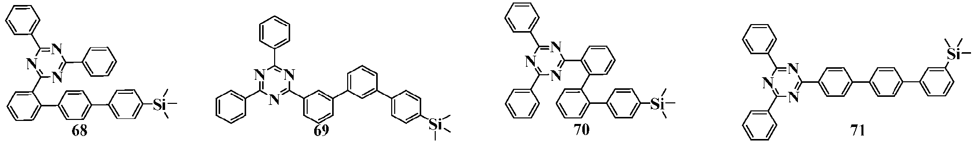

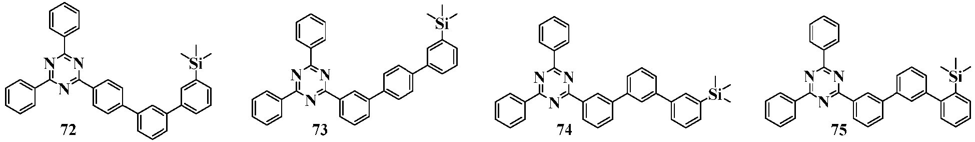

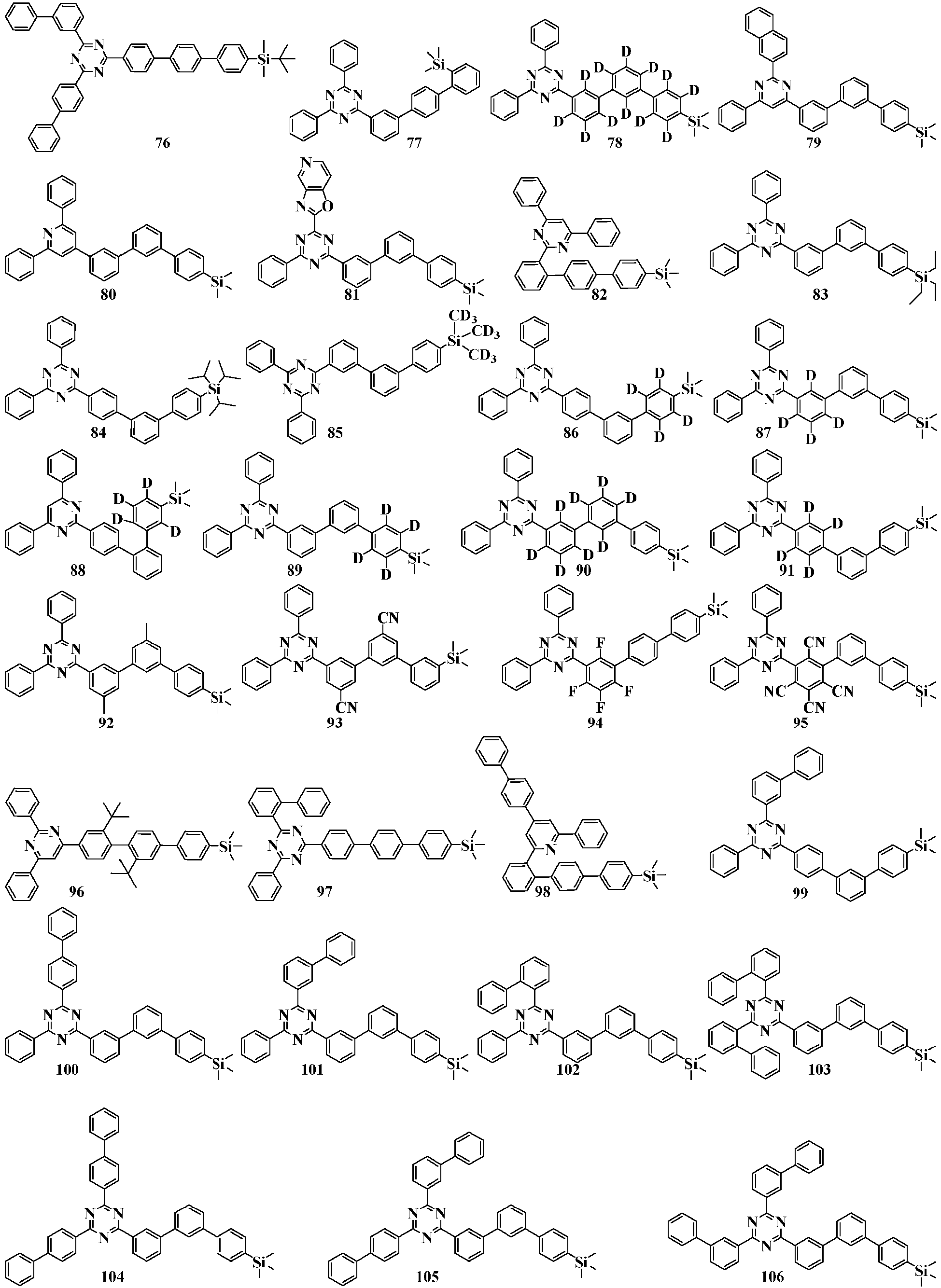

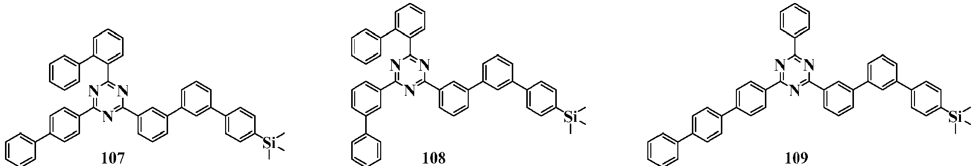

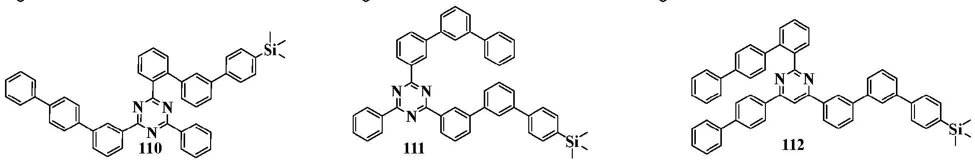

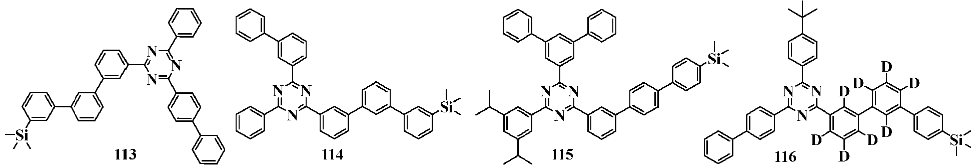

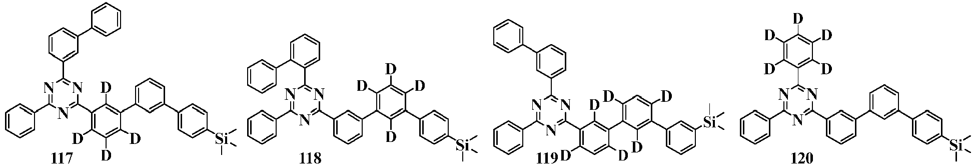

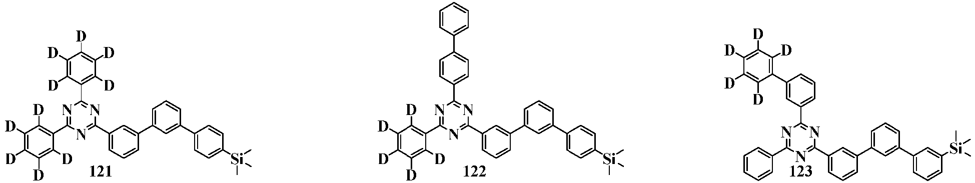

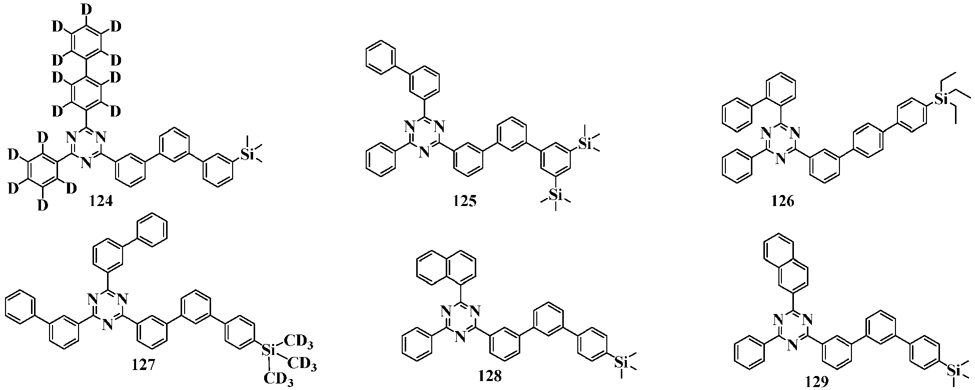

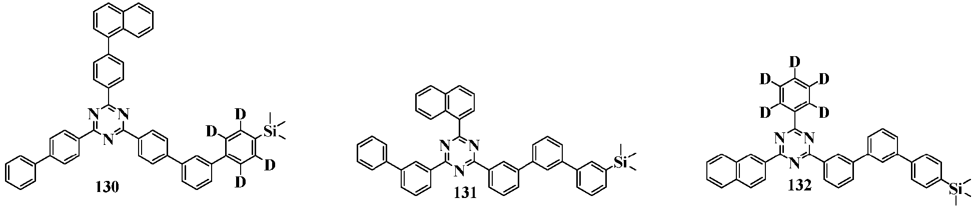

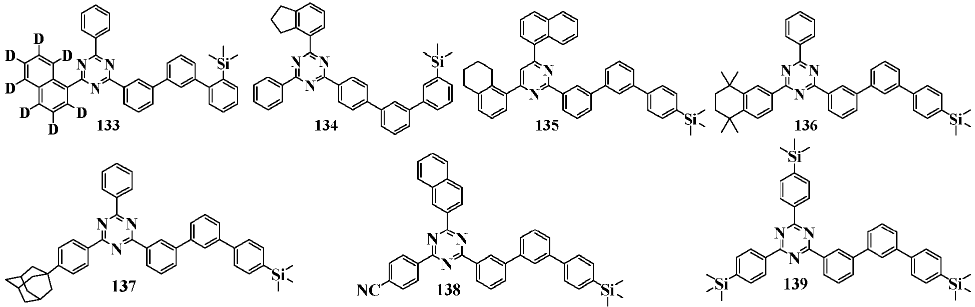

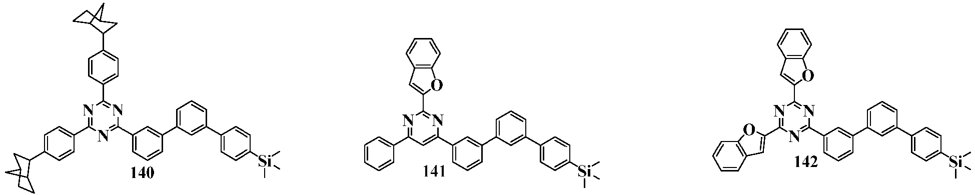

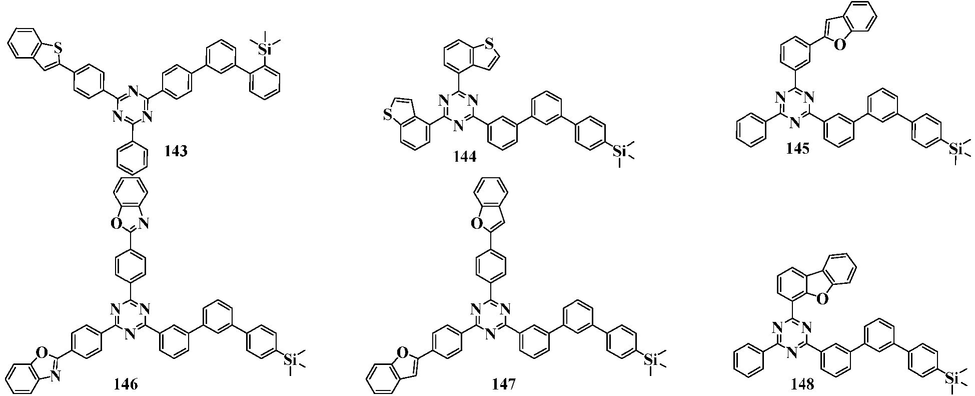

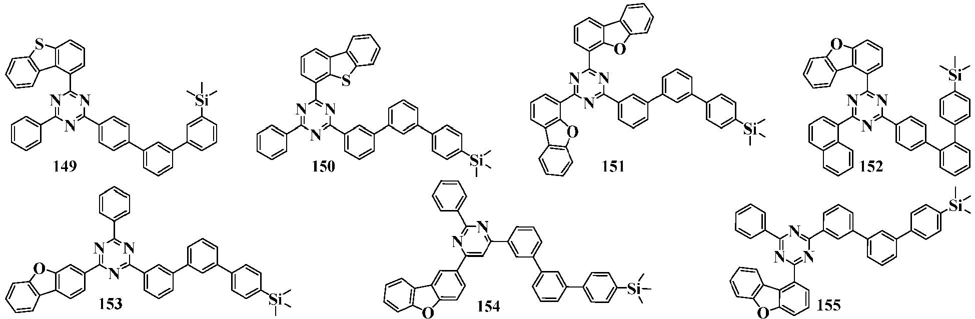

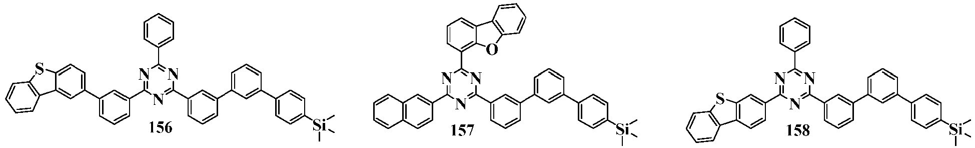

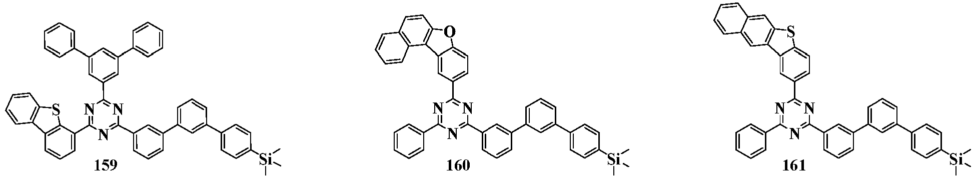

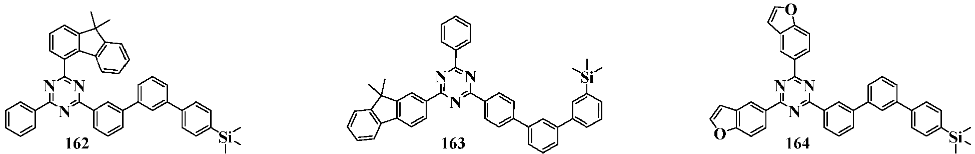

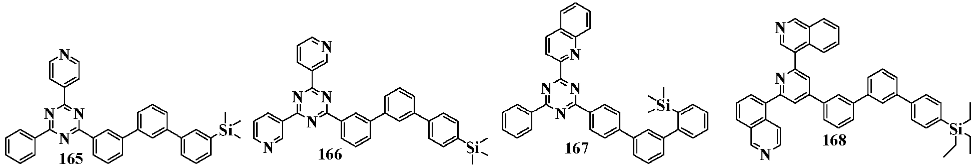

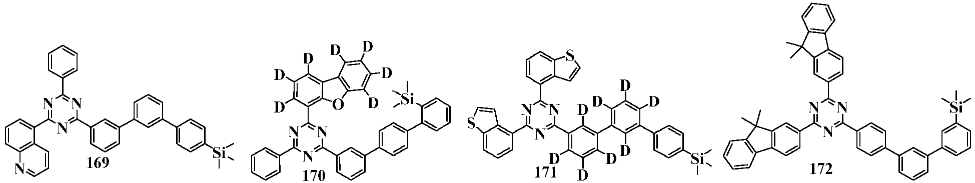

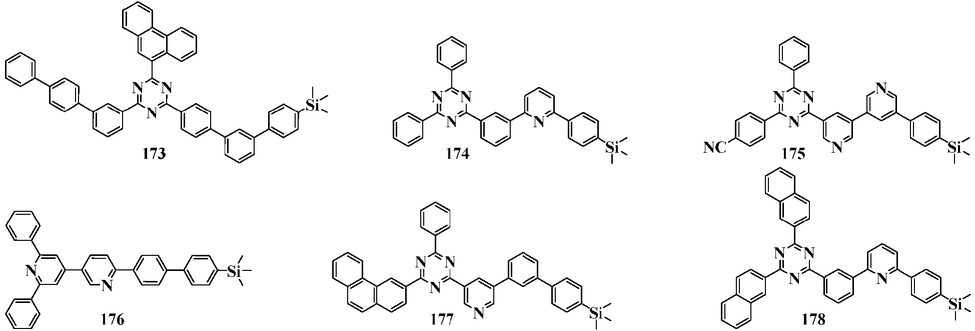

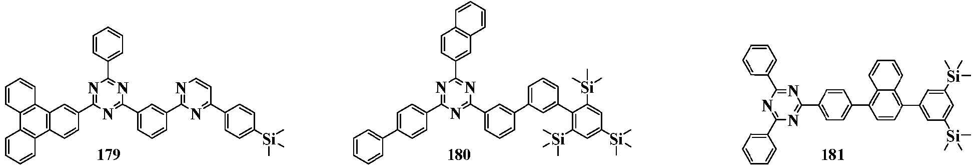

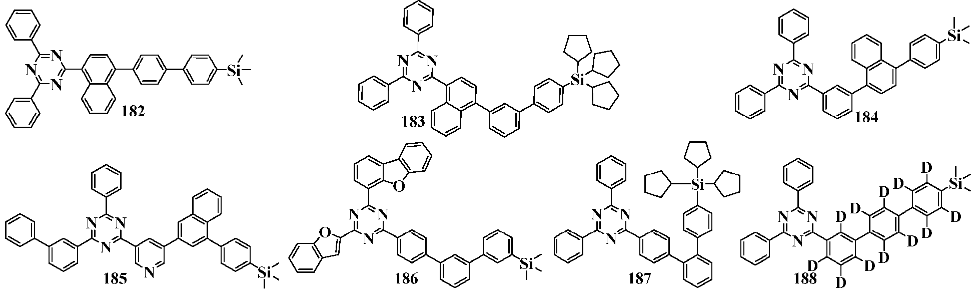

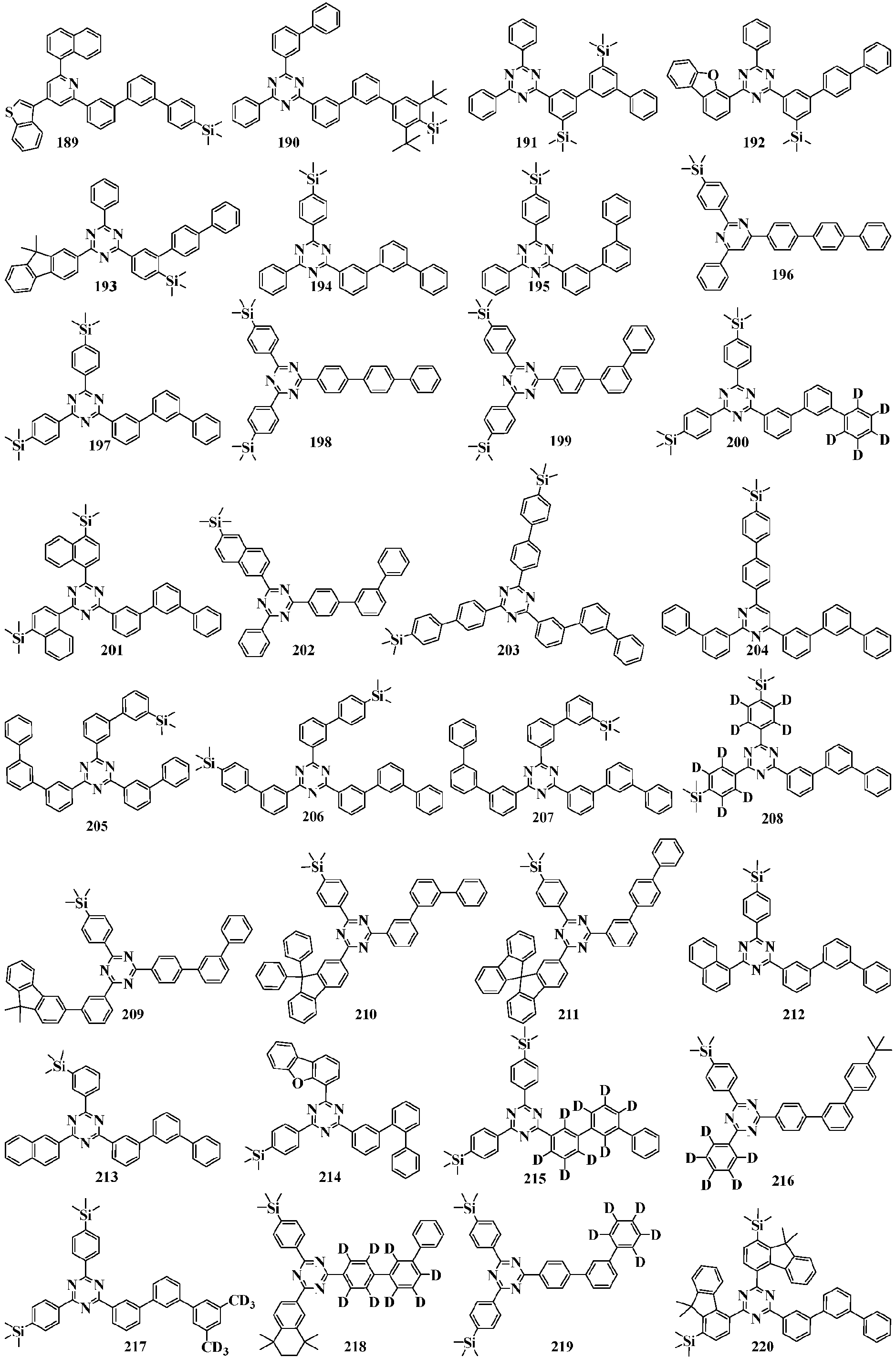

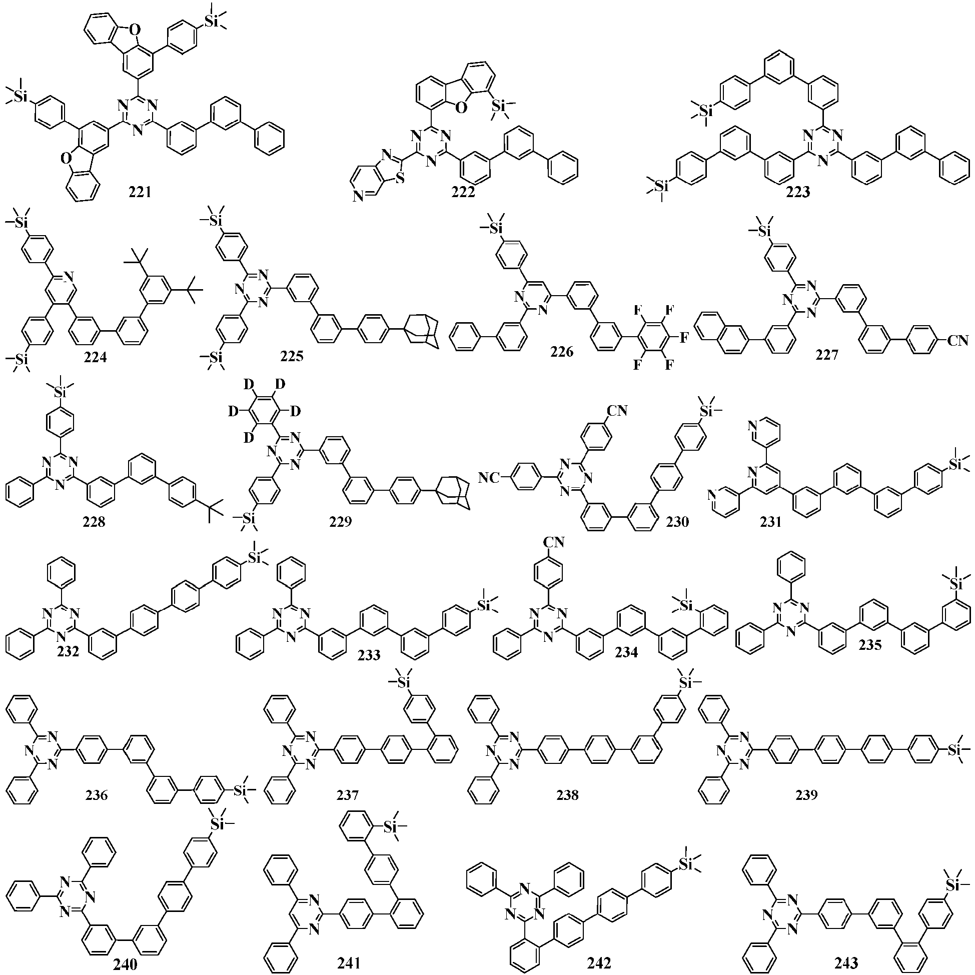

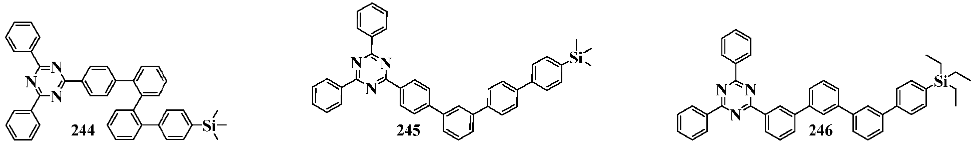

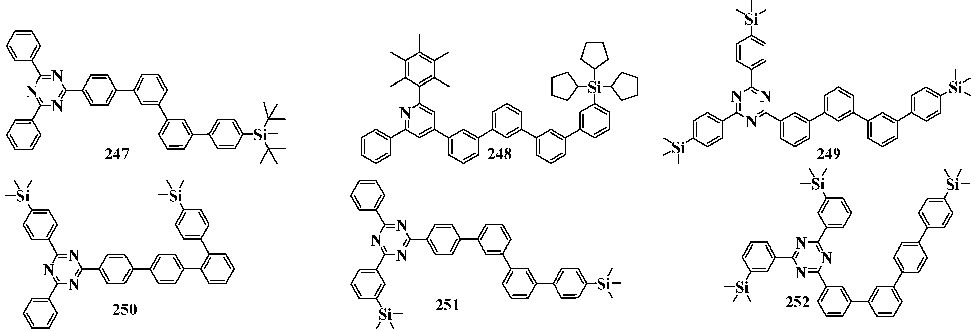

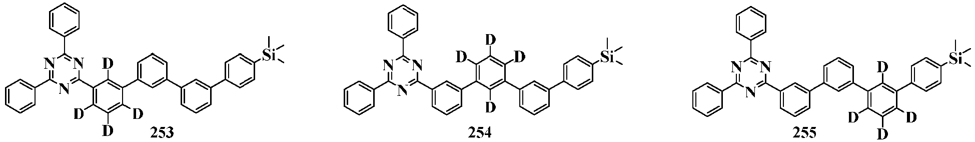

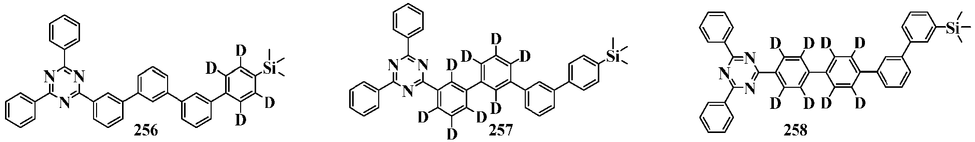

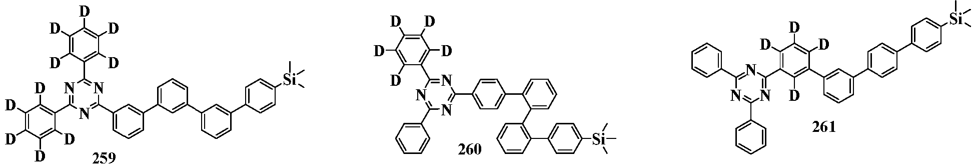

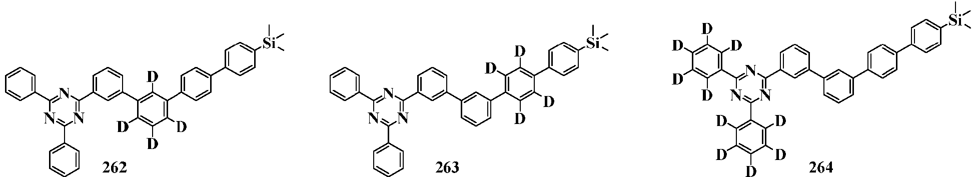

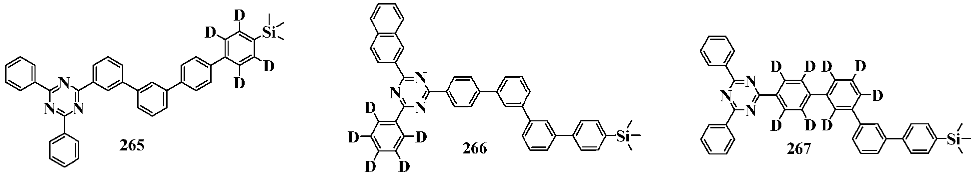

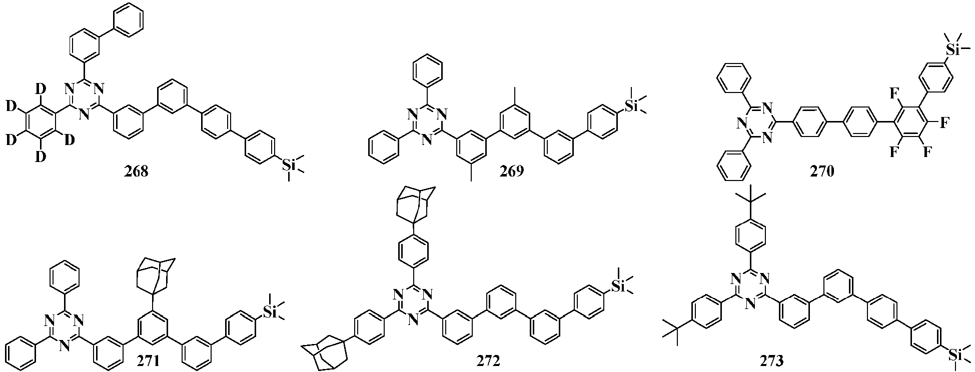

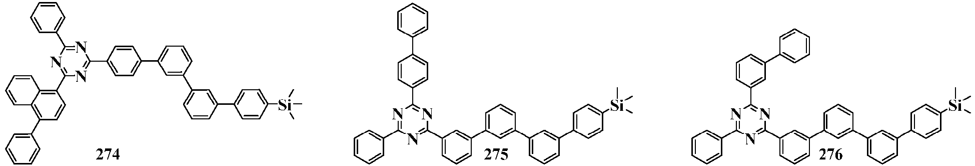

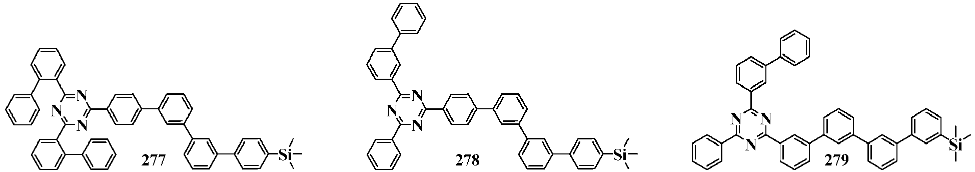

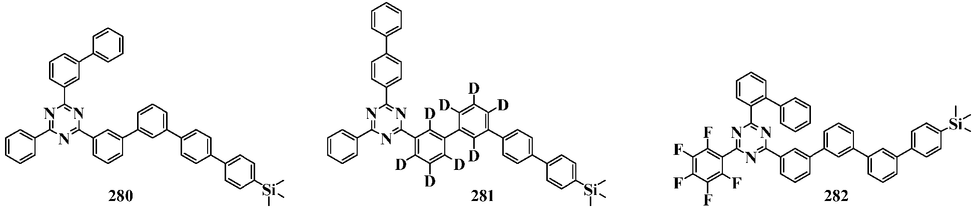

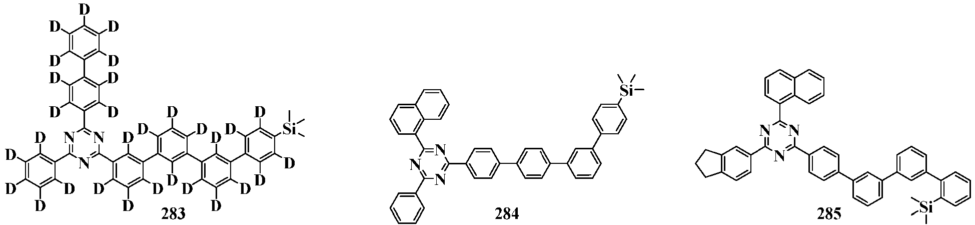

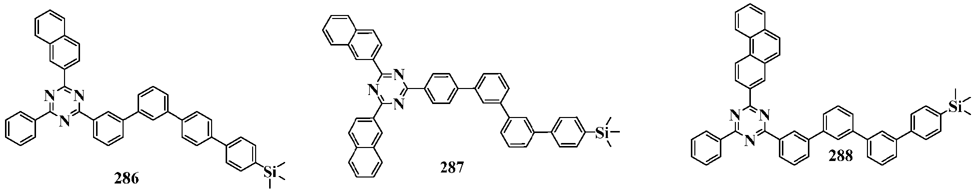

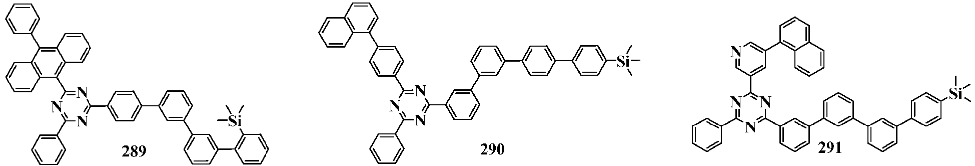

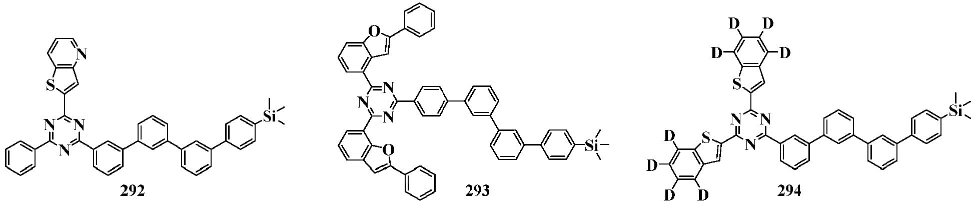

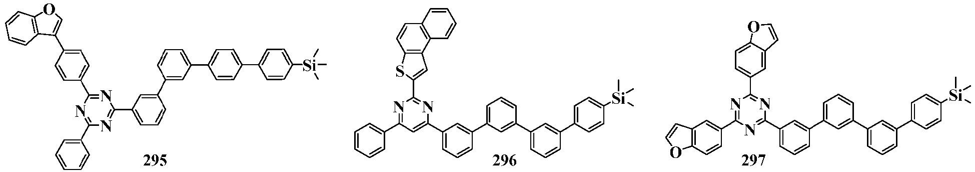

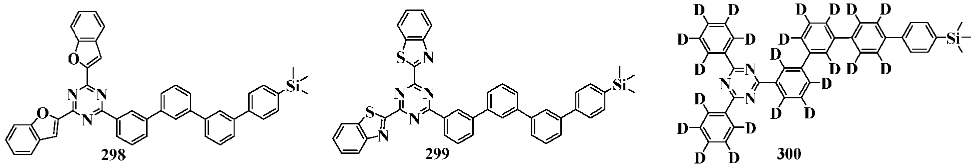

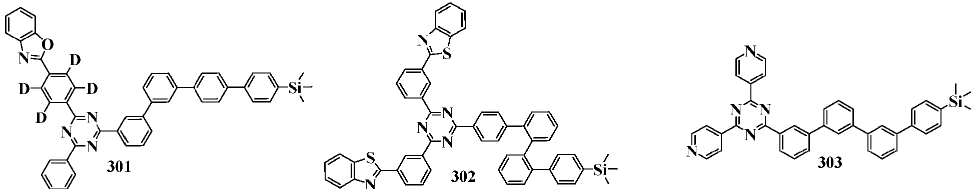

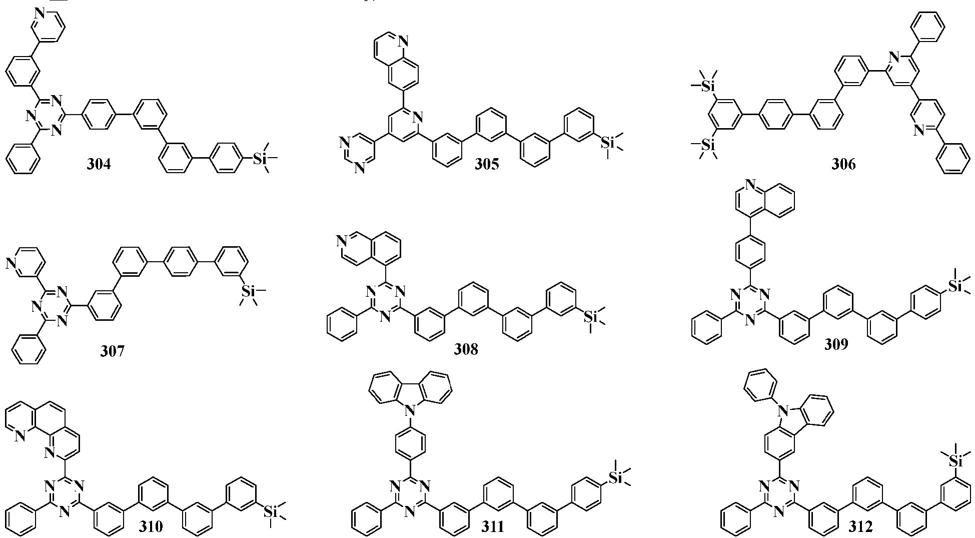

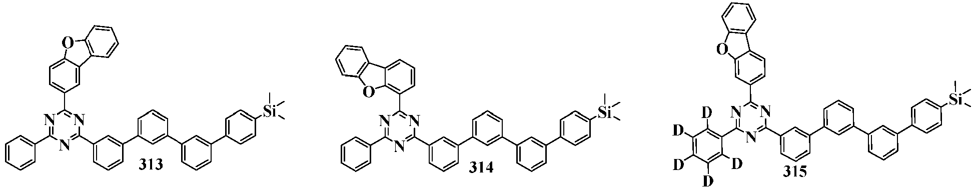

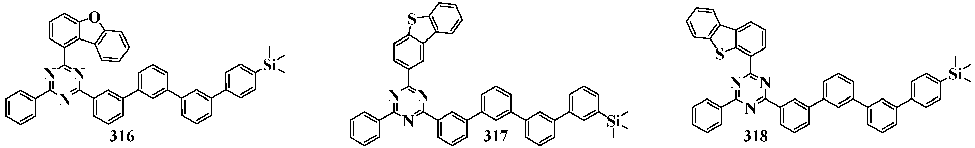

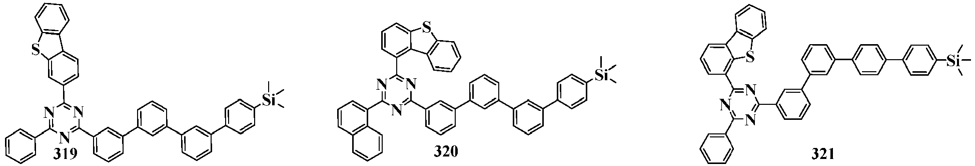

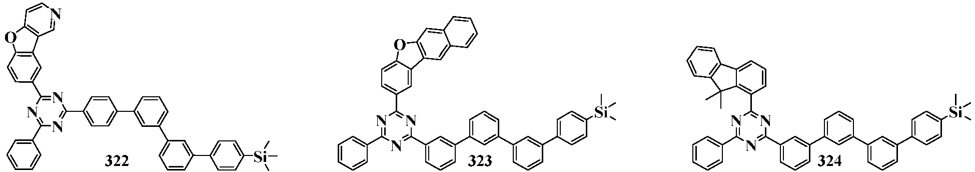

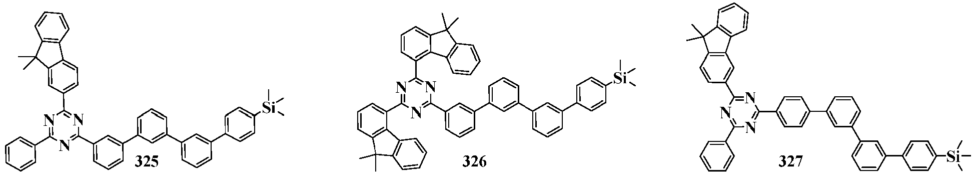

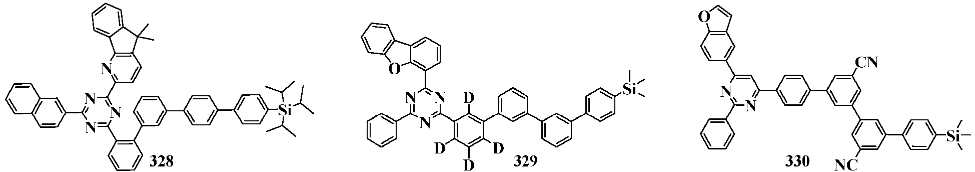

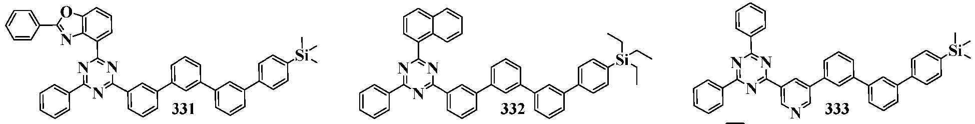

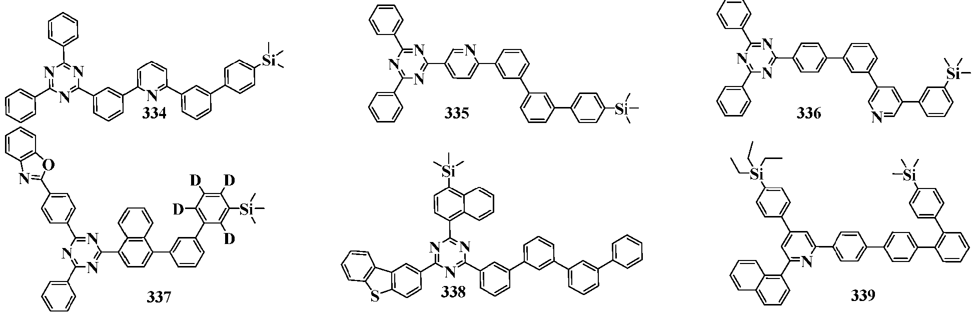

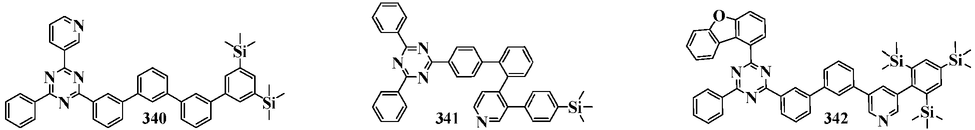

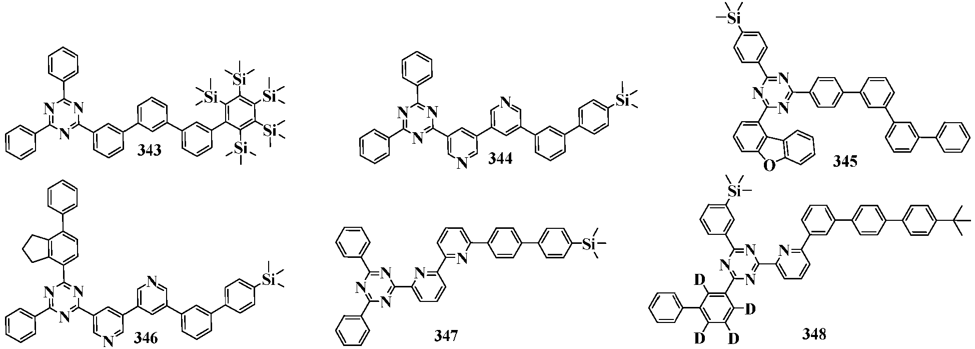

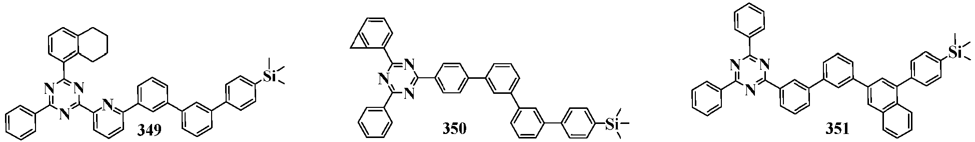

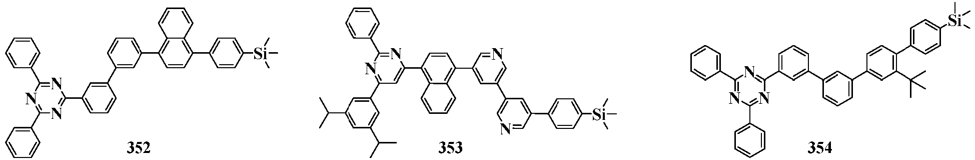

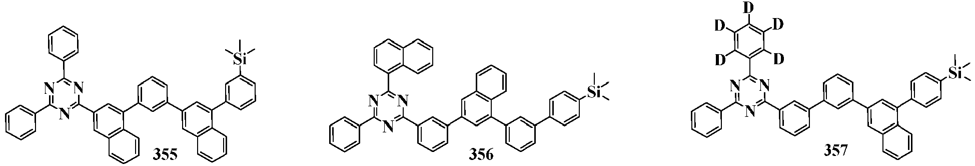

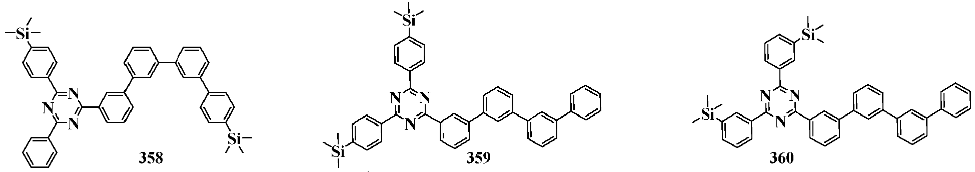

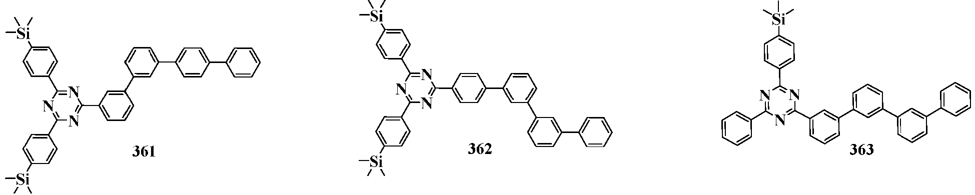

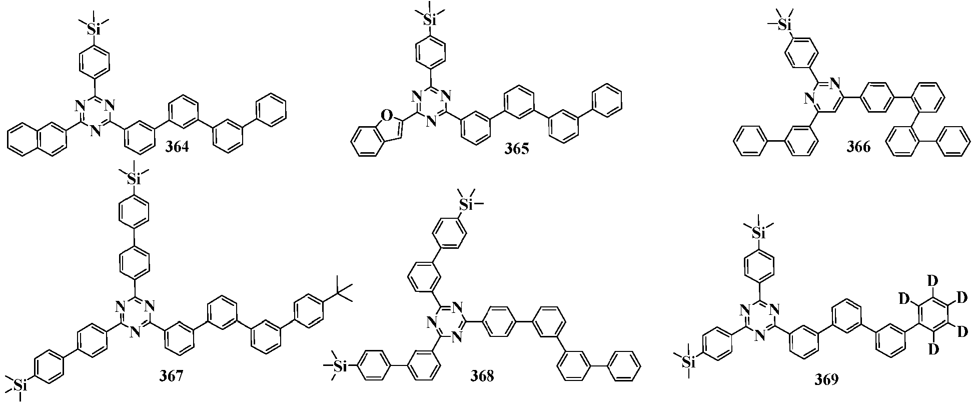

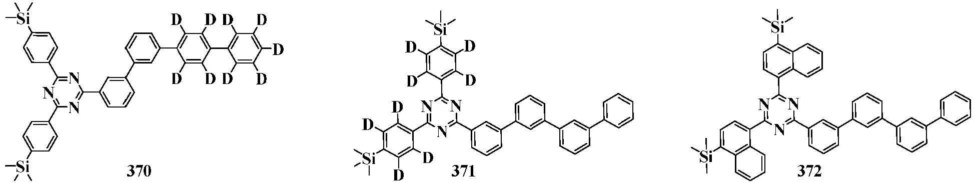

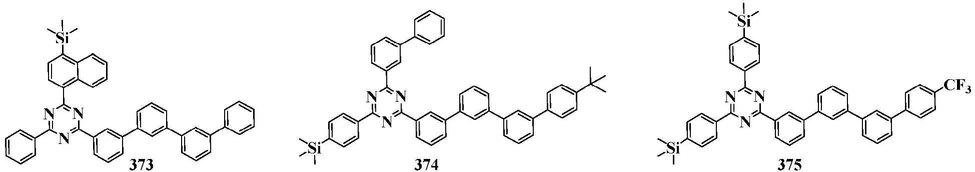

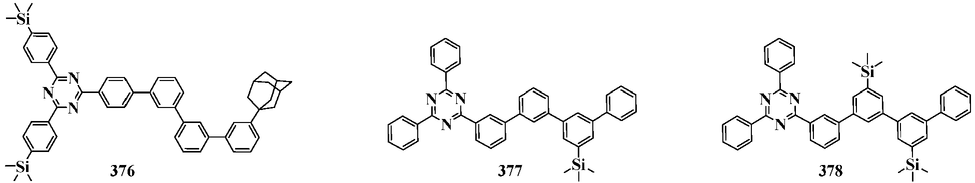

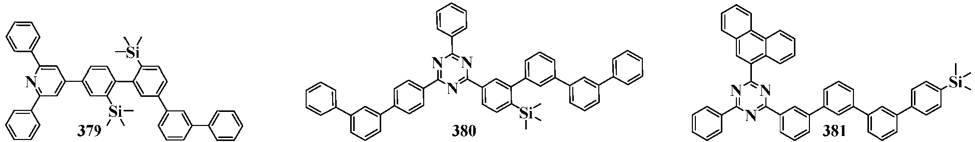

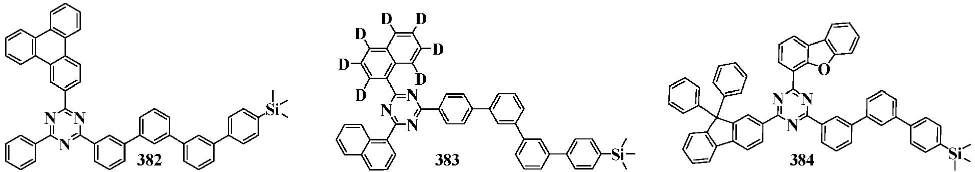

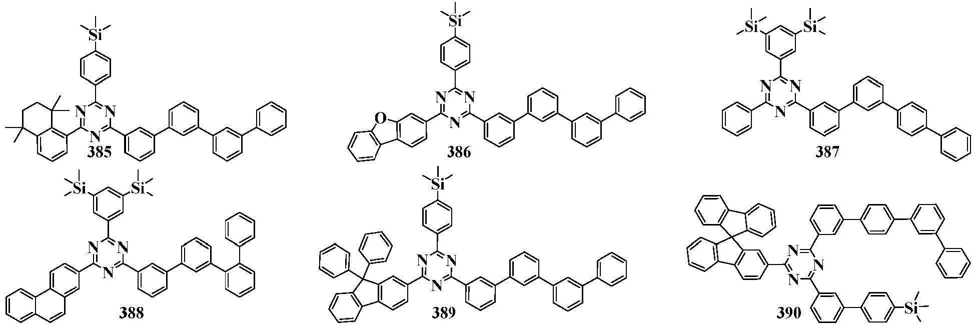

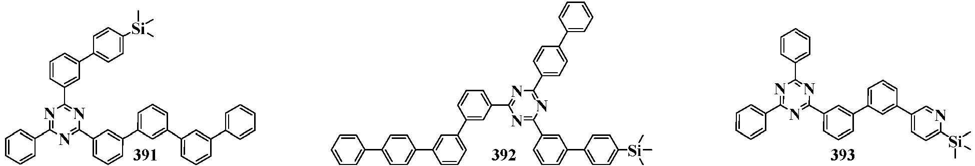

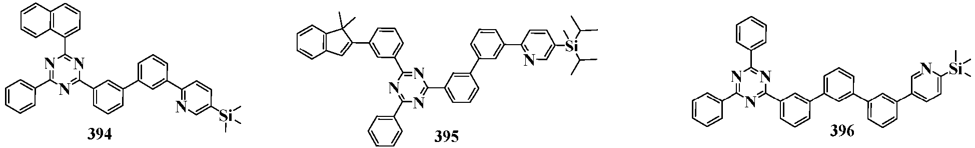

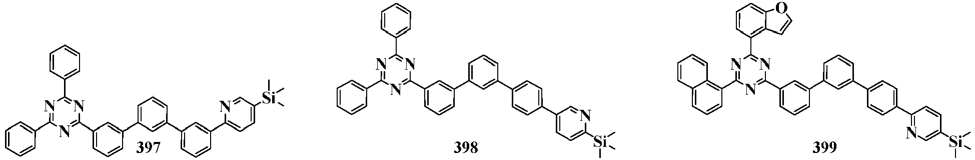

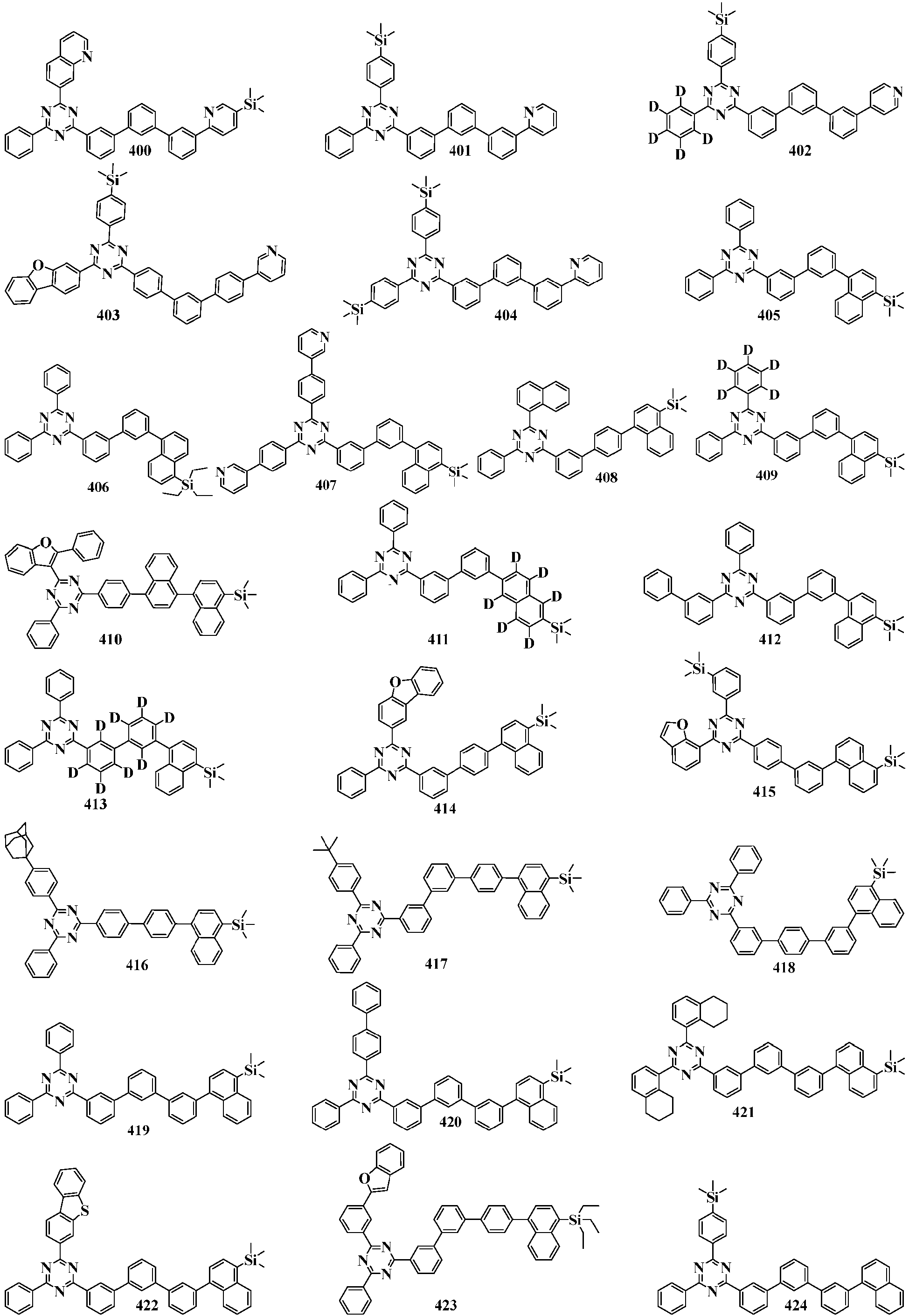

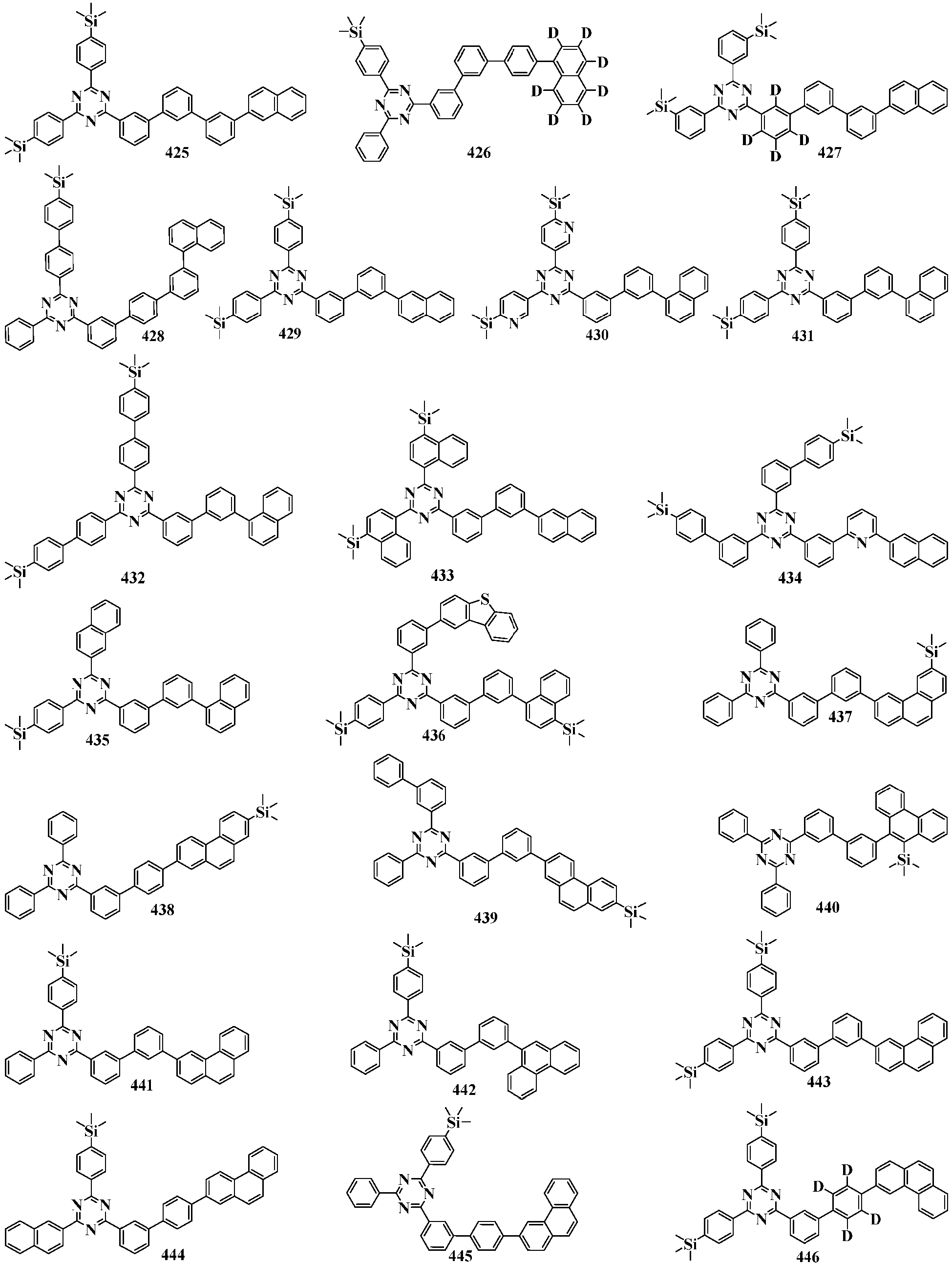

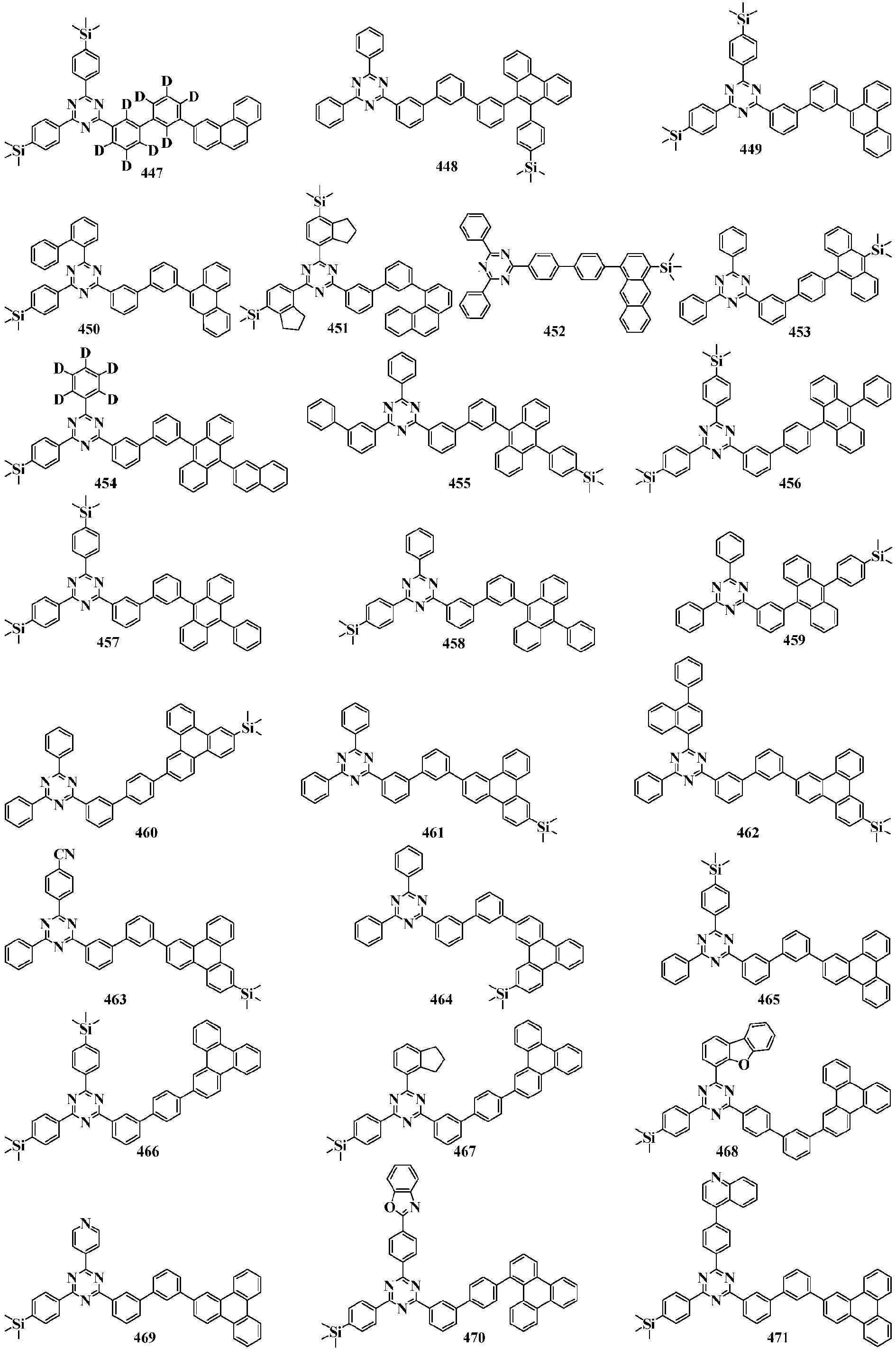

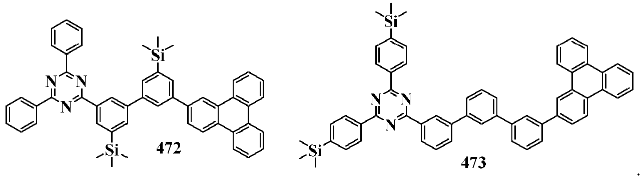

Some specific chemical structures of the heterocyclic compound of the present disclosure represented by Formula I are exemplified above. However, the present disclosure is not limited to the exemplified chemical structures. Any structures based on the structure represented by Formula I and having substituents which are the groups defined above are included.

The present disclosure further provides an organic electroluminescent device, wherein the organic electroluminescent device includes an anode, a cathode facing the anode and an organic layer, wherein the organic layer includes any one or more of the heterocyclic compounds of the present disclosure.

The organic electroluminescent device of the present disclosure includes at least the anode, the cathode facing the anode and the organic layer. One organic electroluminescent device may include one or more organic layers, wherein the organic layer may be located between the anode and the cathode or outside at least one electrode of the anode or the cathode, and preferably, the organic layer includes any one or more of the heterocyclic compounds of the present disclosure.

Preferably, the organic layer is located between the anode and the cathode and includes any one or more of the heterocyclic compounds of the present disclosure.

Preferably, the organic layer includes a light-emitting layer, an electron transport region and a hole transport region, wherein the light-emitting layer or the electron transport region includes any one or more of the heterocyclic compounds of the present disclosure.

Specifically, the organic layer located between the anode and the cathode may include a light-emitting layer, an electron transport region and a hole transport region, wherein the hole transport region includes a hole injection layer, a hole transport layer, a light-emitting auxiliary layer and an electron blocking layer, and the electron transport region includes a hole blocking layer, an electron transport layer and an electron injection layer; the organic layer located outside the at least one electrode of the anode or the cathode includes a light extraction layer. Each of the above functional layers may include a single film or multiple films. Each film may be composed of only one material or multiple materials.

Preferably, the organic layer includes a light-emitting layer, wherein the light-emitting layer includes any one or more of the heterocyclic compounds of the present disclosure.

Preferably, the light-emitting layer includes a host material and a guest material, wherein the host material includes any one or more of the heterocyclic compounds of the present disclosure.

Preferably, the organic layer includes an electron transport region, wherein the electron transport region includes at least one of an electron injection layer, an electron transport layer and a hole blocking layer, wherein the at least one of the electron injection layer, the electron transport layer and the hole blocking layer includes any one or more of the heterocyclic compounds of the present disclosure.

Preferably, the organic layer includes at least one of an electron transport layer or a hole blocking layer, wherein the at least one of the electron transport layer or the hole blocking layer includes any one or more of the heterocyclic compounds of the present disclosure.

Preferably, the organic layer includes an electron transport layer, wherein the electron transport layer includes any one or more of the heterocyclic compounds of the present disclosure.

Preferably, the organic layer includes a hole blocking layer, wherein the hole blocking layer includes any one or more of the heterocyclic compounds of the present disclosure.

Preferably, the organic layer includes a light extraction layer, wherein the light extraction layer includes any one or more of the heterocyclic compounds of the present disclosure.

A material of each film in the organic electroluminescent device is not particularly limited in the present disclosure, which may be a substance known in the art. The above-mentioned organic functional layers of the organic electroluminescent device and electrodes on two sides of the device are separately described below.

The anode in the present disclosure needs a relatively high work function to improve injection efficiency of holes. A material of the anode may be selected from the following materials such as a metal oxide, a combination of a metal and an oxide, and a metal or an alloy thereof. Specific examples may include, but are not limited to, indium tin oxide (ITO), indium zinc oxide (IZO), aluminium (Al), titanium (Ti), gold (Au), platinum (Pt), copper (Cu), silver (Ag), indium tin oxide/silver/indium tin oxide (ITO/Ag/ITO) , and the like.

The cathode in the present disclosure needs a relatively low work function to improve injection efficiency of electrons. A material of the cathode may be selected from the following materials such as a metal or an alloy thereof. Specific examples may include, but are not limited to, aluminium (Al), silver (Ag), calcium (Ca), indium (In), magnesium: silver (Mg:Ag) , and the like.

A material of the hole injection layer in the present disclosure needs a relatively good hole injection ability and a relatively proper HOMO energy level to reduce an interface barrier between the anode and the hole transport layer and improve the hole injection ability. The material of the hole injection layer may be selected from the following materials such as an arylamine derivative, a metal oxide, a phthalocyanine metal complex, a multi-cyano conjugated organic compound or a polymer. Specific examples may include, but are not limited to, 2,3,6,7,10,11-hexacyano-1,4,5,8,9,12-hexaazatriphenylene (HAT-CN), copper phthalocyanine (CuPC), 4,4',4"-tris(N,N-2-naphthylphenylamino)triphenylamine (2-TNATA), 4,4',4"-tris(N-3-methylphenyl-N-phenylamino)triphenylamine (m-MTDATA), 2,3, 5,6-tetrafluoro-7,7', 8, 8'-tetracyanodquinodimethane (F4-TCNQ), poly(4-vinyltriphenylamine) (PVTPA), and the like.

A material of the hole transport layer in the present disclosure needs a relatively high hole mobility to inject holes. The material of the hole transport layer may be selected from the following materials such as an arylamine derivative, a carbazole derivative, a fluorene derivative or a polymer. Specific examples may include, but are not limited to, N,N'-bis(1-naphthalenyl)-N,N'-bisphenyl-(1,1'-biphenyl)-4,4'-diamine (NPB), N,N'-bis(naphthalene-2-yl)-N,N'-diphenyl-[1,1'-biphenyl]-4,4'-diamine (β-NPB), N,N,N',N'-tetra-1-naphthalenyl[1,1'-biphenyl]-4,4'-diamine (α-TNB), N,N'-bis(3-methylphenyl)-N,N'-diphenyl-(1,1'-biphenyl)-4,4'-diamine (TPD), 4,4'-cyclohexylidenebis[N,N-bis(4-methylphenyl)aniline] (TAPC), 4,4',4"-tris(carbazol-9-yl)-triphenylamine (TCTA), polyvinylcarbazole (PVC), and the like.

A material of the electron blocking layer in the present disclosure needs a relatively good hole transport ability and electron blocking ability to effectively transport holes and prevent electrons from escaping to an interface of the light-emitting layer. The material of the electron blocking layer may be selected from the following materials such as an arylamine derivative or a carbazole derivative. Specific examples may include, but are not limited to, N,N-bis([1,1'-biphenyl]-4-yl)-(9H-carbazol-9-yl)-[1,1'-biphenyl]-4-amine, N-(4'-(9H-carbazol-9-yl)-[1,1'-biphenyl]-4-N-([1,1'-biphenyl]-4-yl)-9,9-dimethyl-9H-fluoren-2-amine, N,N'-bis(naphthalene-1-yl)-N,N'-diphenyl-benzidine (NPD) , and the like.

As a material of the light-emitting layer in the present disclosure, a red, green or blue light-emitting material may be used, and a guest (doped) material and a host material are generally contained. The guest material may be a simple fluorescent material, phosphorescent material or TADF material, or may be formed by a combination of the fluorescent and phosphorescent materials. The host material of the light-emitting layer needs not only a bipolar charge transport property but also a proper energy level to efficiently transfer excitation energy to the guest light-emitting material. The material of this type may include a diphenylvinylaryl derivative, a stilbene derivative, a carbazole derivative, a triarylamine derivative, an anthracene derivative and a pyrene derivative, preferably at least one of the heterocyclic compounds of the present disclosure. Specific examples may include, but are not limited to, 4,4'-bis(9-carbazole)biphenyl (CBP), 4,4'-bis(9-carbazolyl)-2,2'-dimethylbiphenyl (CDBP), 9-(4-*tert*-butylphenyl)-3,6-bis(triphenylsilyl)-9H-carbazole (CzSi), 9,9'-(2,6-pyridinediyldi-3,1-phenylene)bis-9H-carbazole (26DCZPPY), 9,9'-diphenyl-9*H*,9*H*-3,3'-bicarbazole (BCzPh), 9-(5-(3-9H-carbazol-9-yl)phenyl)pyridine-3-yl)-9H-carbazole (CPPyC), 4,4'-bis(carbazol-9-yl)-2,2'-dimethylbiphenyl (CDBP), 1,3-bis(N-carbazolyl)benzene (MCP), 9,9-dimethyl-N,N-diphenyl-7-(4-(1-phenyl-1H-benzo[d]imadazol-2-yl)phenyl)-9H-fluoren-2-a mine (EFIN), 10-(4'-(diphenylamino)biphenyl-4-yl)acridine-9(10H)-ketone (ADBP), tris[4-(pyrenyl)-phenyl]amine (TPyPA), 9,10-di(2-naphthyl)anthracene (ADN), 2-(*tert*-butyl)-9,10-di(2-naphthyl)anthracene (TBADN), 1-(7-[9,9'-bianthracene]-10-yl-9,9-dioctyl-9H-fluoren-2-yl)pyrene (BAnF8Pye), 9,9,9',9'-tetra(4-methylphenyl)-2,2'-bi-9H-fluorene (BDAF), tris(6-fluoro-8-hydroquinolinato)aluminium (6FAlq3), tris(8-hydroxyquinolinato)aluminium (Alq3), bis(10-hydroxybenzo[H]quinolinato)beryllium (BeBq2), bis(8-hydroxyquinolinato)zinc (Znq2), and the like.

The guest material may be selected from, but is not limited to, any one or more of the following structures: a metal complex (for example, an iridium complex, a platinum complex, an osmium complex or a rhodium complex), an anthracene derivative, a pyrene derivative or a perylene derivative. Specific examples may include, but are not limited to, bis(2-(naphthalene-2-yl)pyridine)(acetylacetonate)iridium (Ir(npy)₂acac), tris[2-phenyl-4-methylquinoline)]iridium (Ir(Mphq)₃), acetylacetonate bis(2-phenylpyridine)irdium (Ir(ppy)₂(acac)), (tris[2-(3-methyl-2-pyridinyl)phenyl]iridium (Ir(3mppy)₃), bis(2-benzo[H]quinoline-C2,N')(acetylacetonate)iridium(III) (Ir(bzq)₂(acac)), tris(2-(3,5-dimethylphenyl)quinoline-C2,N')iridium(III) (Ir(dmpq)₃), (bis(1-phenyl-isoquinoline)(acetylacetonate)iridium(III) (Ir(piq)₂(acac)), 2,5,8,11-tetra-*tert*-butylperylene (TBPe), rubrene, 9-(9-phenylcarbazol-3-yl)-10-(naphthalene-1-yl) (PCAN), 1,4-bis(4-(9H-carbazol-9-yl)styryl)benzene (BCzSB), 1,1'-(4,4'-(4-phenyl-4H-1,2,4-triazole-3,5-diyl)bis(4,1-phenylene))bis(1H-phenoxazine) (2PXZ-TAZ) , and the like.

The hole blocking layer in the present disclosure has a relatively good electron transport ability and hole blocking ability to effectively transport electrons and prevent holes from escaping to the interface of the light-emitting layer. A material of the hole blocking layer may be selected from the following materials: a metal complex, a quinoline derivative, an imidazole derivative, a phenanthroline derivative, a triazole derivative and an azabenzene derivative, preferably at least one of the heterocyclic compounds of the present disclosure. Specific examples may include, but are not limited to, bis(2-methyl-8-quinolinolato-N1,O8)-(1,1'-biphenyl-4-olato)aluminum (BAlq), 1,3,5-tris(N-phenyl-2-benzimidazole)benzene (TPBi), 2,9-dimethyl-4,7-biphenyl-1,10-phenanthroline (BCP), 3,3'-[5'-[3-(3-pyridyl)phenyl][1,1':3',1"-terphenyl]-3,3"-diyl]dipyridine (TmPyPB), and the like.

An electron transport material in the present disclosure needs a relatively high electron mobility to inject electrons. The material of the electron transport layer may be selected from the following materials: a quinoline derivative, an imidazole derivative, a phenanthroline derivative, a triazole derivative, a metal chelate, an azabenzene derivative, a phenazine derivative, a silicon-containing heterocyclic compound and a boron-containing heterocyclic compound, preferably at least one of the heterocyclic compounds of the present disclosure. Specific examples may include, but are not limited to, tris(8-hydroxyquinolinato)aluminium (Alq₃), 2,9-bis(naphthalene-2-yl)-4,7-biphenyl-1,10-phenanthroline (NBphen), 1,3,5-tris(4-pyridine-3-ylphenyl)benzene (TpPyPB), 1,3,5-tris(4-pyridylquinoline-2-yl)benzene (TPyQB), 3-(biphenyl-4-yl)-4-phenyl-5-(4-*tert*-butylphenyl)-1,2,4-triazole (TAZ) 1,3,5-tris(N-phenyl-2-benzimidazole)benzene (TPBi), tris[2,4,6-trimethyl-3-(3-pyridyl)phenyl]borine (3TPYMB), and the like.

An electron injection material in the present disclosure needs a relatively good electron injection ability and a relatively proper LUMO energy level to reduce an interface barrier between the cathode and the electron transport layer and improve the electron injection ability. The material of the electron injection layer includes, but is not limited to, the following materials: a metal, an alkali metal, an alkaline-earth metal, a metal compound, a metal oxide, a metal halide, an alkaline-earth metal compound, an alkaline-earth metal oxide, an alkaline-earth metal halide, an alkali metal compound, an alkali metal oxide and an alkali metal halide. Specific examples may include, but are not limited to, lithium (Li), strontium (Sr), ytterbium (Yb), lithium fluoride (LiF), sodium fluoride (NaF), cesium fluoride (CsF), calcium fluoride (CaF₂), 8-hydroxyquinolinolato-lithium (Liq), tris(8-hydroxyquinolinato)aluminium (Alq₃), cesium carbonate (Cs₂CO₃), rubidium acetate (CH₃COORb), lithium oxide (Li₂O), barium oxide (BaO), and the like.

The light extraction layer in the present disclosure has an effect of coupling light to improve light emission efficiency. A material of the light extraction layer may include the following materials: a metal compound, a triarylamine derivative, a benzidine derivative and a carbazole derivative, preferably at least one of the heterocyclic compounds of the present disclosure. Specific examples may include tris(8-hydroxyquinolinato)aluminium (Alq₃), N,N'-bis(naphthalene-1-yl)-N,N'-bis(phenyl)-2,2'-dimethylbenzidine (NPD), 4,4'-bis(9-carbazole)biphenyl (CBP), and the like.

A method for preparing the films in the organic electroluminescent device of the present disclosure is not particularly limited, which may use, but is not limited to, a vacuum evaporation method, a sputtering method, a spin coating method, a spraying method, a screen printing method, and a laser transfer printing method.

The organic electroluminescent device of the present disclosure is mainly applied to the field of information display technology and the field of lighting and is widely applied to various information displays in the aspect of information display, such as a mobile phone, a tablet computer, a flat-panel television, a smart watch, VR, an in-vehicle system, a digital camera, a wearable device, and the like.

### Synthesis Example

Note of raw materials and reagents: The raw materials or reagents used in the following synthesis examples are not particularly limited in the present disclosure and may be commercially available products or be prepared by preparation methods well-known to those skilled in the art.

Instruments: G2-Si quadrupole tandem time-of-flight high-resolution mass spectrometer (WATERS CORPORATION, UK); Vario EL cube elemental analyzer (ELEMENTAR CO., GERMANY).

A core structure of the compound of the present disclosure represented by Formula I may be prepared by the following reaction route. Substituents may be bonded by methods known in the art, and types, positions and numbers of the substituents may be changed according to technologies known in the art.

### Synthetic route:

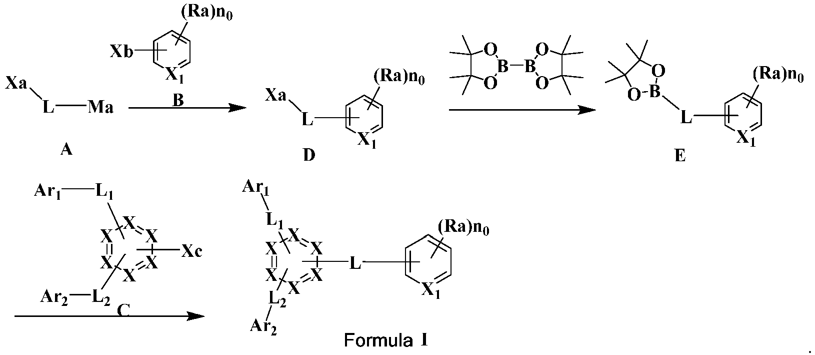

Main reaction types involved in the present disclosure are a Suzuki coupling reaction and a Miyaura borylation reaction. The raw materials in the synthetic route provided in the present disclosure may be commercially available products, or may be prepared by well-known preparation methods in the art.

For example, when L is selected from 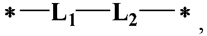 the raw material A may be prepared by, but is not limited to, the following synthetic route:

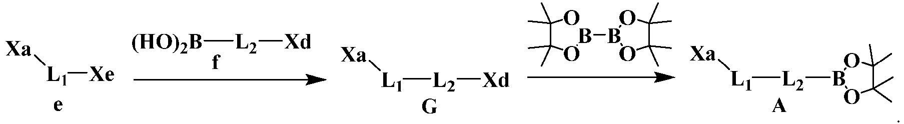

The raw material C may be prepared by the following synthetic route: 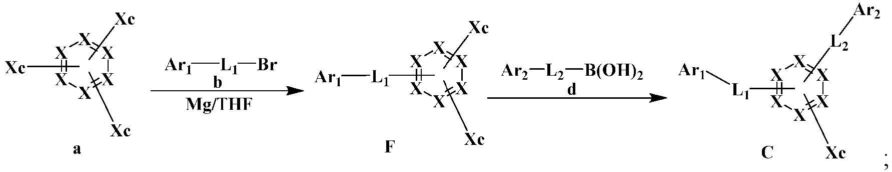
wherein when Ar₁ is the same as Ar₂, the above Ar₁ and Ar₂ groups may be introduced together into one step to obtain the raw material C, that is: 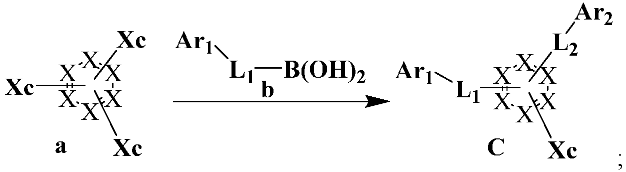
wherein Xa, Xb, Xc, Xd and Xe are independently selected from Cl, Br or I, and Ma is independently selected from

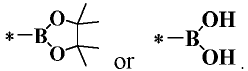

Alternatively, an order of the above reaction may also be changed to obtain the heterocyclic compound of the present disclosure represented by Formula I.

### Synthesis Example 1: Preparation of Intermediate C-5

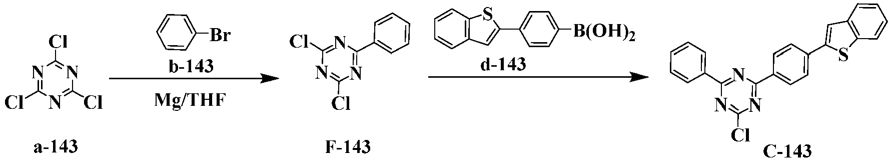

In step 1, under nitrogen protection, an anhydrous tetrahydrofuran solvent (50 mL) was added to magnesium turnings (5.04 g, 210 mmol), and then two grains of iodine were added; a tetrahydrofuran solution (100 mL) of b-143 (31.40 g, 200 mmol) was slowly added dropwise, and a Grignard reaction was initiated; after the dropwise addition was finished, the mixture was reacted for 7.5 h at room temperature; after the reaction was finished, the mixture was cooled to room temperature.

In step 2, under nitrogen protection, a-143 (36.88 g, 200 mmol) was added to a reaction flask, and then a tetrahydrofuran solvent (200 mL) was added; the system was reduced to -5 °C, and then the Gregnard reagent prepared in step 1 was slowly added dropwise for 2 to 3 h; after the dropwise addition was finished, the mixture was reacted for 5 h at -5 °C; after the reaction was finished, the reaction solution was poured into 12% dilute hydrochloric acid; the mixture was fully stirred for 30 min and extracted with dichloromethane (300 mL×3 times), an organic phase was separated and dried with anhydrous magnesium sulfate, a solvent was concentrated by distillation at reduced pressure, and a filter cake was recrystallized with tetrahydrofuran after vacuum filtration to obtain Intermediate F-143 (28.03 g, with a yield of 62%) with an HPLC purity of ≥ 99.78%. Mass spectrometry (m/z): 224.9843 (theoretical value: 224.9861).

In step 3, under nitrogen protection, Intermediate F-143 (24.87 g, 110 mmol), d-143 (25.41 g, 100 mmol) and anhydrous potassium carbonate (27.64 g, 200 mmol) were added to a reaction flask, and then a toluene solution (250 ml) was added; after air was purged with nitrogen for three times, tetrakis(triphenylphosphine)palladium (1.16 g, 1.0 mmol) was added, and the mixture was stirred, heated and reacted for 8.5 h. After the reaction was finished, the mixture was cooled to room temperature, a solvent was concentrated by distillation at reduced pressure, a filter cake was washed with ethanol after vacuum filtration, and the obtained filter cake was recrystallized with toluene to obtain Intermediate C-143 (25.99 g, with a yield of 65%) with an HPLC purity of ≥ 99.80%. Mass spectrometry (m/z): 399.0584 (theoretical value: 399.0597).

According to the above method for preparing Intermediate C-143, the raw material b-143 and the raw material d-143 in Synthesis Example 1 were replaced with the raw materials b and the raw materials d in the table to synthesize the following Intermediates C:

| Raw Material a | Raw Material b | Raw Material d | Intermediate C | Yield/ HPLC Purity | Mass Spectrometry (m/z) |
|---|---|---|---|---|---|
| 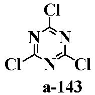 | 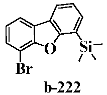 | 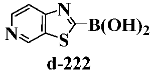 | 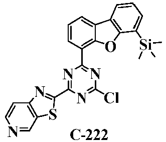 | 29.22 g; HPLC purity ≥ 99.75% | measured value: 487.0706 |
| | | | | | theoretical value: 487.0690 |
| 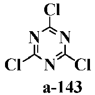 | 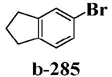 | 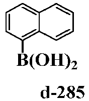 | 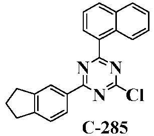 | 23.56 g; HPLC purity ≥ 99.88% | measured value: 357.1049 |
| | | | | | theoretical value: 357.1033 |
| 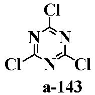 | 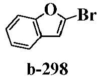 | 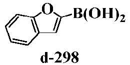 | 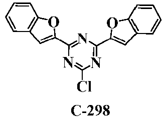 | 23.25 g; HPLC purity ≥ 99.83% | measured value: 347.0453 |
| | | | | | theoretical value: 347.0462 |
| 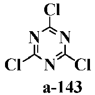 | 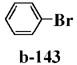 | 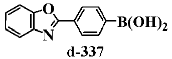 | 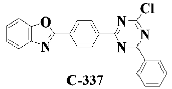 | 24.85 g; HPLC purity ≥ 99.79% | measured value: 400.1075 |
| | | | | | theoretical value: 400.1091 |
| 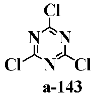 | 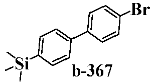 | 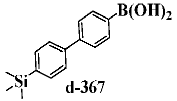 | 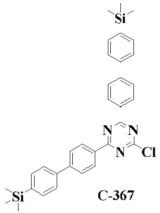 | 35.47 g; HPLC purity ≥ 99.86% | measured value: 563.1967 |
| | | | | | theoretical value: 563.1980 |
| 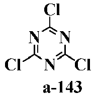 | 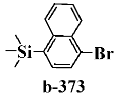 | 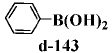 | 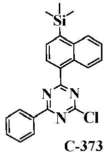 | 25.29 g; HPLC purity ≥ 99.87% | measured value: 389.1110 |
| | | | | | theoretical value: 389.1115 |
| 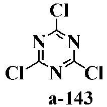 | 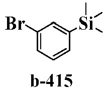 | 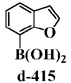 | 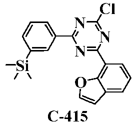 | 24.08 g; HPLC purity ≥ 99.80% | measured value: 379.0923 |
| | | | | | theoretical value: 379.0908 |
| 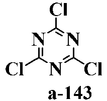 | 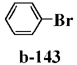 | 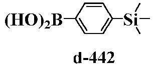 | 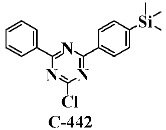 | 23.05 g; HPLC purity ≥ 99.89% | measured value: 339.0967 |
| | | | | | theoretical value: 339.0959 |
| 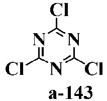 | 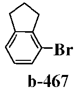 | 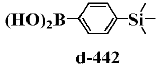 | 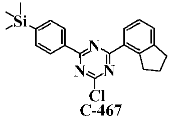 | 23.87 g; HPLC purity ≥ 99.81% | measured value: 379.1258 |
| | | | | | theoretical value: 379.1272 |

### Synthesis Example 2: Preparation of Compound 1

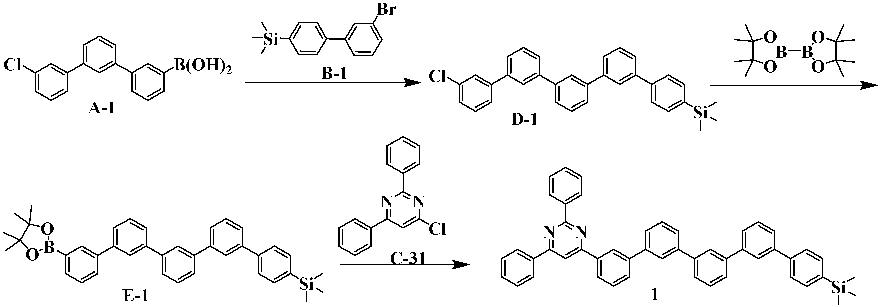

### Preparation of D-1:

Under nitrogen protection, A-1 (37.03 g, 120 mmol), B-1 (36.63 g, 120 mmol) and potassium carbonate (33.17 g, 240 mmol) were added to a reaction flask. Then, a toluene/ethanol/water mixed solvent (v toluene : v ethanol : v water = 2:1:1) (400 mL) was added. Then, tetrakis(triphenylphosphine)palladium (0.14 g, 0.12 mmol) was added, and the mixture was reacted for 3.5 h under conditions of stir and reflux. After the reaction was finished, the reactant was cooled to room temperature, distilled water was added, the mixture stood still to separate layers, a solvent of the separated organic phase was concentrated by distillation at reduced pressure, a filter cake was washed with ethanol after vacuum filtration, and the obtained filter cake was recrystallized with toluene/ethanol (v toluene : v ethanol = 10:1) to obtain D-1 (41.67 g, 71%) with an HPLC purity of ≥ 99.81%. Mass spectrometry (m/z): 488.1742 (theoretical value: 488.1727).

### Preparation of E-1:

Under nitrogen protection, D-1 (39.13 g, 80 mmol), bis(pinacolato)diboron (21.58 g, 85 mmol) and KOAc (23.55 g, 240 mmol) were added to a reaction flask. Then, 1,4-dioxane (350 mL) was added. After air was purged with nitrogen for three times, Pd(dppf)Cl₂ (0.73 g, 1.0 mmol) was added, and the mixture was stirred and reacted for 6 h under a condition of heating. After the reaction was finished, the reactant was cooled to room temperature, distilled water was added, the mixture was extracted with dichloromethane (700 mL×3 times), an organic phase was separated and dried with anhydrous magnesium sulfate, and the obtained solid was recrystallized with toluene to obtain E-1 (39.02 g, 84%) with an HPLC purity ≥ 99.92%. Mass spectrometry (m/z): 580.2951 (theoretical value: 580.2969).

### Preparation of Compound 1:

Under nitrogen protection, E-1 (29.03 g, 50 mmol), C-1 (13.34 g, 50 mmol) and potassium carbonate (13.82 g, 100 mmol) were added to a reaction flask. Then, a toluene/ethanol/water mixed solvent (v toluene:v ethanol: v water = 2:1:1) (200 mL) was added. Then, Pd₂(dba)₃ (0.92 g, 1.0 mmol) and P(t-Bu)₃ (4.0 mL, 2.0 mmol) (0.5 M toluene solution) were added, and the mixture was heated, stirred and reacted for 7.5 h. After the reaction was finished, the reactant was cooled to room temperature, distilled water was added, the mixture stood still to separate layers, a solvent of the separated organic phase was concentrated by distillation at reduced pressure, a filter cake was washed with ethanol and distilled water after vacuum filtration, and the obtained filter cake was recrystallized with toluene to obtain Compound 1 (21.92 g, 64%) with an HPLC purity of ≥ 99.92%. Mass spectrometry (m/z): 684.2978 (theoretical value: 684.2961). Theoretical element content (%) of C₄₉H₄₀N₂Si: C, 85.92; H, 5.89; N, 4.09. Measured element content (%): C, 85.88; H, 5.85; N, 4.12.

### Synthesis Example 3: Preparation of Compound 17

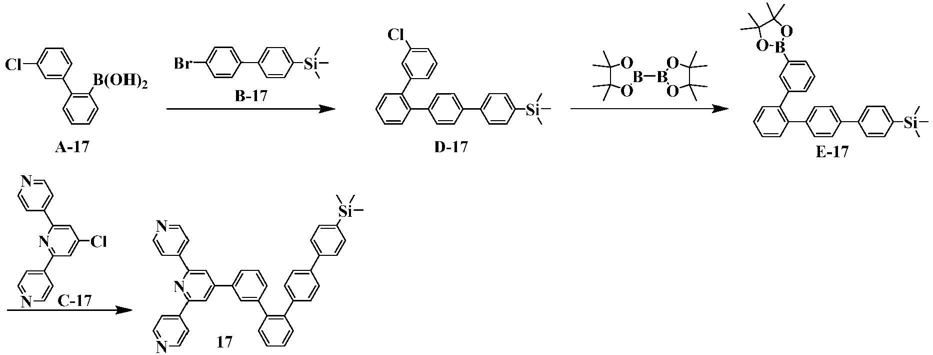

According to the same preparation method as the method for preparing Compound 1 in Synthesis Example 2, A-1, B-1 and C-1 were replaced with equalmolar A-17, B-17 and C-17, respectively, and other steps were the same to obtain Compound 17 (19.21 g) with a solid purity of ≥ 99.90% detected by HPLC. Mass spectrometry (m/z): 609.2588 (theoretical value: 609.2600). Theoretical element content (%) of C₄₂H₃₅N₃Si: C, 82.72; H, 5.79; N, 6.89. Measured element content (%): C, 82.75; H, 5.76; N, 6.87.

### Synthesis Example 4: Preparation of Compound 31

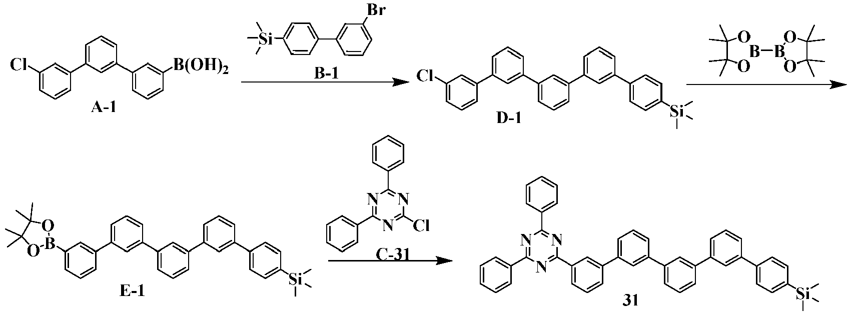

According to the same preparation method as the method for preparing Compound 1 in Synthesis Example 2, C-1 was replaced with equalmolar C-31, and other steps were the same to obtain Compound 31 (20.92 g) with a solid purity of ≥ 99.93% detected by HPLC. Mass spectrometry (m/z): 685.2901 (theoretical value: 685.2913). Theoretical element content (%) of C₄₈H₃₉N₃Si: C, 84.05; H, 5.73; N, 6.13. Measured element content (%): C, 84.08; H, 5.77; N, 6.09.

### Synthesis Example 5: Preparation of Compound 63

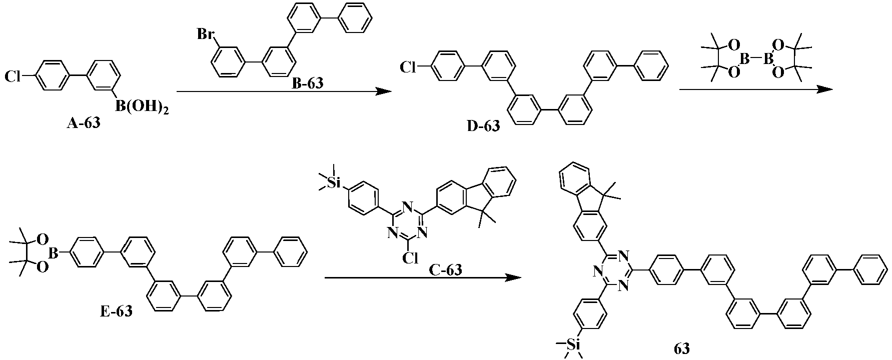

According to the same preparation method as the method for preparing Compound 1 in Synthesis Example 2, A-1, B-1 and C-1 were replaced with equalmolar A-63, B-63 and C-63, respectively, and other steps were the same to obtain Compound 63 (26.35 g) with a solid purity of ≥ 99.90% detected by HPLC. Mass spectrometry (m/z): 877.3872 (theoretical value: 877.3852). Theoretical element content (%) of C₆₃H₅₁N₃Si: C, 86.16; H, 5.85; N, 4.78. Measured element content (%): C, 86.11; H, 5.83; N, 4.75.

### Synthesis Example 6: Preparation of Compound 74

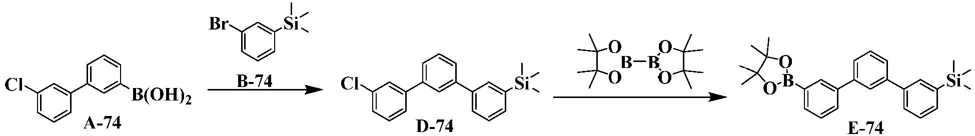

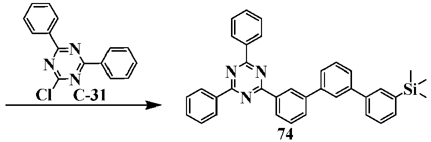

According to the same preparation method as the method for preparing Compound 1 in Synthesis Example 2, A-1, B-1 and C-1 were replaced with equalmolar A-74, B-74 and C-31, respectively, and other steps were the same to obtain Compound 74 (17.35 g) with a solid purity of ≥ 99.98% detected by HPLC. Mass spectrometry (m/z): 533.2269 (theoretical value: 533.2287). Theoretical element content (%) of C₃₆H₃₁N₃Si: C, 81.01; H, 5.85; N, 7.87. Measured element content (%): C, 81.05; H, 5.86; N, 7.85.

### Synthesis Example 7: Preparation of Compound 76

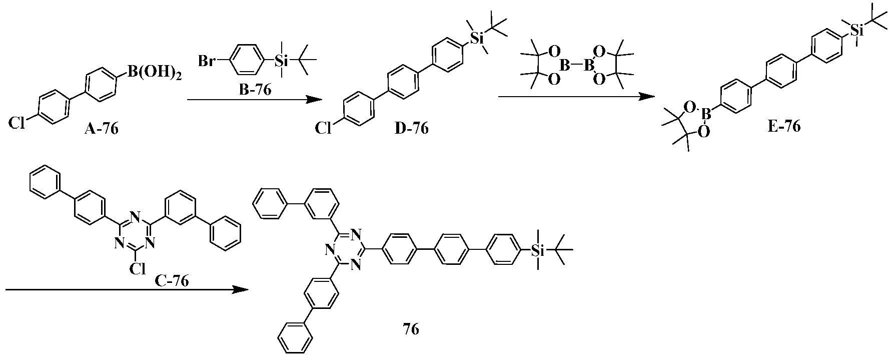

According to the same preparation method as the method for preparing Compound 1 in Synthesis Example 2, A-1, B-1 and C-1 were replaced with equalmolar A-76, B-76 and C-76, respectively, and other steps were the same to obtain Compound 76 (22.20 g) with a solid purity of ≥ 99.92% detected by HPLC. Mass spectrometry (m/z): 727.3363 (theoretical value: 727.3383). Theoretical element content (%) of C₅₁H₄₅N₃Si: C, 84.14; H, 6.23; N, 5.77. Measured element content (%): C, 84.18; H, 6.20; N, 5.74.

### Synthesis Example 8: Preparation of Compound 99

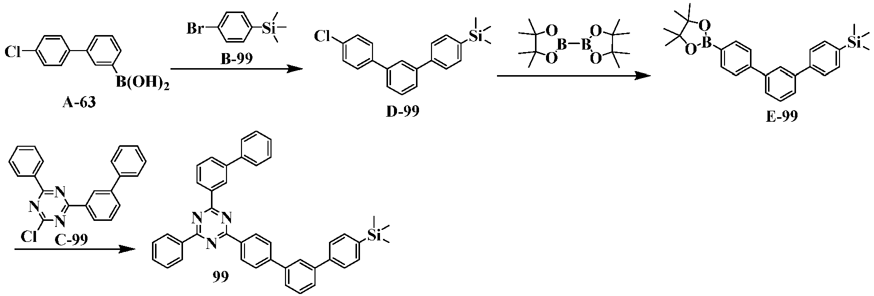

According to the same preparation method as the method for preparing Compound 1 in Synthesis Example 2, A-1, B-1 and C-1 were replaced with equalmolar A-63, B-99 and C-99, respectively, and other steps were the same to obtain Compound 99 (19.64 g) with a solid purity of ≥ 99.97% detected by HPLC. Mass spectrometry (m/z): 609.2589 (theoretical value: 609.2600). Theoretical element content (%) of C₄₂H₃₅N₃Si: C, 82.72; H, 5.79; N, 6.89. Measured element content (%): C, 82.69; H, 5.83; N, 6.90.

### Synthesis Example 9: Preparation of Compound 126

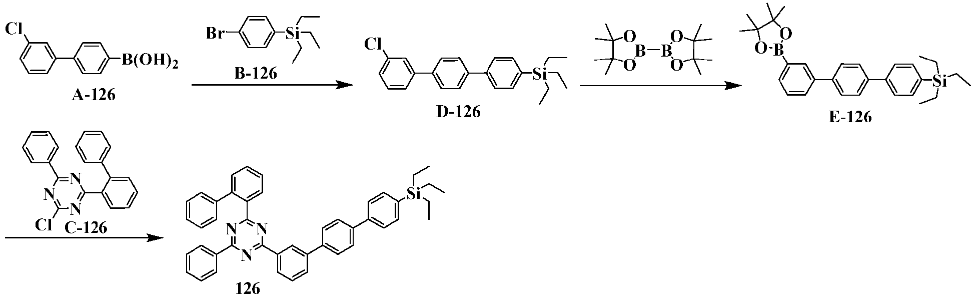

According to the same preparation method as the method for preparing Compound 1 in Synthesis Example 2, A-1, B-1 and C-1 were replaced with equalmolar A-126, B-126 and C-126, respectively, and other steps were the same to obtain Compound 126 (20.20 g) with a solid purity of ≥ 99.94% detected by HPLC. Mass spectrometry (m/z): 651.3083 (theoretical value: 651.3070). Theoretical element content (%) of C₄₅H₄₁N₃Si: C, 82.91; H, 6.34; N, 6.45. Measured element content (%): C, 82.95; H, 6.30; N, 6.47.

### Synthesis Example 10: Preparation of Compound 133

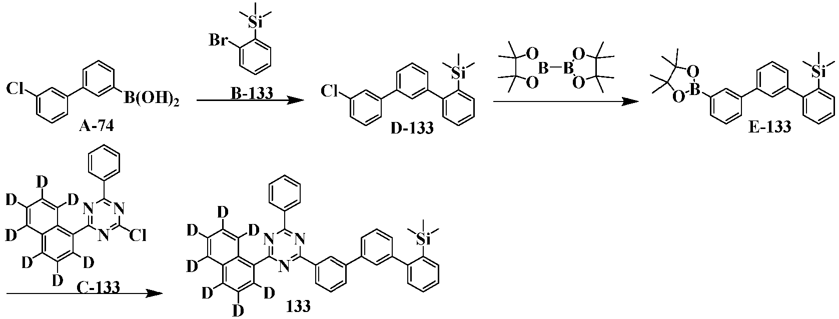

According to the same preparation method as the method for preparing Compound 1 in Synthesis Example 2, A-1, B-1 and C-1 were replaced with equalmolar A-74, B-133 and C-133, respectively, and other steps were the same to obtain Compound 133 (18.61 g) with a solid purity of ≥ 99.91% detected by HPLC. Mass spectrometry (m/z): 590.2875 (theoretical value: 590.2883). Theoretical element content (%) of C₄₀H₂₆D₇N₃Si: C, 81.31; H, 6.82; N, 7.11. Measured element content (%): C, 81.32; H, 6.86; N, 7.07.

### Synthesis Example 11: Preparation of Compound 136

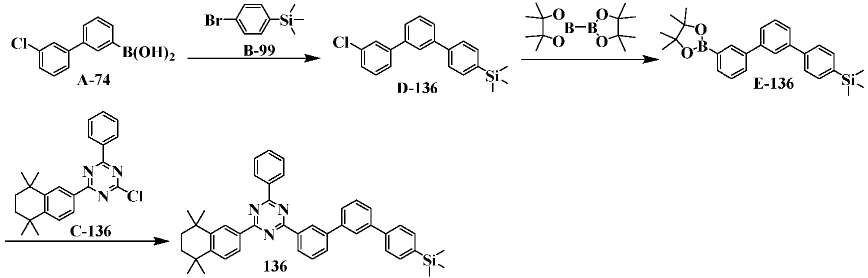

According to the same preparation method as the method for preparing Compound 1 in Synthesis Example 2, A-1, B-1 and C-1 were replaced with equalmolar A-74, B-99 and C-136, respectively, and other steps were the same to obtain Compound 136 (20.28 g) with a solid purity of ≥ 99.93% detected by HPLC. Mass spectrometry (m/z): 643.3399 (theoretical value: 643.3383). Theoretical element content (%) of C₄₄H₄₅N₃Si: C, 82.07; H, 7.04; N, 6.53. Measured element content (%): C, 82.03; H, 7.05; N, 6.50.

### Synthesis Example 12: Preparation of Compound 139

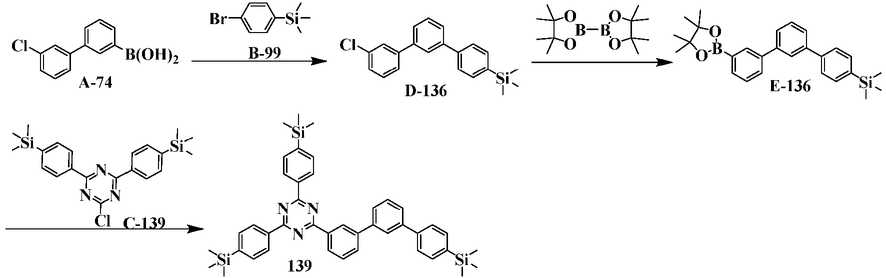

According to the same preparation method as the method for preparing Compound 1 in Synthesis Example 2, A-1, B-1 and C-1 were replaced with equalmolar A-74, B-99 and C-139, respectively, and other steps were the same to obtain Compound 139 (21.67 g) with a solid purity of ≥ 99.87% detected by HPLC. Mass spectrometry (m/z): 677.3057 (theoretical value: 677.3078). Theoretical element content (%) of C₄₂H₄₇N₃Si₃: C, 74.39; H, 6.99; N, 6.20. Measured element content (%): C, 74.42; H, 6.95; N, 6.23.

### Synthesis Example 13: Preparation of Compound 143

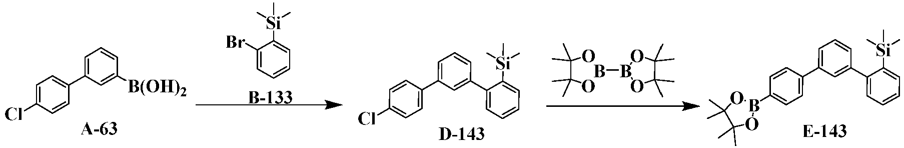

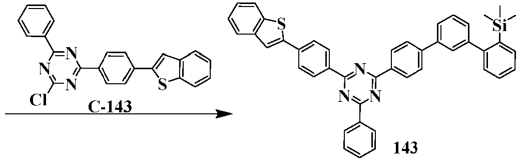

According to the same preparation method as the method for preparing Compound 1 in Synthesis Example 2, A-1, B-1 and C-1 were replaced with equalmolar A-63, B-133 and C-143, respectively, and other steps were the same to obtain Compound 143 (20.97 g) with a solid purity of ≥ 99.90% detected by HPLC. Mass spectrometry (m/z): 665.2315 (theoretical value: 665.2321). Theoretical element content (%) of C₄₄H₃₅N₃SSi: C, 79.36; H, 5.30; N, 6.31. Measured element content (%): C, 79.32; H, 5.27; N, 6.35.

### Synthesis Example 14: Preparation of Compound 154

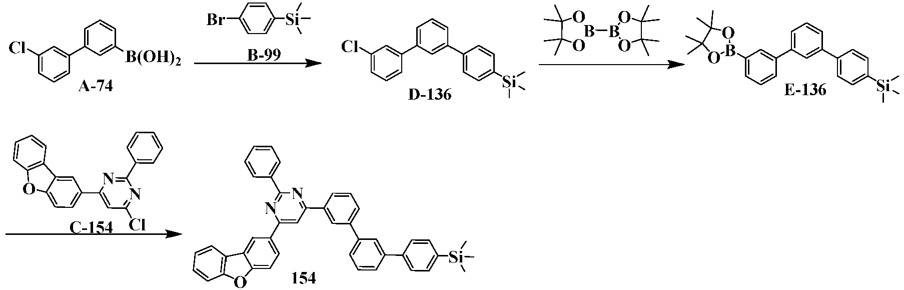

According to the same preparation method as the method for preparing Compound 1 in Synthesis Example 2, A-1, B-1 and C-1 were replaced with equalmolar A-74, B-99 and C-154, respectively, and other steps were the same to obtain Compound 154 (19.31 g) with a solid purity of ≥ 99.92% detected by HPLC. Mass spectrometry (m/z): 622.2459 (theoretical value: 622.2440). Theoretical element content (%) of C₄₃H₃₄N₃OSi: C, 82.92; H, 5.50; N, 4.50. Measured element content (%): C, 82.90; H, 5.48; N, 4.55.

### Synthesis Example 15: Preparation of Compound 159

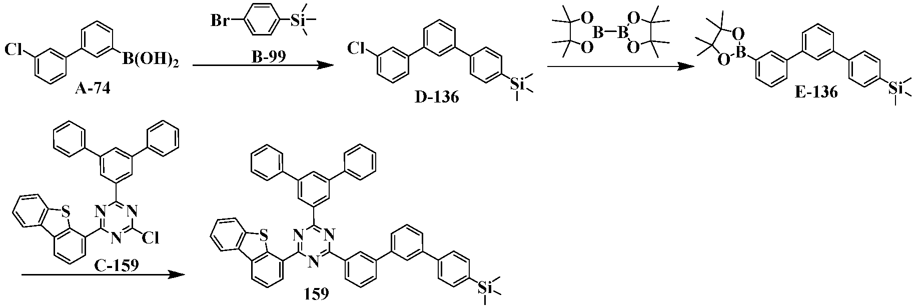

According to the same preparation method as the method for preparing Compound 1 in Synthesis Example 2, A-1, B-1 and C-1 were replaced with equalmolar A-74, B-99 and C-159, respectively, and other steps were the same to obtain Compound 159 (24.15 g) with a solid purity of ≥ 99.87% detected by HPLC. Mass spectrometry (m/z): 791.2775 (theoretical value: 791.2790). Theoretical element content (%) of C₅₄H₄₁N₃SSi: C, 81.88; H, 5.22; N, 5.31. Measured element content (%): C, 81.85; H, 5.20; N, 5.27.

### Synthesis Example 16: Preparation of Compound 160

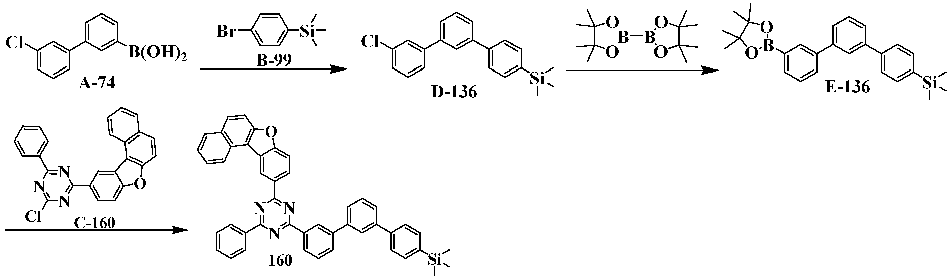

According to the same preparation method as the method for preparing Compound 1 in Synthesis Example 2, A-1, B-1 and C-1 were replaced with equalmolar A-74, B-99 and C-160, respectively, and other steps were the same to obtain Compound 160 (20.21 g) with a solid purity of ≥ 99.85% detected by HPLC. Mass spectrometry (m/z): 673.2568 (theoretical value: 673.2549). Theoretical element content (%) of C₄₆H₃₅N₃OSi: C, 81.99; H, 5.24; N, 6.24. Measured element content (%): C, 82.03; H, 5.20; N, 6.22.

### Synthesis Example 17: Preparation of Compound 173

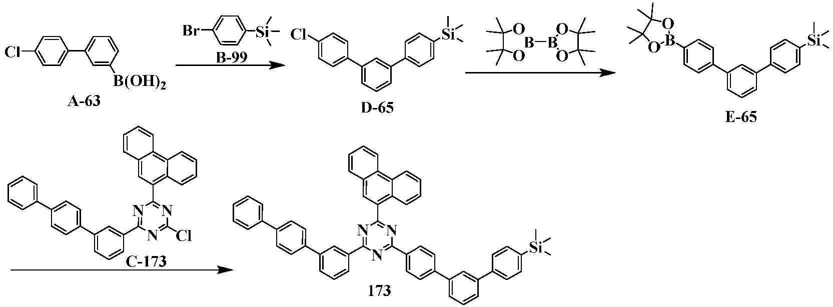

According to the same preparation method as the method for preparing Compound 1 in Synthesis Example 2, A-1, B-1 and C-1 were replaced with equalmolar A-63, B-99 and C-173, respectively, and other steps were the same to obtain Compound 173 (23.58 g) with a solid purity of ≥ 99.92% detected by HPLC. Mass spectrometry (m/z): 785.3238 (theoretical value: 785.3226). Theoretical element content (%) of C₅₆H₄₃N₃Si: C, 85.57; H, 5.51; N, 5.35. Measured element content (%): C, 85.55; H, 5.48; N, 5.39.

### Synthesis Example 18: Preparation of Compound 175

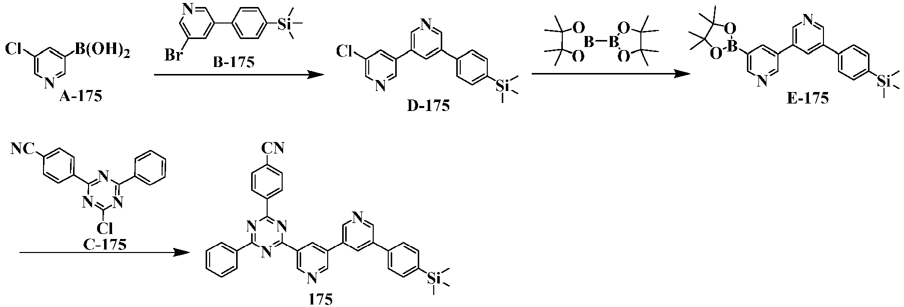

According to the same preparation method as the method for preparing Compound 1 in Synthesis Example 2, A-1, B-1 and C-1 were replaced with equalmolar A-175, B-175 and C-31, respectively, and other steps were the same to obtain Compound 175 (17.07 g) with a solid purity of ≥ 99.90% detected by HPLC. Mass spectrometry (m/z): 560.2122 (theoretical value: 560.2145). Theoretical element content (%) of C₃₅H₂₈N₆Si: C, 74.97; H, 5.03; N, 14.99. Measured element content (%): C, 74.93; H, 5.08; N, 14.95.

### Synthesis Example 19: Preparation of Compound 184

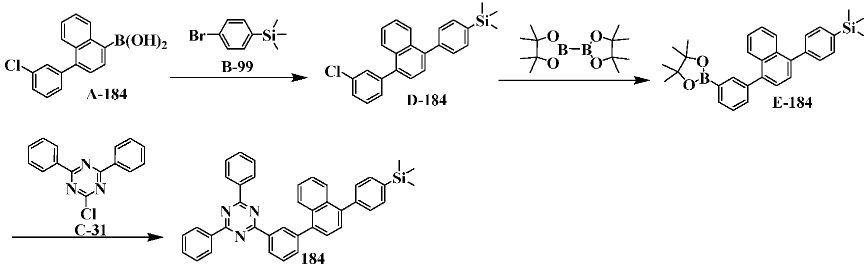

According to the same preparation method as the method for preparing Compound 1 in Synthesis Example 2, A-1, B-1 and C-1 were replaced with equalmolar A-184, B-99 and C-31, respectively, and other steps were the same to obtain Compound 184 (17.51 g) with a solid purity of ≥ 99.93% detected by HPLC. Mass spectrometry (m/z): 583.2460 (theoretical value: 583.2444). Theoretical element content (%) of C₄₀H₃₃N₃Si: C, 82.29; H, 5.70; N, 7.20. Measured element content (%): C, 82.25; H, 5.73; N, 7.17.

### Synthesis Example 20: Preparation of Compound 222

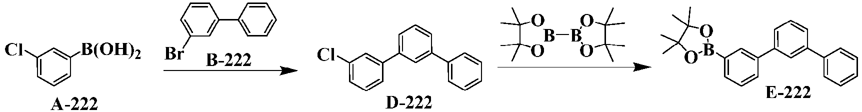

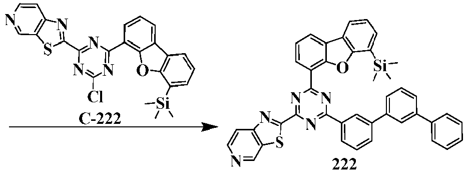

According to the same preparation method as the method for preparing Compound 1 in Synthesis Example 2, A-1, B-1 and C-1 were replaced with equalmolar A-222, B-222 and C-222, respectively, and other steps were the same to obtain Compound 222 (19.43 g) with a solid purity of ≥ 99.82% detected by HPLC. Mass spectrometry (m/z): 681.2035 (theoretical value: 681.2019). Theoretical element content (%) of C₄₂H₃₁N₅OSSi: C, 73.98; H, 4.58; N, 10.27. Measured element content (%): C, 73.95; H, 4.62; N, 10.23.

### Synthesis Example 21: Preparation of Compound 225

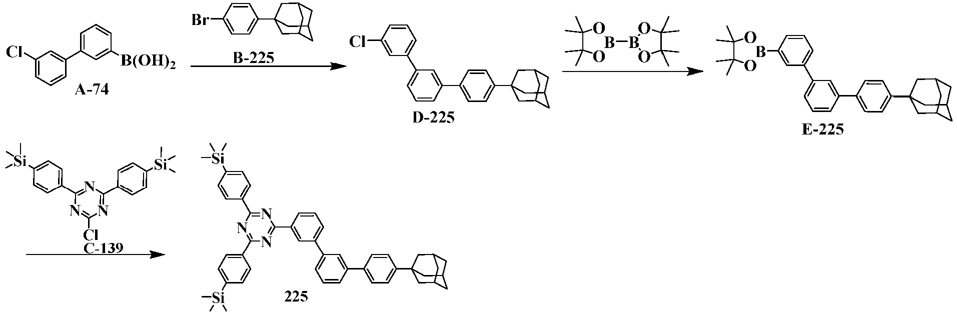

According to the same preparation method as the method for preparing Compound 1 in Synthesis Example 2, A-1, B-1 and C-1 were replaced with equalmolar A-74, B-225 and C-139, respectively, and other steps were the same to obtain Compound 225 (22.57 g) with a solid purity of ≥ 99.94% detected by HPLC. Mass spectrometry (m/z): 739.3759 (theoretical value: 739.3778). Theoretical element content (%) of C₄₉H₅₃N₃Si₂: C, 79.52; H, 7.22; N, 5.68. Measured element content (%): C, 79.47; H, 7.25; N, 5.70.

### Synthesis Example 22: Preparation of Compound 233

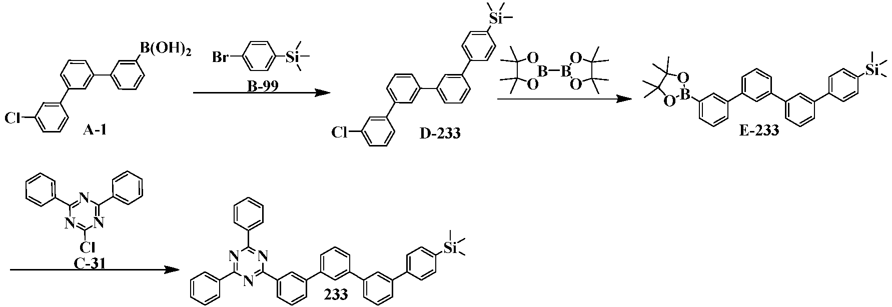

According to the same preparation method as the method for preparing Compound 1 in Synthesis Example 2, B-1 and C-1 were replaced with equalmolar B-99 and C-31, respectively, and other steps were the same to obtain Compound 233 (19.82 g) with a solid purity of ≥ 99.98% detected by HPLC. Mass spectrometry (m/z): 609.2605 (theoretical value: 609.2600). Theoretical element content (%) of C₄₂H₃₅N₃Si: C, 82.72; H, 5.79; N, 6.89. Measured element content (%): C, 82.75; H, 5.76; N, 6.93.

### Synthesis Example 23: Preparation of Compound 240

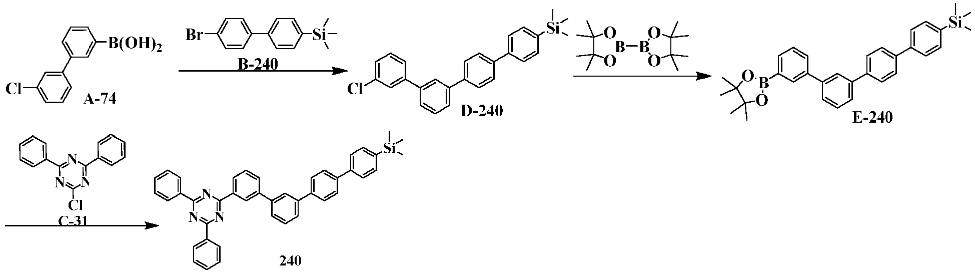

According to the same preparation method as the method for preparing Compound 1 in Synthesis Example 2, A-1, B-1 and C-1 were replaced with equalmolar A-74, B-240 and C-31, respectively, and other steps were the same to obtain Compound 240 (19.82 g) with a solid purity of ≥ 99.96% detected by HPLC. Mass spectrometry (m/z): 609.2590 (theoretical value: 609.2600). Theoretical element content (%) of C₄₂H₃₅N₃Si: C, 82.72; H, 5.79; N, 6.89. Measured element content (%): C, 82.75; H, 5.75; N, 6.93.

### Synthesis Example 24: Preparation of Compound 258

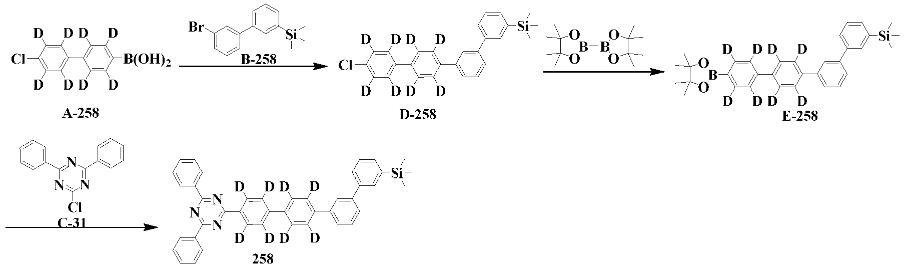

According to the same preparation method as the method for preparing Compound 1 in Synthesis Example 2, A-1, B-1 and C-1 were replaced with equalmolar A-258, B-258 and C-31, respectively, and other steps were the same to obtain Compound 258 (18.53 g) with a solid purity of ≥ 99.94% detected by HPLC. Mass spectrometry (m/z): 617.3117 (theoretical value: 617.3102). Theoretical element content (%) of C₄₂H₂₇D₈N₃Si: C, 81.64; H, 7.01; N, 6.80. Measured element content (%): C, 81.60; H, 7.05; N, 6.82.

### Synthesis Example 25: Preparation of Compound 264

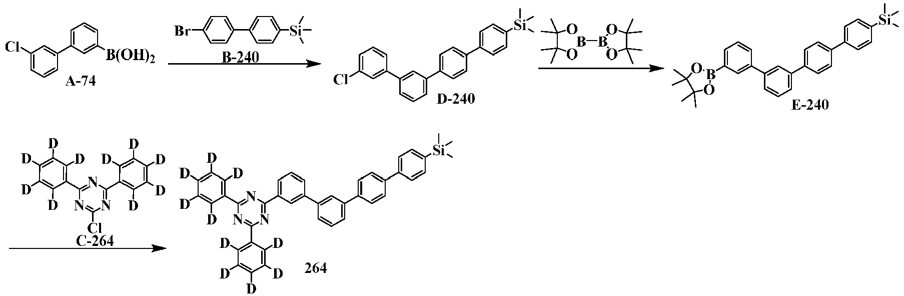

According to the same preparation method as the method for preparing Compound 1 in Synthesis Example 2, A-1, B-1 and C-1 were replaced with equalmolar A-74, B-240 and C-264, respectively, and other steps were the same to obtain Compound 264 (18.60 g) with a solid purity of ≥ 99.89% detected by HPLC. Mass spectrometry (m/z): 619.3239 (theoretical value: 619.3228). Theoretical element content (%) of C₄₂H₂₅D₁₀N₃Si: C, 81.38; H, 7.31; N, 6.78. Measured element content (%): C, 81.35; H, 7.34; N, 6.75.

### Synthesis Example 26: Preparation of Compound 285

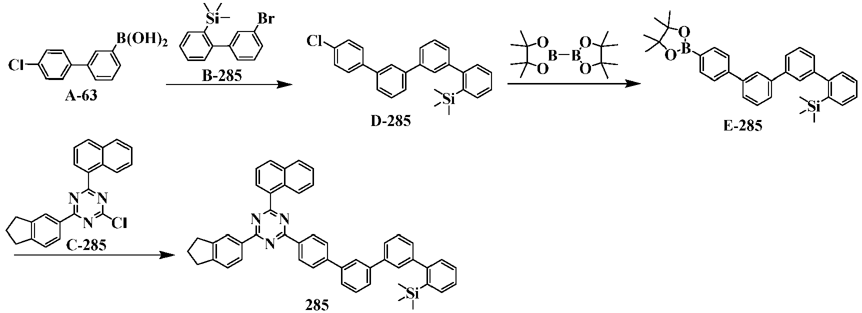

According to the same preparation method as the method for preparing Compound 1 in Synthesis Example 2, A-1, B-1 and C-1 were replaced with equalmolar A-63, B-285 and C-285, respectively, and other steps were the same to obtain Compound 285 (20.64 g) with a solid purity of ≥ 99.86% detected by HPLC. Mass spectrometry (m/z): 699.3051 (theoretical value: 699.3070). Theoretical element content (%) of C₄₉H₄₁N₃Si: C, 84.08; H, 5.90; N, 6.00. Measured element content (%): C, 84.05; H, 5.92; N, 6.04.

### Synthesis Example 27: Preparation of Compound 298

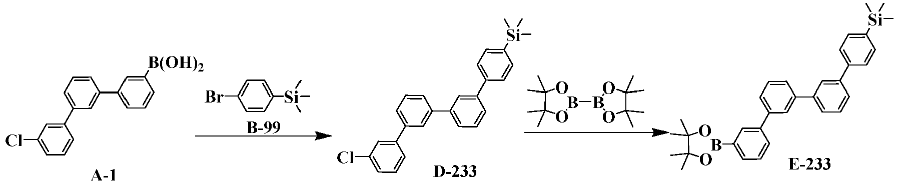

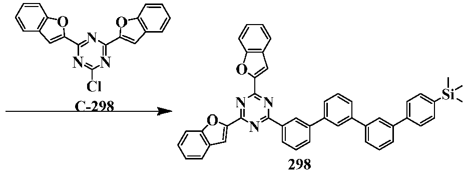

According to the same preparation method as the method for preparing Compound 1 in Synthesis Example 2, B-1 and C-1 were replaced with equalmolar B-99 and C-298, respectively, and other steps were the same to obtain Compound 298 (21.04 g) with a solid purity of ≥ 99.95% detected by HPLC. Mass spectrometry (m/z): 689.2480 (theoretical value: 689.2499). Theoretical element content (%) of C₄₆H₃₅N₃O₂Si: C, 80.09; H, 5.11; N, 6.09. Measured element content (%): C, 80.12; H, 5.06; N, 6.13.

### Synthesis Example 28: Preparation of Compound 308

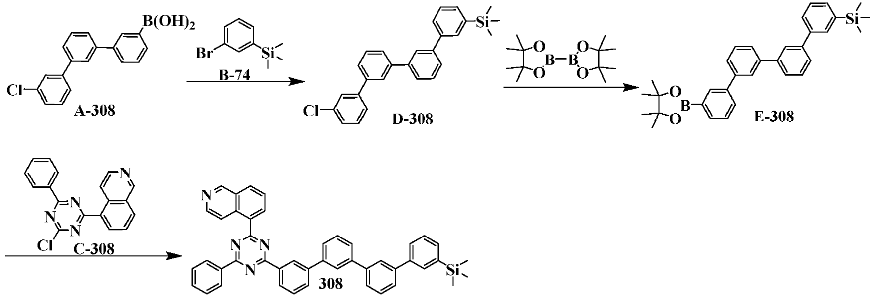

According to the same preparation method as the method for preparing Compound 1 in Synthesis Example 2, A-1, B-1 and C-1 were replaced with equalmolar A-308, B-74 and C-308, respectively, and other steps were the same to obtain Compound 308 (20.48 g) with a solid purity of ≥ 99.92% detected by HPLC. Mass spectrometry (m/z): 660.2716 (theoretical value: 660.2709). Theoretical element content (%) of C₄₅H₃₆N₄Si: C, 81.78; H, 5.49; N, 8.48. Measured element content (%): C, 81.82; H, 5.52; N, 8.50.

### Synthesis Example 29: Preparation of Compound 316

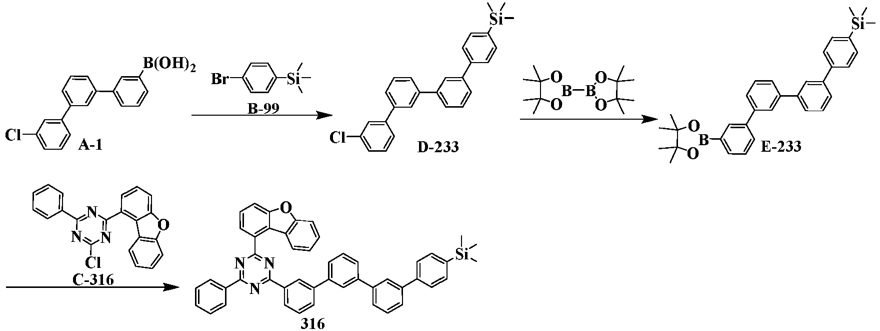

According to the same preparation method as the method for preparing Compound 1 in Synthesis Example 2, B-1 and C-1 were replaced with equalmolar B-99 and C-316, respectively, and other steps were the same to obtain Compound 316 (20.99 g) with a solid purity of ≥ 99.95% detected by HPLC. Mass spectrometry (m/z): 699.2689 (theoretical value: 699.2706). Theoretical element content (%) of C₄₈H₃₇N₃OSi: C, 82.37; H, 5.33; N, 6.00. Measured element content (%): C, 82.40; H, 5.35; N, 6.05.

### Synthesis Example 30: Preparation of Compound 328

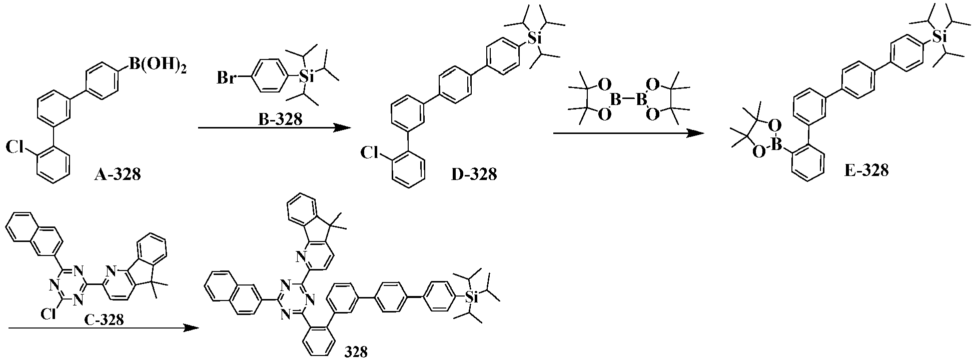

According to the same preparation method as the method for preparing Compound 1 in Synthesis Example 2, A-1, B-1 and C-1 were replaced with equalmolar A-328, B-328 and C-328, respectively, and other steps were the same to obtain Compound 328 (24.11 g) with a solid purity of ≥ 99.80% detected by HPLC. Mass spectrometry (m/z): 860.4255 (theoretical value: 860.4274). Theoretical element content (%) of C₆₀H₅₆N₄Si: C, 83.68; H, 6.55; N, 6.51. Measured element content (%): C, 83.65; H, 6.50; N, 6.47.

### Synthesis Example 31: Preparation of Compound 337

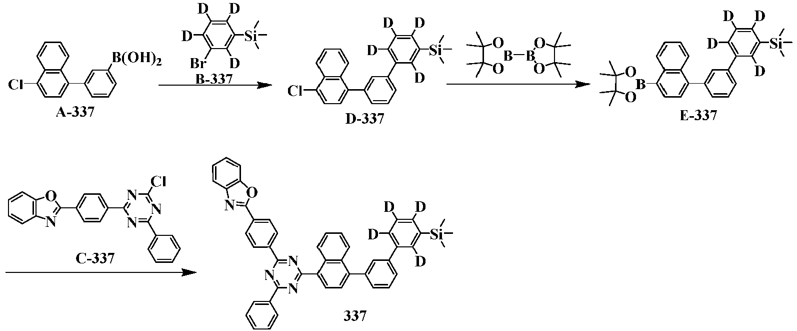

According to the same preparation method as the method for preparing Compound 1 in Synthesis Example 2, A-1, B-1 and C-1 were replaced with equalmolar A-337, B-337 and C-337, respectively, and other steps were the same to obtain Compound 337 (22.20 g) with a solid purity of ≥ 99.83% detected by HPLC. Mass spectrometry (m/z): 704.2914 (theoretical value: 704.2909). Theoretical element content (%) of C₄₇H₃₂D₄N₄OSi: C, 80.08; H, 5.72; N, 7.95. Measured element content (%): C, 80.12; H, 5.75; N, 7.99.

### Synthesis Example 32: Preparation of Compound 340

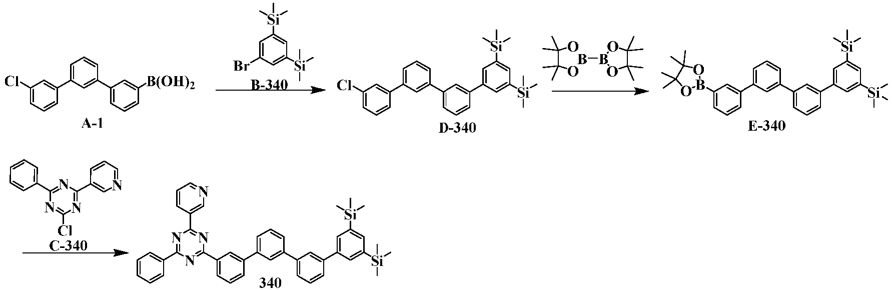

According to the same preparation method as the method for preparing Compound 1 in Synthesis Example 2, B-1 and C-1 were replaced with equalmolar B-340 and C-340, respectively, and other steps were the same to obtain Compound 340 (21.85 g) with a solid purity of ≥ 99.93% detected by HPLC. Mass spectrometry (m/z): 682.2930 (theoretical value: 682.2948). Theoretical element content (%) of C₄₄H₄₂N₄Si₂: C, 77.37; H, 6.20; N, 8.20. Measured element content (%): C, 77.41; H, 6.22; N, 8.16.

### Synthesis Example 33: Preparation of Compound 367

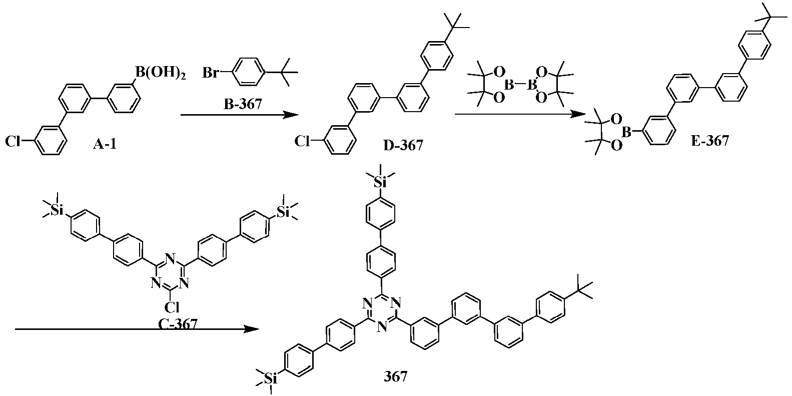

According to the same preparation method as the method for preparing Compound 1 in Synthesis Example 2, B-1 and C-1 were replaced with equalmolar B-367 and C-367, respectively, and other steps were the same to obtain Compound 367 (25.05 g) with a solid purity of ≥ 99.91% detected by HPLC. Mass spectrometry (m/z): 889.4253 (theoretical value: 889.4248). Theoretical element content (%) of C₆₁H₅₉N₃Si₂: C, 82.29; H, 6.68; N, 4.72. Measured element content (%): C, 82.33; H, 6.65; N, 4.75.

### Synthesis Example 34: Preparation of Compound 373

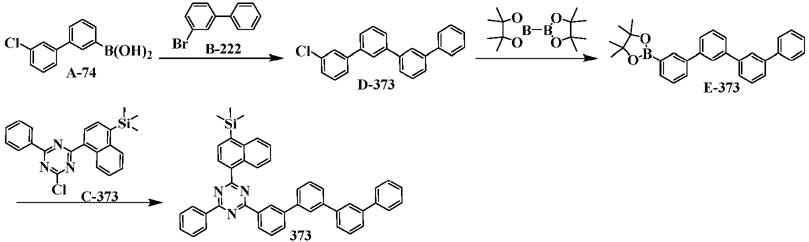

According to the same preparation method as the method for preparing Compound 1 in Synthesis Example 2, A-1, B-1 and C-1 were replaced with equalmolar A-74, B-222 and C-373, respectively, and other steps were the same to obtain Compound 373 (20.19 g) with a solid purity of ≥ 99.93% detected by HPLC. Mass spectrometry (m/z): 659.2740 (theoretical value: 659.2757). Theoretical element content (%) of C₄₆H₃₇N₃Si: C, 83.72; H, 5.65; N, 6.37. Measured element content (%): C, 83.75; H, 5.62; N, 6.34.

### Synthesis Example 35: Preparation of Compound 415

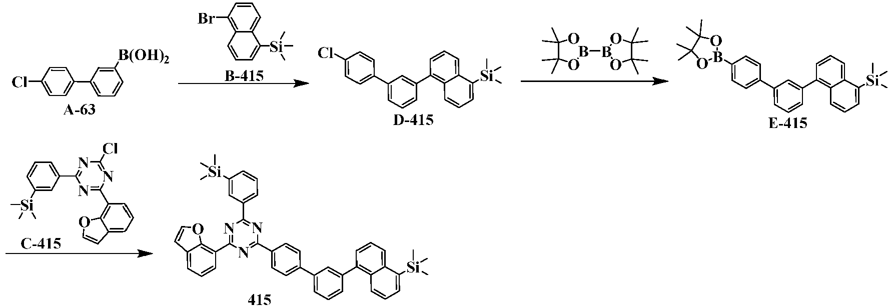

According to the same preparation method as the method for preparing Compound 1 in Synthesis Example 2, A-1, B-1 and C-1 were replaced with equalmolar A-63, B-415 and C-415, respectively, and other steps were the same to obtain Compound 415 (21.64 g) with a solid purity of ≥ 99.89% detected by HPLC. Mass spectrometry (m/z): 695.2780 (theoretical value: 695.2788). Theoretical element content (%) of C₄₅H₄₁N₃OSi₂: C, 77.66; H, 5.94; N, 6.04. Measured element content (%): C, 77.69; H, 5.90; N, 6.07.

### Synthesis Example 36: Preparation of Compound 425

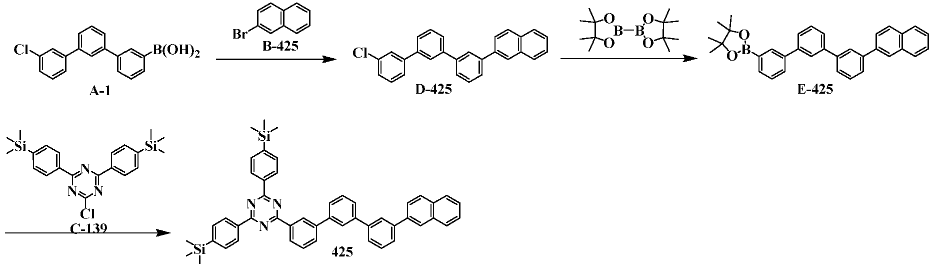

According to the same preparation method as the method for preparing Compound 1 in Synthesis Example 2, B-1 and C-1 were replaced with equalmolar B-425 and C-139, respectively, and other steps were the same to obtain Compound 425 (23.42 g) with a solid purity of ≥ 99.94% detected by HPLC. Mass spectrometry (m/z): 731.3134 (theoretical value: 731.3152). Theoretical element content (%) of C₄₉H₄₅N₃Si₂: C, 80.39; H, 6.20; N, 5.74. Measured element content (%): C, 80.42; H, 6.17; N, 5.78.

### Synthesis Example 37: Preparation of Compound 442

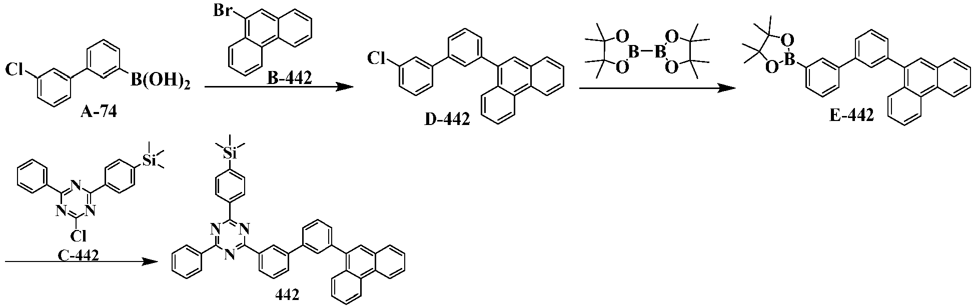

According to the same preparation method as the method for preparing Compound 1 in Synthesis Example 2, A-1, B-1 and C-1 were replaced with equalmolar A-74, B-442 and C-442, respectively, and other steps were the same to obtain Compound 442 (20.61 g) with a solid purity of ≥ 99.96% detected by HPLC. Mass spectrometry (m/z): 633.2612 (theoretical value: 633.2600). Theoretical element content (%) of C₄₄H₃₅N₃Si: C, 83.37; H, 5.57; N, 6.63. Measured element content (%): C, 83.35; H, 5.60; N, 6.64.

### Synthesis Example 38: Preparation of Compound 455

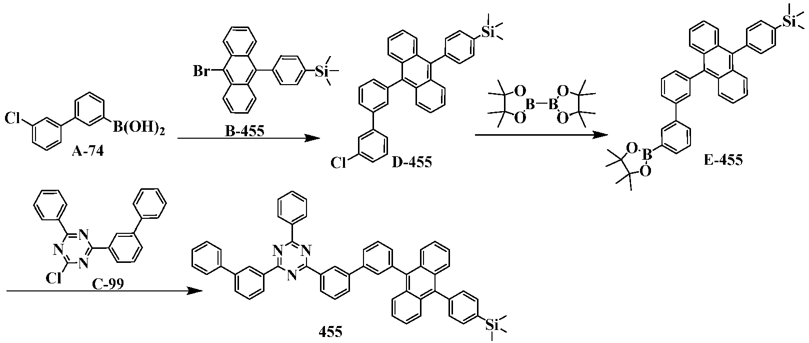

According to the same preparation method as the method for preparing Compound 1 in Synthesis Example 2, A-1, B-1 and C-1 were replaced with equalmolar A-74, B-455 and C-99, respectively, and other steps were the same to obtain Compound 455 (23.58 g) with a solid purity of ≥ 99.83% detected by HPLC. Mass spectrometry (m/z): 785.3208 (theoretical value: 785.3226). Theoretical element content (%) of C₅₆H₄₃N₃Si: C, 85.57; H, 5.51; N, 5.35. Measured element content (%): C, 85.60; H, 5.55; N, 5.32.

### Synthesis Example 39: Preparation of Compound 467

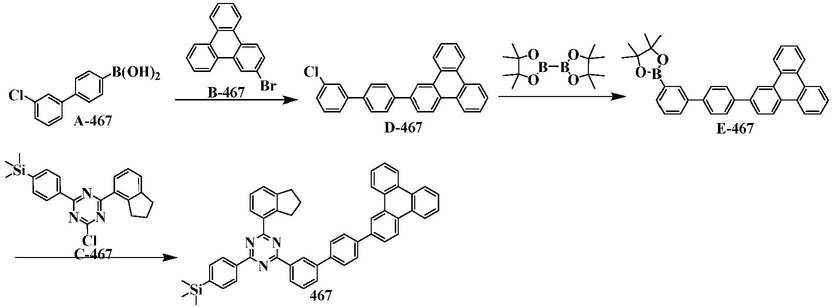

According to the same preparation method as the method for preparing Compound 1 in Synthesis Example 2, A-1, B-1 and C-1 were replaced with equalmolar A-467, B-467 and C-467, respectively, and other steps were the same to obtain Compound 467 (21.71 g) with a solid purity of ≥ 99.86% detected by HPLC. Mass spectrometry (m/z): 723.3085 (theoretical value: 723.3070). Theoretical element content (%) of C₅₁H₄₁N₃Si: C, 84.61; H, 5.71; N, 5.80. Measured element content (%): C, 84.58; H, 5.75; N, 5.76.

### Device Example

The following describes the application effect of the OLED material synthesized in the present disclosure in the organic electroluminescent device in detail by using the device examples and the comparative device examples. It is to be understood that these examples are merely an explanation of the present disclosure, and are not intended to limit the scope of the present disclosure.

Test method: the driving voltage and the luminescence efficiency were tested using a combined IVL test system composed of test software, a computer, a K2400 digital source meter manufactured by Keithley, USA and a PR788 spectral scanning photometer manufactured by Photo Research, USA. The lifetime was tested using an M6000 OLED lifetime test system manufactured by McScience. The test was conducted in an atmospheric environment and at room temperature.

The materials used in preparing the organic electroluminescent devices and comparative devices are as follows:

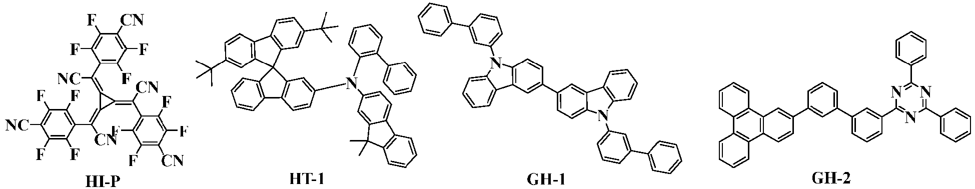

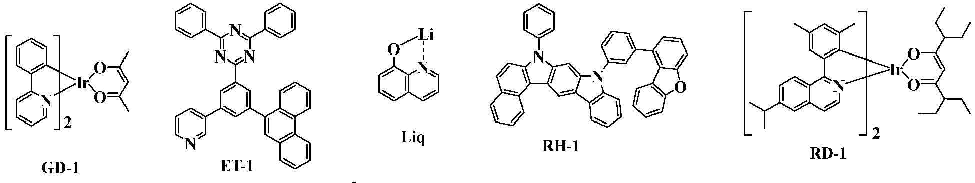

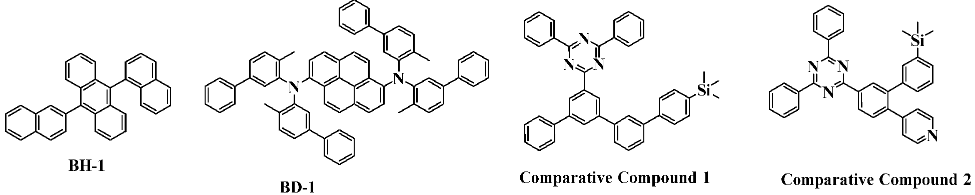

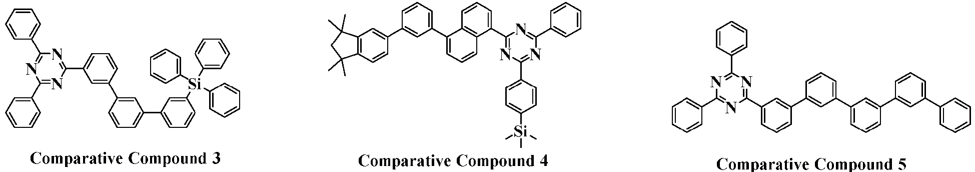

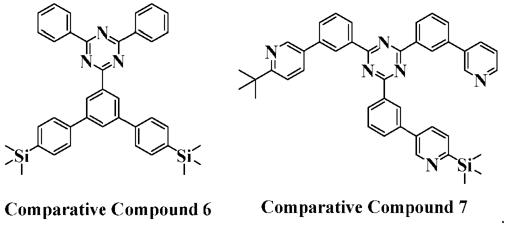

### Device Example 1-1: Preparation of a green light organic electroluminescent device

An ITO transparent glass substrate was ultrasonically cleaned twice with a 5% glass cleaning solution, 20 minutes each time, and then ultrasonically cleaned twice with deionized water, 10 minutes each time. The ITO transparent glass substrate was ultrasonically cleaned for 20 minutes with acetone and isopropyltone in sequence and dried at 120 °C. All organic materials were sublimated, each with a purity above 99.99%. A mixed material (mass ratio of HI-P:HT-1 = 3:97) of HI-P and HT-1 was vacuum evaporated on the ITO transparent glass substrate as a hole injection layer with a thickness of evaporation of 10 nm. HT-1 with a thickness of 120 nm was vacuum evaporated on the hole injection layer as a hole transport layer. A mixed material (mass ratio of GH-2 : GH-1 : GD-1 = 46 : 46 : 8) of GH-2, GH-1 and GD-1 was evaporated on the hole transport layer to form a light-emitting layer with a thickness of evaporation of 40 nm. Then, a mixed material (mass ratio of Compound 1 : Liq = 1:1) of Compound 1 and Liq in the present disclosure was vacuum evaporated on the light-emitting layer as an electron transport layer with a thickness of evaporation of 30 nm. Then, LiF with a thickness of 1 nm was evaporated as an electron injection layer. Al was vacuum evaporated on the electron injection layer as a cathode with a thickness of evaporation of 120 nm.

### Device Examples 1-2 to 1-38 : Preparation of green light organic electroluminescent devices

Compound 1 in Device Example 1-1 was replaced with Compound 17, Compound 31, Compound 63, Compound 74, Compound 76, Compound 99, Compound 126, Compound 133, Compound 136, Compound 139, Compound 143, Compound 154, Compound 159, Compound 160, Compound 173, Compound 175, Compound 184, Compound 222, Compound 225, Compound 233, Compound 240, Compound 258, Compound 264, Compound 285, Compound 298, Compound 308, Compound 316, Compound 328, Compound 337, Compound 340, Compound 367, Compound 373, Compound 415, Compound 425, Compound 442, Compound 455 and Compound 467 in the present disclosure as electron transport layers, and other manufacturing processes were completely the same to prepare the organic electroluminescent devices.

### Comparative Device Examples 1 to 3

Compound 1 in Device Example 1-1 was replaced with Comparative Compound 1, Comparative Compound 2 and Comparative Compound 7 as electron transport layers, and other manufacturing processes were completely the same to prepare the organic electroluminescent devices.

**Table 1 Luminescence characteristic test data of organic electroluminescent devices prepared in Device Examples 1-1 to 1-38 and Comparative Device Examples 1 to 3**

| Example | Material of Electron Transport Layer | Voltage [V] (@10 mA/cm²) | Luminescence Efficiency [cd/A] (@10 mA/cm²) | Lifetime [T95, h] (@10 mA/cm²) |
|---|---|---|---|---|
| Device Example 1-1 | Compound 1:Liq | 4.4 | 57.7 | 263.4 |
| Device Example 1-2 | Compound 17:Liq | 4.5 | 57.9 | 264.8 |
| Device Example 1-3 | Compound 31:Liq | 4.4 | 66.3 | 305.0 |
| Device Example 1-4 | Compound 63:Liq | 4.6 | 66.0 | 303.6 |
| Device Example 1-5 | Compound 74:Liq | 4.3 | 58.6 | 267.5 |
| Device Example 1-6 | Compound 76:Liq | 4.6 | 59.5 | 271.1 |
| Device Example 1-7 | Compound 99:Liq | 4.3 | 59.0 | 268.4 |
| Device Example 1-8 | Compound 126:Liq | 4.4 | 58.8 | 269.5 |
| Device Example 1-9 | Compound 133:Liq | 4.5 | 62.9 | 285.2 |
| Device Example 1-10 | Compound 136:Liq | 4.5 | 61.6 | 277.6 |
| Device Example 1-11 | Compound 139:Liq | 4.4 | 62.7 | 284.6 |
| Device Example 1-12 | Compound 143:Liq | 4.6 | 62.2 | 278.0 |
| Device Example 1-13 | Compound 154:Liq | 4.7 | 59.8 | 273.2 |
| Device Example 1-14 | Compound 159:Liq | 4.6 | 62.5 | 280.2 |
| Device Example 1-15 | Compound 160:Liq | 4.6 | 62.1 | 283.7 |
| Device Example 1-16 | Compound 173:Liq | 4.8 | 61.9 | 281.3 |
| Device Example 1-17 | Compound 175:Liq | 4.5 | 59.3 | 270.6 |
| Device Example 1-18 | Compound 184:Liq | 4.5 | 59.7 | 271.8 |
| Device Example 1-19 | Compound 222:Liq | 4.7 | 61.2 | 275.0 |
| Device Example 1-20 | Compound 225:Liq | 4.6 | 65.6 | 302.7 |
| Device Example 1-21 | Compound 233:Liq | 4.3 | 63.2 | 288.2 |
| Device Example 1-22 | Compound 240:Liq | 4.4 | 63.1 | 287.5 |
| Device Example 1-23 | Compound 258:Liq | 4.3 | 65.9 | 301.5 |
| Device Example 1-24 | Compound 264:Liq | 4.3 | 65.8 | 302.4 |
| Device Example 1-25 | Compound 285:Liq | 4.5 | 64.4 | 292.5 |
| Device Example 1-26 | Compound 298:Liq | 4.5 | 64.5 | 291.5 |
| Device Example 1-27 | Compound 308:Liq | 4.5 | 63.4 | 288.6 |
| Device Example 1-28 | Compound 316:Liq | 4.5 | 65.2 | 298.4 |
| Device Example 1-29 | Compound 328:Liq | 4.7 | 64.0 | 290.9 |
| Device Example 1-30 | Compound 337:Liq | 4.5 | 58.2 | 265.0 |
| Device Example 1-31 | Compound 340:Liq | 4.7 | 62.4 | 275.7 |
| Device Example 1-32 | Compound 367:Liq | 4.4 | 65.3 | 299.7 |
| Device Example 1-33 | Compound 373:Liq | 4.5 | 63.5 | 289.4 |
| Device Example 1-34 | Compound 415:Liq | 4.5 | 58.2 | 265.0 |
| Device Example 1-35 | Compound 425:Liq | 4.7 | 60.2 | 278.3 |
| Device Example 1-36 | Compound 442:Liq | 4.6 | 59.5 | 272.5 |
| Device Example 1-37 | Compound 455:Liq | 4.7 | 61.4 | 281.8 |
| Device Example 1-38 | Compound 467:Liq | 4.5 | 59.6 | 274.6 |
| Comparative Example 1 | Comparative Compound 1:Liq | 5.1 | 45.6 | 210.7 |
| Comparative Example 2 | Comparative Compound 2:Liq | 5.3 | 42.5 | 202..2 |
| Comparative Example 3 | Comparative Compound 4:Liq | 5.0 | 47.1 | 219.5 |

According to the results in Table 1, the heterocyclic compound provided in the present disclosure has a relatively good electron mobility, which can effectively balance transport of holes and electrons and improve the luminescence efficiency, and the heterocyclic compound provided in the present disclosure has a proper energy level, which can reduce injection and transport barriers of the electrons, reduce the drive voltage, avoid the increase of the power consumption of the device due to an excessively high local voltage and prolong the service life of the device.

### Device Example 2-1: Preparation of a green light organic electroluminescent device

A mixed material (mass ratio of HI-P:HT-1 = 3:97) of HI-P and HT-1 was vacuum evaporated on the cleaned ITO transparent glass substrate as a hole injection layer with a thickness of evaporation of 10 nm. HT-1 with a thickness of 120 nm was vacuum evaporated on the hole injection layer as a hole transport layer. A mixed material (mass ratio of GH-2:GH-1:GD-1 = 46:46:8) of GH-2, GH-1 and GD-1 was evaporated on the hole transport layer to form a light-emitting layer with a thickness of evaporation of 40 nm. Then, Compound 1 in the present disclosure was vacuum evaporated on the light-emitting layer as a hole blocking layer with a thickness of evaporation of 10 nm. Then, a mixed material (mass ratio of ET-1 : Liq = 1:1) of ET-1 and Liq was vacuum evaporated on the hole blocking layer as an electron transport layer with a thickness of evaporation of 30 nm. Then, LiF with a thickness of 1 nm was evaporated as an electron injection layer. Al was vacuum evaporated on the electron injection layer as a cathode with a thickness of evaporation of 120 nm.

### Device Examples 2-2 to 2-20 : Preparation of green light organic electroluminescent devices

Compound 1 in Device Example 2-1 was replaced with Compound 31, Compound 74, Compound 99, Compound 139, Compound 159, Compound 184, Compound 225, Compound 233, Compound 258, Compound 285, Compound 298, Compound 308, Compound 316, Compound 337, Compound 340, Compound 373, Compound 425, Compound 442 and Compound 455 in the present disclosure as hole blocking layers, and other manufacturing processes were completely the same to prepare the organic electroluminescent devices.

### Comparative Device Examples 4 to 6

Compound 1 in Device Example 2-1 was replaced with Comparative Compound 2 and Comparative Compound 3 as hole blocking layers, and other manufacturing processes were completely the same to prepare the organic electroluminescent devices.

**Table 2 Luminescence characteristic test data of organic electroluminescent devices prepared in Device Examples 2-1 to 2-20 and Comparative Device Examples 4 and 5**

| Example | Material of Hole Blocking Layer | Voltage [V] (@ 10 mA/cm²) | Luminescence Efficiency [cd/A] (@10 mA/cm²) | Lifetime [T95, h] (@10 mA/cm²) |
|---|---|---|---|---|
| Device Example 2-1 | Compound 1 | 4.2 | 60.5 | 281.5 |
| Device Example 2-2 | Compound 31 | 4.1 | 72.8 | 314.5 |
| Device Example 2-3 | Compound 74 | 4.0 | 61.5 | 284.6 |
| Device Example 2-4 | Compound 99 | 4.0 | 62.2 | 285.3 |
| Device Example 2-5 | Compound 139 | 4.1 | 66.0 | 297.5 |
| Device Example 2-6 | Compound 159 | 4.3 | 65.8 | 294.2 |
| Device Example 2-7 | Compound 184 | 4.1 | 63.3 | 288.8 |
| Device Example 2-8 | Compound 225 | 4.3 | 72.0 | 313.7 |
| Device Example 2-9 | Compound 233 | 4.0 | 66.4 | 298.4 |
| Device Example 2-10 | Compound 258 | 4.1 | 72.4 | 312.2 |
| Device Example 2-11 | Compound 285 | 4.2 | 69.8 | 305.1 |
| Device Example 2-12 | Compound 298 | 4.2 | 70.3 | 302.6 |
| Device Example 2-13 | Compound 308 | 4.1 | 67.0 | 298.7 |
| Device Example 2-14 | Compound 316 | 4.2 | 71.4 | 308.5 |
| Device Example 2-15 | Compound 337 | 4.4 | 68.5 | 305.7 |
| Device Example 2-16 | Compound 340 | 4.1 | 70.5 | 306.4 |
| Device Example 2-17 | Compound 373 | 4.1 | 67.2 | 299.7 |
| Device Example 2-18 | Compound 425 | 4.3 | 68.0 | 301.4 |
| Device Example 2-19 | Compound 442 | 4.3 | 69.1 | 303.5 |
| Device Example 2-20 | Compound 455 | 4.2 | 67.8 | 300.3 |
| Comparative Example 4 | Comparative Compound 2 | 4.8 | 45.8 | 216.9 |
| Comparative Example 5 | Comparative Compound 3 | 4.9 | 46.4 | 220.7 |

According to the results in Table 2, the heterocyclic compound provided in the present disclosure has a proper energy level, which can effectively confine the holes in the light-emitting layer, avoid the escape of the holes to one side of the electron transport layer, reduce a probability of recombination at an interface of the light-emitting layer, improve an effective recombination rate of excitons, improve the luminescence efficiency, avoid a leakage current phenomenon and prolong the lifetime of the device.

### Device Example 3-1: Preparation of a red light organic electroluminescent device

A mixed material (mass ratio of HI-P : HT-1 = 3:97) of HI-P and HT-1 was vacuum evaporated on the cleaned ITO transparent glass substrate as a hole injection layer with a thickness of evaporation of 10 nm. HT-1 with a thickness of 120 nm was vacuum evaporated on the hole injection layer as a hole transport layer. A mixed material (mass ratio of Compound 1:RH-1:RD-1 in the present disclosure = 49:49:2) of Compound 1, RH-1 and RD-1 in the present disclosure was evaporated on the hole transport layer to form a light-emitting layer with a thickness of evaporation of 30 nm. Then, a mixed material (mass ratio of ET-1 : Liq = 1:1) of ET-1 and Liq was vacuum evaporated on the light-emitting layer as an electron transport layer with a thickness of evaporation of 30 nm. Then, LiF with a thickness of 1 nm was evaporated as an electron injection layer. Al was vacuum evaporated on the electron injection layer as a cathode with a thickness of evaporation of 120 nm.

### Device Examples 3-2 to 3-30 : Preparation of red light organic electroluminescent devices

Compound 1 in Device Example 3-1 was replaced with Compound 17, Compound 31, Compound 63, Compound 74, Compound 99, Compound 126, Compound 133, Compound 136, Compound 139, Compound 143, Compound 154, Compound 159, Compound 173, Compound 175, Compound 184, Compound 225, Compound 233, Compound 240, Compound 258, Compound 264, Compound 298, Compound 308, Compound 316, Compound 337, Compound 340, Compound 367, Compound 373, Compound 425 and Compound 442 in the present disclosure as red light host materials, and other manufacturing processes were completely the same to prepare the organic electroluminescent devices.

### Comparative Device Examples 6 to 8

Compound 1 in Device Example 3-1 was replaced with Comparative Compound 1, Comparative Compound 5 and Comparative Compound 7 as red light host materials, and other manufacturing processes were completely the same to prepare the organic electroluminescent devices.

**Table 3 Luminescence characteristic test data of organic electroluminescent devices prepared in Device Examples 3-1 to 3-30 and Comparative Device Examples 6 to 8**

| Example | Host Material of Light-emitting Layer | Voltage [V] (@10 mA/cm²) | Luminescence Efficiency [cd/A] (@10 mA/cm²) | Lifetime [T95, h] (@10 mA/cm²) |
|---|---|---|---|---|
| Device Example 3-1 | Compound 1:RH-1 | 4.3 | 29.3 | 260.3 |
| Device Example 3-2 | Compound 17:RH-1 | 4.5 | 29.7 | 261.4 |
| Device Example 3-3 | Compound 31:RH-1 | 4.4 | 35.7 | 282.2 |
| Device Example 3-4 | Compound 63:RH-1 | 4.4 | 32.3 | 268.9 |
| Device Example 3-5 | Compound 74:RH-1 | 4.3 | 30.2 | 263.0 |
| Device Example 3-6 | Compound 99:RH-1 | 4.4 | 30.6 | 263.4 |
| Device Example 3-7 | Compound 126:RH-1 | 4.5 | 30.8 | 264.5 |
| Device Example 3-8 | Compound 133:RH-1 | 4.6 | 32.8 | 271.4 |
| Device Example 3-9 | Compound 136:RH-1 | 4.5 | 31.6 | 266.9 |
| Device Example 3-10 | Compound 139:RH-1 | 4.4 | 31.1 | 264.9 |
| Device Example 3-11 | Compound 143:RH-1 | 4.4 | 33.3 | 272.5 |
| Device Example 3-12 | Compound 154:RH-1 | 4.4 | 32.0 | 268.3 |
| Device Example 3-13 | Compound 159:RH-1 | 4.3 | 33.9 | 274.3 |
| Device Example 3-14 | Compound 173:RH-1 | 4.5 | 32.6 | 270.2 |
| Device Example 3-15 | Compound 175:RH-1 | 4.4 | 31.4 | 265.6 |
| Device Example 3-16 | Compound 184:RH-1 | 4.3 | 30.3 | 262.9 |
| Device Example 3-17 | Compound 225:RH-1 | 4.4 | 34.9 | 278.6 |
| Device Example 3-18 | Compound 233:RH-1 | 4.3 | 33.4 | 273.4 |
| Device Example 3-19 | Compound 240:RH-1 | 4.3 | 33.9 | 275.0 |
| Device Example 3-20 | Compound 258:RH-1 | 4.6 | 34.9 | 277.0 |
| Device Example 3-21 | Compound 264:RH-1 | 4.2 | 35.6 | 281.8 |
| Device Example 3-22 | Compound 298:RH-1 | 4.2 | 35.2 | 280.6 |
| Device Example 3-23 | Compound 308:RH-1 | 4.3 | 33.6 | 273.7 |
| Device Example 3-24 | Compound 316:RH-1 | 4.2 | 35.0 | 279.4 |
| Device Example 3-25 | Compound 337:RH-1 | 4.3 | 29.9 | 261.8 |
| Device Example 3-26 | Compound 340:RH-1 | 4.4 | 34.3 | 276.8 |
| Device Example 3-27 | Compound 367:RH-1 | 4.3 | 34.0 | 275.1 |
| Device Example 3-28 | Compound 373:RH-1 | 4.3 | 31.6 | 267.0 |
| Device Example 3-29 | Compound 425:RH-1 | 4.5 | 32.7 | 270.6 |
| Device Example 3-30 | Compound 442:RH-1 | 4.5 | 33.1 | 274.5 |
| Comparative Example 6 | Comparative Compound 1:RH-1 | 4.9 | 24.8 | 207.5 |
| Comparative Example 7 | Comparative Compound 5:RH-1 | 4.7 | 23.9 | 195.2 |
| Comparative Example 8 | Comparative Compound 7:RH-1 | 4.7 | 25.2 | 210.1 |

According to the results in Table 3, the heterocyclic compound provided in the present disclosure has a proper energy level, which can match with an energy level of an adjacent functional layer, balance transport of electrons and holes and reduce injection barriers of the holes and the electrons so that the holes and the electrons are effectively recombined in the light-emitting layer, thereby reducing the drive voltage, improving the luminescence efficiency and prolonging the lifetime of the device.

### Device Example 4-1: Preparation of a blue light organic electroluminescent device

A mixed material (mass ratio of HI-P:HT-1 = 3:97) of HI-P and HT-1 was vacuum evaporated on the cleaned and dried ITO/Ag/ITO glass substrate as a hole injection layer with a thickness of evaporation of 10 nm. HT-1 with a thickness of 120 nm was vacuum evaporated on the hole injection layer as a hole transport layer. A mixed material (mass ratio of BH-1: BD-1 = 98:2) of BH-1 and BD-1 was evaporated on the hole transport layer to form a light-emitting layer with a thickness of evaporation of 20 nm. Then, a mixed material (mass ratio of ET-1: Liq = 1:1) of ET-1 and Liq was vacuum evaporated on the light-emitting layer as an electron transport layer with a thickness of evaporation of 30 nm. Then, Yb with a thickness of 1 nm was evaporated as an electron injection layer. A Mg : Ag alloy (mass ratio of Mg : Ag = 1 : 9) was vacuum evaporated on the electron injection layer as a cathode with a thickness of evaporation of 13 nm. Then, Compound 31 in the present disclosure was vacuum evaporated on the cathode as a light extraction layer with a thickness of evaporation of 75 nm.

### Device Examples 4-2 to 4-10 : Preparation of blue light organic electroluminescent devices

Compound 31 in Device Example 4-1 was replaced with Compound 74, Compound 139, Compound 160, Compound 233, Compound 264, Compound 298, Compound 316, Compound 337 and Compound 415 in the present disclosure as materials of light extraction layers, and other manufacturing processes were completely the same to prepare the organic electroluminescent devices.

### Comparative Device Example 9

Compound 31 in Device Example 4-1 was replaced with Comparative Compound 6 as a material of a light extraction layer, and other manufacturing processes were completely the same to prepare the organic electroluminescent device.

**Table 4 Luminescence characteristic test data of organic electroluminescent devices prepared in Device Examples 4-1 to 4-10 and Comparative Device Example 9**

| Example | Material of Light Extraction Layer | Luminescence Efficiency [cd/A] (@10 mA/cm²) | Lifetime [T95, h] (@10 mA/cm²) |
|---|---|---|---|
| Example 4-1 | Compound 31 | 9.0 | 134.6 |
| Example 4-2 | Compound 74 | 9.2 | 135.8 |
| Example 4-3 | Compound 139 | 9.4 | 138.0 |
| Example 4-4 | Compound 160 | 9.6 | 140.3 |
| Example 4-5 | Compound 233 | 8.9 | 133.4 |
| Example 4-6 | Compound 264 | 9.3 | 136.9 |
| Example 4-7 | Compound 298 | 9.6 | 139.5 |
| Example 4-8 | Compound 316 | 9.5 | 138.8 |
| Example 4-9 | Compound 337 | 8.6 | 130.5 |
| Example 4-10 | Compound 415 | 8.7 | 131.2 |
| Comparative Example 9 | Comparative Compound 6 | 7.0 | 105.6 |

According to the results in Table 4, when the heterocyclic compound provided in the present disclosure is applied to the light extraction layer, the luminescence efficiency of the organic electroluminescent device can be improved, and the lifetime of the device can be prolonged.

## Claims

1. A heterocyclic compound, wherein the heterocyclic compound has a structure represented by Formula I: 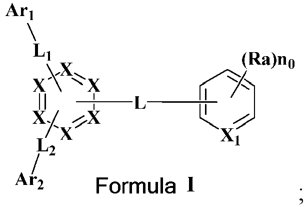
wherein in Formula I, X is independently selected from C(R₂) or an N atom, at least one X is selected from an N atom, and X bonded to Ar₁, Ar₂ or L is selected from a C atom;
X₁ is independently selected from a C atom or an N atom;
R₂ is independently selected from any one of hydrogen, deuterium, tritium, halogen, cyano, nitro, substituted or unsubstituted C1 to C12 alkyl, substituted or unsubstituted silyl, substituted or unsubstituted C1 to C12 alkoxy, substituted or unsubstituted C3 to C12 cycloalkyl, substituted or unsubstituted C2 to C12 heterocycloalkyl, substituted or unsubstituted C6 to C30 aryl or substituted or unsubstituted C2 to C30 heteroaryl;
Ar₁ and Ar₂ are independently selected from any one of substituted or unsubstituted C6 to C30 aryl or substituted or unsubstituted C2 to C30 heteroaryl;
L₁ and L₂ are independently selected from any one of a single bond, substituted or unsubstituted C6 to C30 arylene or substituted or unsubstituted C2 to C30 heteroarylene;
Ra is independently selected from any one of hydrogen, deuterium, halogen, cyano, nitro, substituted or unsubstituted C1 to C12 alkyl, substituted or unsubstituted silyl, substituted or unsubstituted C1 to C12 alkoxy, substituted or unsubstituted C3 to C12 cycloalkyl or substituted or unsubstituted C2 to C12 heterocycloalkyl;
n₀ is independently selected from 0, 1, 2, 3, 4 or 5, when n₀ is greater than 1, two or more Ra are the same as or different from each other, or two adjacent Ra are joined to form a substituted or unsubstituted benzene ring;
L is independently selected from any one of the following groups: 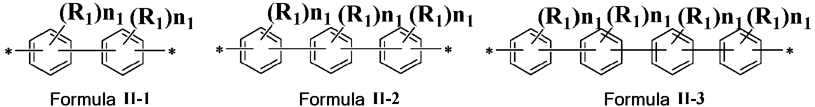 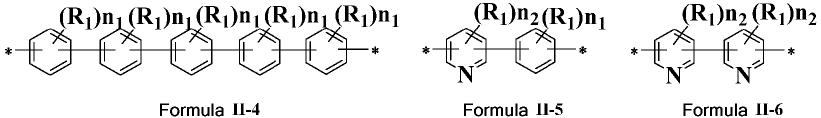 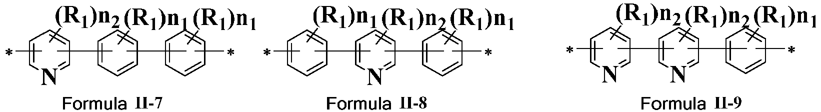 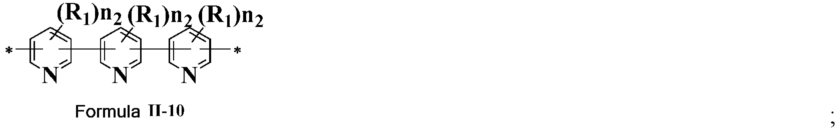
wherein R₁ is independently selected from any one of hydrogen, deuterium, halogen, cyano, nitro, substituted or unsubstituted C1 to C12 alkyl, substituted or unsubstituted silyl, substituted or unsubstituted C1 to C12 alkoxy, substituted or unsubstituted C3 to C12 cycloalkyl or substituted or unsubstituted C2 to C12 heterocycloalkyl;
n₁ is independently selected from 0, 1, 2, 3 or 4; n₂ is independently selected from 0, 1, 2 or 3; when n₁ is greater than 1, two or more R₁ are the same as or different from each other, or two adjacent R₁ are joined to form a substituted or unsubstituted benzene ring;
with the proviso that one or more hydrogens in at least one group of Ar₁, Ar₂, L, L₁, L₂, R₂ or Ra are substituted with a group represented by Formula III: 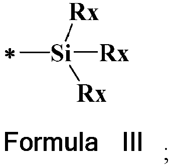 Rx is independently selected from any one of hydrogen, deuterium, cyano, nitro, halogen, substituted or unsubstituted C1 to C12 alkyl, substituted or unsubstituted C2 to C12 alkenyl, substituted or unsubstituted C3 to C12 cycloalkyl or substituted or unsubstituted C2 to C12 heterocycloalkyl; and
the following compound is excluded from the heterocyclic compound:

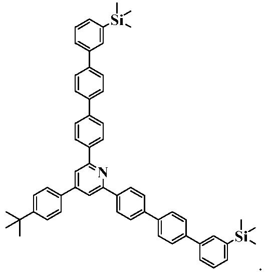

2. The heterocyclic compound according to claim 1, wherein the heterocyclic compound in Formula I is selected from any one of the structures represented by the following Formula III-1 to Formula III-5: 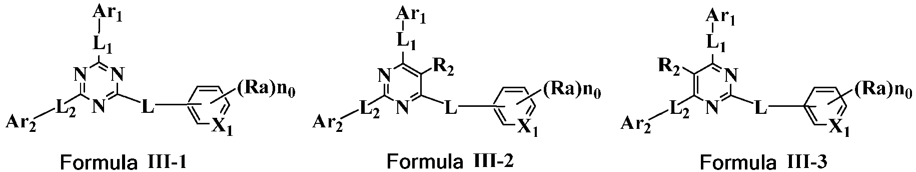 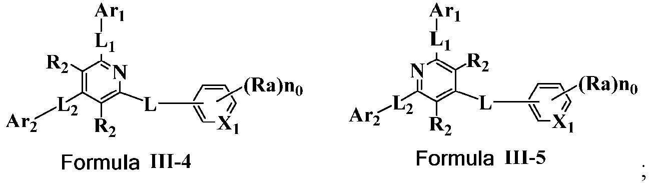 wherein definitions of Ar₁, Ar₂, R₂, L, L₁, L₂, X₁, Ra and n₀ are the same as that in Formula I.

3. The heterocyclic compound according to claim 1, wherein Ar₁ and Ar₂ are independently selected from any one of the following groups: 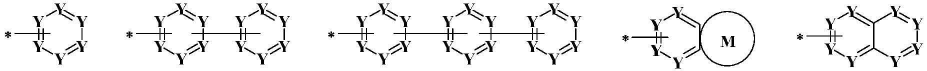 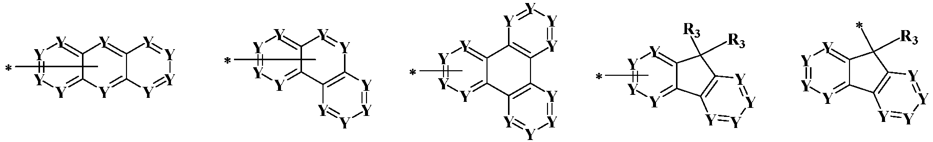 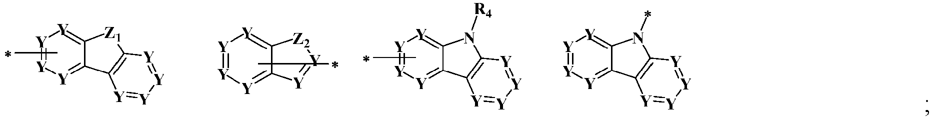
wherein Y is independently selected from C(R₅) or an N atom, and Y at a linkage site is selected from a C atom;
the ring M is selected from an unsubstituted C3 to C7 aliphatic ring or a C3 to C7 aliphatic ring substituted with one or more R₆;
Z₁ is selected from an O atom or an S atom, and Z₂ is selected from O, S, C(R₇)₂ or N(R₈);
R₃ and R₇ are independently selected from any one of hydrogen, deuterium, substituted or unsubstituted C1 to C12 alkyl, substituted or unsubstituted C1 to C12 alkoxy, substituted or unsubstituted C3 to C12 cycloalkyl, substituted or unsubstituted silyl, substituted or unsubstituted C2 to C12 heterocycloalkyl, substituted or unsubstituted C6 to C30 aryl or
substituted or unsubstituted C2 to C30 heteroaryl, or two adjacent R₃ are joined to form a substituted or unsubstituted spirocyclic structure;
R₄ and R₈ are independently selected from any one of substituted or unsubstituted C1 to C12 alkyl, substituted or unsubstituted C3 to C12 cycloalkyl, substituted or unsubstituted silyl, substituted or unsubstituted C2 to C12 heterocycloalkyl, substituted or unsubstituted C6 to C30 aryl or substituted or unsubstituted C2 to C30 heteroaryl; and
R₅ and R₆ are independently selected from any one of hydrogen, deuterium, tritium, cyano, nitro, halogen, substituted or unsubstituted C1 to C12 alkyl, substituted or unsubstituted C1 to C12 alkoxy, substituted or unsubstituted silyl, substituted or unsubstituted C3 to C12 cycloalkyl, substituted or unsubstituted C2 to C12 heterocycloalkyl, substituted or unsubstituted C6 to C30 aryl or substituted or unsubstituted C2 to C30 heteroaryl, or two adjacent R₅ are joined to form a substituted or unsubstituted ring.

4. The heterocyclic compound according to claim 1, wherein Ar₁ and Ar₂ are independently selected from any one of the following groups: 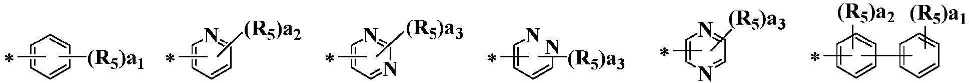 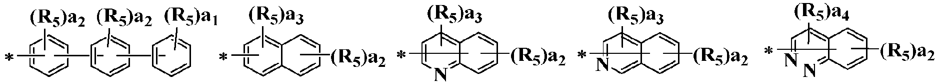 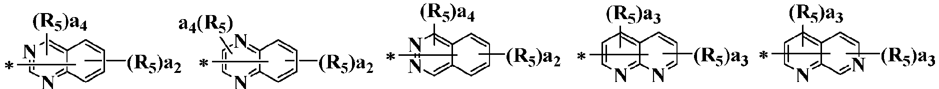 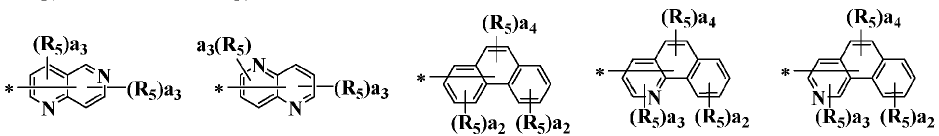 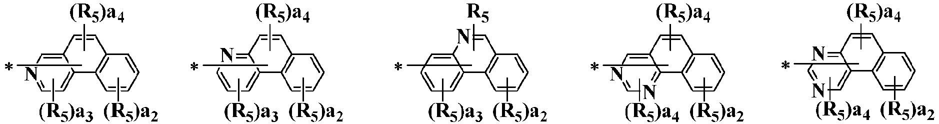 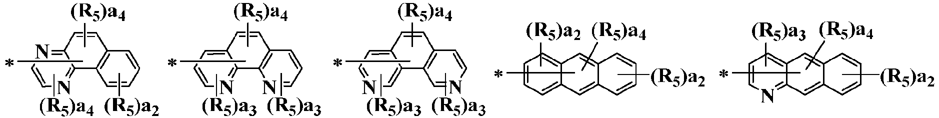 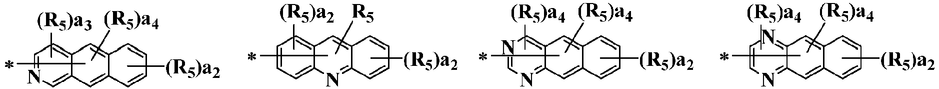 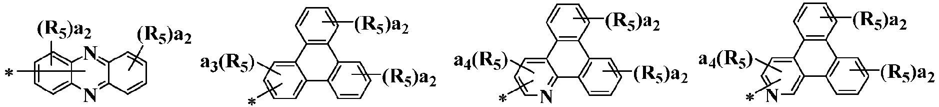 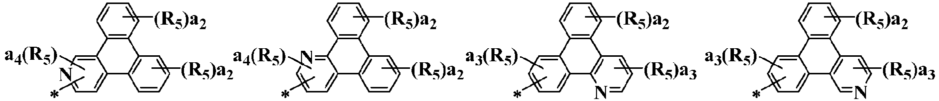 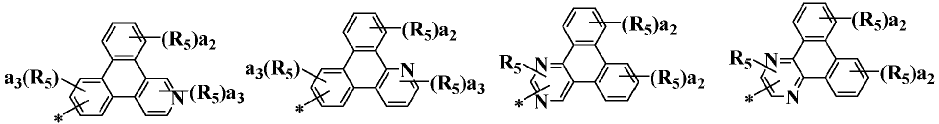 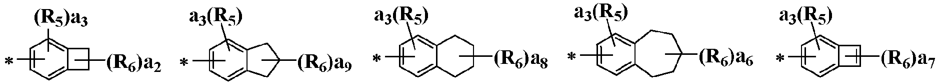 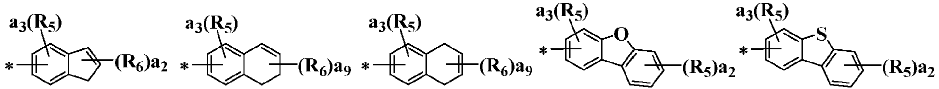 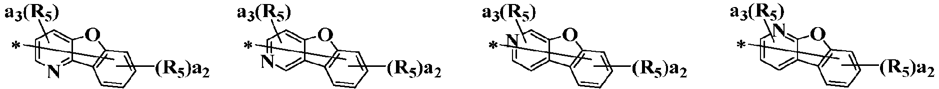 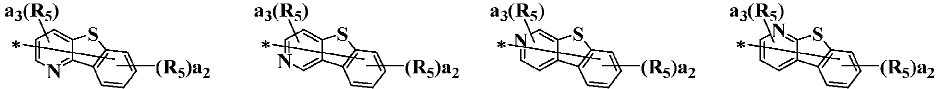 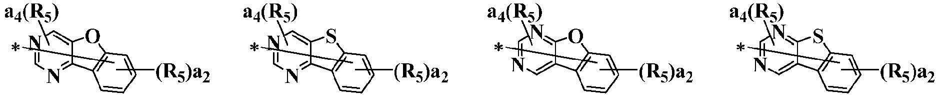 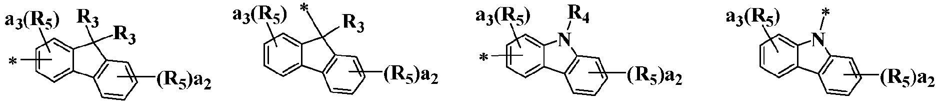 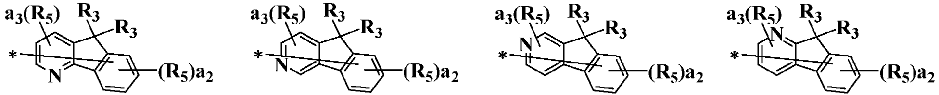 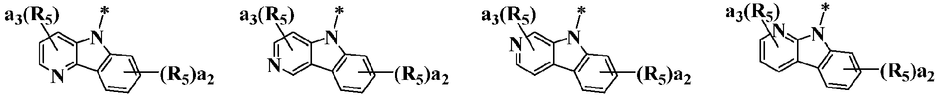 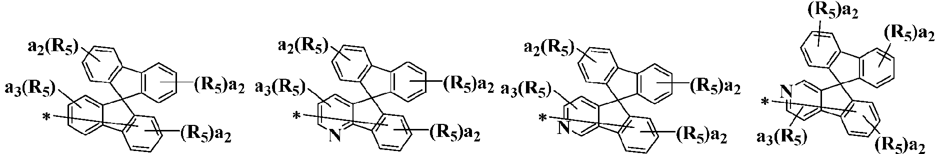 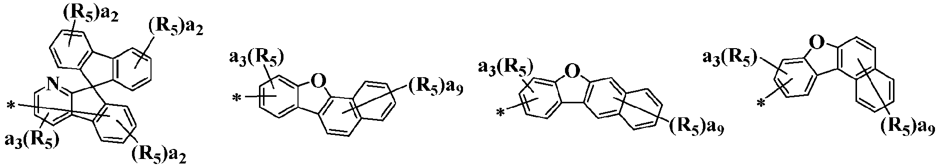 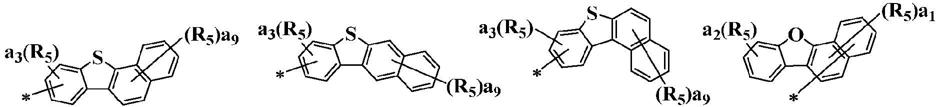 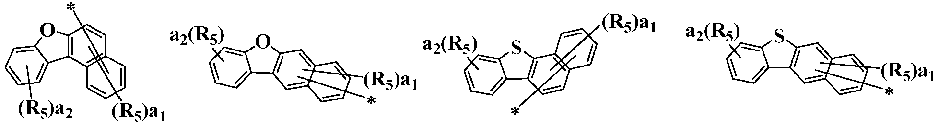 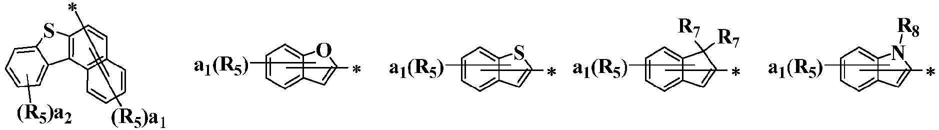 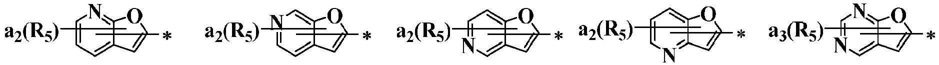 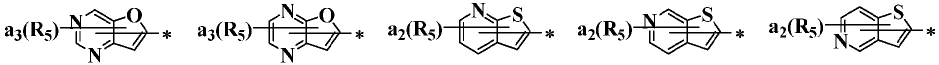 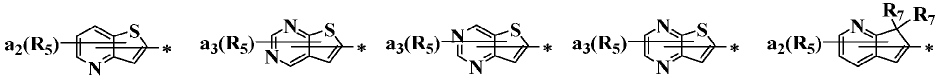 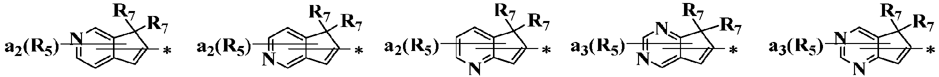 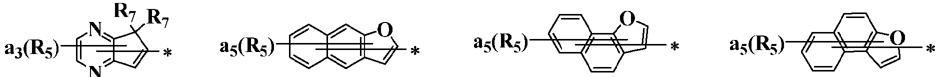 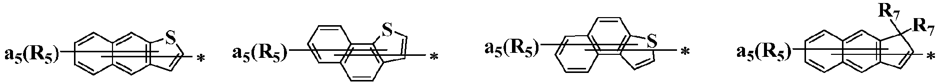 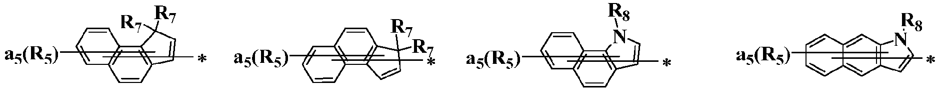 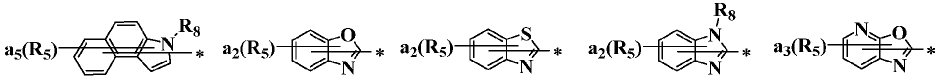 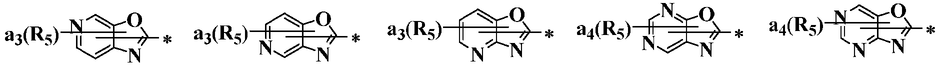 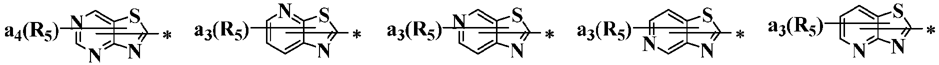 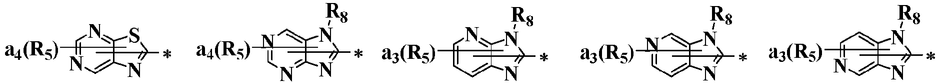 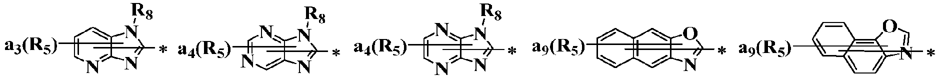 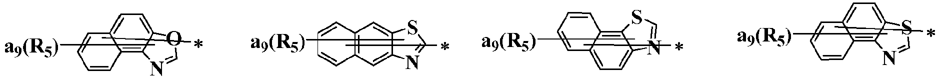 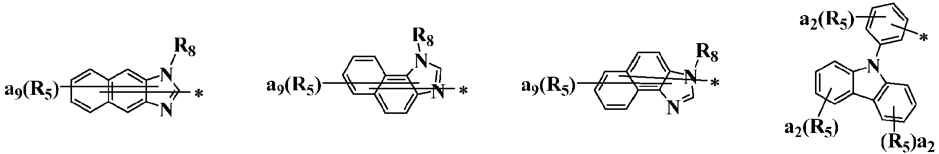 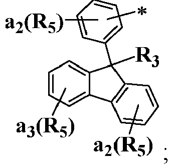
wherein R₃ and R₇ are independently selected from any one of hydrogen, deuterium, substituted or unsubstituted C1 to C12 alkyl, substituted or unsubstituted C1 to C12 alkoxy, substituted or unsubstituted C3 to C12 cycloalkyl, substituted or unsubstituted silyl, substituted or unsubstituted C2 to C12 heterocycloalkyl, substituted or unsubstituted C6 to C30 aryl or substituted or unsubstituted C2 to C30 heteroaryl, or two adjacent R₃ are joined to form the following spirocyclic structures: 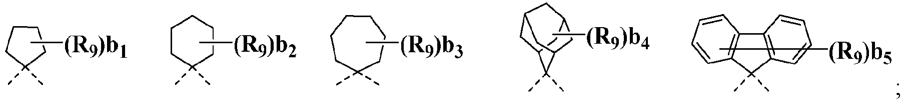
wherein R₉ is independently selected from any one of hydrogen, deuterium, tritium, cyano, nitro, halogen, substituted or unsubstituted C1 to C12 alkyl, substituted or unsubstituted C1 to C12 alkoxy, substituted or unsubstituted silyl, substituted or unsubstituted C3 to C12 cycloalkyl, substituted or unsubstituted C2 to C12 heterocycloalkyl, substituted or unsubstituted C6 to C30 aryl or substituted or unsubstituted C2 to C30 heteroaryl;
b₁ is selected from 0, 1, 2, 3, 4, 5, 6, 7 or 8, b₂ is selected from 0, 1, 2, 3, 4, 5, 6, 7, 8, 9 or 10, b₃ is selected from 0, 1, 2, 3, 4, 5, 6, 7, 8, 9, 10, 11 or 12, and b₄ is selected from 0, 1, 2, 3, 4, 5, 6, 7, 8, 9, 10, 11, 12, 13 or 14;
R₄ and R₈ are independently selected from any one of substituted or unsubstituted C1 to C12 alkyl, substituted or unsubstituted C3 to C12 cycloalkyl, substituted or unsubstituted silyl, substituted or unsubstituted C2 to C12 heterocycloalkyl, substituted or unsubstituted C6 to C30 aryl or substituted or unsubstituted C2 to C30 heteroaryl;
R₅ and R₆ are independently selected from any one of hydrogen, deuterium, tritium, cyano, nitro, halogen, substituted or unsubstituted C1 to C12 alkyl, substituted or unsubstituted C1 to C12 alkoxy, substituted or unsubstituted silyl, substituted or unsubstituted C3 to C12 cycloalkyl, substituted or unsubstituted C2 to C12 heterocycloalkyl, substituted or unsubstituted C6 to C30 aryl or substituted or unsubstituted C2 to C30 heteroaryl; and
a₁ is independently selected from 0, 1, 2, 3, 4 or 5, a₂ is independently selected from 0, 1, 2, 3 or 4, a₃ is independently selected from 0, 1, 2 or 3, a₄ is independently selected from 0, 1 or 2, as is independently selected from 0, 1, 2, 3, 4, 5, 6 or 7, a₆ is independently selected from 0, 1, 2, 3, 4, 5, 6, 7, 8, 9 or 10, a₇ is independently selected from 0, 1 or 2, a₈ is independently selected from 0, 1, 2, 3, 4, 5, 6, 7 or 8, and a₉ is independently selected from 0, 1, 2, 3, 4, 5 or 6.

5. The heterocyclic compound according to claim 1, wherein Ra is independently selected from any one of hydrogen, deuterium, cyano, nitro, fluorine, chlorine, bromine, iodine, methyl, ethyl, *n*-propyl, isopropyl, *n*-butyl, isobutyl, *tert*-butyl, cyclopropyl, cyclobutyl, cyclopentyl, cyclohexyl, cycloheptyl, adamantyl, norbornyl, trimethylsilyl, triethylsilyl, tri-*tert*-butylsilyl, deuterated trimethylsilyl, deuterated triethylsilyl, deuterated tri-*tert*-butylsilyl, deuterated methyl, deuterated ethyl, deuterated *n*-propyl, deuterated isopropyl, deuterated *n*-butyl, deuterated isobutyl, deuterated *tert*-butyl, deuterated adamantyl, deuterated norbornyl, fluorine-substituted methyl, fluorine-substituted ethyl, fluorine-substituted isopropyl, fluorine-substituted *tert*-butyl, methyl-substituted adamantyl or methyl-substituted norbornyl or selected from the group represented by Formula III, and when two or more Ra are present, two adjacent Ra can be joined to form a substituted or unsubstituted benzene ring.

6. The heterocyclic compound according to claim 1, wherein Formula III is selected from any one of the following groups:

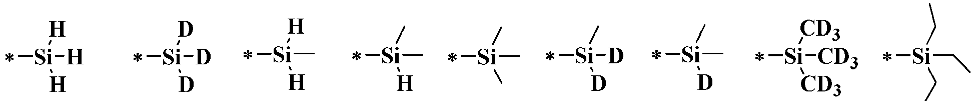

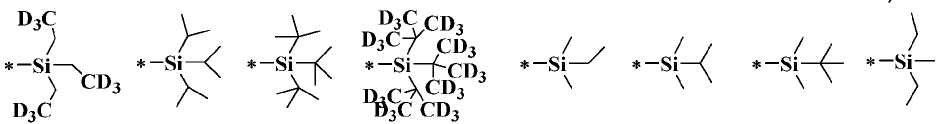

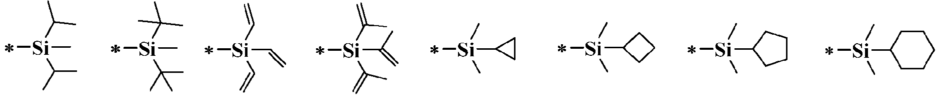

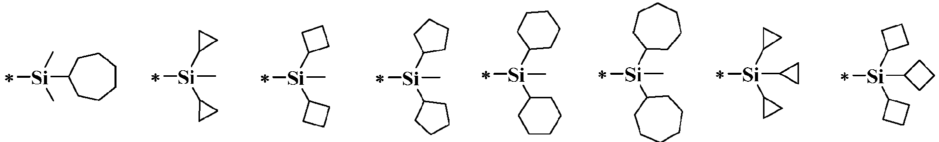

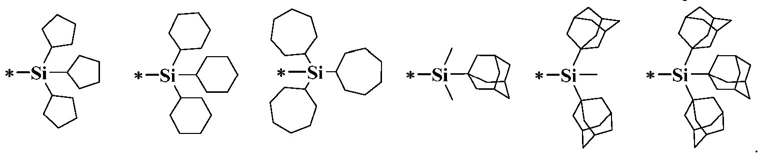

7. The heterocyclic compound according to claim 1, wherein L₁ and L₂ are independently selected from any one of a single bond or the following group: 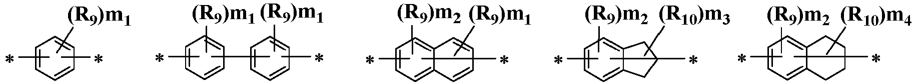 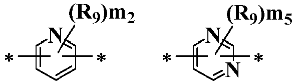
wherein R₉ is independently selected from any one of hydrogen, deuterium, cyano, nitro, halogen, substituted or unsubstituted C1 to C12 alkyl, substituted or unsubstituted C1 to C12 alkoxy, substituted or unsubstituted C3 to C12 cycloalkyl, substituted or unsubstituted silyl, substituted or unsubstituted C2 to C12 heterocycloalkyl, substituted or unsubstituted C6 to C30 aryl or substituted or unsubstituted C2 to C30 heteroaryl; and
m₁ is independently selected from 0, 1, 2, 3 or 4, m₂ is independently selected from 0, 1, 2 or 3, m₃ is independently selected from 0, 1, 2, 3, 4, 5 or 6, m₄ is independently selected from 0, 1, 2, 3, 4, 5, 6, 7 or 8, and m₅ is independently selected from 0, 1 or 2.

8. The heterocyclic compound according to claim 1, wherein the heterocyclic compound is selected from any one of the following structures:

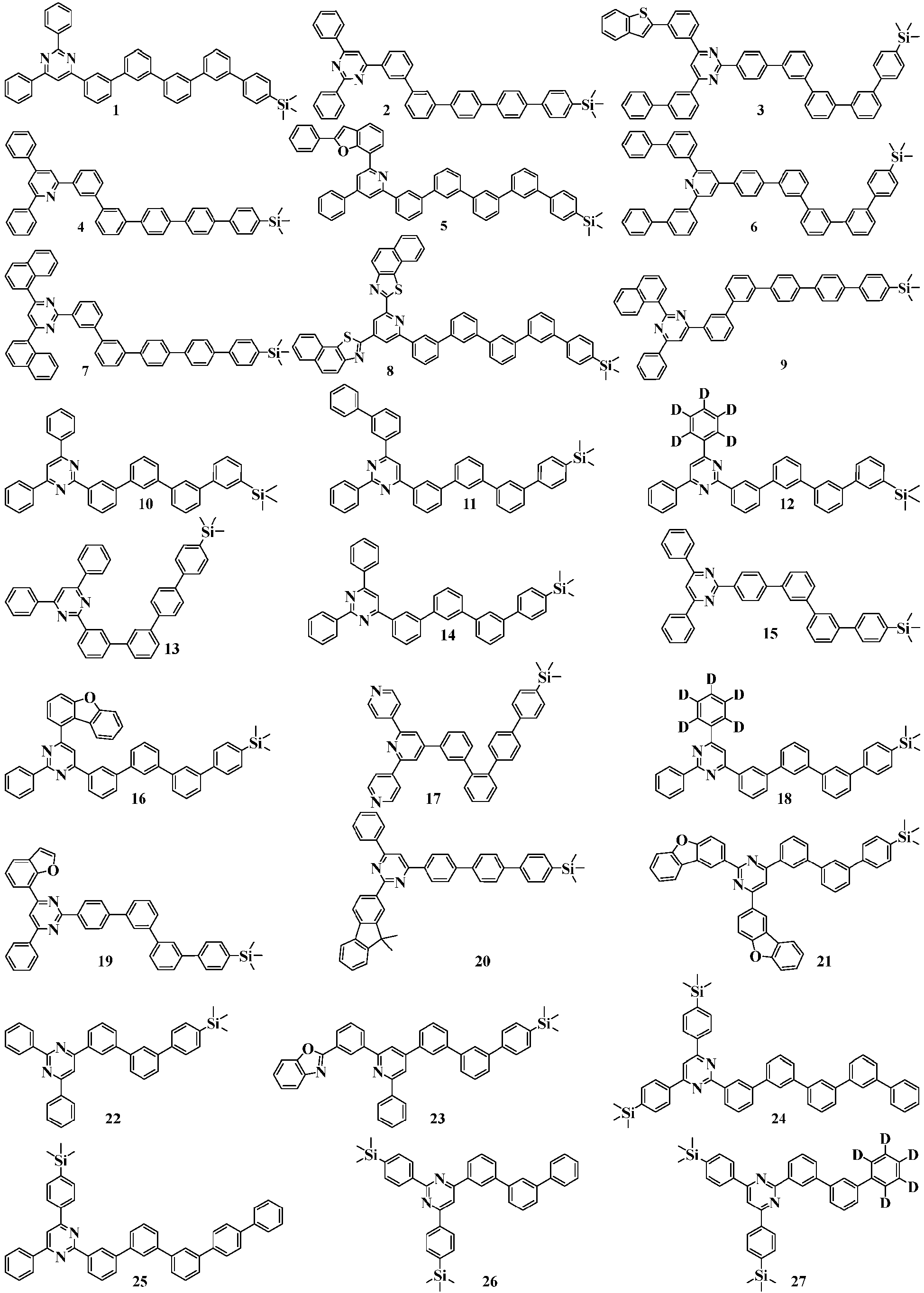

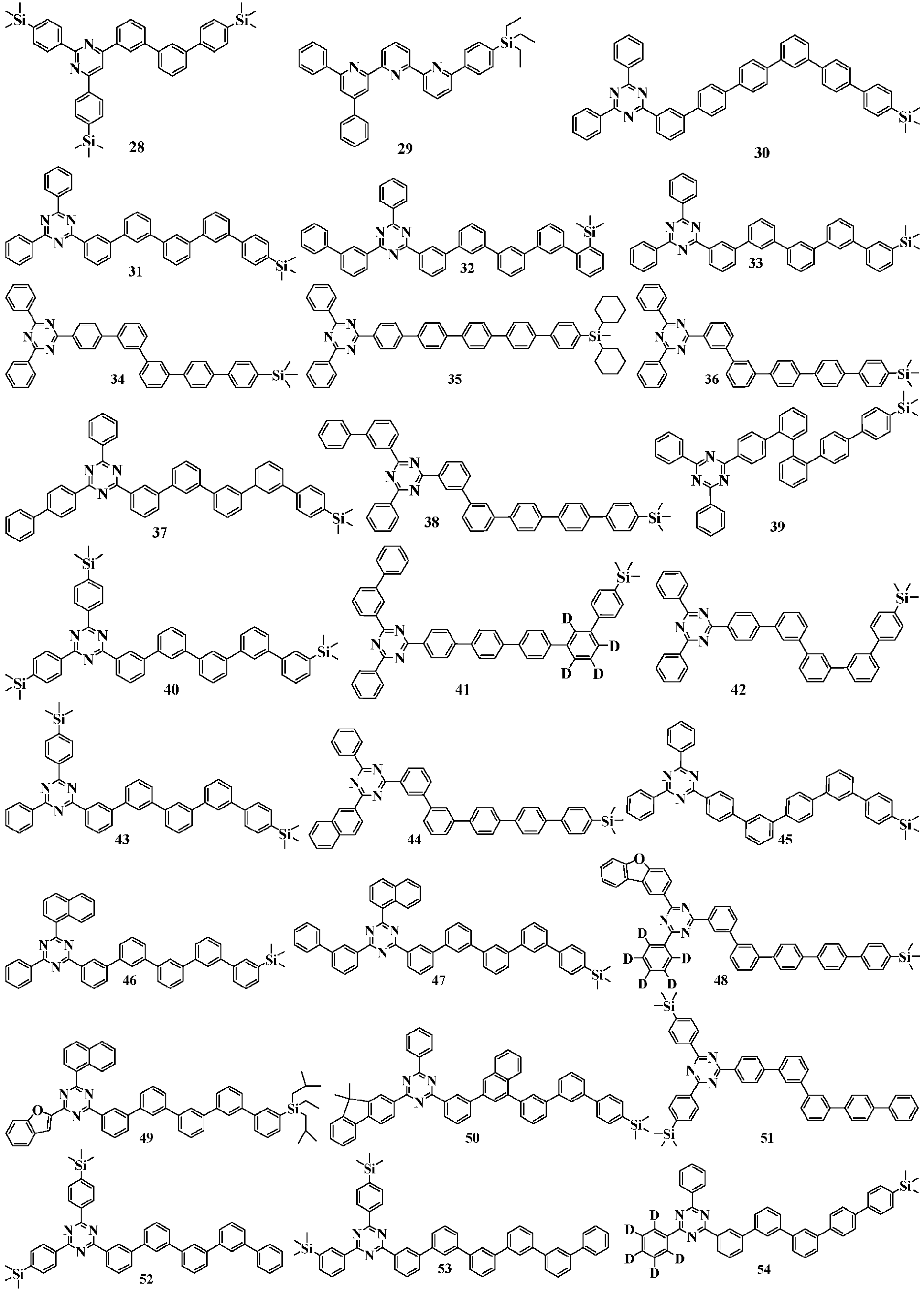

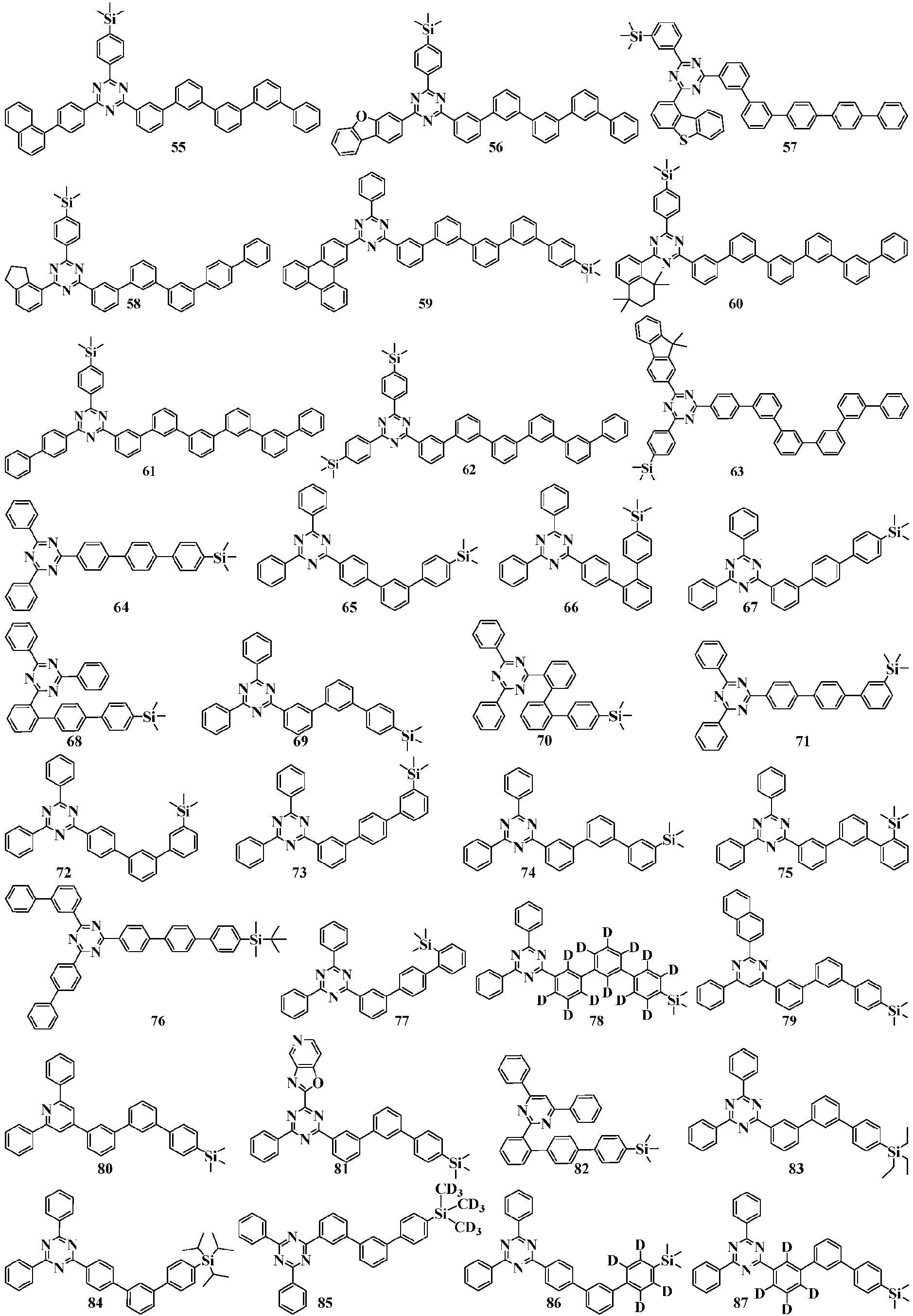

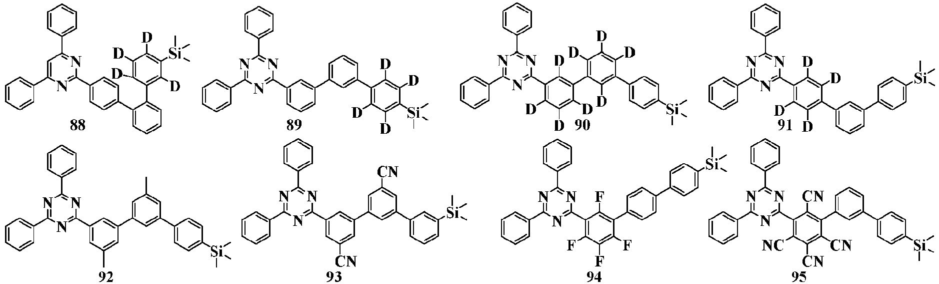

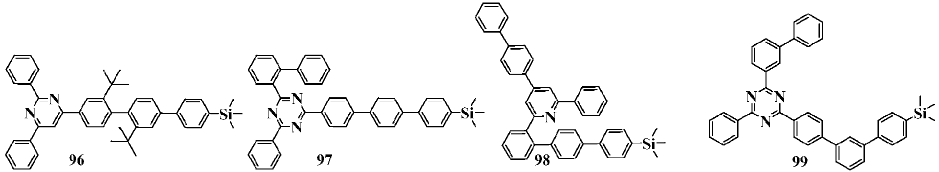

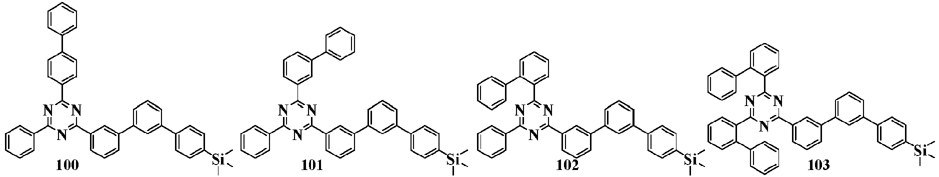

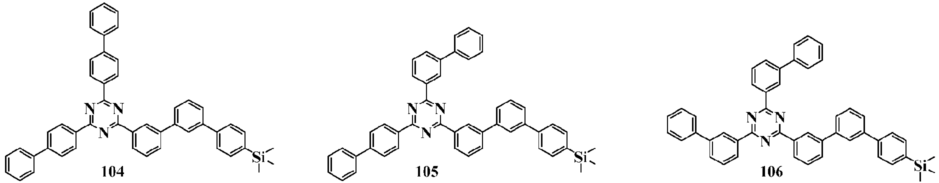

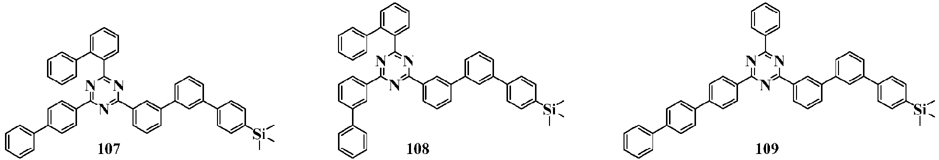

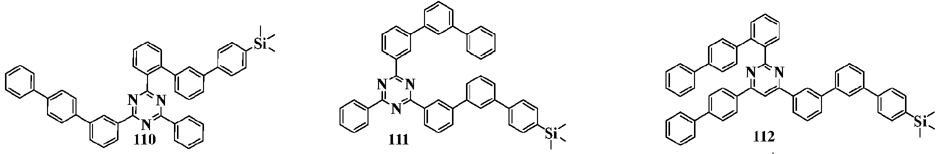

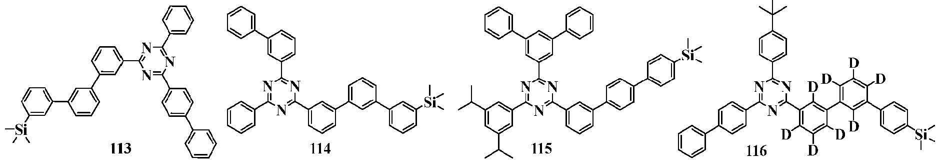

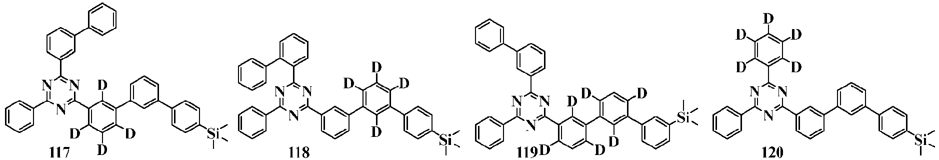

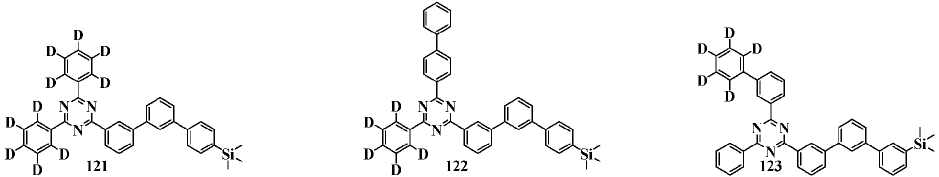

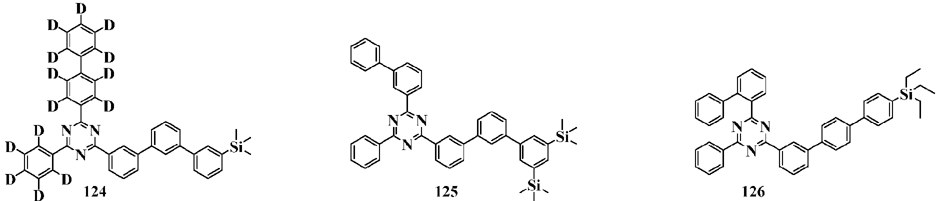

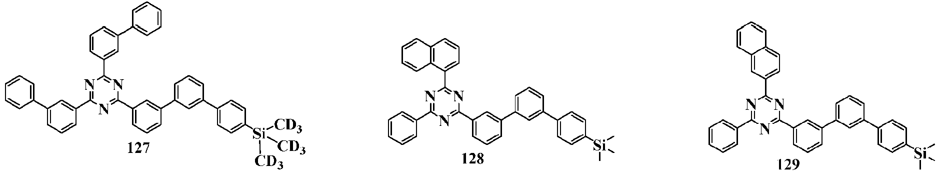

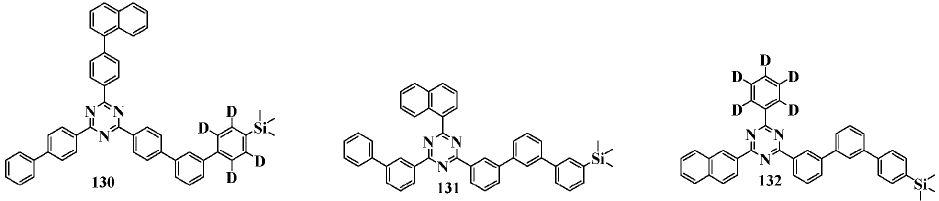

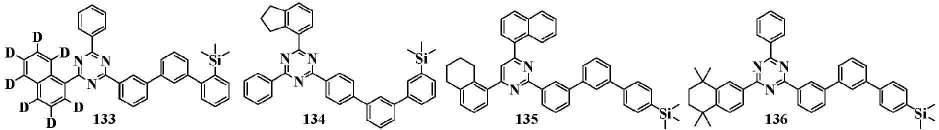

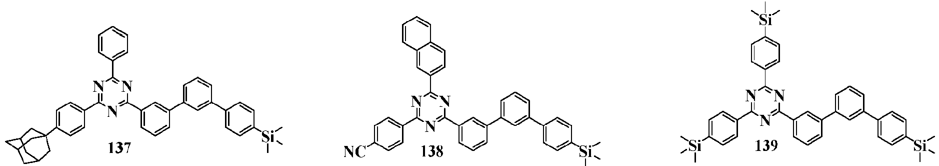

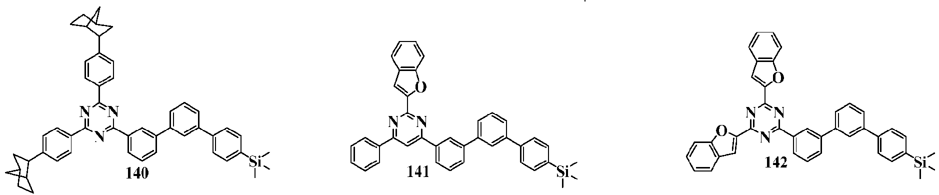

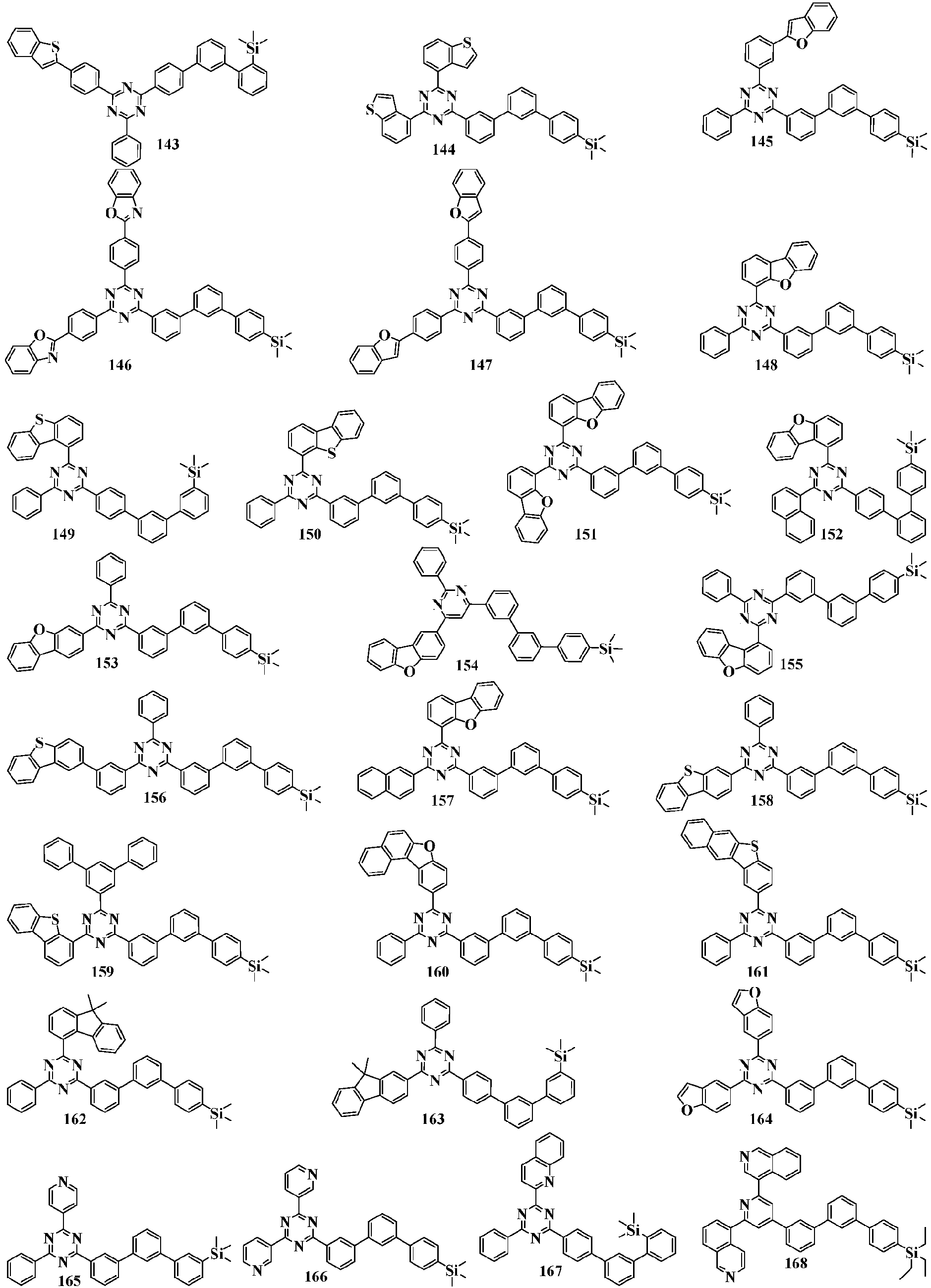

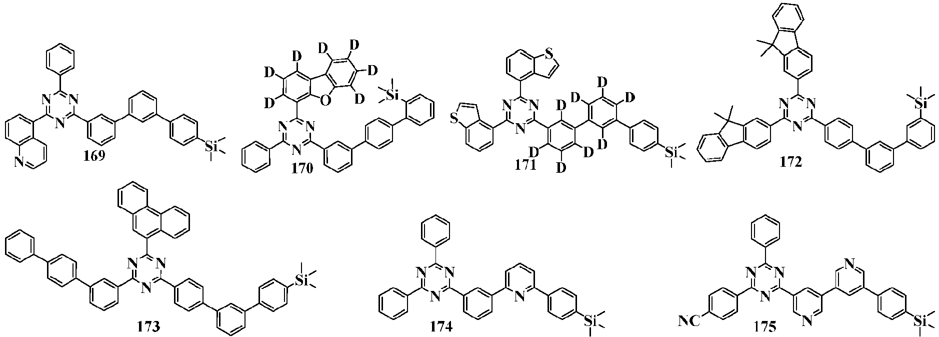

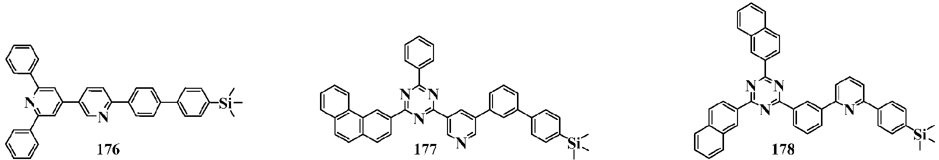

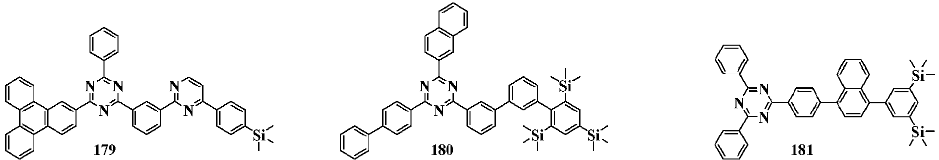

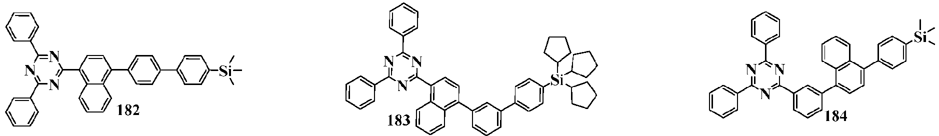

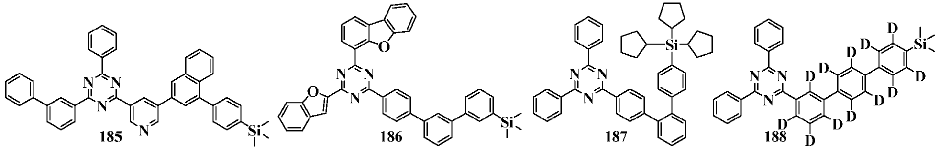

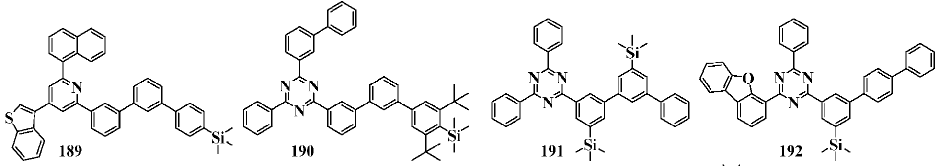

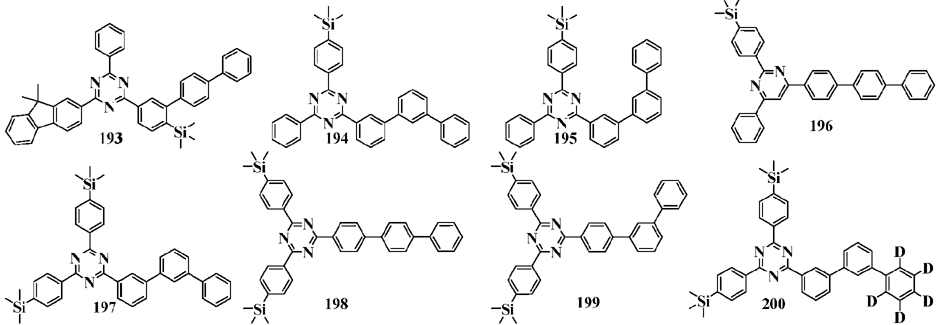

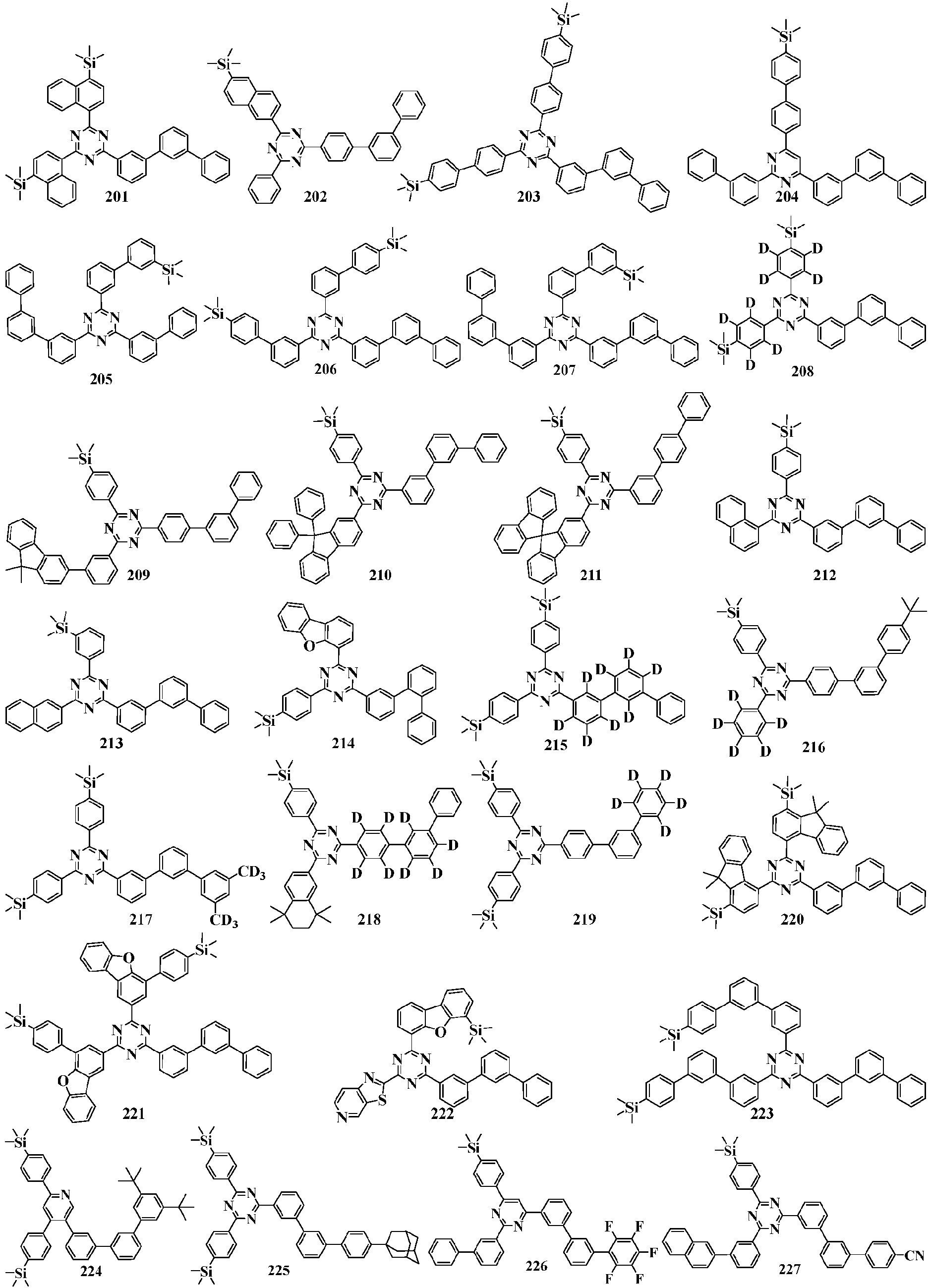

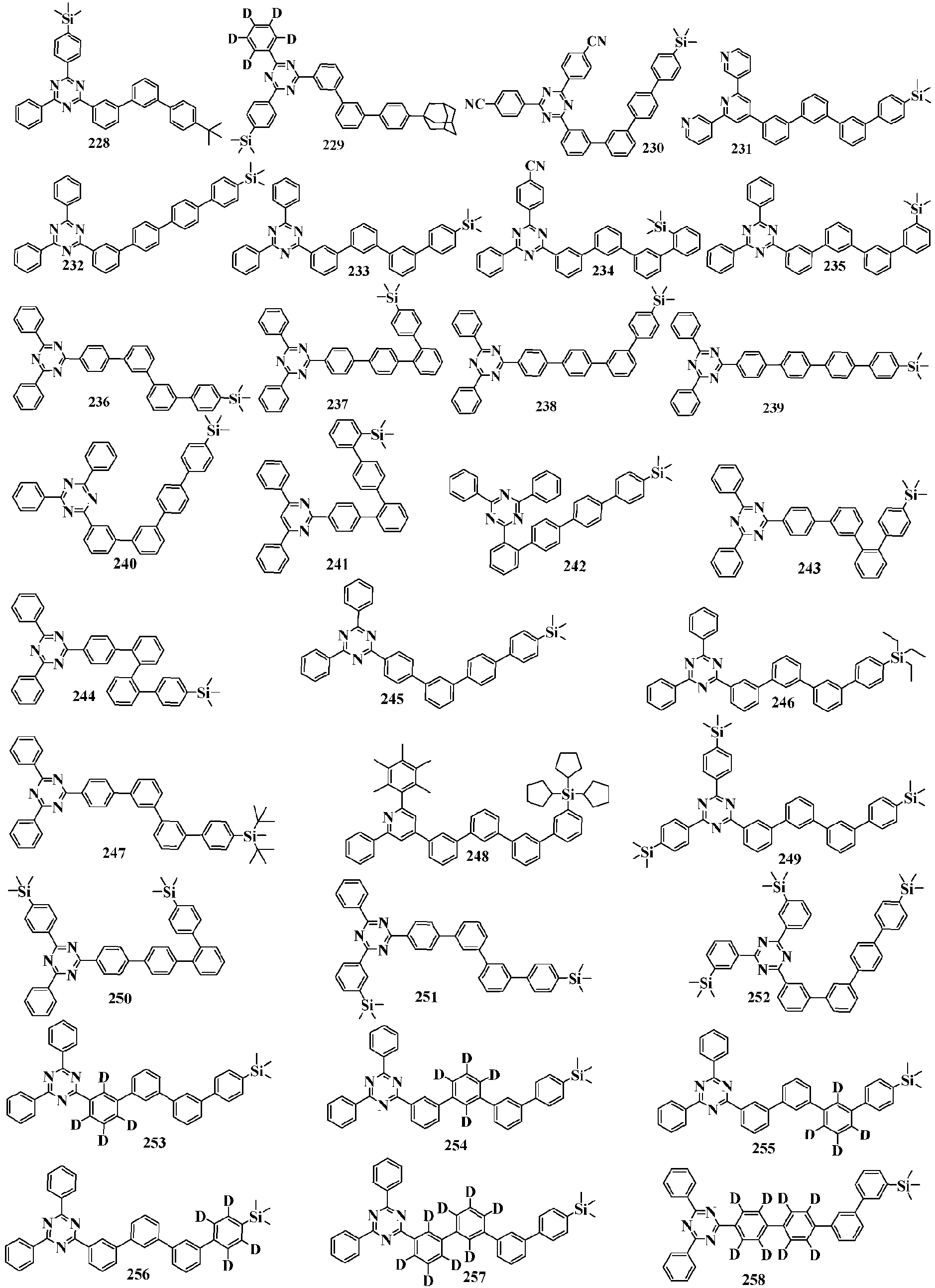

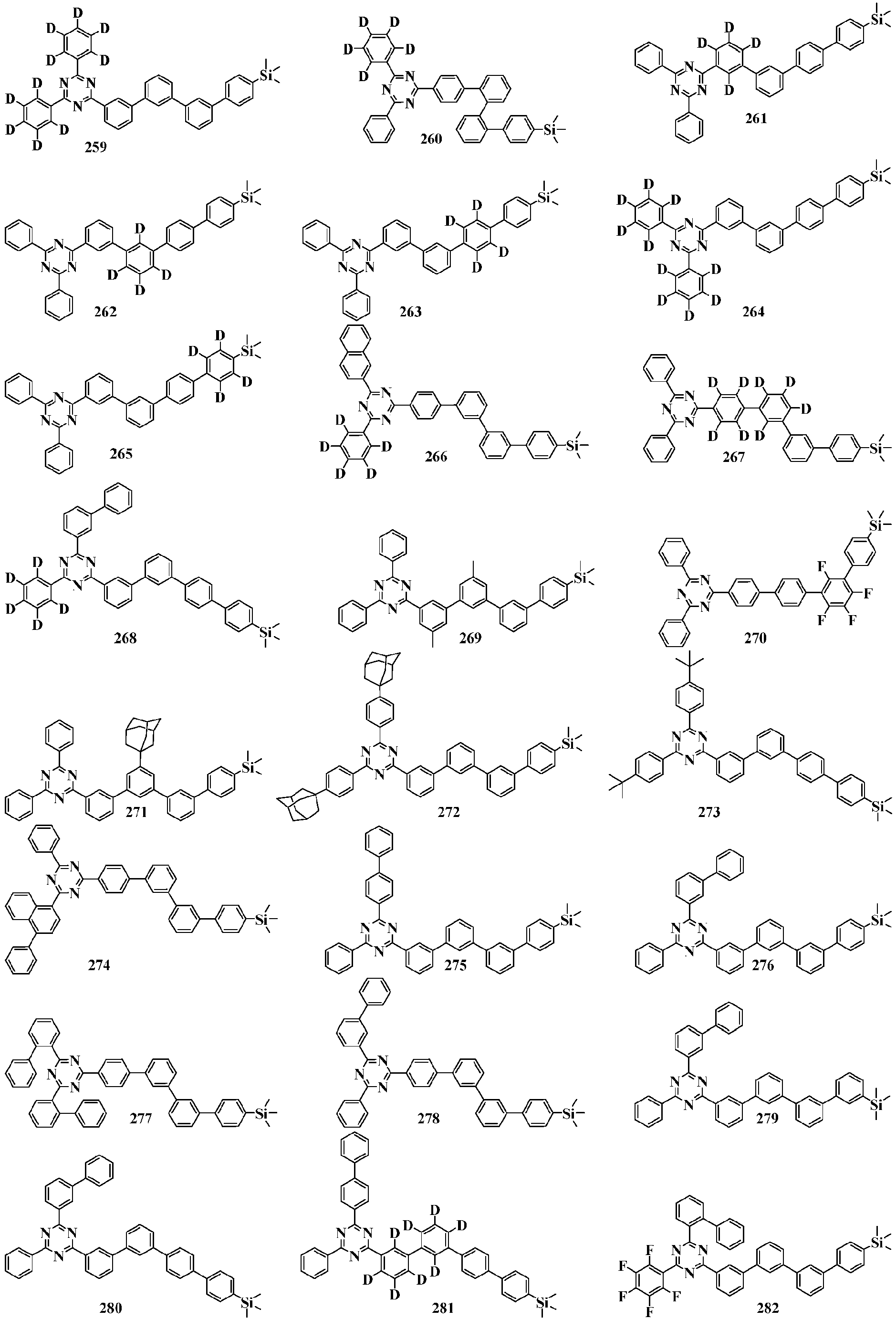

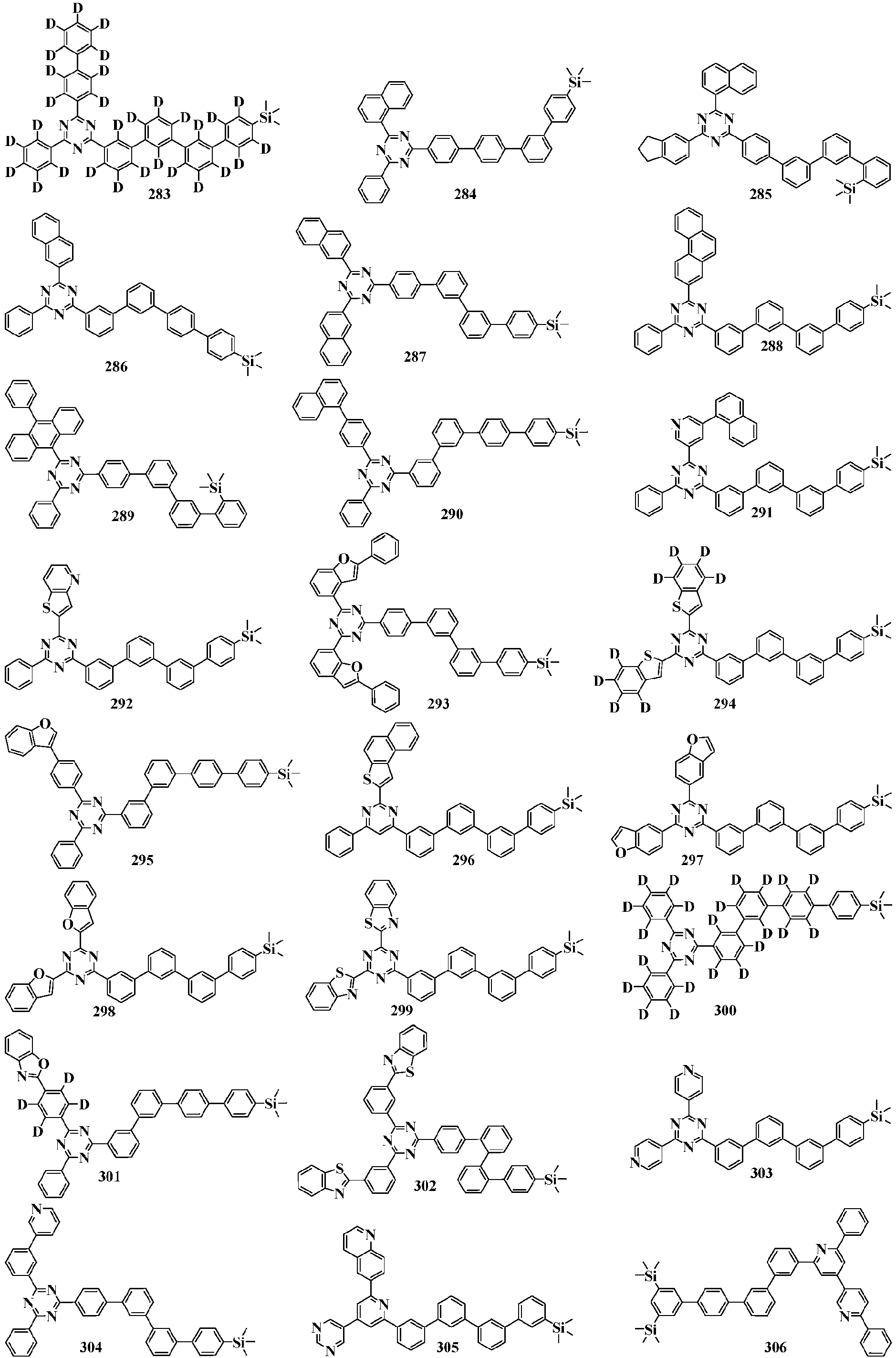

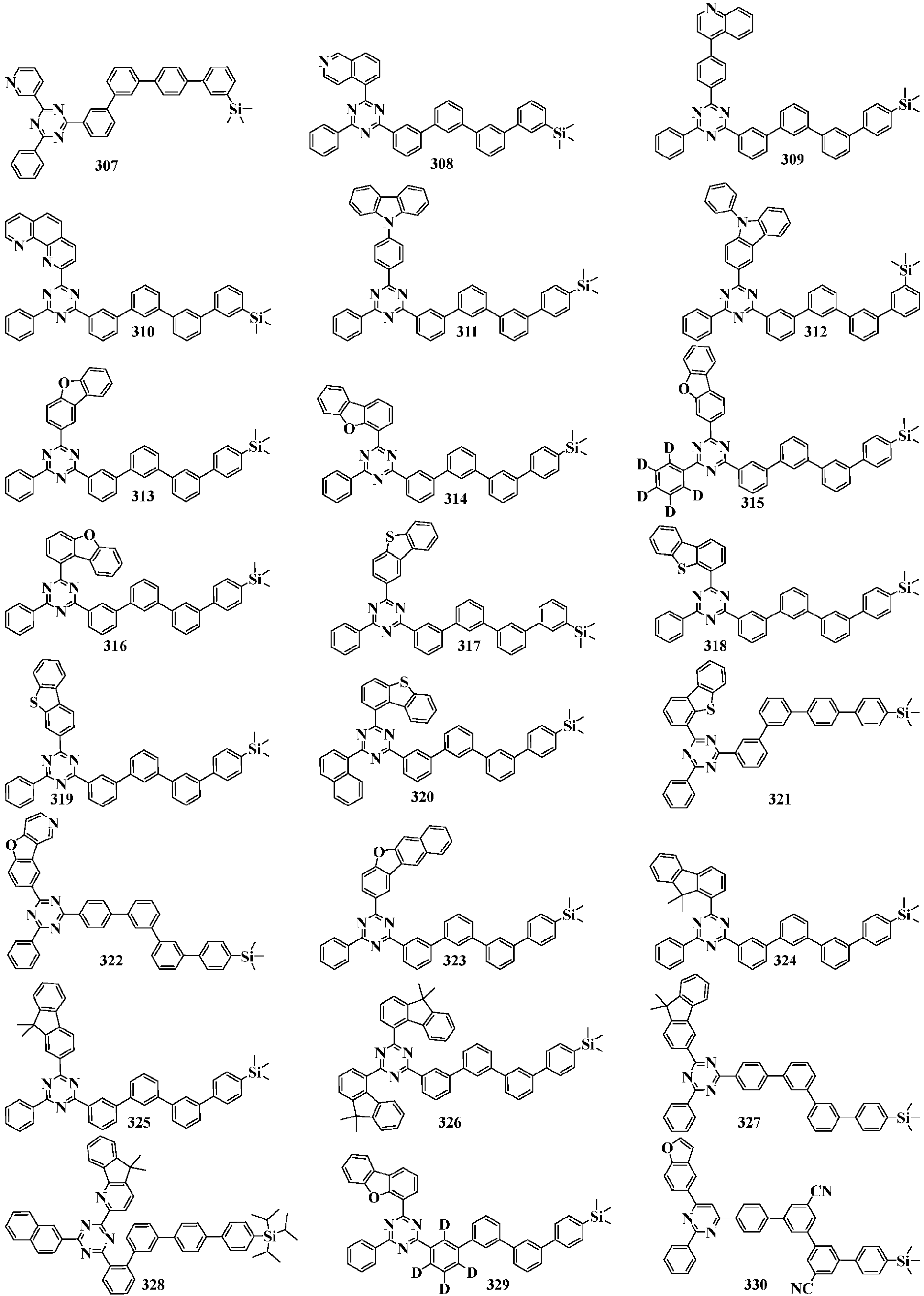

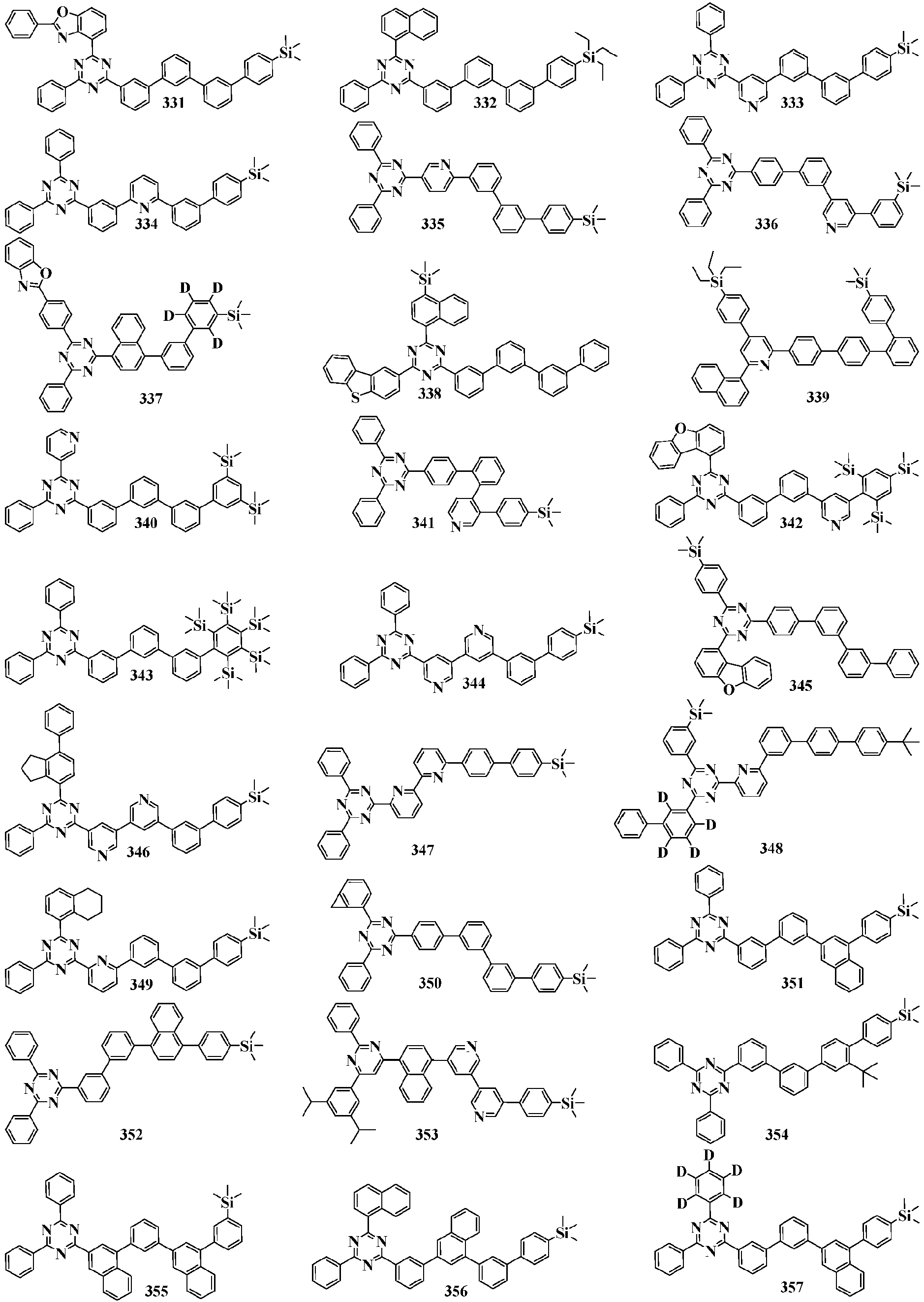

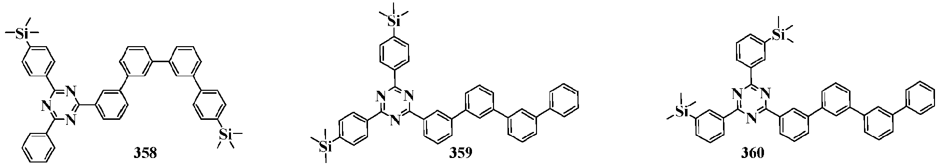

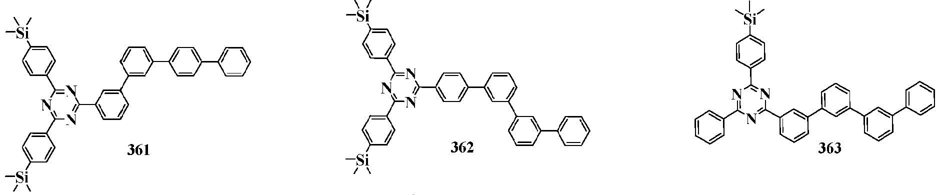

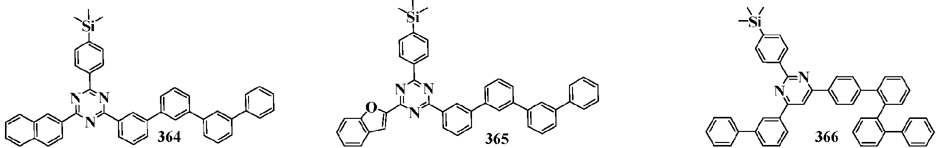

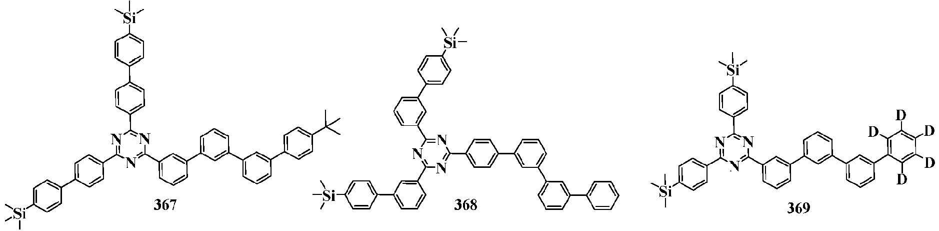

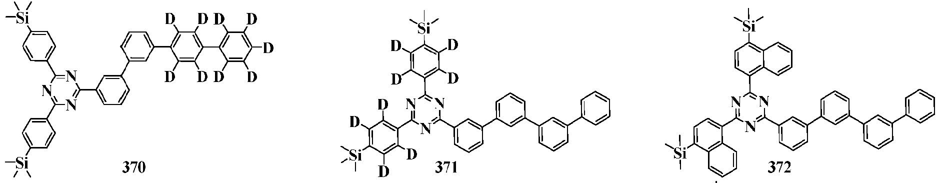

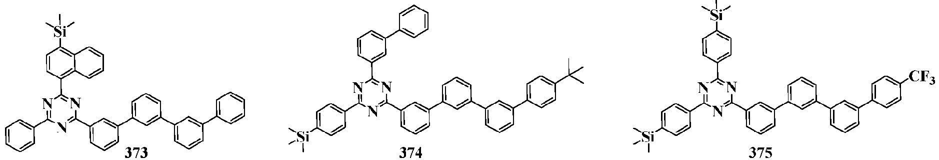

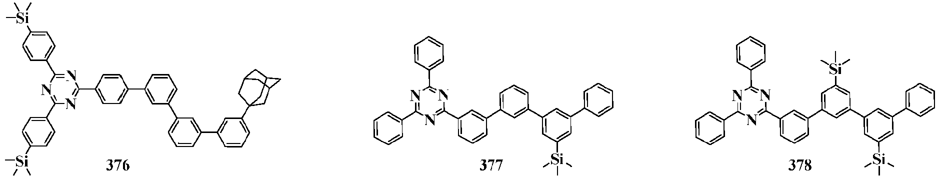

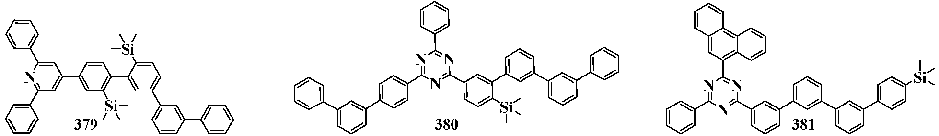

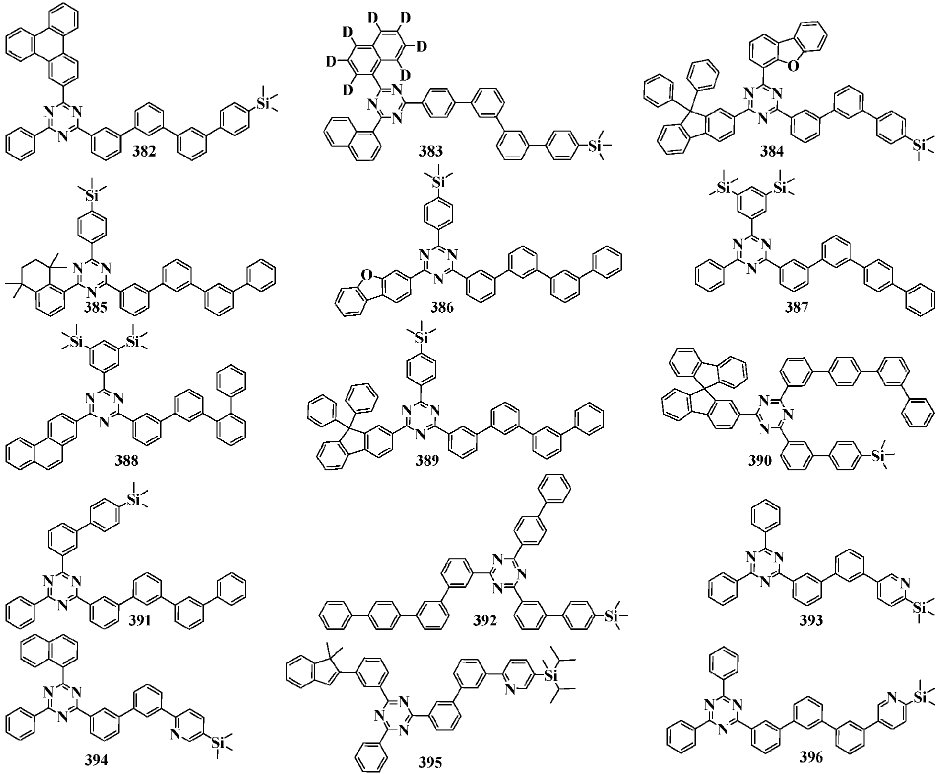

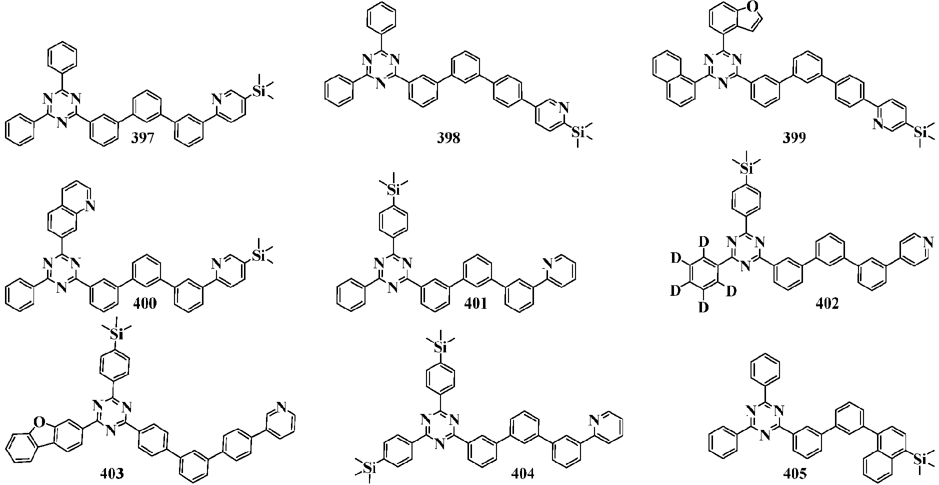

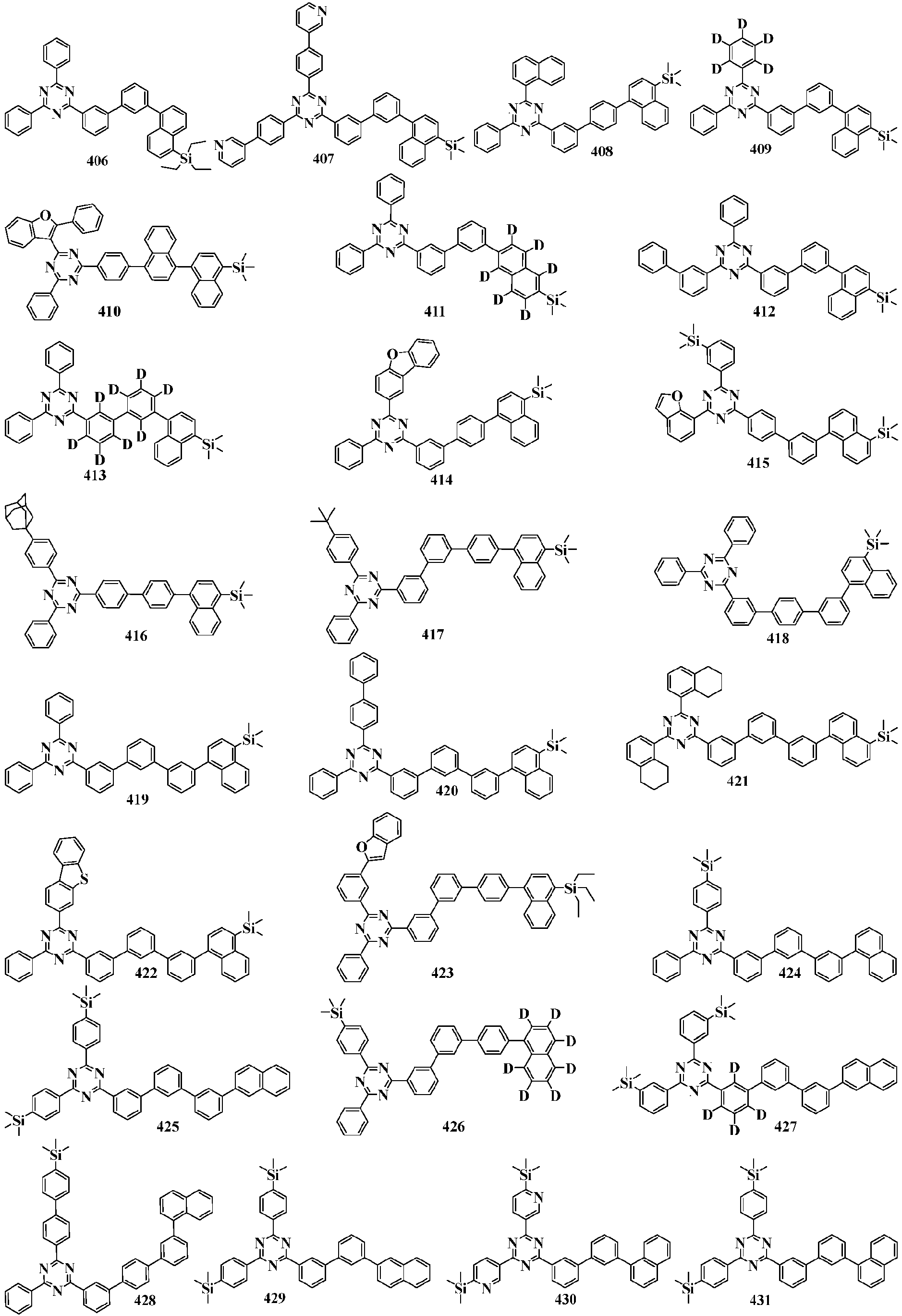

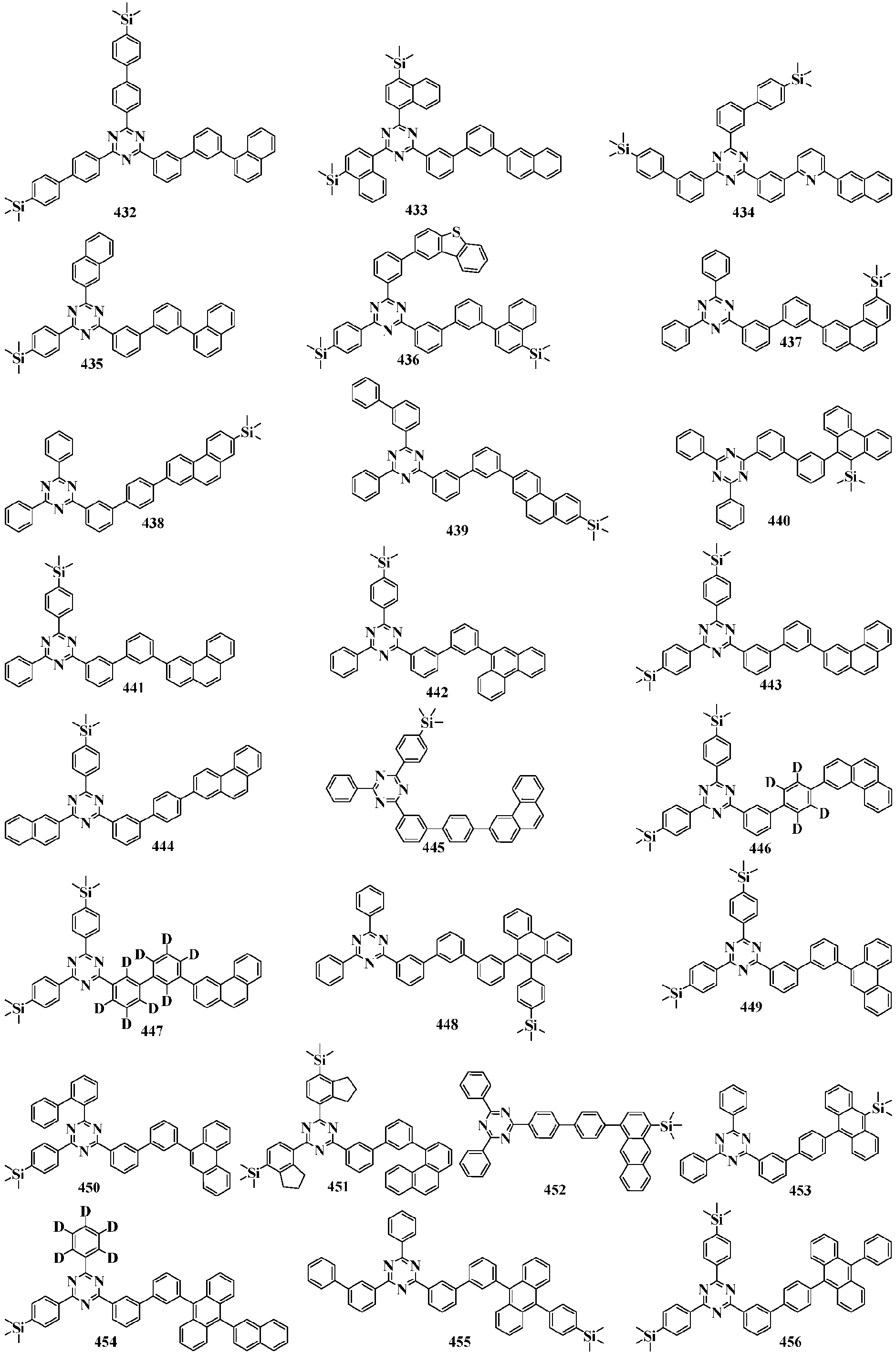

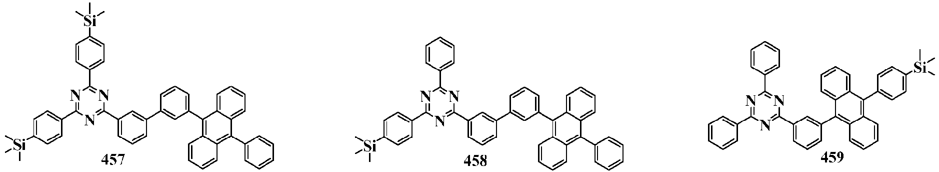

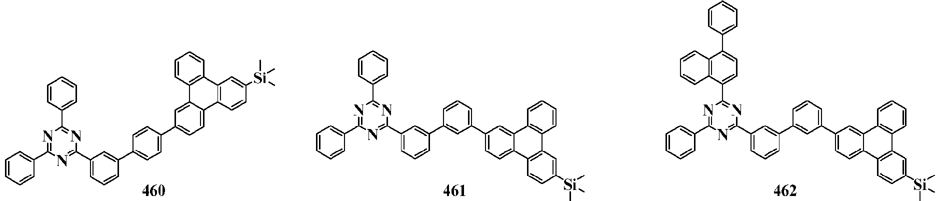

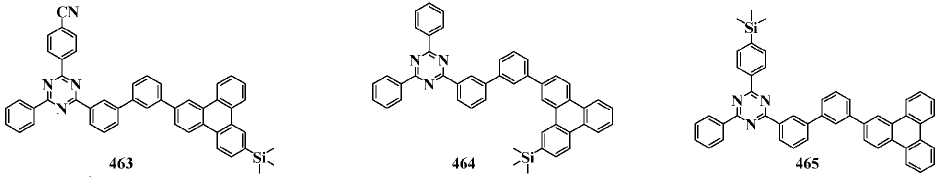

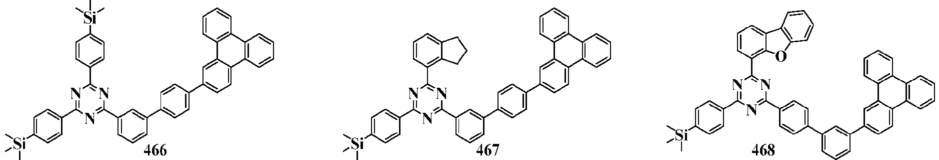

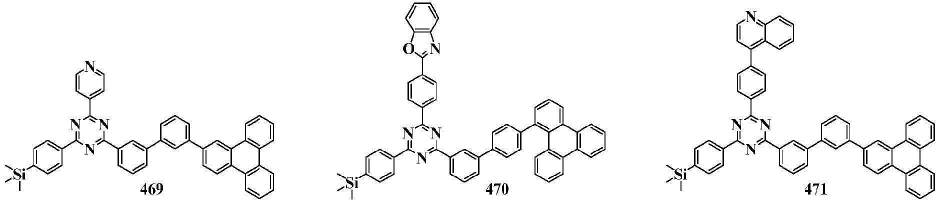

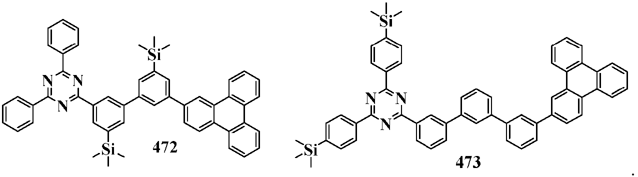

9. An organic electroluminescent device, comprising an anode, a cathode facing the anode and an organic layer located between the anode and the cathode or outside at least one electrode of the anode or the cathode, wherein the organic layer comprises any one or more of the heterocyclic compounds according to any one of claims 1 to 8.

10. The organic electroluminescent device according to claim 9, wherein the organic layer is located between the anode and the cathode and comprises at least one of a light-emitting layer, an electron transport layer or a hole blocking layer, wherein the at least one of the light-emitting layer, the electron transport layer or the hole blocking layer comprises any one or more of the heterocyclic compounds according to any one of claims 1 to 8.

## Patentansprüche

1. Heterocyclische Verbindung, wobei die heterocyclische Verbindung eine durch Formel I dargestellte Struktur aufweist: 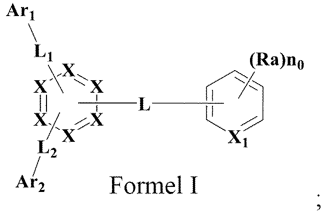
wobei in Formel I X unabhängig aus C(R₂) oder einem N-Atom ausgewählt ist, mindestens ein X aus einem N-Atom ausgewählt ist und X, das an Ar1, Ar2 oder L gebunden ist, aus einem C-Atom ausgewählt ist;
wobei X₁ unabhängig aus einem C-Atom oder einem N-Atom ausgewählt ist;
wobei R₂ aus einer beliebigen der folgenden Gruppen unabhängig ausgewählt ist: Wasserstoff, Deuterium, Tritium, Halogen, Cyano, Nitro, substituierte oder unsubstituierte C1-bis C12-Alkylgruppen, substituierte oder unsubstituierte Silylgruppen, substituierte oder unsubstituierte C1- bis C12-Alkoxygruppen, substituierte oder unsubstituierte C3- bis C12-Cycloalkylgruppen, substituierte oder unsubstituierte C2- bis C12-Heterocycloalkylgruppen, substituierte oder unsubstituierte C6- bis C30-Arylgruppen oder substituierte oder unsubstituierte C2- bis C30-Heteroarylgruppen;
wobei Ar₁ und Ar₂ aus einer beliebigen der folgenden Gruppen unabhängig ausgewählt sind: substituierte oder unsubstituierte C6- bis C30-Arylgruppen oder substituierte oder unsubstituierte C2- bis C30-Heteroarylgruppen;
wobei L₁ und L₂ aus einer beliebigen der folgenden Gruppen unabhängig ausgewählt sind: eine Einfachbindung, substituierte oder unsubstituierte C6- bis C30-Arylenegruppen oder substituierte oder unsubstituierte C2- bis C30-Heteroarylenegruppen;
wobei Ra aus einer beliebigen der folgenden Gruppen unabhängig ausgewählt ist: Wasserstoff, Deuterium, Halogen, Cyano, Nitro, substituierte oder unsubstituierte C1- bis C12-Alkylgruppen, substituierte oder unsubstituierte Silylgruppen, substituierte oder unsubstituierte C1- bis C12-Alkoxygruppen, substituierte oder unsubstituierte C3- bis C12-Cycloalkylgruppen oder substituierte oder unsubstituierte C2- bis C12-Heterocycloalkylgruppen;
wobei n₀ aus 0, 1, 2, 3, 4 oder 5 unabhängig ausgewählt ist, wobei, wenn n₀ größer als 1 ist, zwei oder mehr Ra gleich oder unterschiedlich zueinander sind, oder zwei benachbarte Ra sich verbinden, um einen substituierten oder unsubstituierten Benzolring zu bilden;
wobei L aus einer beliebigen der folgenden Gruppen unabhängig ausgewählt: 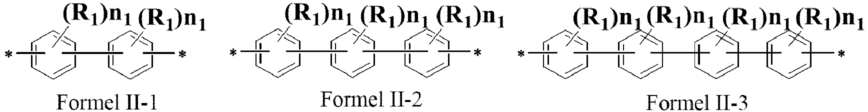 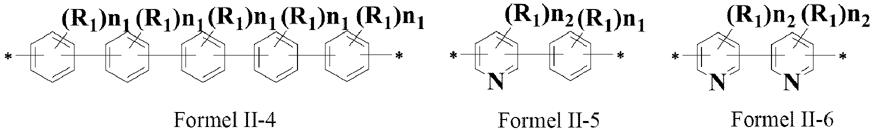 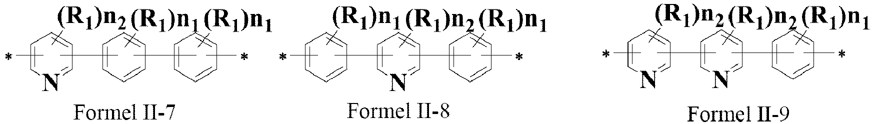 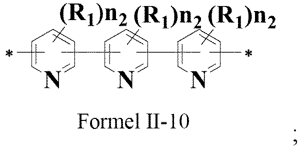
wobei R₁ unabhängig aus einer beliebigen Gruppe von Wasserstoff, Deuterium, Halogen, Cyano, Nitro, substituierter oder unsubstituierter C1- bis C12-Alkylgruppe, substituierter oder unsubstituierter Silylgruppe, substituierter oder unsubstituierter C1- bis C12-Alkoxygruppe, substituierter oder unsubstituierter C3- bis C12-Cycloalkylgruppe oder substituierter oder unsubstituierter C2- bis C12-Heterocycloalkylgruppe ausgewählt ist;
wobei n₁ aus 0, 1, 2, 3 oder 4 unabhängig ausgewählt; wobei n₂ aus 0, 1, 2 oder 3 unabhängig ausgewählt; wobei, wenn n₁ größer als 1 ist, zwei oder mehr R₁ gleich oder unterschiedlich zueinander sind, oder sich zwei benachbarte R1 zu einer substituierten oder unsubstituierten Benzolringstruktur verbinden;
wobei mit der Bedingung, dass ein oder mehrere Wasserstoffe in mindestens einer Gruppe von Ar₁, Ar₂, L, L₁, L₂, R₂ oder Ra durch eine durch Formel III dargestellte Gruppe ersetzt sind: 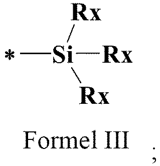 Rx aus einer beliebigen Gruppe von Wasserstoff, Deuterium, Cyano, Nitro, Halogen, substituierter oder unsubstituierter C1- bis C12-Alkylgruppe, substituierter oder unsubstituierter C2- bis C12-Alkenylgruppe, substituierter oder unsubstituierter C3- bis C12-Cycloalkylgruppe oder substituierter oder unsubstituierter C2- bis C12-Heterocycloalkylgruppe unabhängig ausgewählt ist; und
die folgende Verbindung von der heterocyclischen Verbindung ausgeschlossen ist:

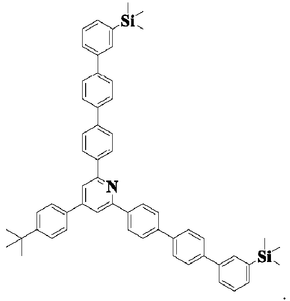

2. Heterocyclische Verbindung nach Anspruch 1, wobei die heterocyclische Verbindung in Formel I aus einer beliebigen der durch die folgenden Formeln III-1 bis III-5 dargestellten Strukturen ausgewählt ist: 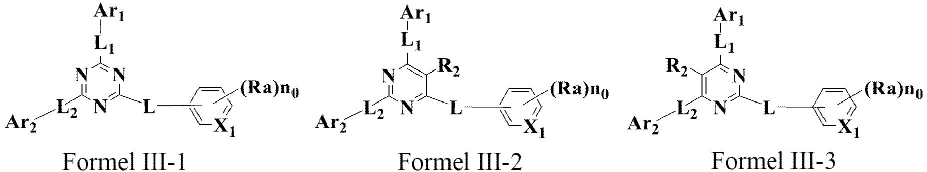 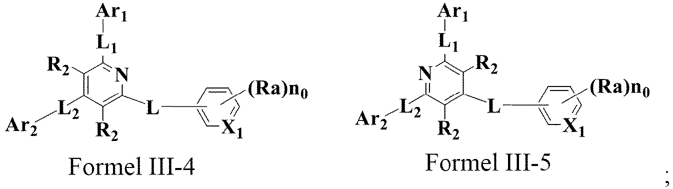 wobei die Definitionen von Ar₁, Ar₂, R₂, L, L₁, L₂, X₁, Ra und n₀ dieselben sind wie in Formel I.

3. Heterocyclische Verbindung nach Anspruch 1, wobei Ar₁ und Ar₂ unabhängig voneinander ausgewählt aus einer beliebigen der folgenden Gruppen sind: 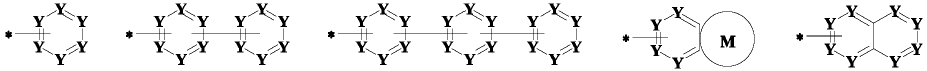 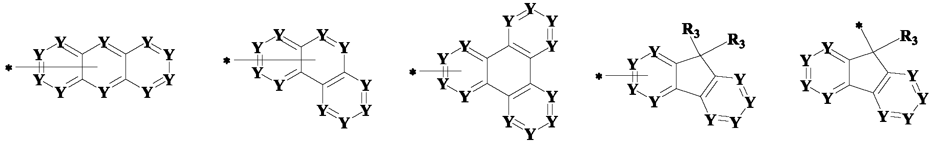 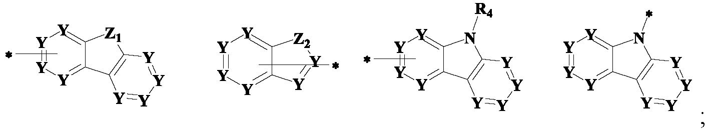
wobei Y unabhängig aus C(R₅) oder einem N-Atom ausgewählt ist und Y an einer Verknüpfungsstelle aus einem C-Atom ausgewählt ist;
wobei der Ring M aus einem unsubstituierten C3- bis C7-aliphatischen Ring oder einem C3- bis C7-aliphatischen Ring, substituiert mit einem oder mehreren R₆, ausgewählt ist;
wobei Z₁ aus einem O-Atom oder einem S-Atom ausgewählt ist, und Z₂ aus O, S, C(R₇)₂ oder N(R₈) ausgewählt ist;
wobei R₃ und R₇ unabhängig aus einer beliebigen Gruppe von Wasserstoff, Deuterium, substituierter oder unsubstituierter C1- bis C12-Alkylgruppe, substituierter oder unsubstituierter C1- bis C12-Alkoxygruppe, substituierter oder unsubstituierter C3- bis C12-Cycloalkylgruppe, substituierter oder unsubstituierter Silylgruppe, substituierter oder unsubstituierter C2- bis C12-Heterocycloalkylgruppe, substituierter oder unsubstituierter C6-bis C30-Arylgruppe oder substituierter oder unsubstituierter C2- bis C30-Heteroarylgruppe ausgewählt sind, oder sich zwei benachbarte R₃ zu einer substituierten oder unsubstituierten spirocyclischen Struktur verbinden;
wobei R₄ und R₈ unabhängig aus einer beliebigen Gruppe von substituierter oder unsubstituierter C1- bis C12-Alkylgruppe, substituierter oder unsubstituierter C3- bis C12-Cycloalkylgruppe, substituierter oder unsubstituierter Silylgruppe, substituierter oder unsubstituierter C2- bis C12-Heterocycloalkylgruppe, substituierter oder unsubstituierter C6-bis C30-Arylgruppe oder substituierter oder unsubstituierter C2- bis C30-Heteroarylgruppe ausgewählt sind; und
wobei R₅ und R₆ unabhängig aus einer beliebigen Gruppe von Wasserstoff, Deuterium, Tritium, Cyano, Nitro, Halogen, substituierter oder unsubstituierter C1- bis C12-Alkylgruppe, substituierter oder unsubstituierter C1- bis C12-Alkoxygruppe, substituierter oder unsubstituierter Silylgruppe, substituierter oder unsubstituierter C3- bis C12-Cycloalkylgruppe, substituierter oder unsubstituierter C2- bis C12-Heterocycloalkylgruppe, substituierter oder unsubstituierter C6- bis C30-Arylgruppe oder substituierter oder unsubstituierter C2- bis C30-Heteroarylgruppe ausgewählt sind, oder sich zwei benachbarte R₅ zu einem substituierten oder unsubstituierten Ring verbinden.

4. Heterocyclische Verbindung nach Anspruch 1, wobei Ar₁ und Ar₂ unabhängig aus einer beliebigen der folgenden Gruppen ausgewählt sind: 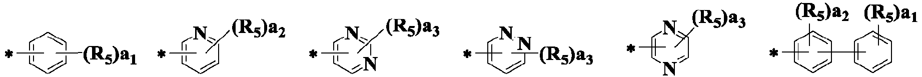 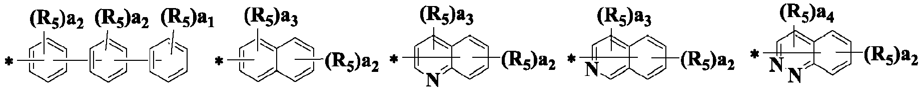 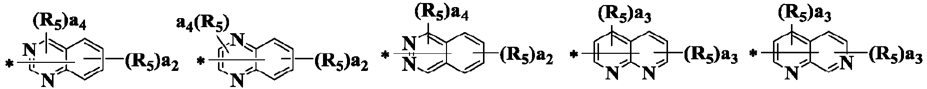 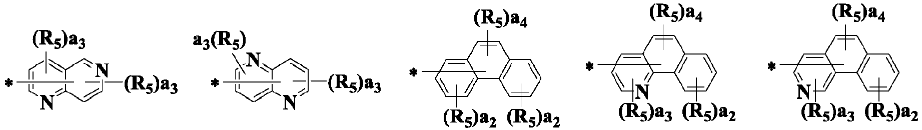 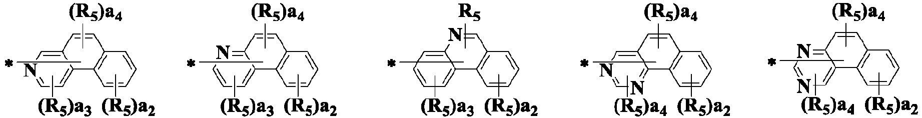 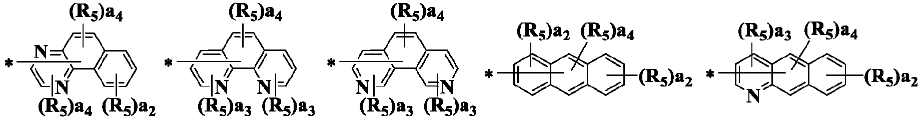 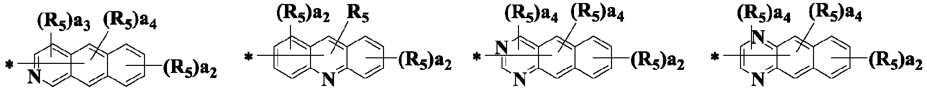 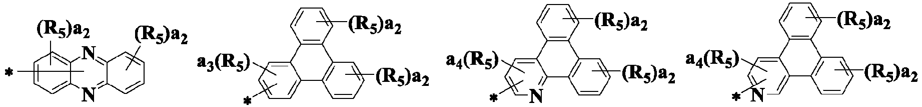 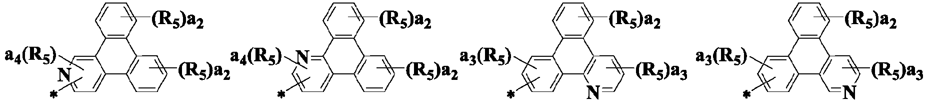 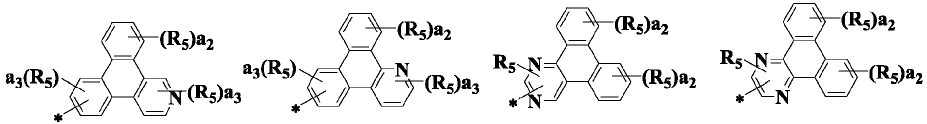 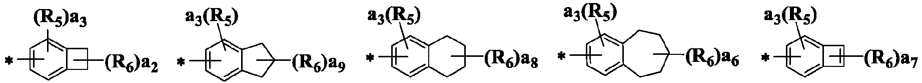 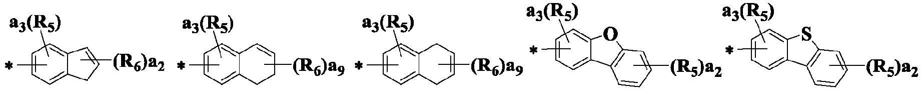 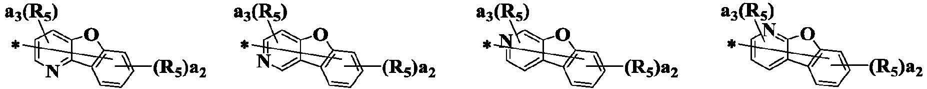 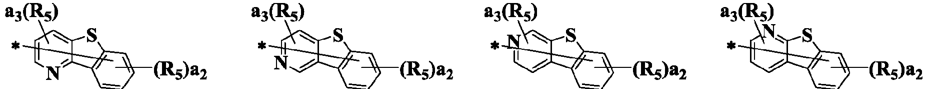 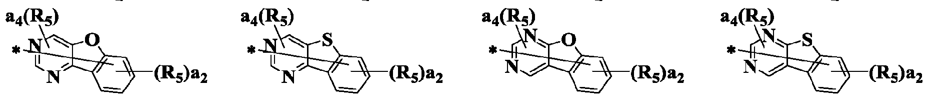 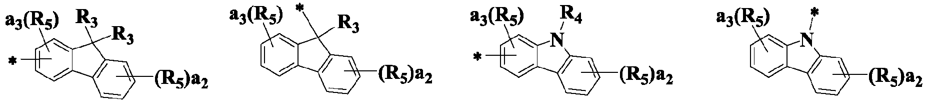 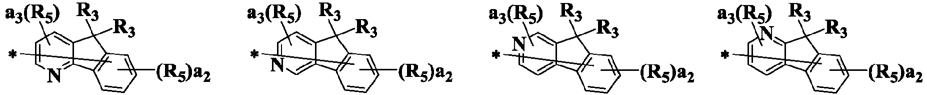 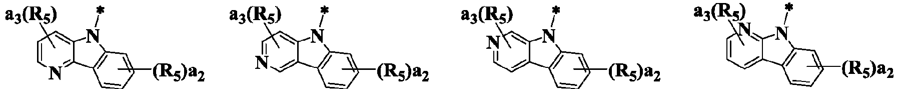 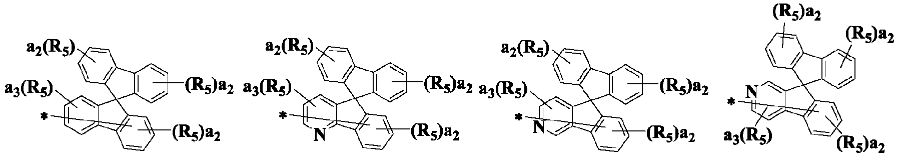 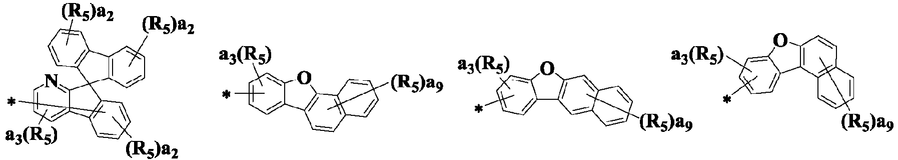 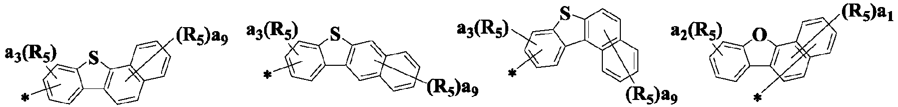 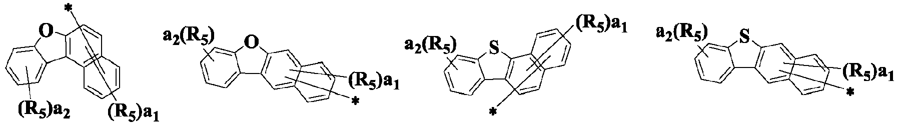 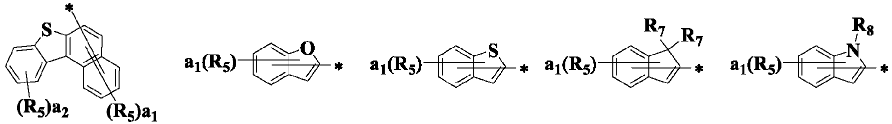 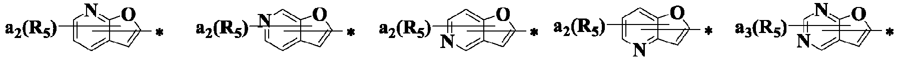 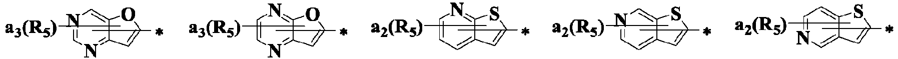 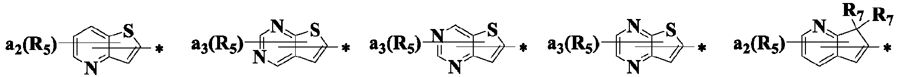 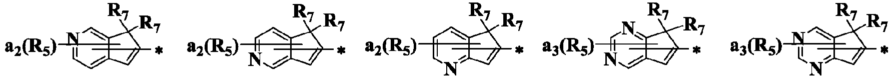 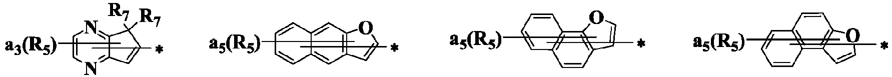 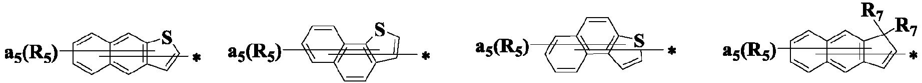 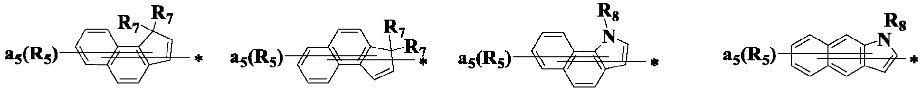 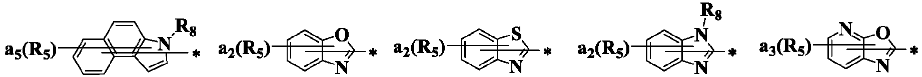 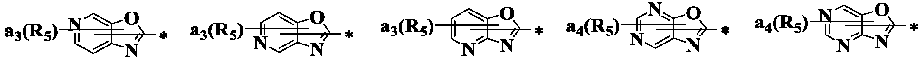 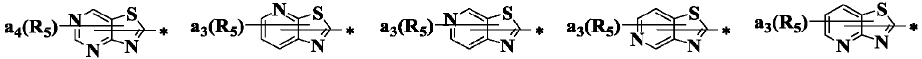 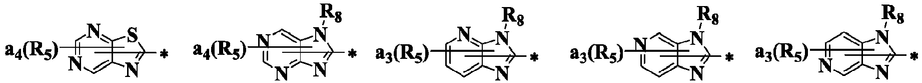 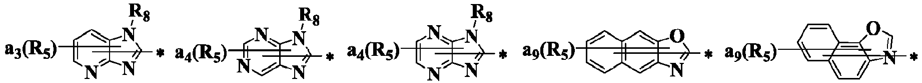 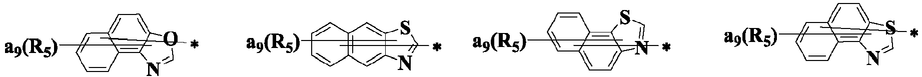 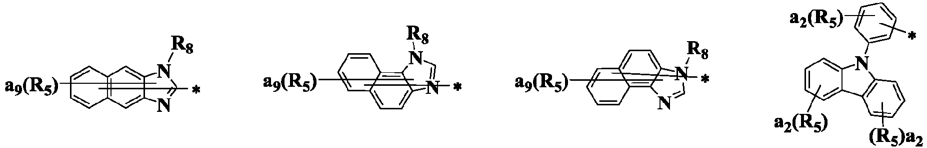 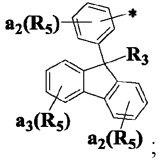
wobei R₃ und R₇ unabhängig voneinander aus einer der folgenden Gruppen ausgewählt sind: Wasserstoff, Deuterium, substituierte oder unsubstituierte C1- bis C12-Alkylgruppe, substituierte oder unsubstituierte C1- bis C12-Alkoxygruppe, substituierte oder unsubstituierte C3- bis C12-Cycloalkylgruppe, substituierte oder unsubstituierte Silylgruppe, substituierte oder unsubstituierte C2- bis C12-Heterocycloalkylgruppe, substituierte oder unsubstituierte C6- bis C30-Arylgruppe oder substituierte oder unsubstituierte C2- bis C30-Heteroarylgruppe, oder sich zwei benachbarte R₃ zu den folgenden spirocyclischen Strukturen verbinden: 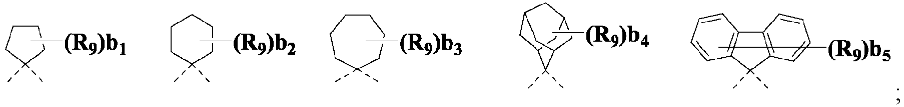
wobei R₉ unabhängig aus einer beliebigen Gruppe von Wasserstoff, Deuterium, Tritium, Cyano, Nitro, Halogen, substituierter oder unsubstituierter C1- bis C12-Alkylgruppe, substituierter oder unsubstituierter C1- bis C12-Alkoxygruppe, substituierter oder unsubstituierter Silylgruppe, substituierter oder unsubstituierter C3- bis C12-Cycloalkylgruppe, substituierter oder unsubstituierter C2- bis C12-Heterocycloalkylgruppe, substituierter oder unsubstituierter C6- bis C30-Arylgruppe oder substituierter oder unsubstituierter C2- bis C30-Heteroarylgruppe ausgewählt ist;
wobei b₁ aus 0, 1, 2, 3, 4, 5, 6, 7 oder 8 ausgewählt ist, b₂ aus 0, 1, 2, 3, 4, 5, 6, 7, 8, 9 oder 10 ausgewählt ist, b₃ aus 0, 1, 2, 3, 4, 5, 6, 7, 8, 9, 10, 11 oder 12 ausgewählt ist, und b₄ aus 0, 1, 2, 3, 4, 5, 6, 7, 8, 9, 10, 11, 12, 13 oder 14 ausgewählt ist;
wobei R₄ und R₈ unabhängig voneinander aus einer beliebigen Gruppe von substituierter oder unsubstituierter C1- bis C12-Alkylgruppe, substituierter oder unsubstituierter C3- bis C12-Cycloalkylgruppe, substituierter oder unsubstituierter Silylgruppe, substituierter oder unsubstituierter C2- bis C12-Heterocycloalkylgruppe, substituierter oder unsubstituierter C6-bis C30-Arylgruppe oder substituierter oder unsubstituierter C2- bis C30-Heteroarylgruppe ausgewählt sind;
wobei R₅ und R₆ unabhängig voneinander aus einer beliebigen Gruppe von Wasserstoff, Deuterium, Tritium, Cyano, Nitro, Halogen, substituierter oder unsubstituierter C1- bis C12-Alkylgruppe, substituierter oder unsubstituierter C1- bis C12-Alkoxygruppe, substituierter oder unsubstituierter Silylgruppe, substituierter oder unsubstituierter C3- bis C12-Cycloalkylgruppe, substituierter oder unsubstituierter C2- bis C12-Heterocycloalkylgruppe, substituierter oder unsubstituierter C6- bis C30-Arylgruppe oder substituierter oder unsubstituierter C2- bis C30-Heteroarylgruppe ausgewählt sind; und
wobei a₁ unabhängig aus 0, 1, 2, 3, 4 oder 5 ausgewählt ist, a₂ unabhängig aus 0, 1, 2, 3 oder 4 ausgewählt ist, a₃ unabhängig aus 0, 1, 2 oder 3 ausgewählt ist, a₄ unabhängig aus 0, 1 oder 2 ausgewählt ist, as unabhängig aus 0, 1, 2, 3, 4, 5, 6 oder 7 ausgewählt ist, a₆ unabhängig aus 0, 1, 2, 3, 4, 5, 6, 7, 8, 9 oder 10 ausgewählt ist, a₇ unabhängig aus 0, 1 oder 2 ausgewählt ist, as unabhängig aus 0, 1, 2, 3, 4, 5, 6, 7 oder 8 ausgewählt ist, und a₉ unabhängig aus 0, 1, 2, 3, 4, 5 oder 6 ausgewählt ist.

5. Heterocyclische Verbindung nach Anspruch 1, wobei Ra unabhängig aus einer beliebigen Gruppe von Wasserstoff, Deuterium, Cyano, Nitro, Fluor, Chlor, Brom, Iod, Methyl, Ethyl, n-Propyl, Isopropyl, n-Butyl, Isobutyl, tert-Butyl, Cyclopropyl, Cyclobutyl, Cyclopentyl, Cyclohexyl, Cycloheptyl, Adamantyl, Norbornyl, Trimethylsilyl, Triethylsilyl, Tri-tert-butylsilyl, deuteriertem Trimethylsilyl, deuteriertem Triethylsilyl, deuteriertem Tri-tert-butylsilyl, deuteriertem Methyl, deuteriertem Ethyl, deuteriertem n-Propyl, deuteriertem Isopropyl, deuteriertem n-Butyl, deuteriertem Isobutyl, deuteriertem tert-Butyl, deuteriertem Adamantyl, deuteriertem Norbornyl, fluor-substituiertem Methyl, fluor-substituiertem Ethyl, fluor-substituiertem Isopropyl, fluor-substituiertem tert-Butyl, methyl-substituiertem Adamantyl oder methyl-substituiertem Norbornyl ausgewählt ist oder aus der durch Formel III dargestellten Gruppe ausgewählt ist, und wobei, wenn zwei oder mehr Ra vorhanden sind, sich zwei benachbarte Ra zu einem substituierten oder unsubstituierten Benzolring verbinden können.

6. Heterocyclische Verbindung nach Anspruch 1, wobei Formel III aus einer beliebigen der folgenden Gruppen ausgewählt ist:

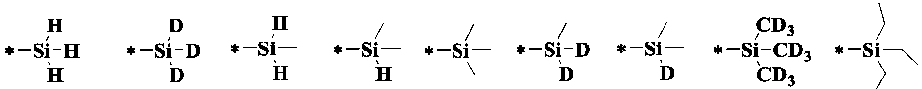

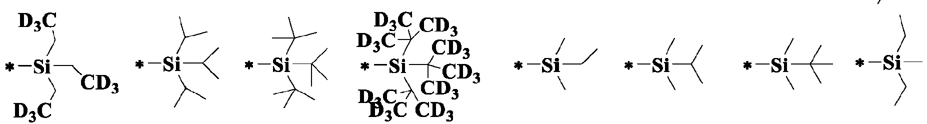

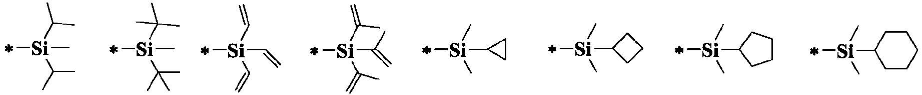

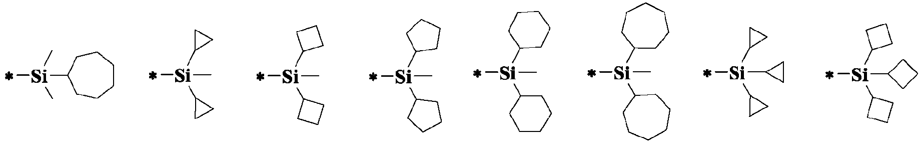

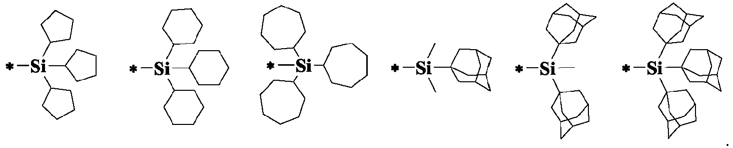

7. Heterocyclische Verbindung nach Anspruch 1, wobei L₁ und L₂ unabhängig voneinander aus einer Einfachbindung oder der folgenden Gruppe ausgewählt sind: 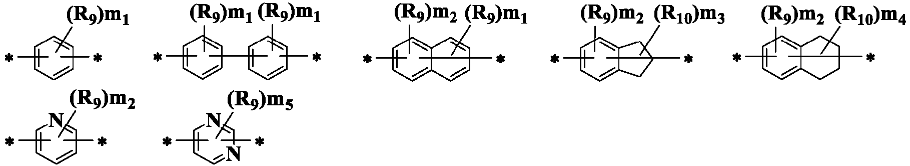
wobei R₉ unabhängig aus einer beliebigen Gruppe von Wasserstoff, Deuterium, Cyano, Nitro, Halogen, substituierter oder unsubstituierter C1- bis C12-Alkylgruppe, substituierter oder unsubstituierter C1- bis C12-Alkoxygruppe, substituierter oder unsubstituierter C3- bis C12-Cycloalkylgruppe, substituierter oder unsubstituierter Silylgruppe, substituierter oder unsubstituierter C2- bis C12-Heterocycloalkylgruppe, substituierter oder unsubstituierter C6-bis C30-Arylgruppe oder substituierter oder unsubstituierter C2- bis C30-Heteroarylgruppe ausgewählt ist; und
wobei m₁ unabhängig aus 0, 1, 2, 3 oder 4 ausgewählt ist, m₂ unabhängig aus 0, 1, 2 oder 3 ausgewählt ist, m₃ unabhängig aus 0, 1, 2, 3, 4, 5 oder 6 ausgewählt ist, m₄ unabhängig aus 0, 1, 2, 3, 4, 5, 6, 7 oder 8 ausgewählt ist, und m₅ unabhängig aus 0, 1 oder 2 ausgewählt ist.

8. Heterocyclische Verbindung nach Anspruch 1, wobei die heterocyclische Verbindung aus einer beliebigen der folgenden Strukturen ausgewählt ist:

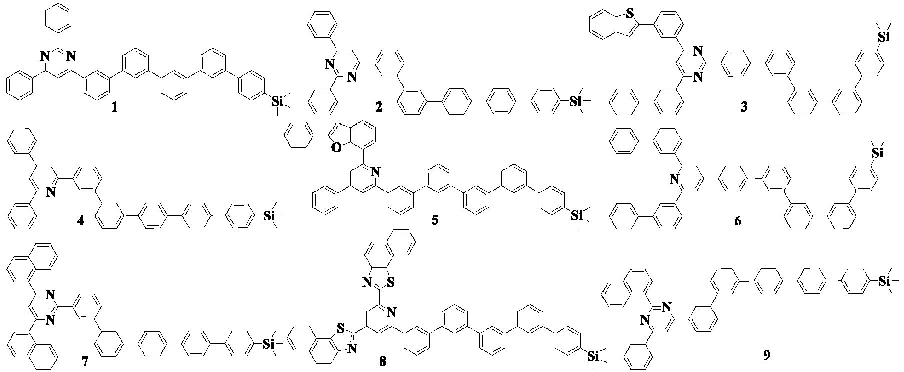

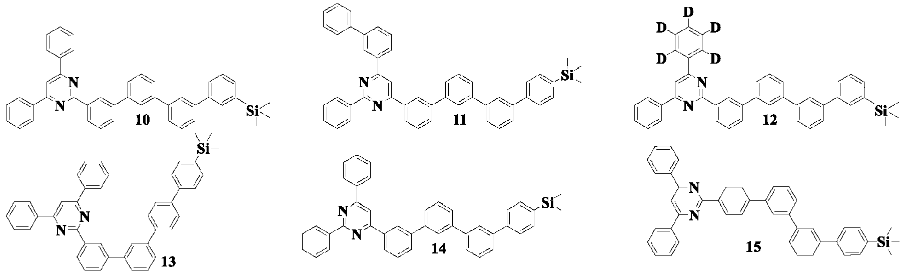

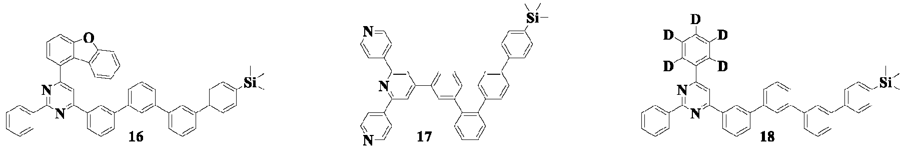

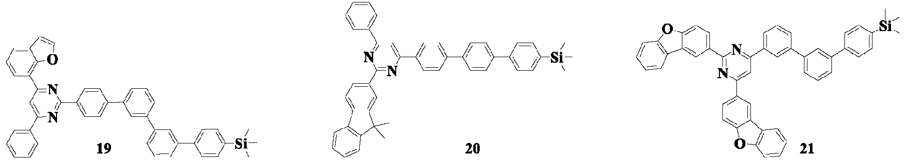

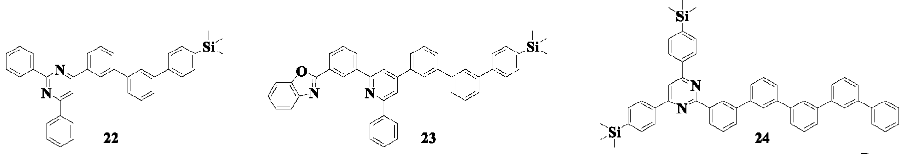

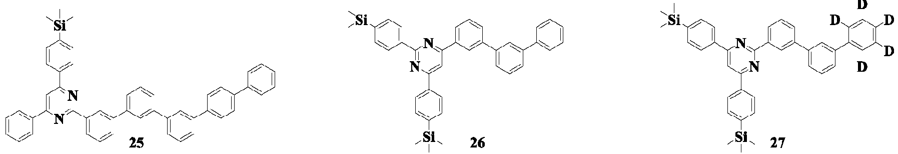

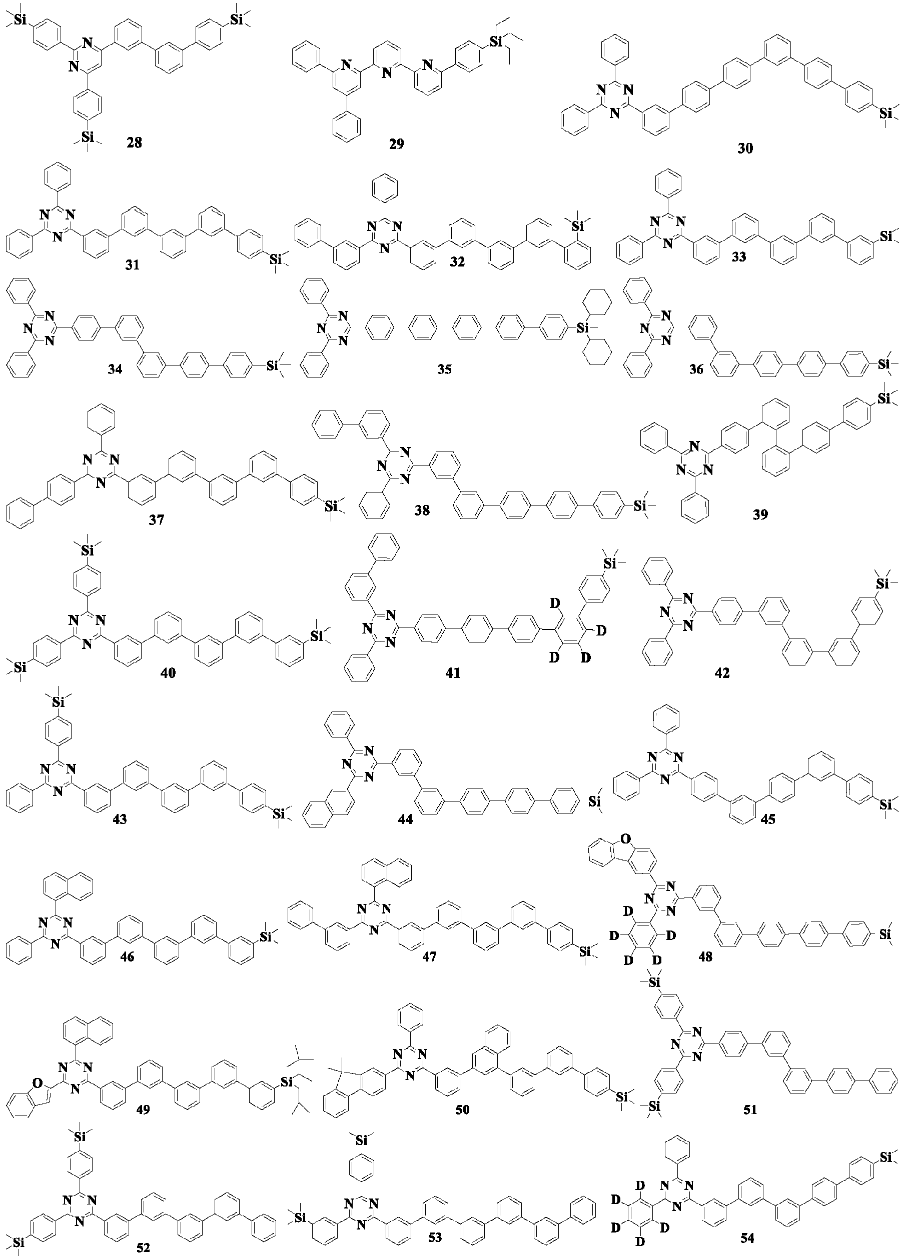

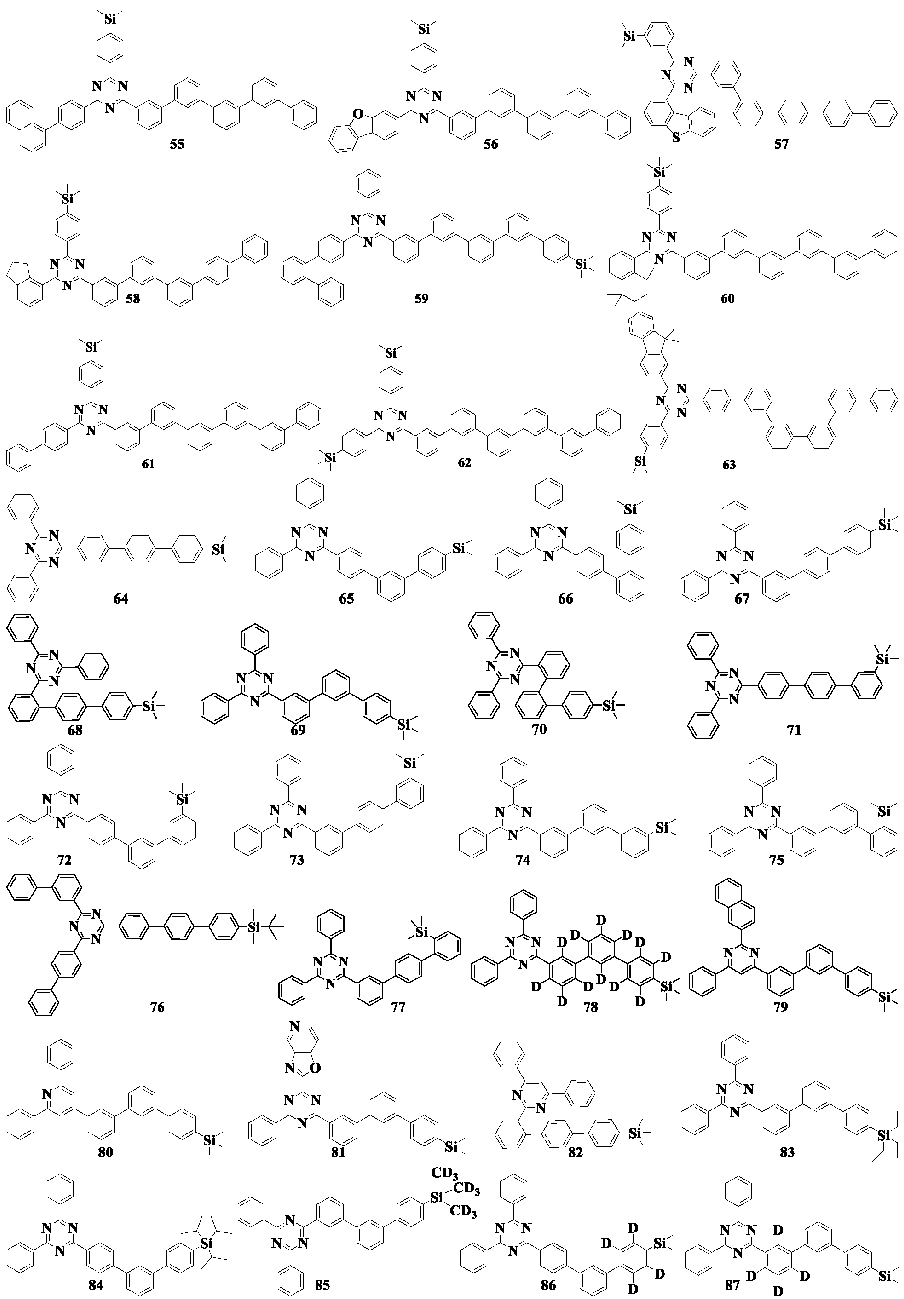

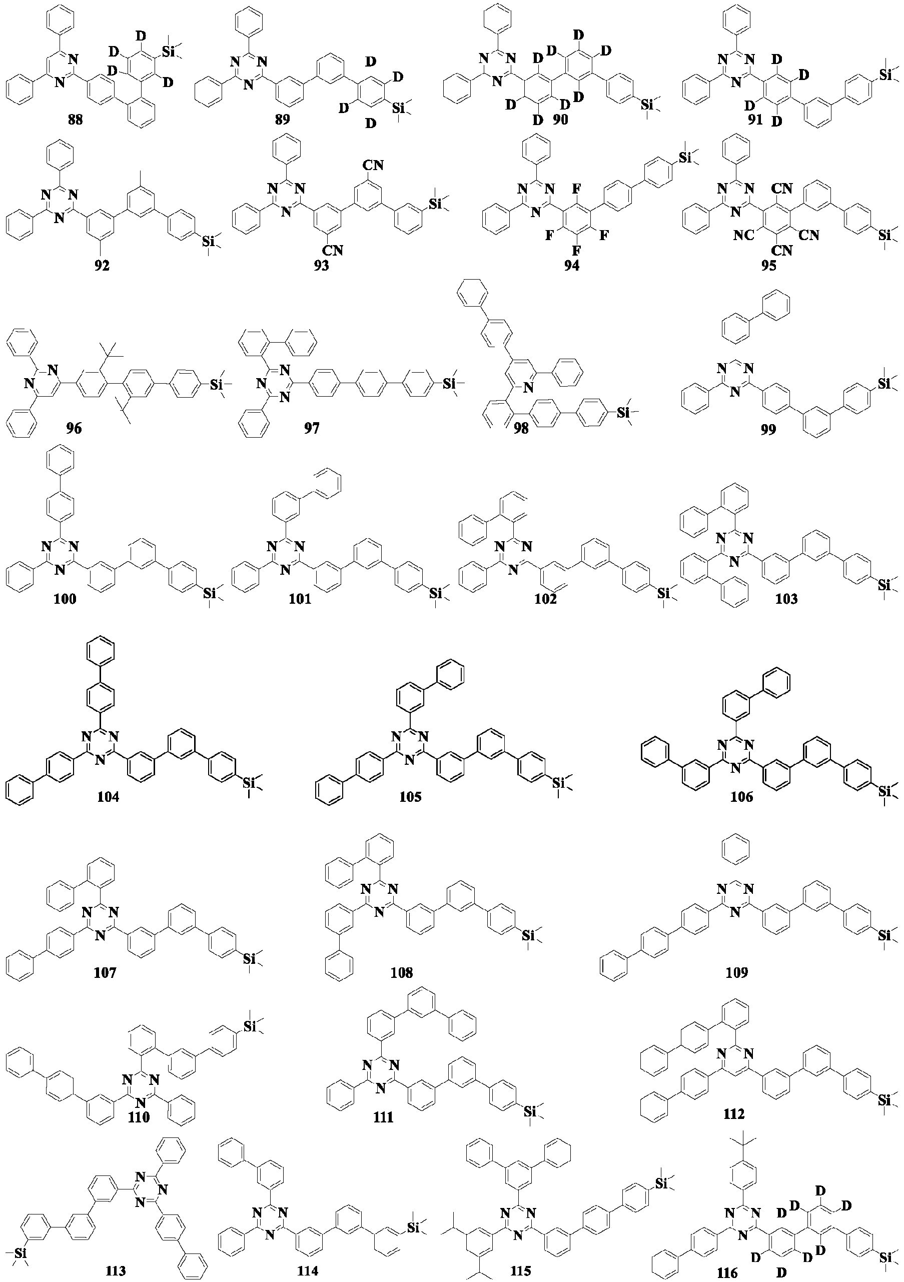

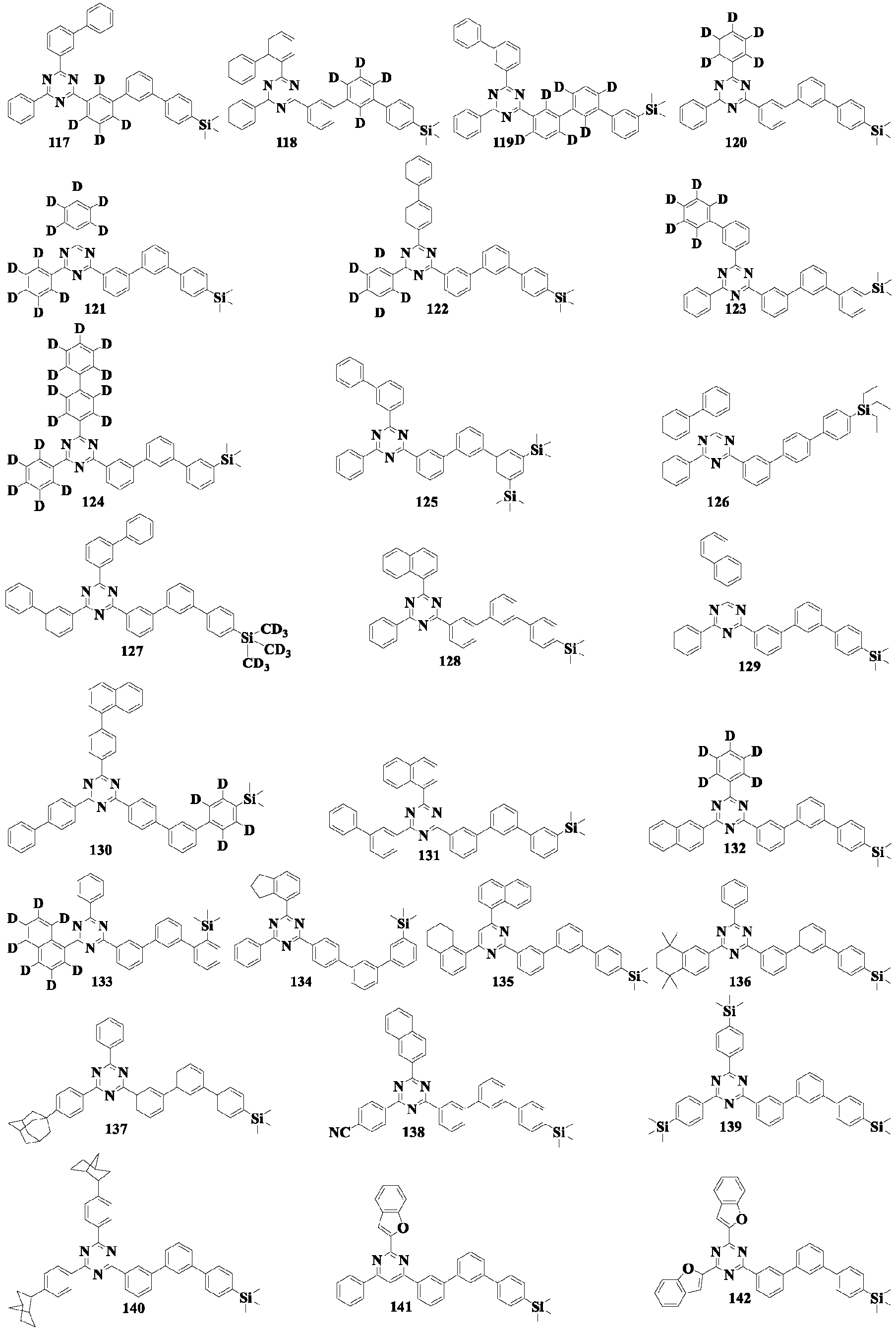

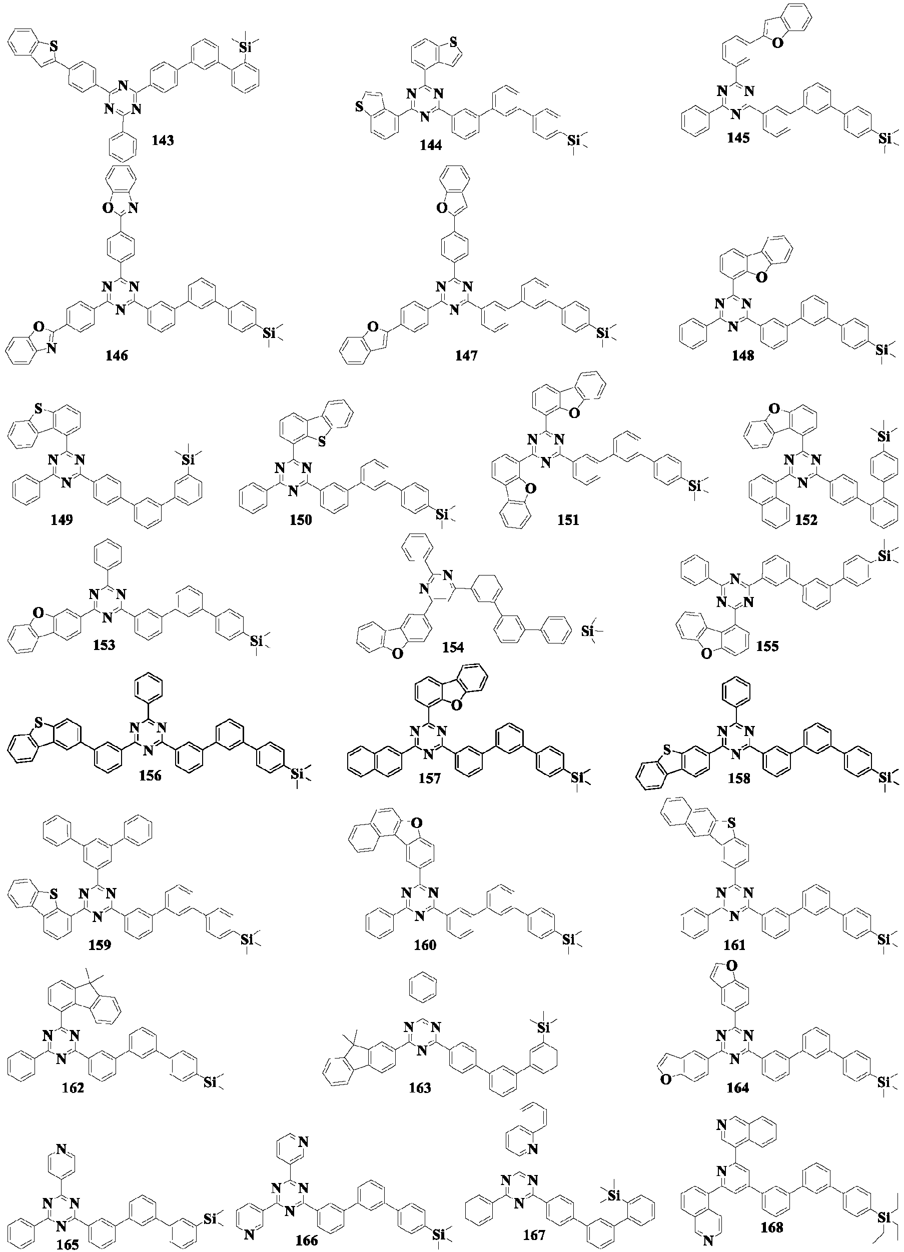

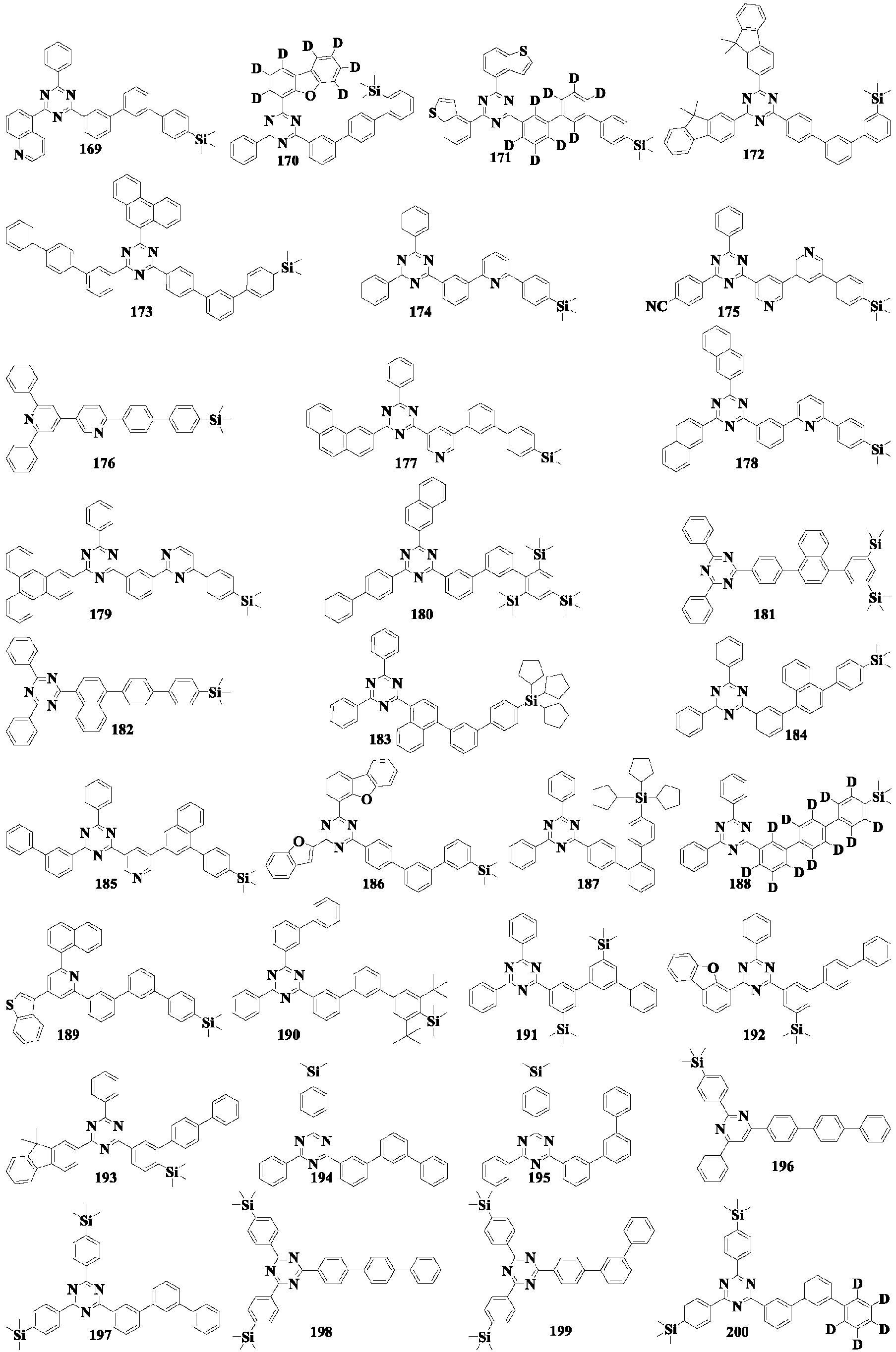

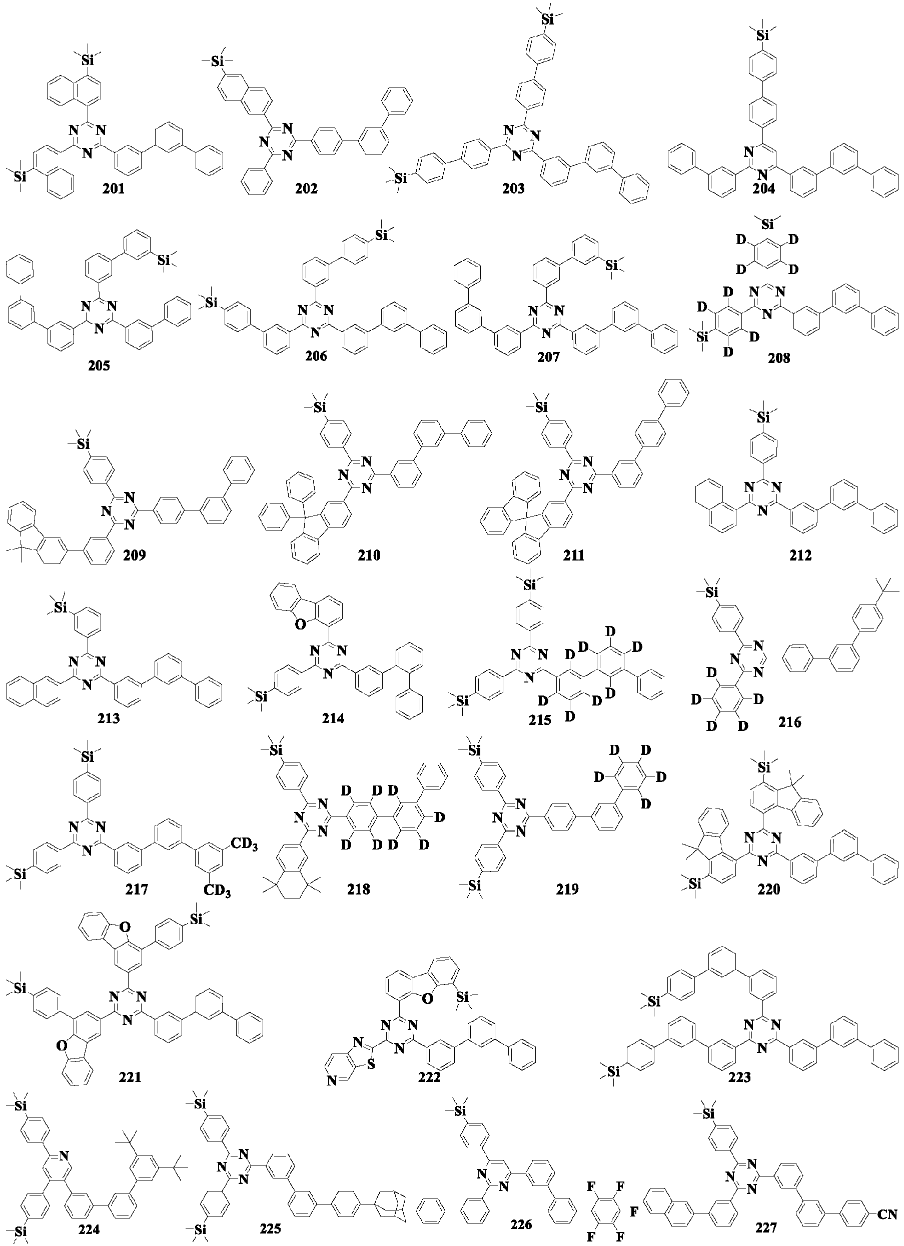

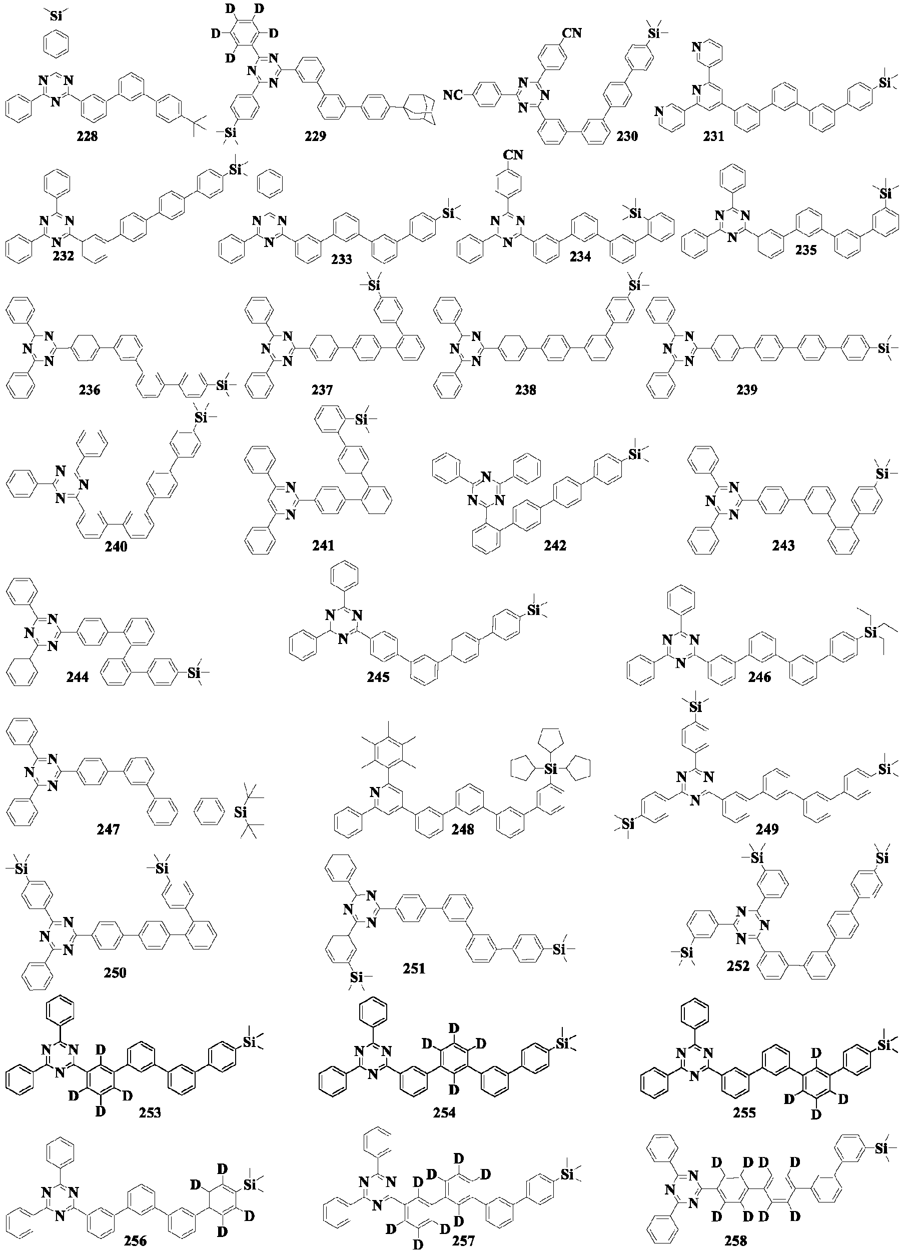

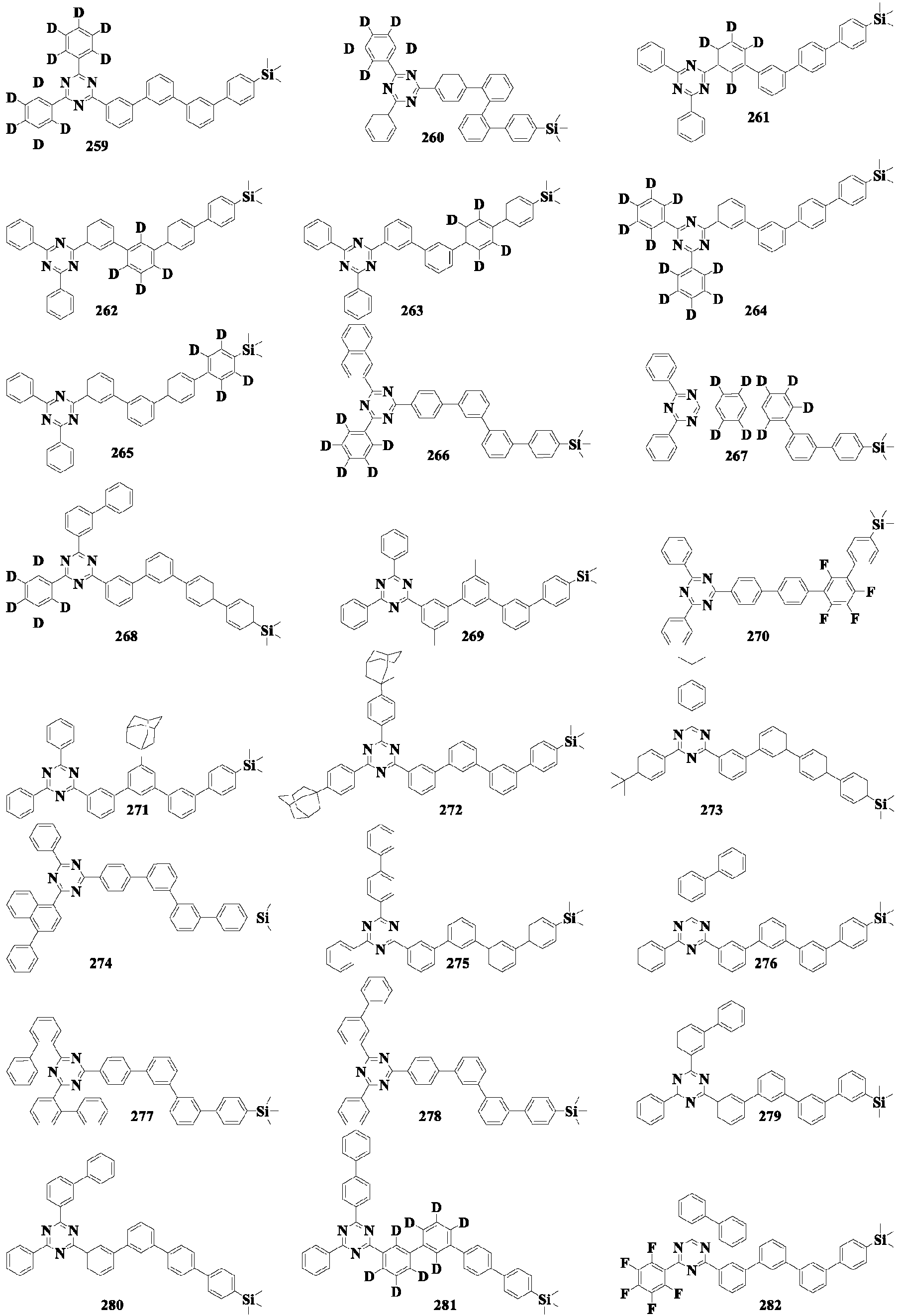

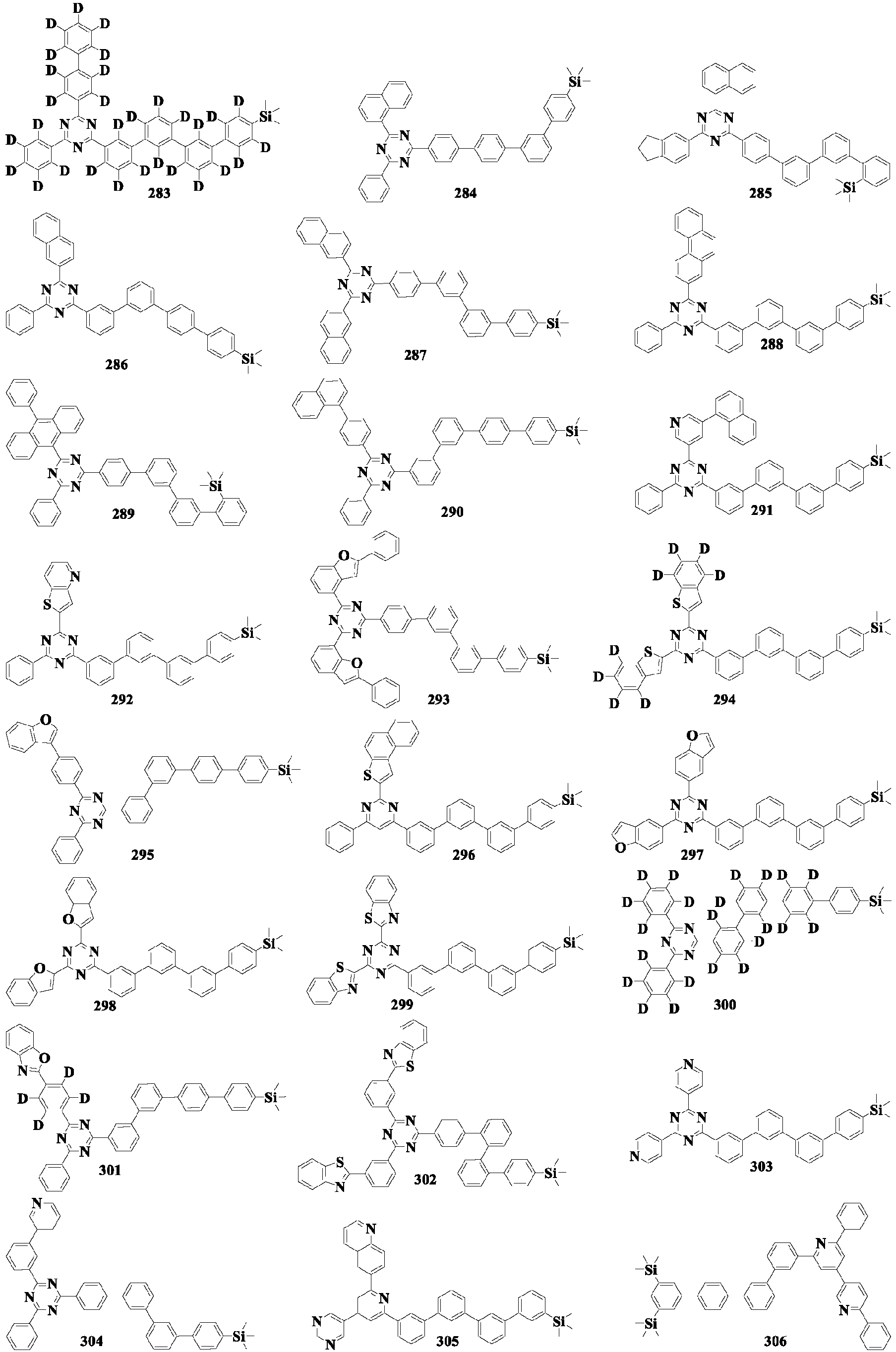

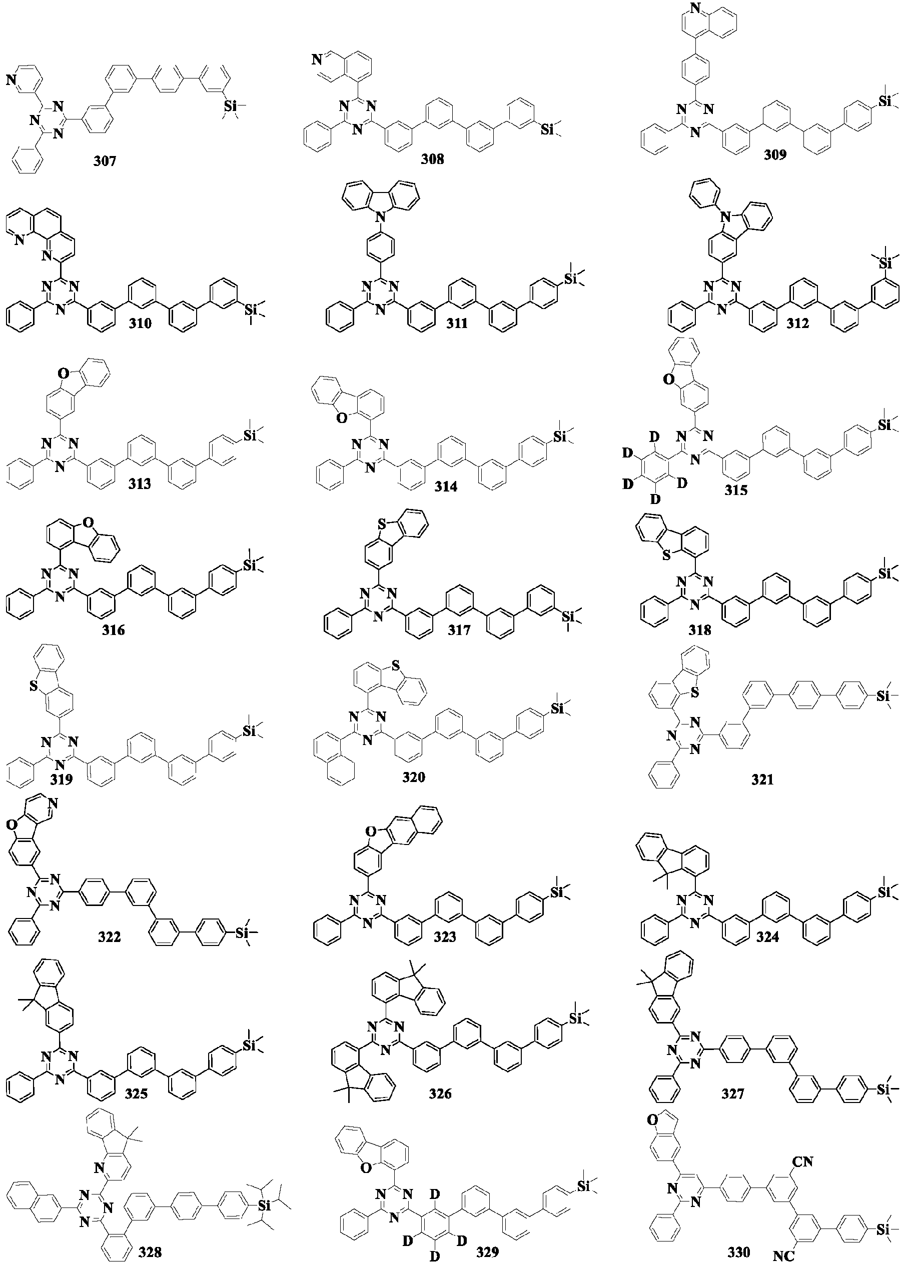

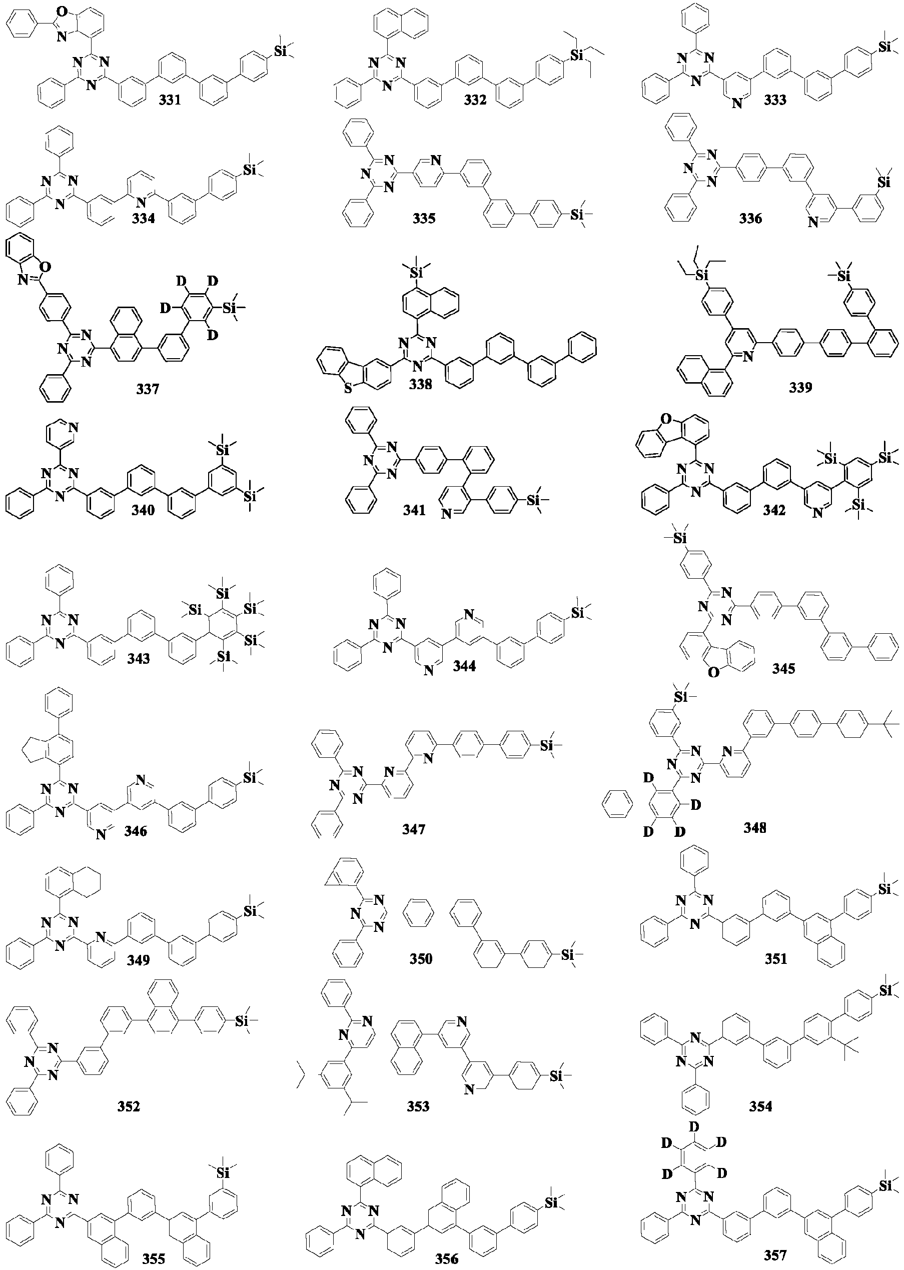

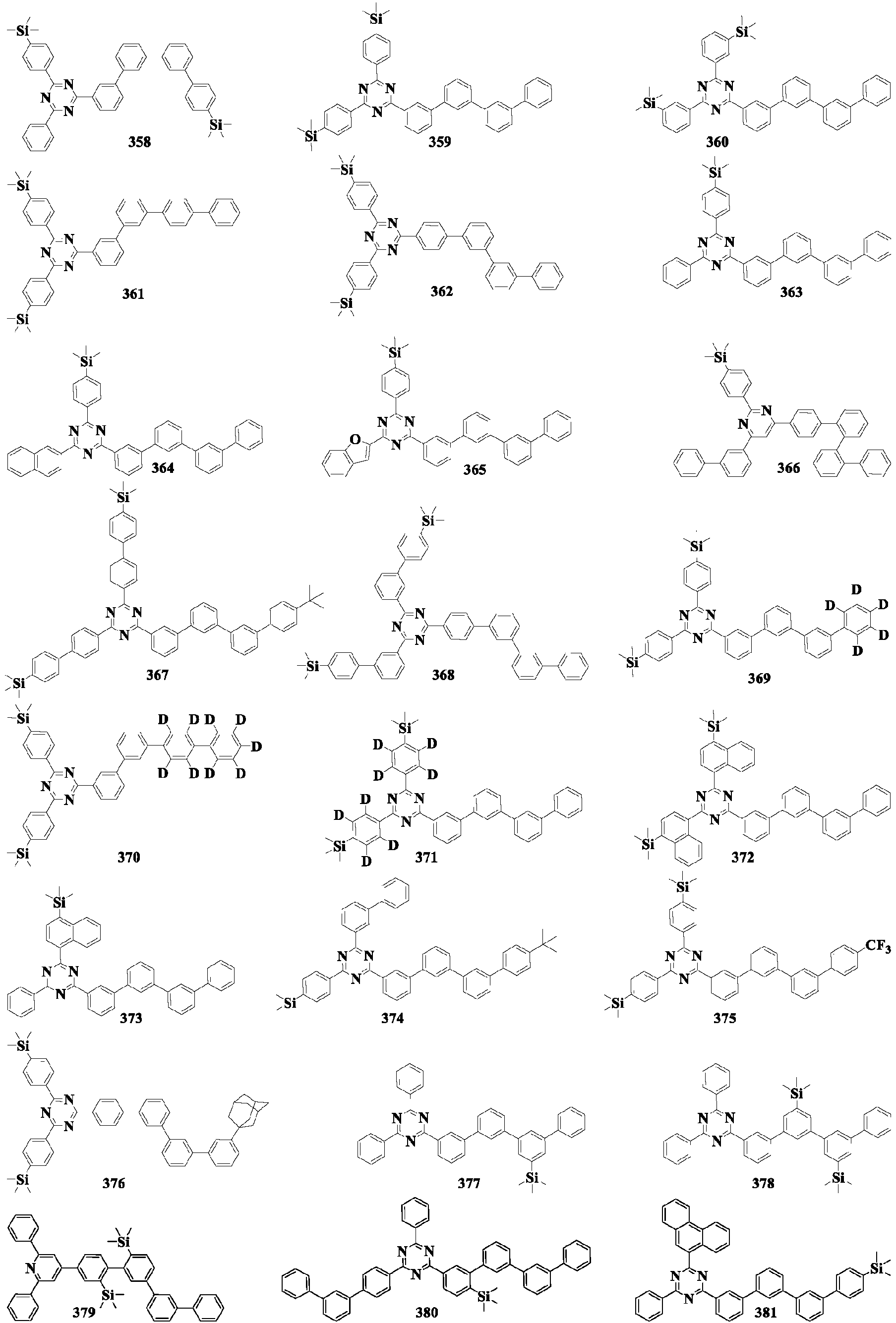

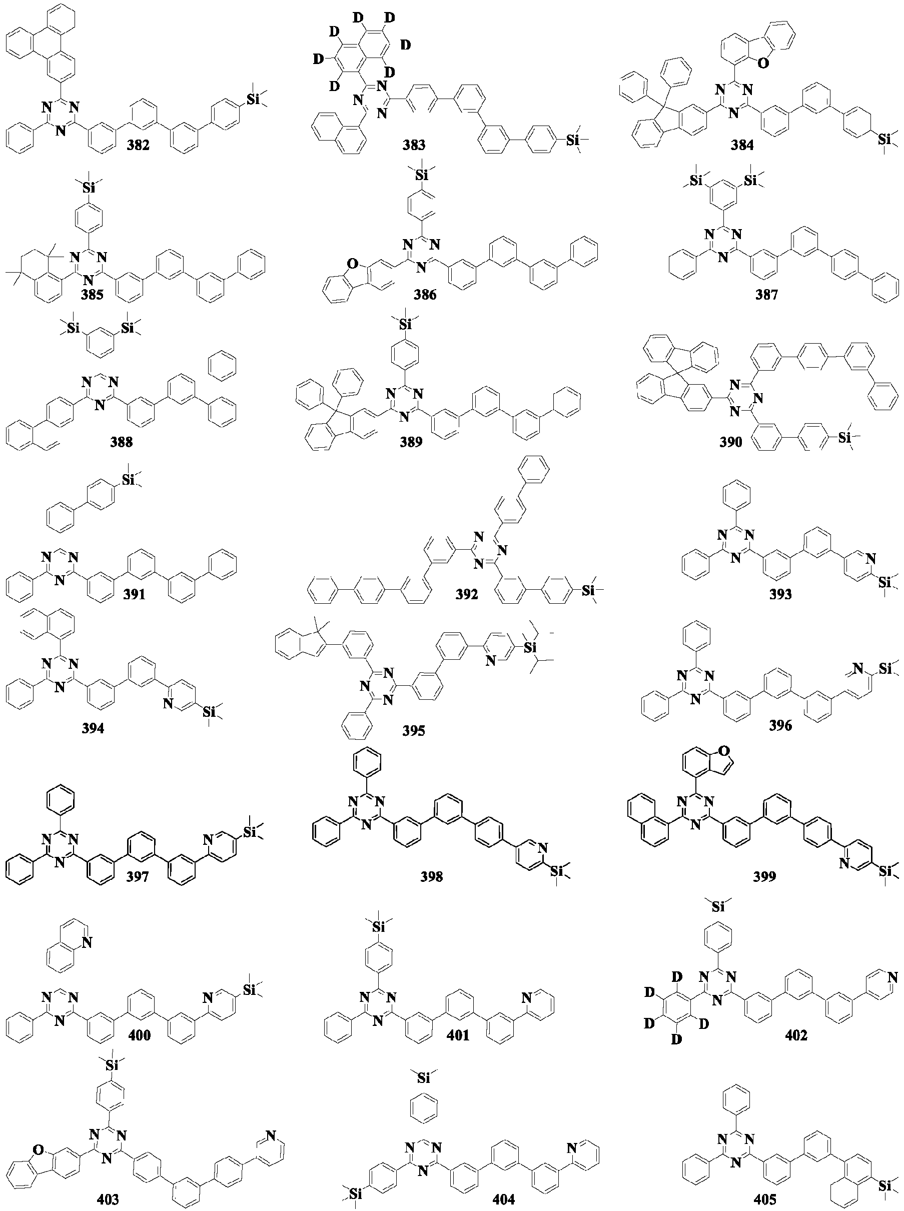

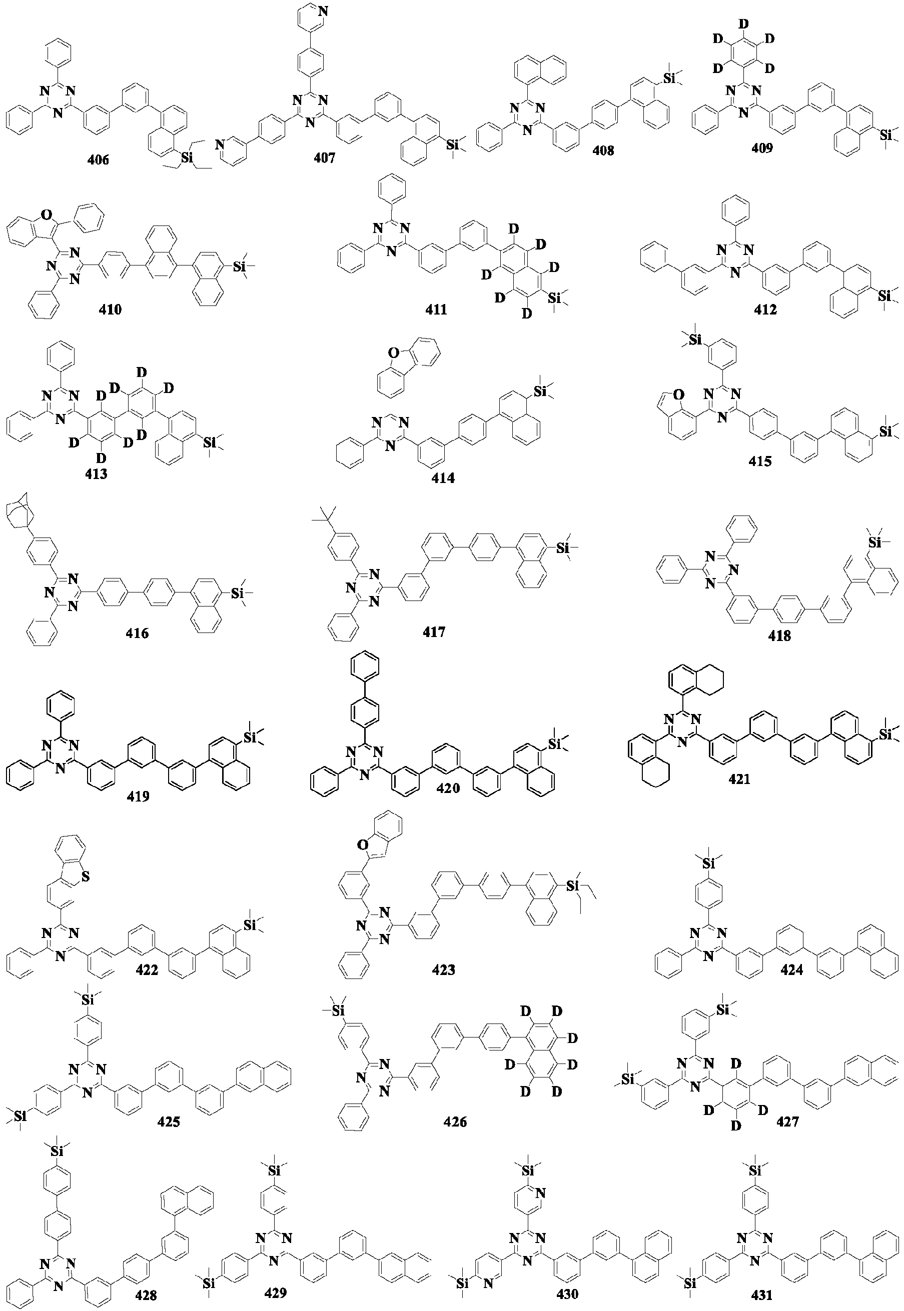

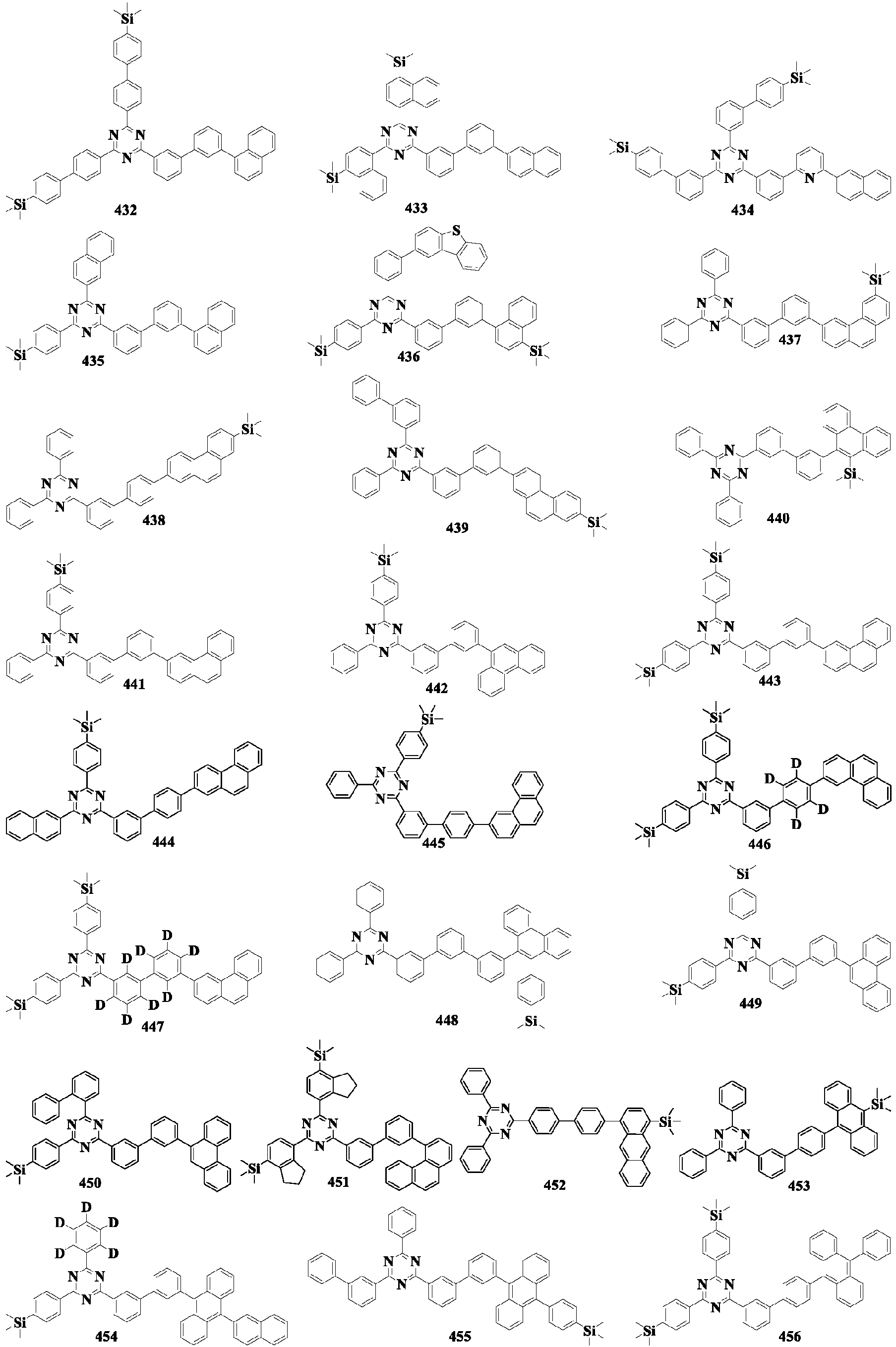

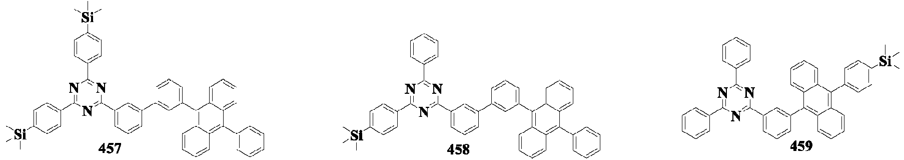

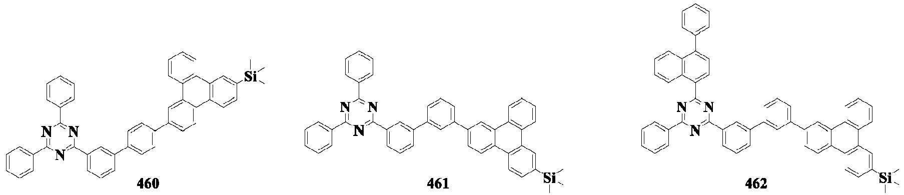

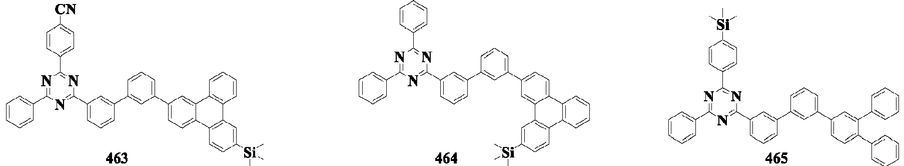

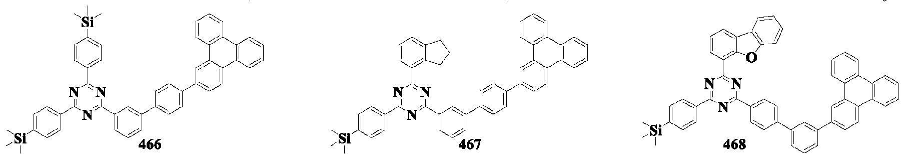

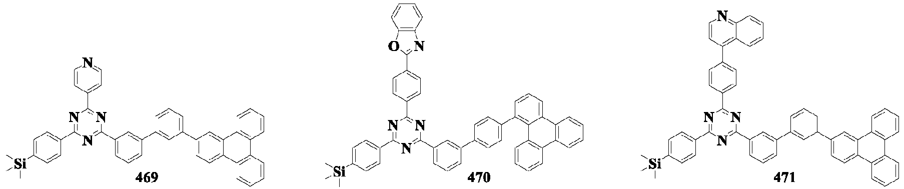

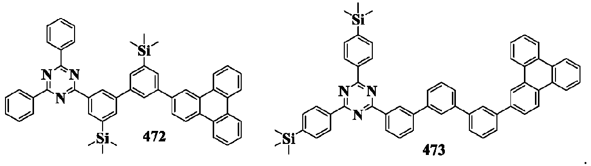

9. Organische elektrolumineszente Vorrichtung, umfassend eine Anode, eine Kathode, die der Anode gegenüberliegt, und eine organische Schicht, die zwischen der Anode und der Kathode oder außerhalb mindestens einer Elektrode der Anode oder der Kathode angeordnet, wobei die organische Schicht eine oder mehrere der heterocyclischen Verbindungen nach einem der Ansprüche 1 bis 8 umfasst.

10. Organische elektrolumineszente Vorrichtung nach Anspruch 9, wobei die organische Schicht zwischen der Anode und der Kathode angeordnet ist und mindestens eine der Schichten Emissionsschicht, Elektronentransportschicht oder Lochblockierschicht umfasst, wobei die mindestens eine Emissionsschicht, Elektronentransportschicht oder Lochblockierschicht eine oder mehrere der heterocyclischen Verbindungen nach einem der Ansprüche 1 bis 8 umfasst.

## Revendications

1. Composé hétérocyclique, dans lequel le composé hétérocyclique est doté de la structure représentée à la Formule I : 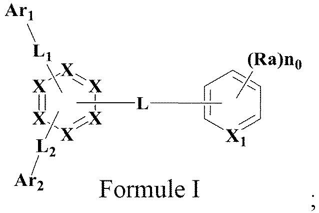
dans lequel dans la Formule I, X est sélectionné de façon indépendante à partir de C(R₂) ou d'un atome N, au moins un X est sélectionné à partir d'un atome N, et X lié à Ar₁, Ar₂ ou L est sélectionné à partir d'un atome C ;
X₁ est sélectionné de façon indépendante à partir d'un atome C ou d'un atome N ;
R₂ est sélectionné de façon indépendante à partir d'un élément quelconque parmi un hydrogène, un deutérium, un tritium, un halogène, un cyano, un nitro, un alkyle C1 à C12 substitué ou non substitué, un silyle substitué ou non substitué, un alcoxy C1 to C12 substitué ou non substitué, un cycloalkyle C3 à C12 substitué ou non substitué, un hétérocycloalkyle C2 à C12 substitué ou non substitué, un aryle C6 à C30 substitué ou non substitué ou un hétéroaryle C2 à C30 substitué ou non substitué ;
Ar₁ et Ar₂ sont sélectionnés de façon indépendante à partir d'un élément quelconque parmi un aryle C6 à C30 substitué ou non substitué ou un hétéroaryle C2 à C30 substitué ou non substitué ;
L₁ et L₂ sont sélectionnés de façon indépendante à partir d'un élément quelconque parmi une liaison simple, un arylène C6 à C30 substitué ou non substitué ou un hétéroarylène C2 à C30 substitué ou non substitué ;
Ra est sélectionné de façon indépendante à partir d'un élément quelconque parmi un hydrogène, un deutérium, un halogène, un cyano, un nitro, un alkyle C1 à C12 substitué ou non substitué, un silyle substitué ou non substitué, un alcoxy C1 to C12 substitué ou non substitué, un cycloalkyle C3 à C12 substitué ou non substitué, un hétérocycloalkyle C2 à C12 substitué ou non substitué ;
n₀ est sélectionné de façon indépendante à partir de 0, 1, 2, 3, 4 ou 5, lorsque n₀ est supérieur à 1, deux Ra ou plus sont identiques ou différents l'un de l'autre, ou deux Ra adjacents sont combinés pour former un cycle benzénique substitué ou non substitué ;
L est sélectionné de façon indépendante à partir de l'un quelconque des groupes suivants : 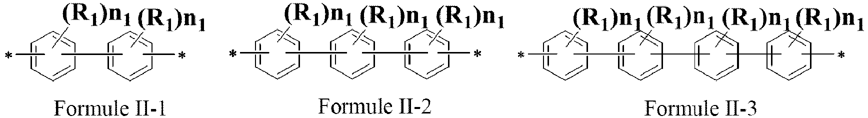 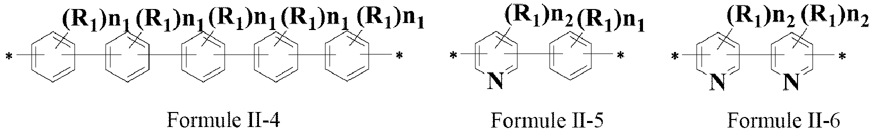 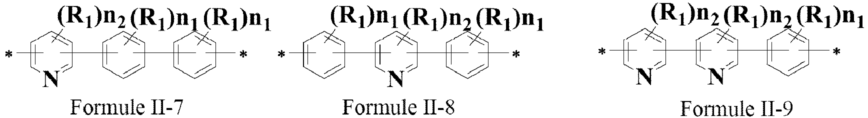 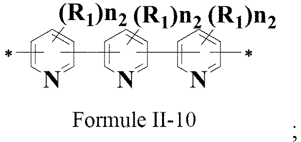
dans lequel R₁ est sélectionné de façon indépendante à partir d'un élément quelconque parmi un hydrogène, un deutérium, un halogène, un cyano, un nitro, un alkyle C1 à C12 substitué ou non substitué, un silyle substitué ou non substitué, un alcoxy C1 to C12 substitué ou non substitué, un cycloalkyle C3 à C12 substitué ou non substitué, un hétérocycloalkyle C2 à C12 substitué ou non substitué ;
n₁ est sélectionné de façon indépendante à partir de 0, 1, 2, 3 ou 4 ; n₂ est sélectionné de façon indépendante à partir de 0, 1, 2 ou 3 ; lorsque n1 est supérieur à 1, deux R₁ ou plus sont identiques ou différents l'un de l'autre, ou deux R₁ adjacents sont combinés pour former un cycle benzénique substitué ou non substitué ;
sous réserve qu'un hydrogène ou plus dans au moins un groupe de Ar₁, Ar₂, L, L₁, L₂, R₂ ou Ra soit substitué avec un groupe représenté par la Formule III : 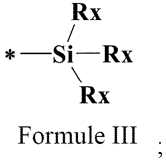 Rx est sélectionné de façon indépendante à partir d'un élément quelconque parmi un hydrogène, un deutérium, un cyano, un nitro, un halogène, un alkyle C1 à C12 substitué ou non substitué, un alkényle C2 à C12 substitué ou non substitué, un cycloalkyle C3 à C12 substitué ou non substitué, un hétérocycloalkyle C2 à C12 substitué ou non substitué ; et
le composé suivant est exclu du composé hétérocyclique :

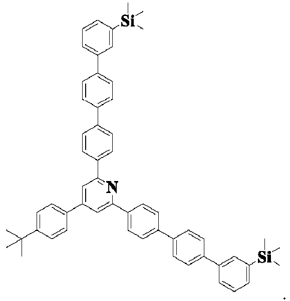

2. Composé hétérocyclique selon la revendication 1, dans lequel le composé hétérocyclique dans la Formule I est sélectionné à partir de l'une quelconque des structures représentées par les Formules III-1 jusqu'à III-5 suivantes : 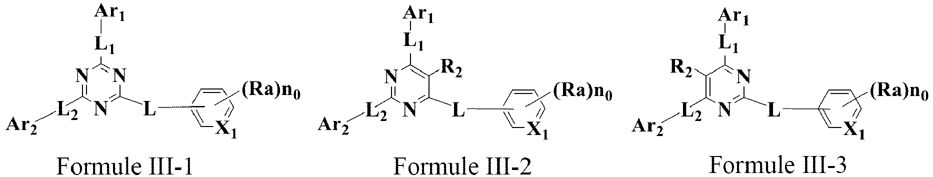 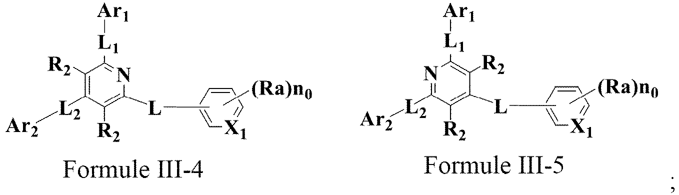 dans lequel les définitions de Ar₁, Ar₂, R₂, L, L₁, L₂, X₁, Ra et n₀ sont identiques à celles dans la Formule I.

3. Composé hétérocyclique selon la revendication 1, dans lequel Ar₁ et Ar₂ sont sélectionnés de façon indépendante à partir d'un élément quelconque parmi les groupes suivants : 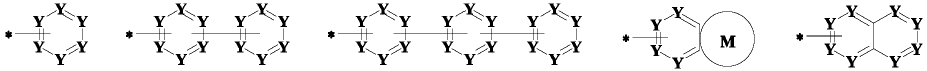 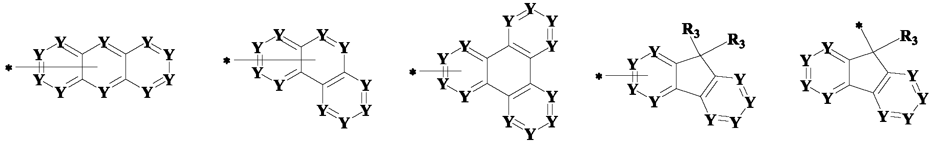 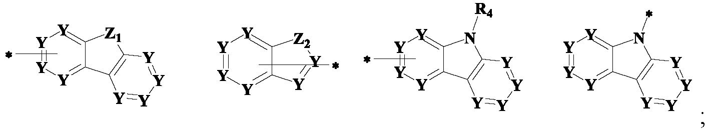
dans lequel Y est sélectionné de façon indépendante à partir de C(R₅) ou un atome N, et Y sur un site de liaison est sélectionné à partir d'un atome C ;
le cycle M est sélectionné à partir d'un cycle aliphatique C3 à C7 non substitué ou un cycle aliphatique C3 à C7 substitué avec un R₆ ou plus ;
Z₁ est sélectionné à partir d'un atome O ou un atome S, et Z₂ est sélectionné à partir de O, S, C(R₇)₂ ou N(R₈) ;
R₃ et R₇ sont sélectionnés de façon indépendante à partir d'un élément quelconque parmi un hydrogène, un deutérium, un alkyle C1 à C12 substitué ou non substitué, un alcoxy C1 à C12 substitué ou non substitué, un cycloalkyle C3 à C12 substitué ou non substitué, un silyle substitué ou non substitué, un hétérocycloalkyle C2 à C12 substitué ou non substitué, un aryle C6 à C30 substitué ou non substitué ou un hétéroaryle C2 à C30 substitué ou non substitué, ou deux R₃ adjacents sont combinés pour former une structure spirocyclique substituée ou non substituée ;
R₄ et R₈ sont sélectionnés de façon indépendante à partir d'un élément quelconque parmi un alkyle C1 à C12 substitué ou non substitué, un cycloalkyle C3 à C12 substitué ou non substitué, un silyle substitué ou non substitué, un hétérocycloalkyle C2 à C12 substitué ou non substitué, un aryle C6 à C30 substitué ou non substitué ou un hétéroaryle C2 à C30 substitué ou non substitué ; et
R₅ et R₆ sont sélectionnés de façon indépendante à partir d'un élément quelconque parmi un hydrogène, un deutérium, un tritium, un cyano, un nitro, un halogène, un alkyle C1 à C12 substitué ou non substitué, un alcoxy C1 à C12 substitué ou non substitué, un silyle substitué ou non substitué, un cycloalkyle C3 à C12 substitué ou non substitué, un hétérocycloalkyle C2 à C12 substitué ou non substitué, un aryle C6 à C30 substitué ou non substitué ou un hétéroaryle C2 à C30 substitué ou non substitué, ou deux R₅ adjacents sont combinés pour former un cycle substitué ou non substitué.

4. Composé hétérocyclique selon la revendication 1, dans lequel Ar₁ et Ar₂ sont sélectionnés de façon indépendante à partir de l'un quelconque des groupes suivants : 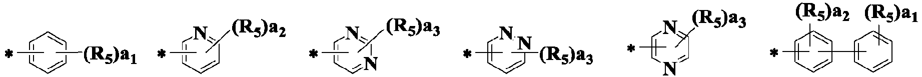 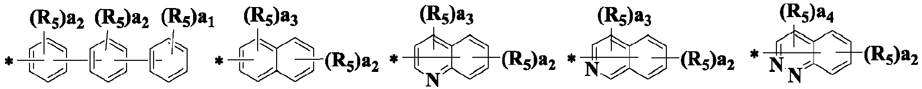 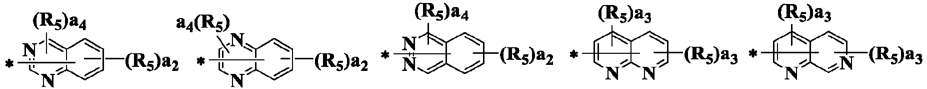 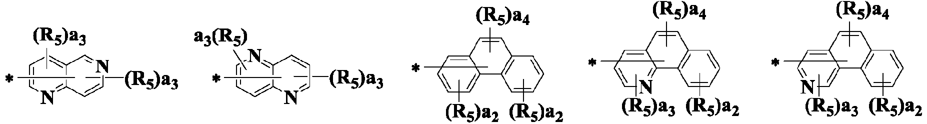 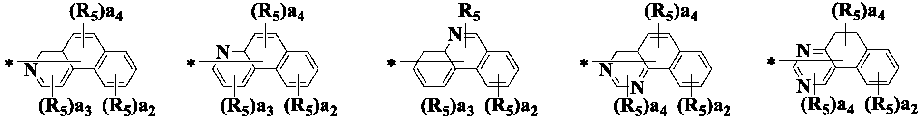 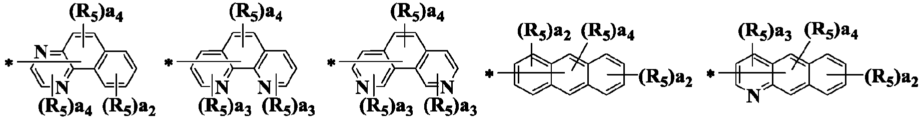 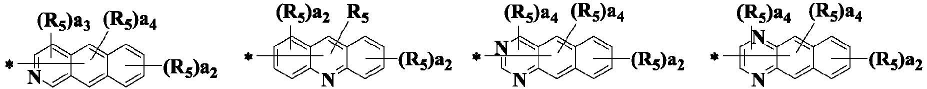 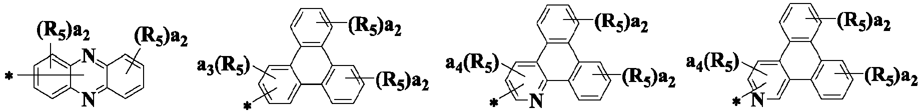 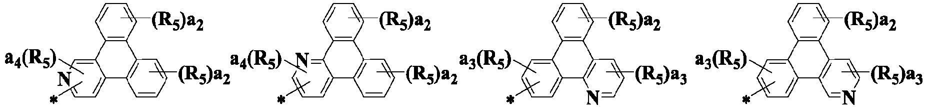 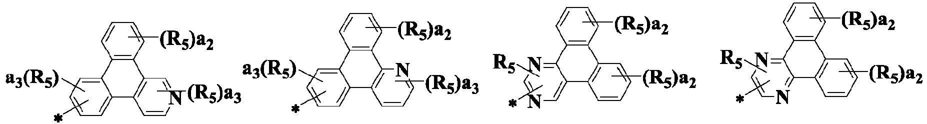 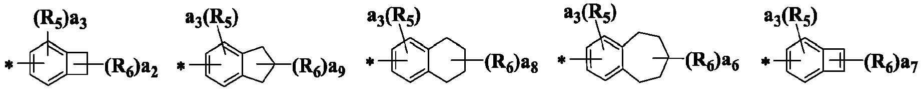 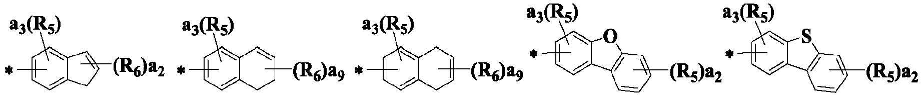 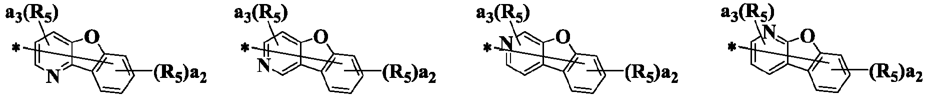 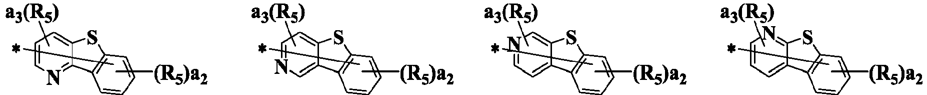 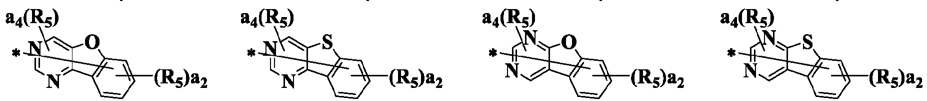 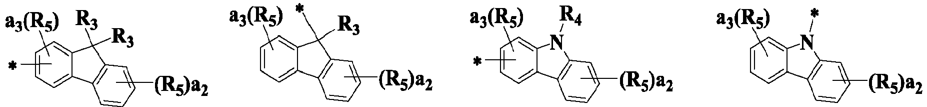 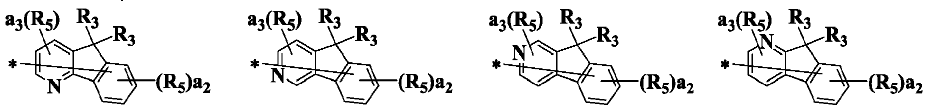 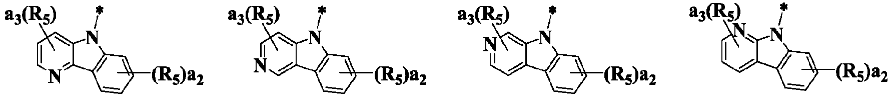 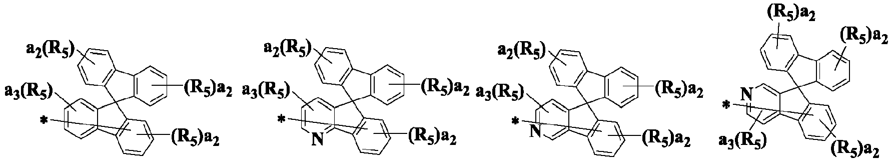 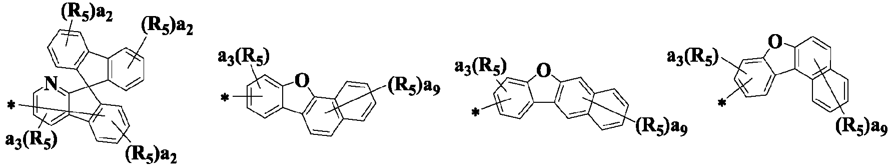 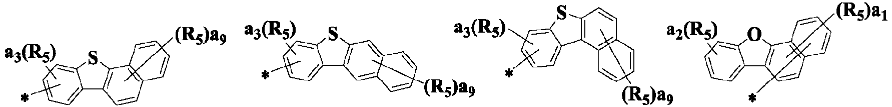 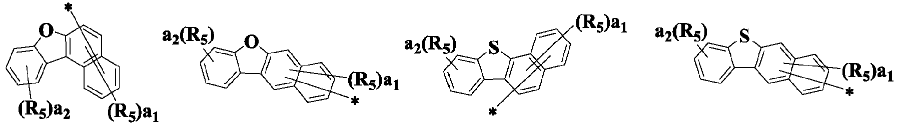 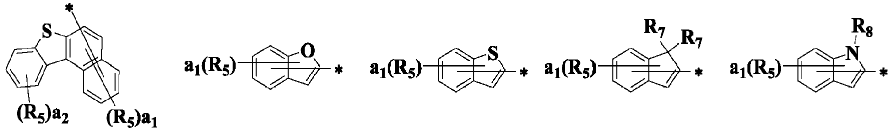 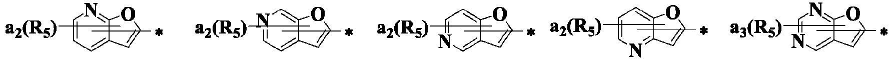 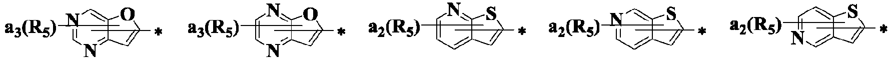 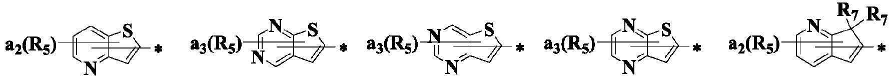 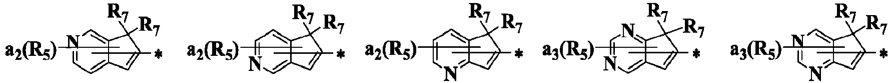 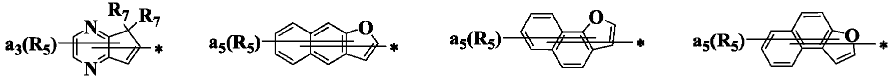 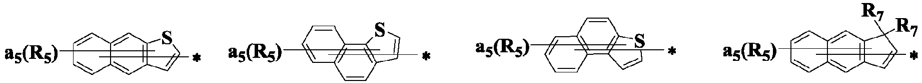 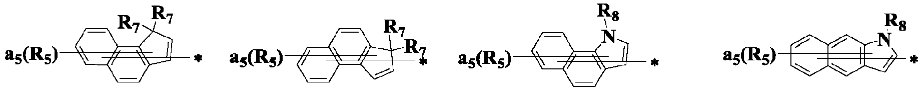 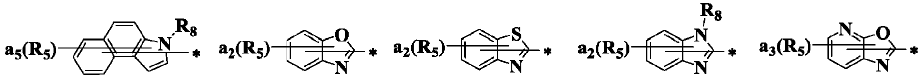 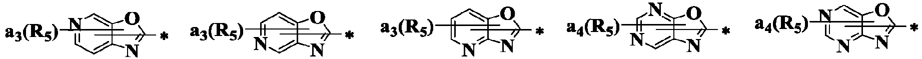 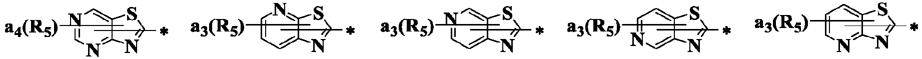 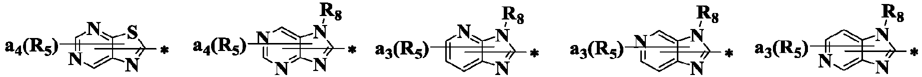 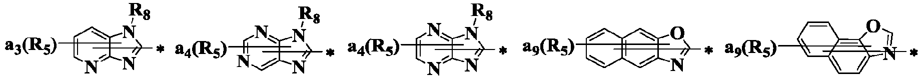 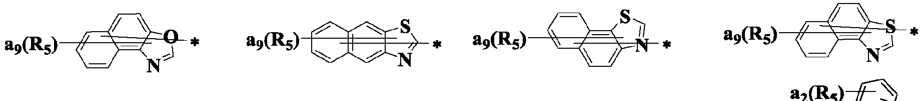 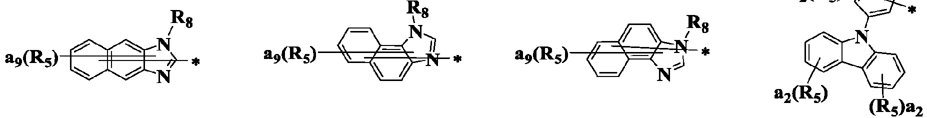 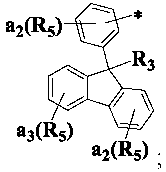
dans lequel R₃ et R₇ sont sélectionnés de façon indépendante à partir d'un élément quelconque parmi un hydrogène, un alkyle C1 à C12 substitué ou non substitué, un alcoxy C1 à C12 substitué ou non substitué, un cycloalkyle C3 à C12 substitué ou non substitué, un hétérocycloalkyle C2 à C12 substitué ou non substitué, un aryle C6 à C30 substitué ou non substitué ou un hétéroaryle C2 à C30 substitué ou non substitué, ou deux R₃ adjacents sont combinés pour former les structures spirocycliques suivantes : 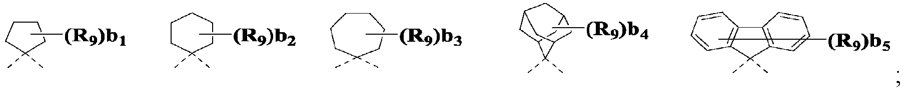
dans lesquelles R₉ est sélectionné de façon indépendante à partir d'un élément quelconque parmi un hydrogène, un deutérium, un tritium, un cyano, un nitro, un halogène, un alkyle C1 à C12 substitué ou non substitué, un alcoxy C1 à C12 substitué ou non substitué, un silyle substitué ou non substitué, un cycloalkyle C3 à C12 substitué ou non substitué, un hétérocycloalkyle C2 à C12 substitué ou non substitué, un aryle C6 à C30 substitué ou non substitué ou un hétéroaryle C2 à C30 substitué ou non substitué ;
b₁ est sélectionné à partir de 0, 1, 2, 3, 4, 5, 6, 7 ou 8, b₂ est sélectionné à partir de 0, 1, 2, 3, 4, 5, 6, 7, 8, 9 ou 10, b₃ est sélectionné à partir de 0, 1, 2, 3, 4, 5, 6, 7, 8, 9, 10, 11 ou 12, et b₄ est sélectionné à partir de 0, 1, 2, 3, 4, 5, 6, 7, 8, 9, 10, 11, 12, 13 ou 14 ;
R₄ et R₈ sont sélectionnés de façon indépendante à partir d'un élément quelconque parmi un alkyle C1 à C12 substitué ou non substitué, un cycloalkyle C3 à C12 substitué ou non substitué, un silyle substitué ou non substitué, un hétérocycloalkyle C2 à C12 substitué ou non substitué, un aryle C6 à C30 substitué ou non substitué ou un hétéroaryle C2 à C30 substitué ou non substitué ;
R₅ et R₆ est sélectionné de façon indépendante à partir d'un élément quelconque parmi un hydrogène, un deutérium, un tritium, un cyano, un nitro, un halogène, un alkyle C1 à C12 substitué ou non substitué, un alcoxy C1 à C12 substitué ou non substitué, un silyle substitué ou non substitué, un cycloalkyle C3 à C12 substitué ou non substitué, un hétérocycloalkyle C2 à C12 substitué ou non substitué, un aryle C6 à C30 substitué ou non substitué ou un hétéroaryle C2 à C30 substitué ou non substitué ; et
a₁ est sélectionné de façon indépendante à partir de 0, 1, 2, 3, 4 ou 5, a₂ est sélectionné de façon indépendante à partir de 0, 1, 2, 3 ou 4, a₃ est sélectionné de façon indépendante à partir de 0, 1, 2 ou 3, a₄ est sélectionné de façon indépendante à partir de 0, 1 ou 2, as est sélectionné de façon indépendante à partir de 0, 1, 2, 3, 4, 5, 6 ou 7, a₆ est sélectionné de façon indépendante à partir de 0, 1, 2, 3, 4, 5, 6, 7, 8, 9 ou 10, a₇ est sélectionné de façon indépendante à partir de 0, 1 ou 2, as est sélectionné de façon indépendante à partir de 0, 1, 2, 3, 4, 5, 6, 7 ou 8, et a₉ est sélectionné de façon indépendante à partir de 0, 1, 2, 3, 4, 5 ou 6.

5. Composé hétérocyclique selon la revendication 1, dans lequel Ra est sélectionné de façon indépendante à partir d'un élément quelconque parmi un hydrogène, un deutérium, un cyano, un nitro, un fluor, un chlore, un brome, un iode, un méthyle, éthyle, un *n*-propyle, un isopropyle, un *n*-butyle, un isobutyle, un *tert-butyle,* un cyclopropyle, un cyclobutyle, un cyclopentyle, un cyclohexyle, un cycloheptyle, un adamantyle, un norbornyle, un triméthylsilyle, un triéthylsilyle, un tri-*tert*-butylsilyle, un triméthylsilyle deutéré, un triéthylsilyle deutéré, un tri-*tert*-butylsilyle deutéré, un méthyle deutéré, un éthyle deutéré, un *n*-propyle deutéré, un isopropyle deutéré, un *n*-butyle deutéré, un isobutyle deutéré, un *tert-*butyle deutéré, un adamantyle deutéré, un norbornyle deutéré, un méthyl substitué au fluor, un éthyle substitué au fluor, isopropyle substitué au fluor, un *tert*-butyle substitué au fluor, un adamantyle substitué au méthyle ou un norbornyle substitué au méthyle ou sélectionné à partir d'un groupe représenté par la Formule III, et lorsque deux Ra ou plus sont présents, deux Ra adjacents peuvent être combinés pour former un cycle benzénique substitué ou non substitué.

6. Composé hétérocyclique selon la revendication 1, dans lequel la Formule III est sélectionnée à partir de l'un quelconque des groupes suivants :

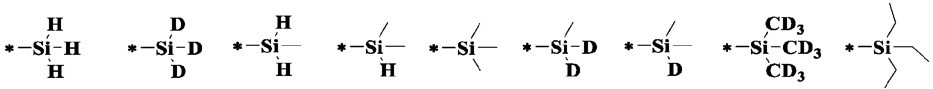

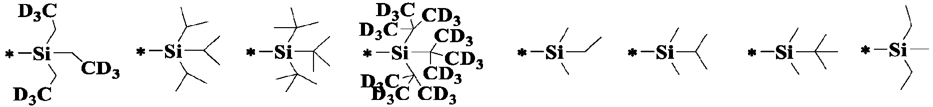

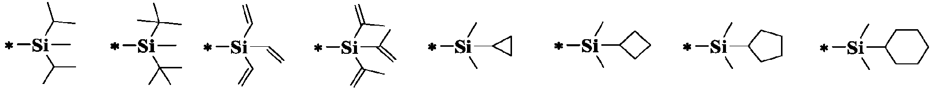

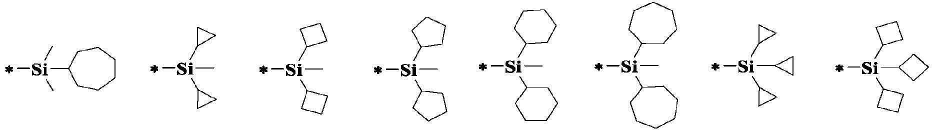

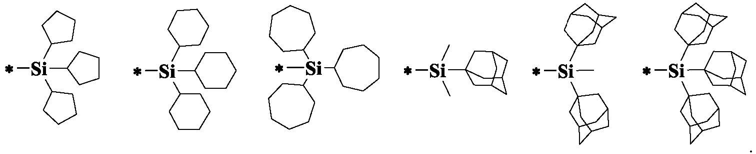

7. Composé hétérocyclique selon la revendication 1, dans lequel L₁ et L₂ sont sélectionnés de façon indépendante à partir d'un élément quelconque parmi une liaison simple ou le groupe suivant : 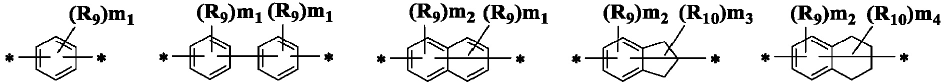 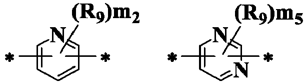
dans lequel R₉ est sélectionné de façon indépendante à partir d'un élément quelconque parmi un hydrogène, un deutérium, un cyano, un nitro, un halogène, un alkyle C1 à C12 substitué ou non substitué, un alcoxy C1 à C12 substitué ou non substitué, un cycloalkyle C3 à C12 substitué ou non substitué, un silyle substitué ou non substitué, un hétérocycloalkyle C2 à C12 substitué ou non substitué, un aryle C6 à C30 substitué ou non substitué ou un hétéroaryle C2 à C30 substitué ou non substitué ; et
m₁ est sélectionné de façon indépendante à partir de 0, 1, 2, 3 ou 4, m₂ est sélectionné de façon indépendante à partir de 0, 1, 2 ou 3, m₃ est sélectionné de façon indépendante à partir de 0, 1, 2, 3, 4, 5 ou 6, m₄ est sélectionné de façon indépendante à partir de 0, 1, 2, 3, 4, 5, 6, 7 ou 8, et m₅ est sélectionné de façon indépendante à partir de 0, 1 ou 2.

8. Composé hétérocyclique selon la revendication 1, dans lequel le composé hétérocyclique est sélectionné de l'une quelconque des structures suivantes :

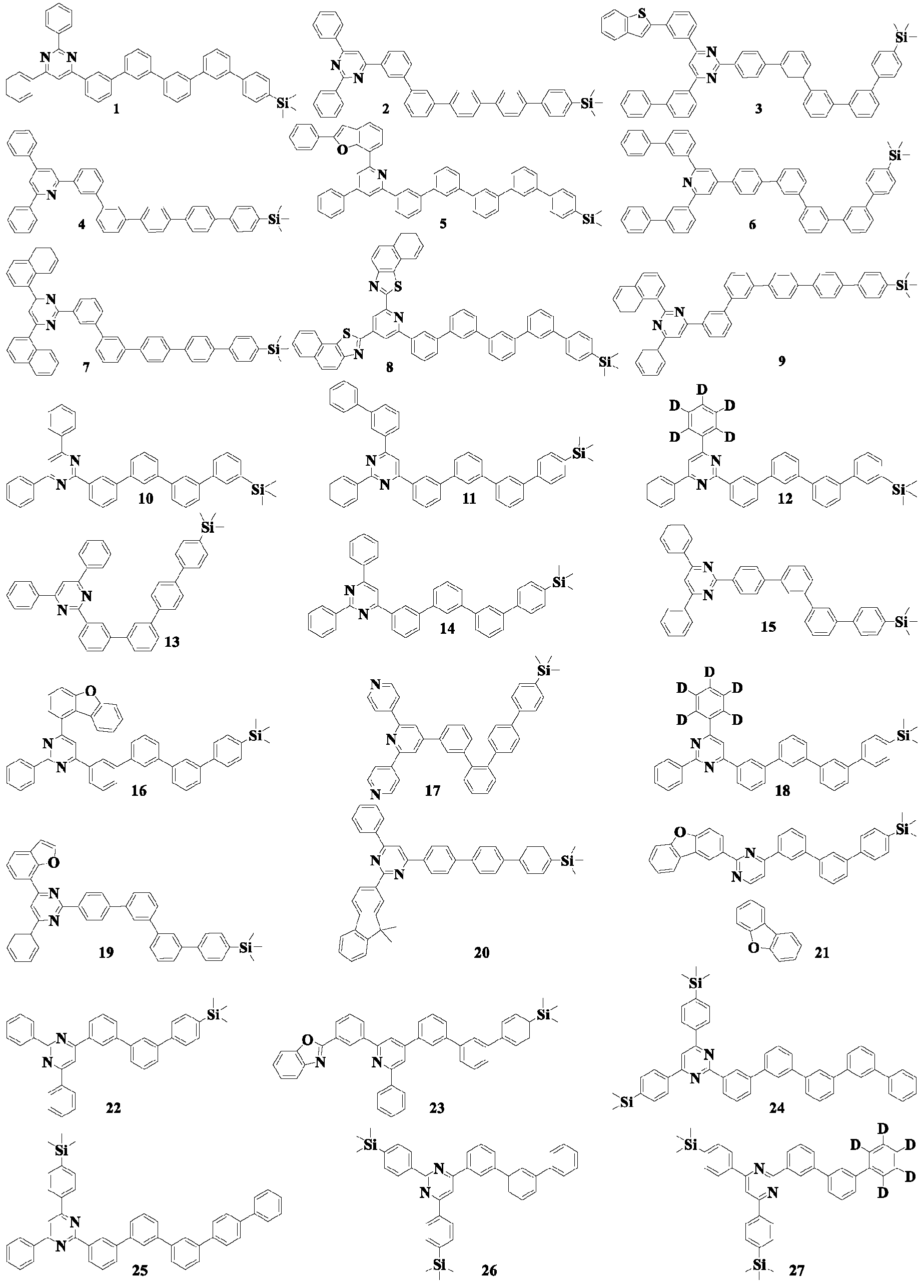

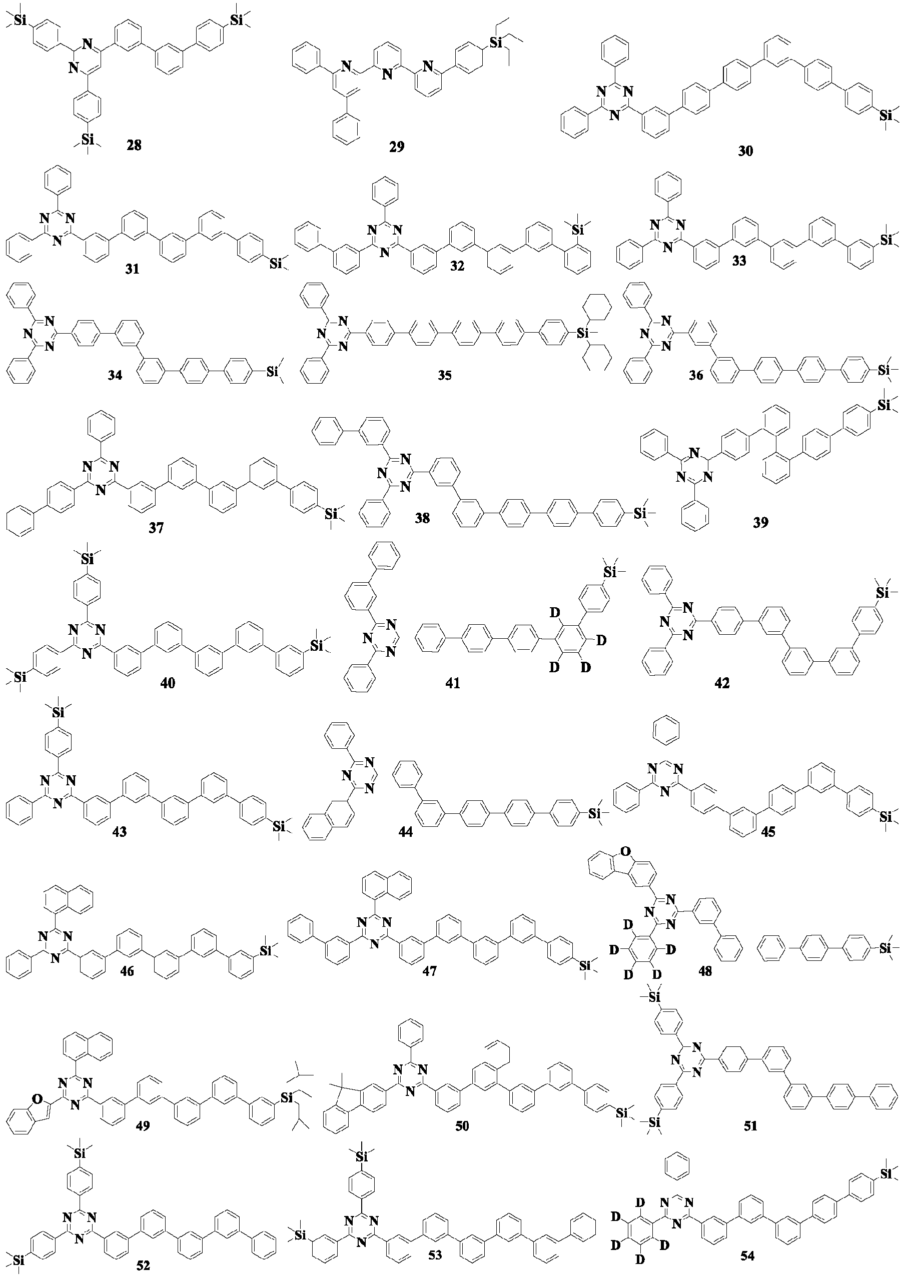

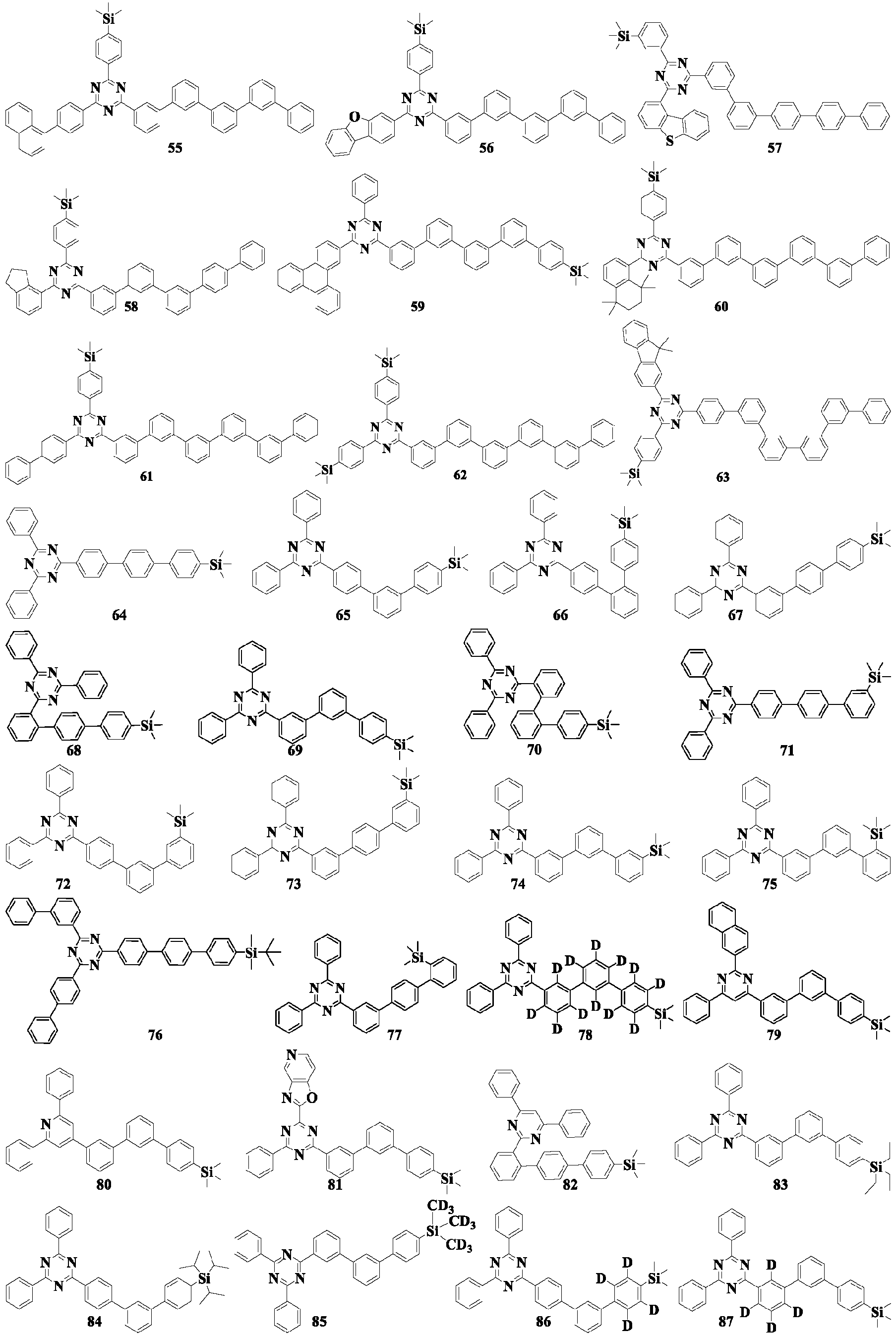

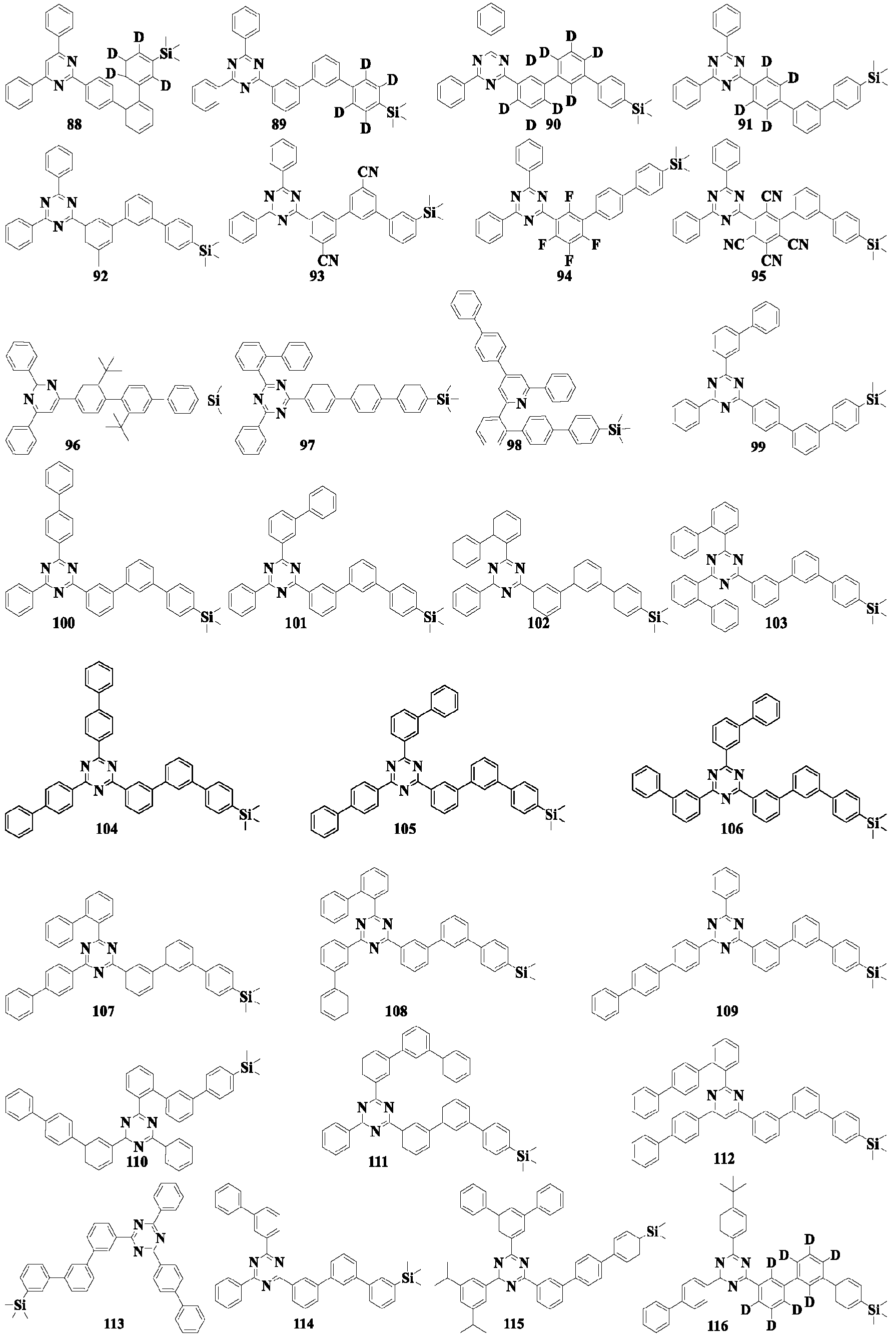

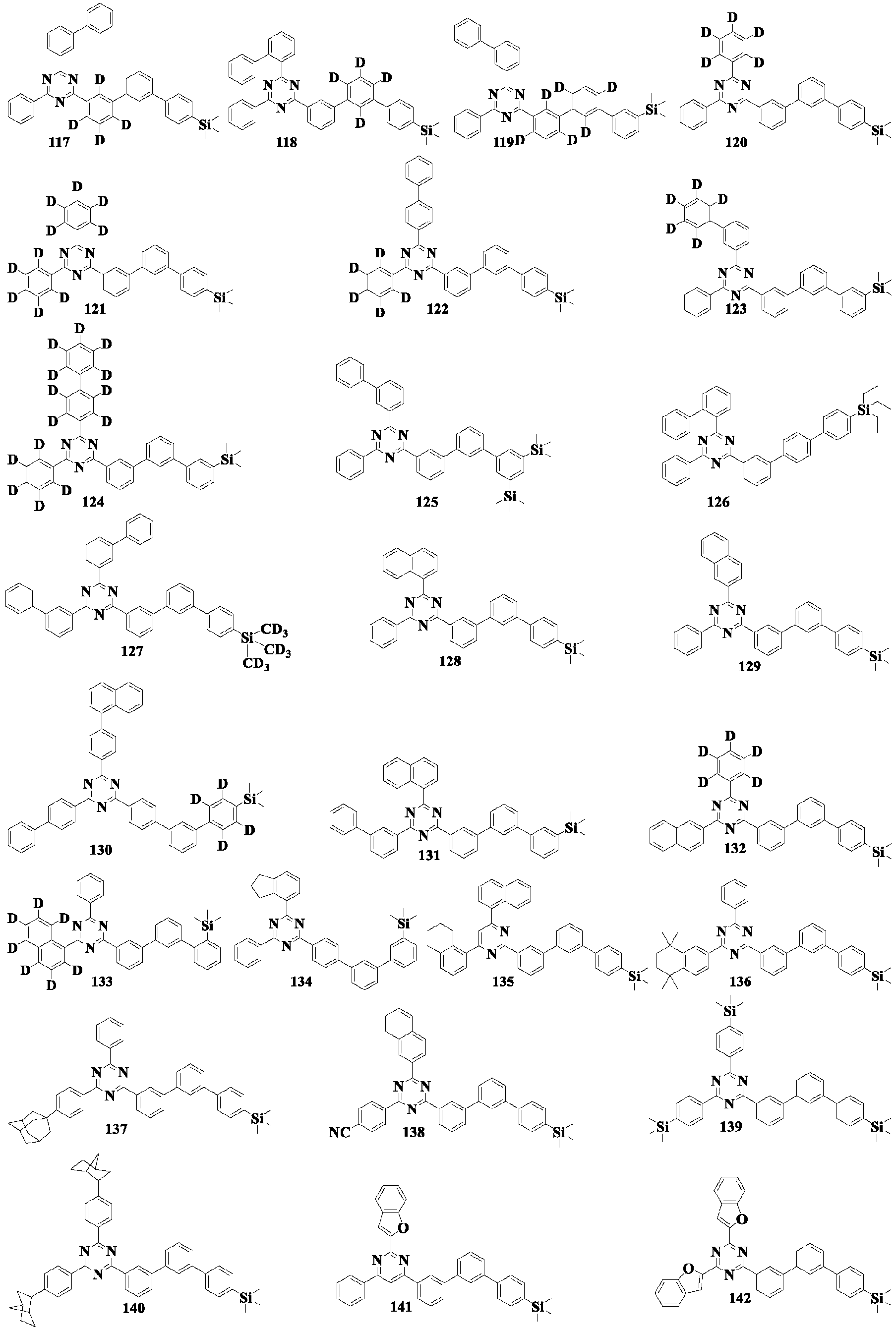

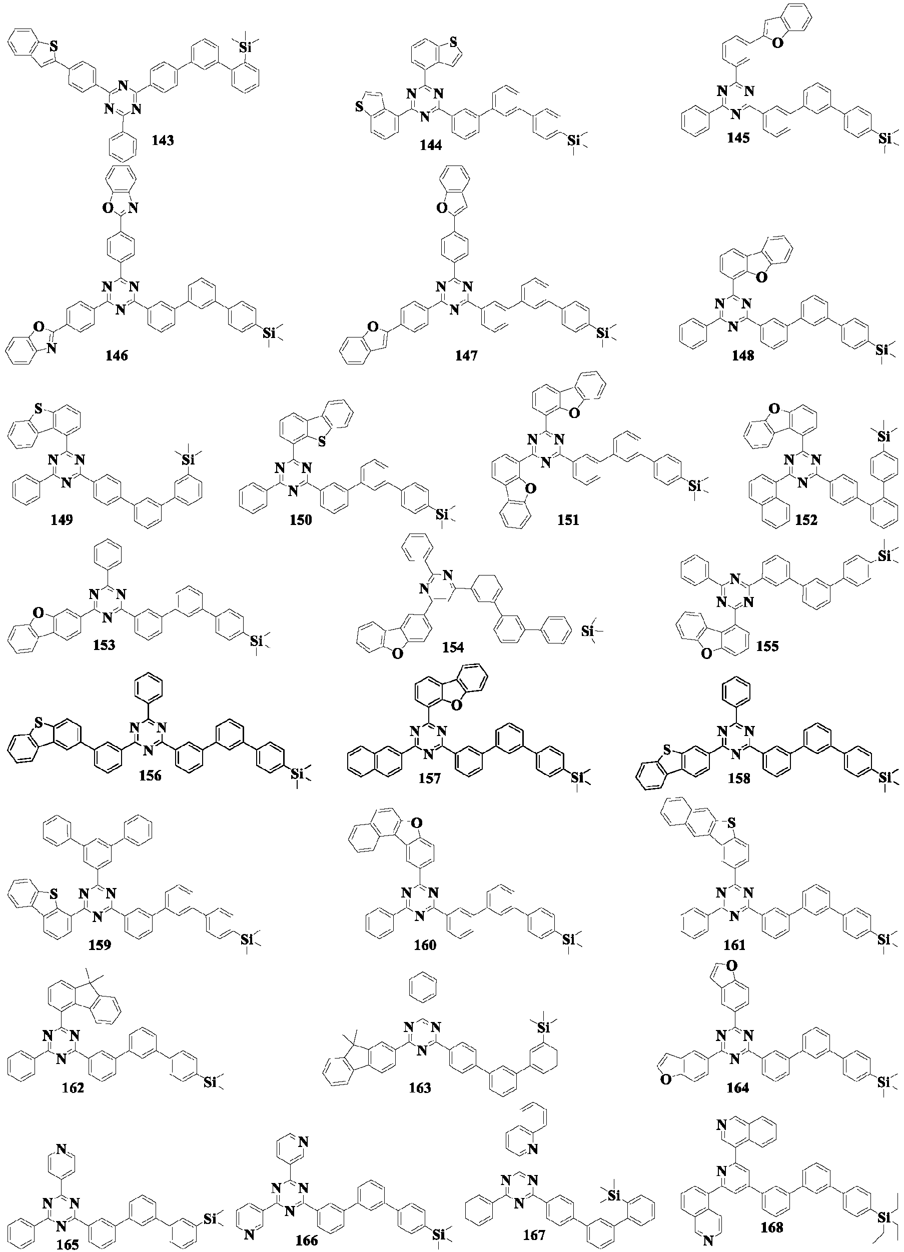

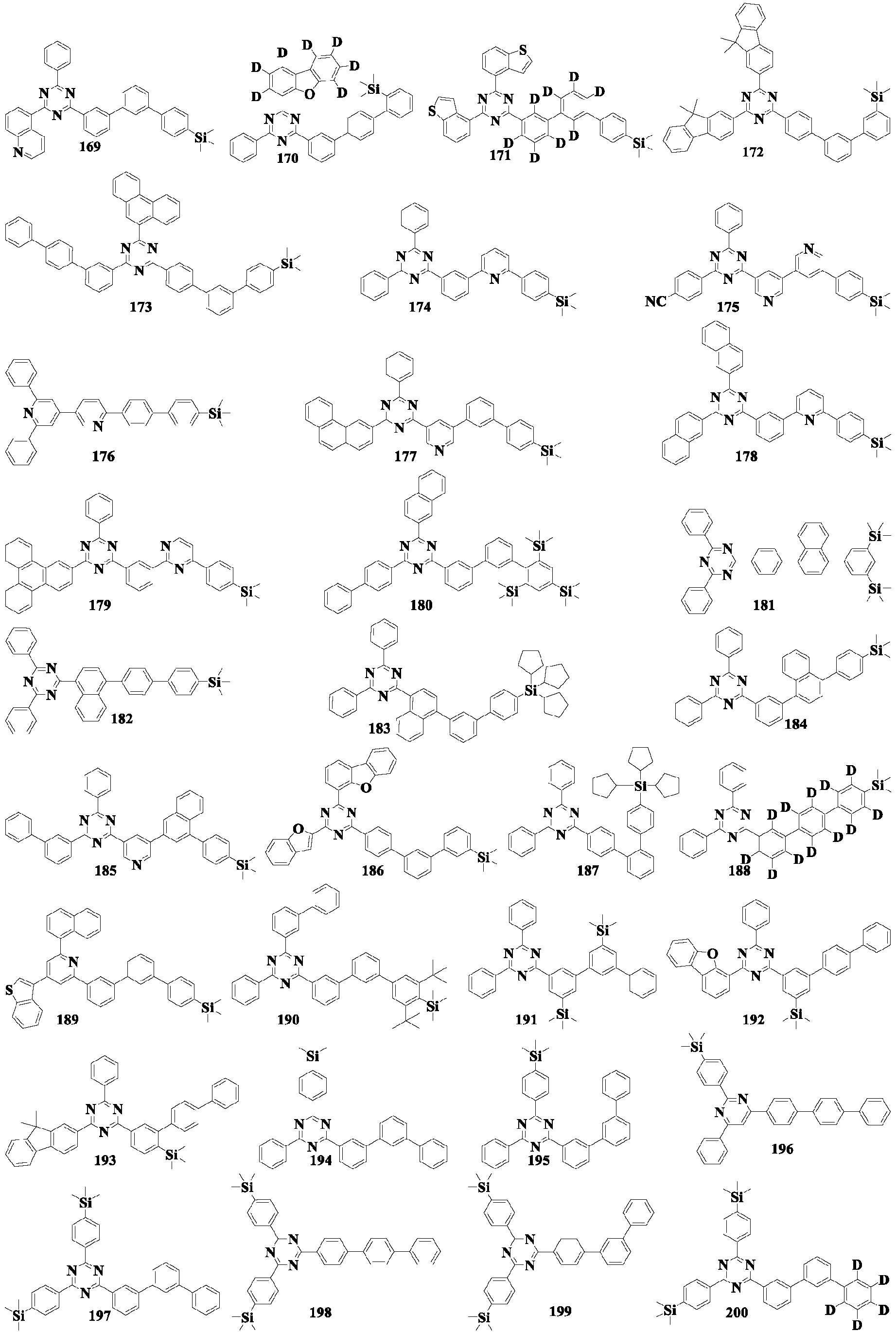

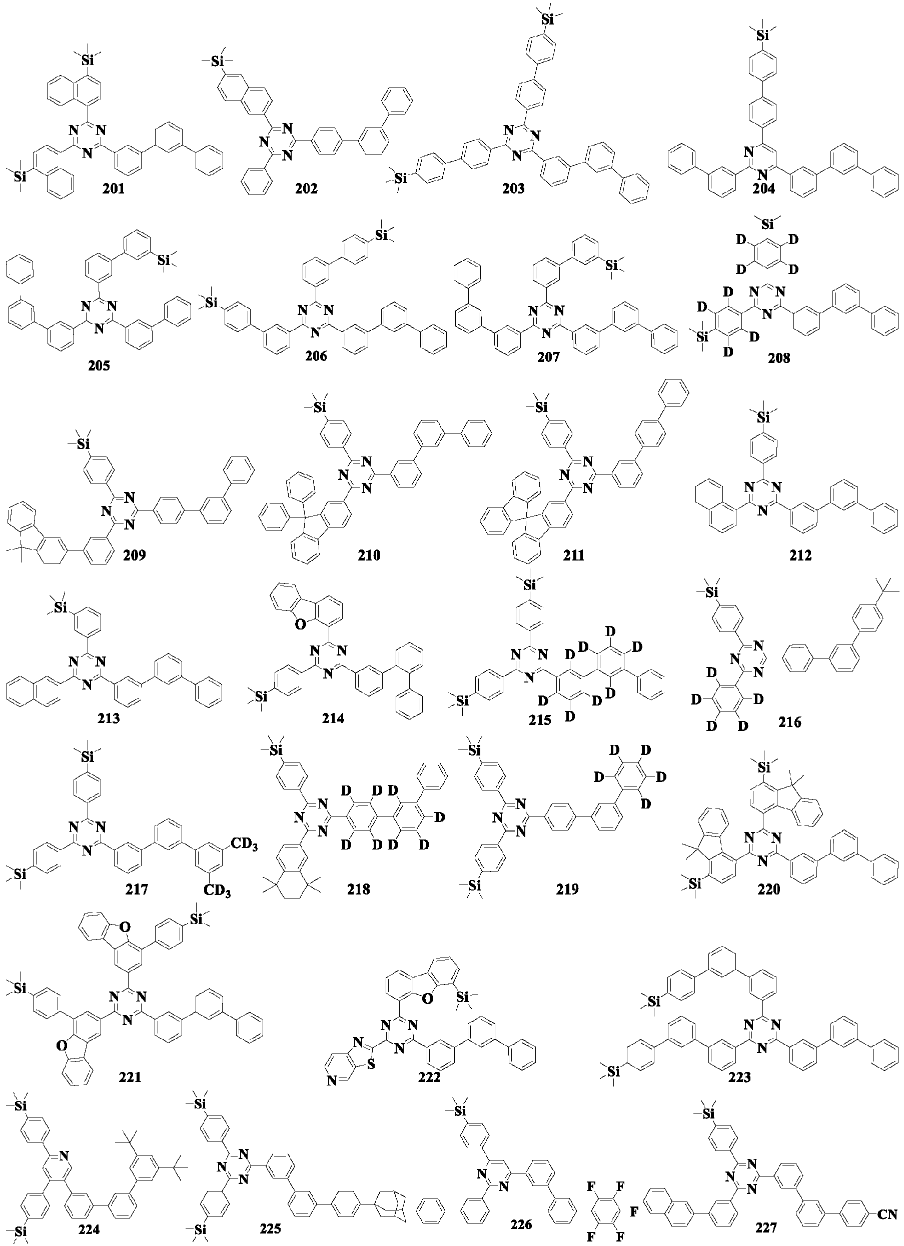

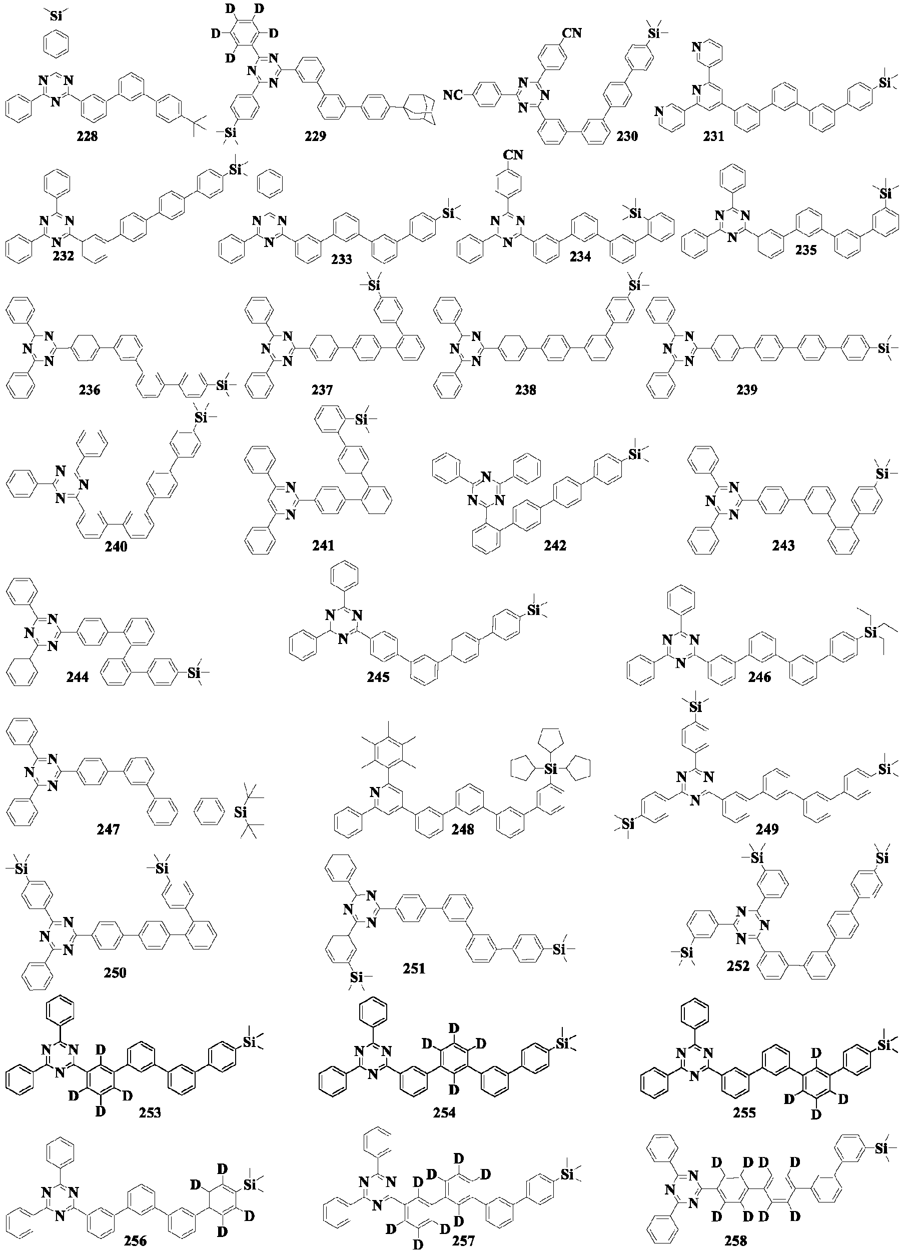

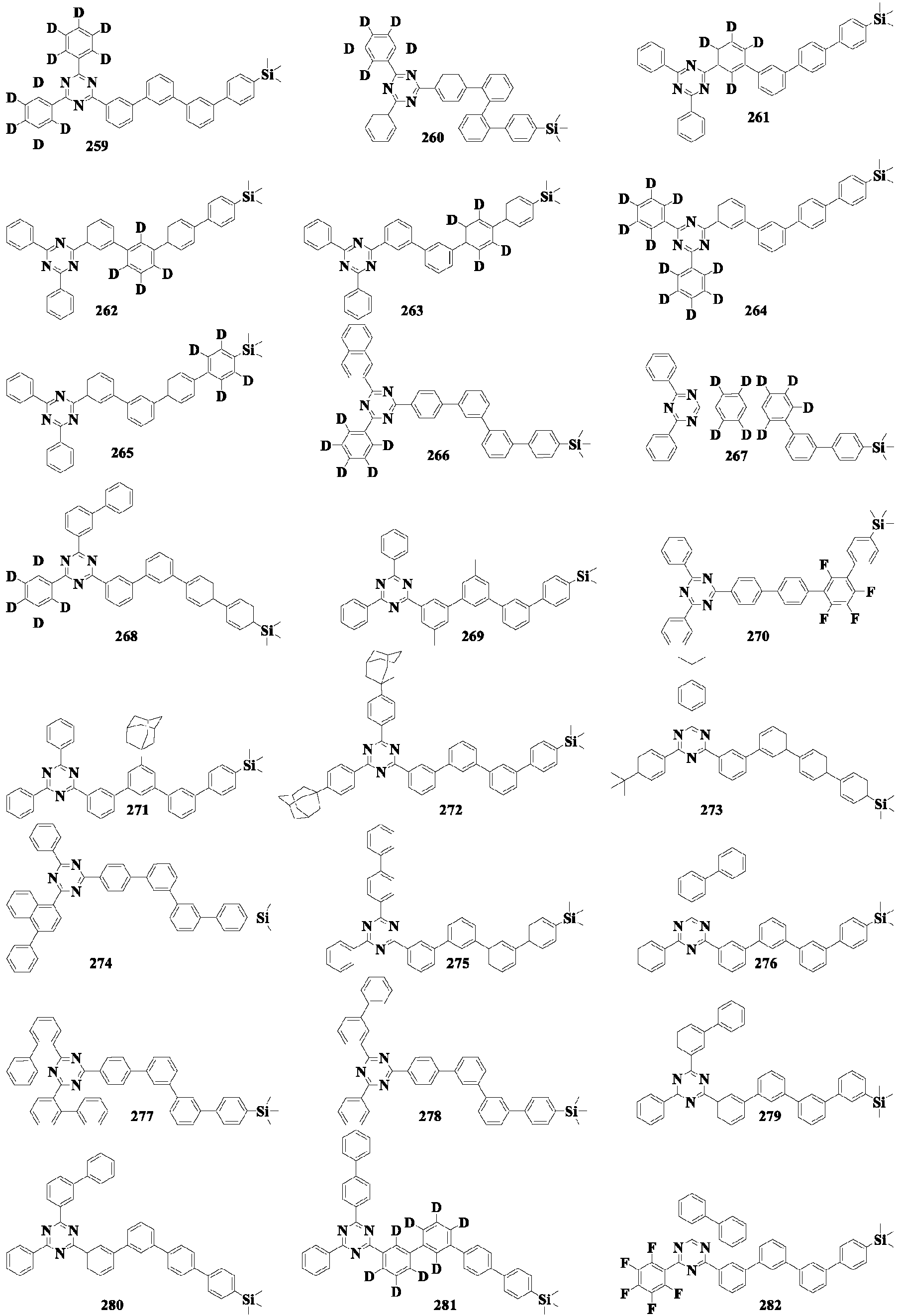

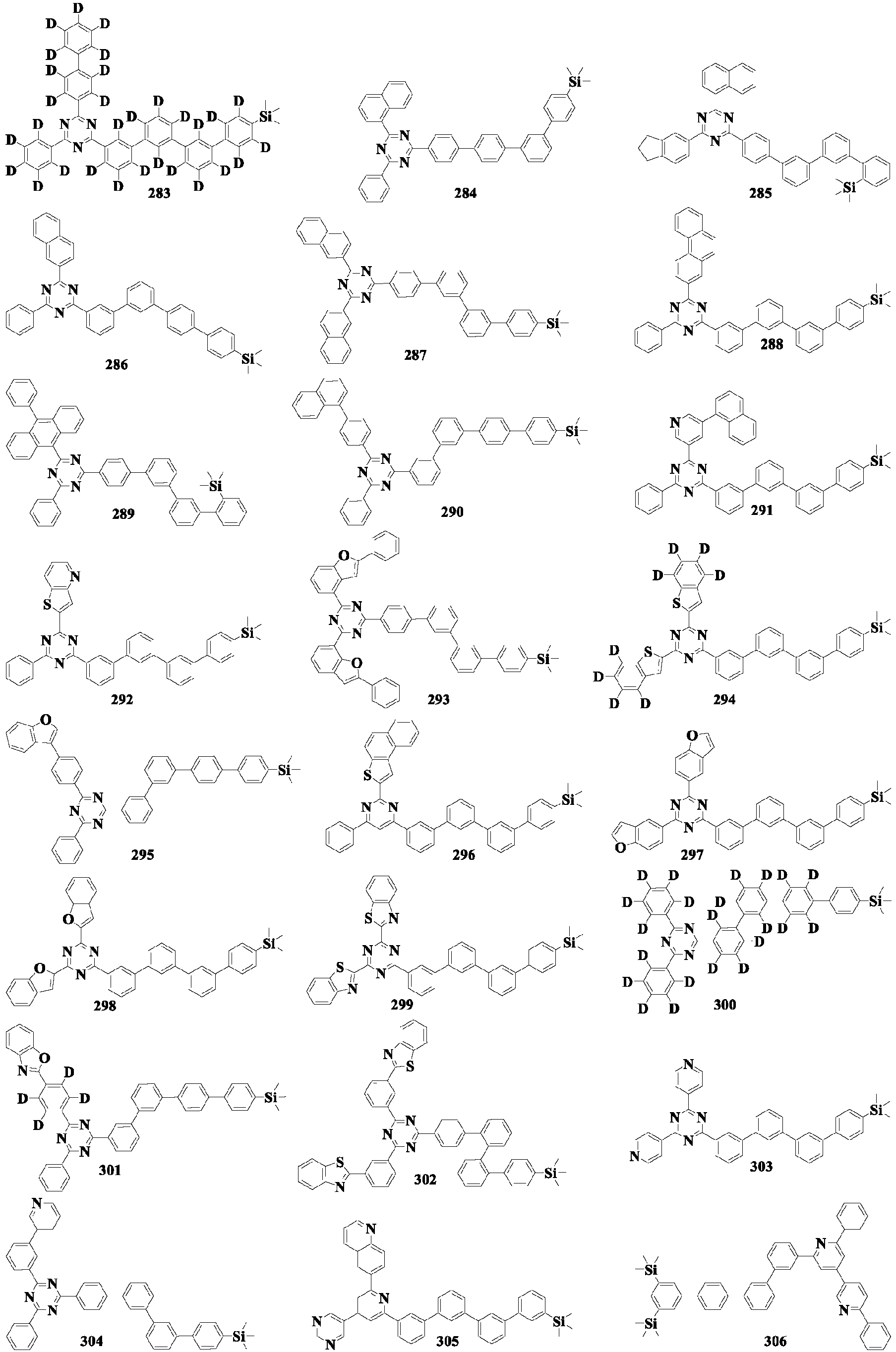

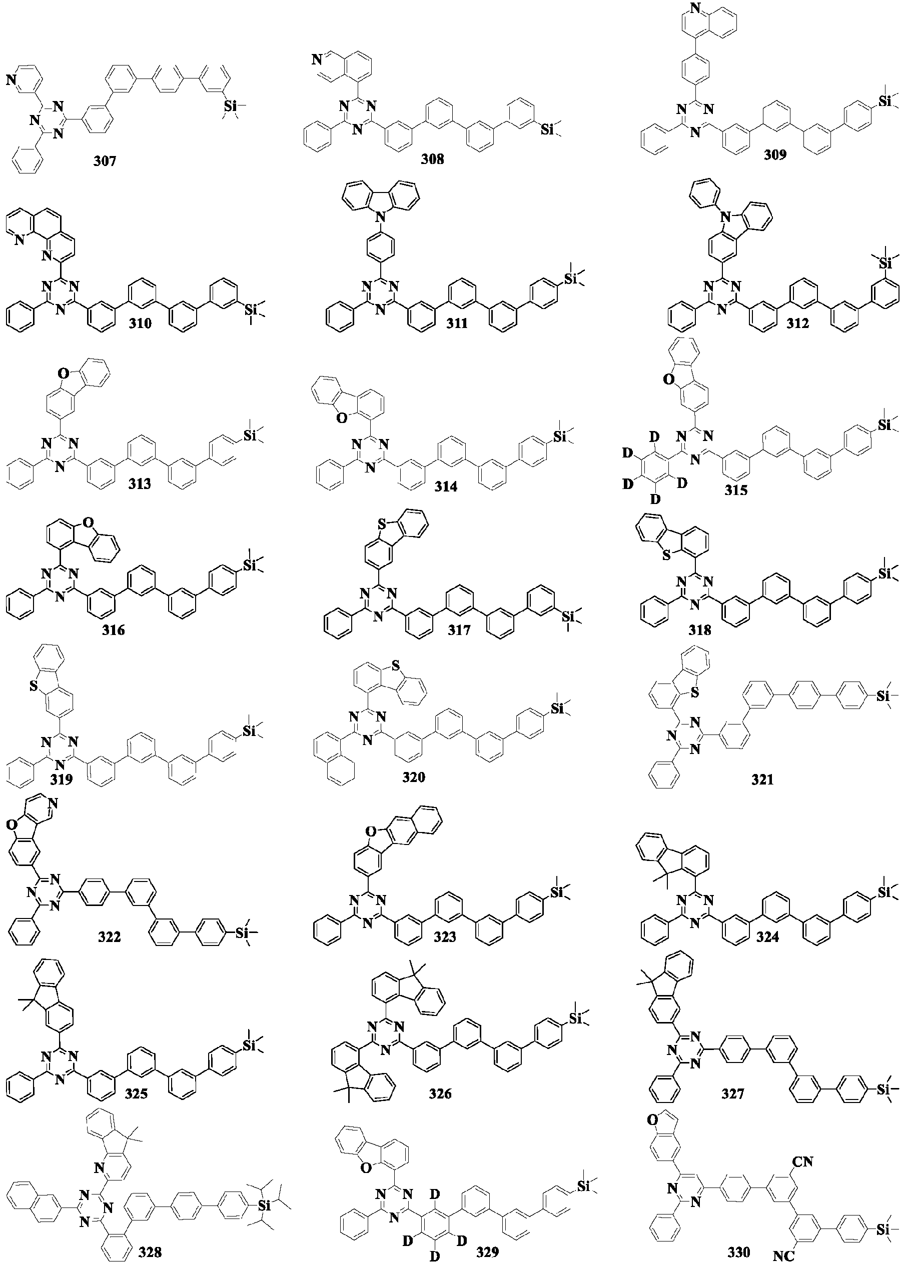

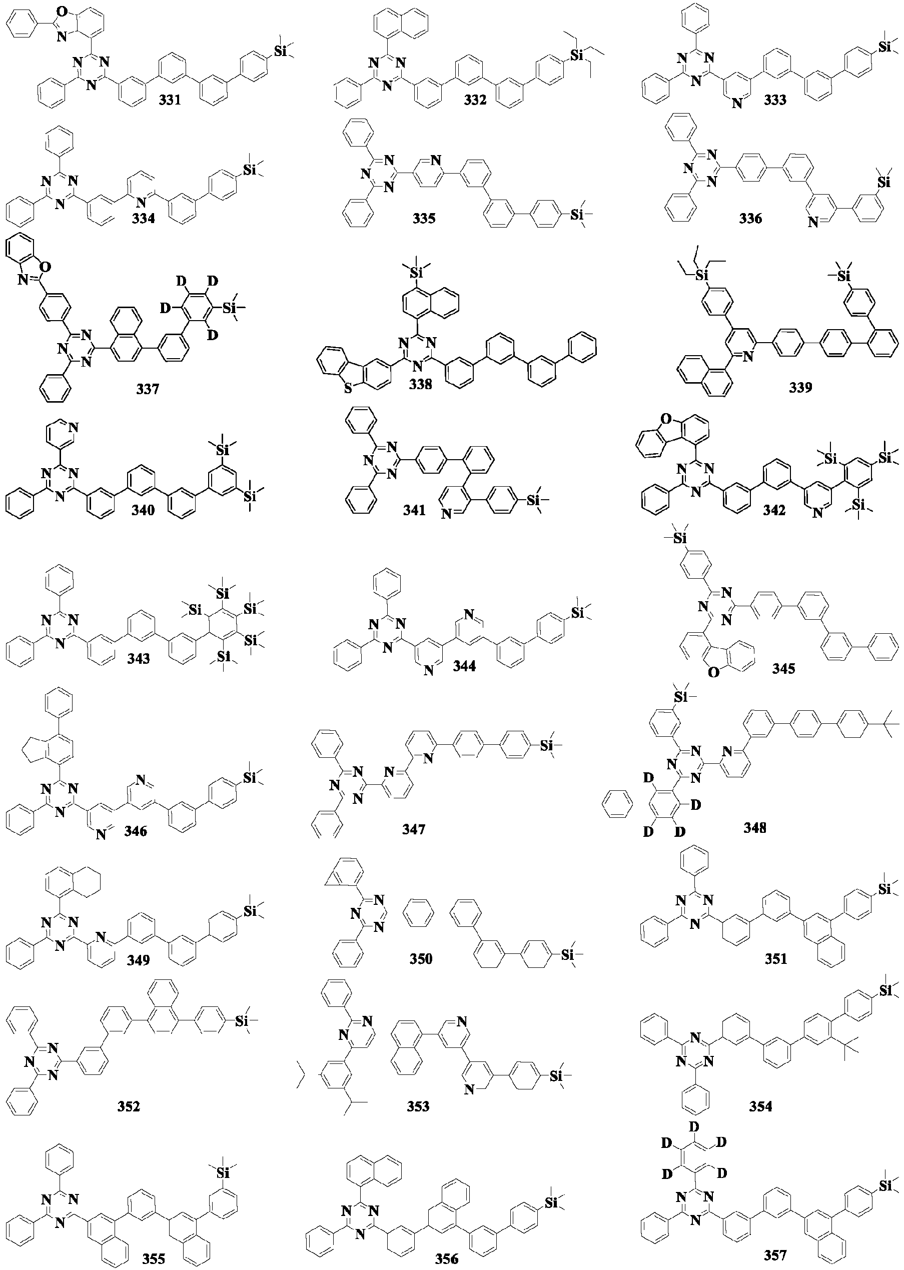

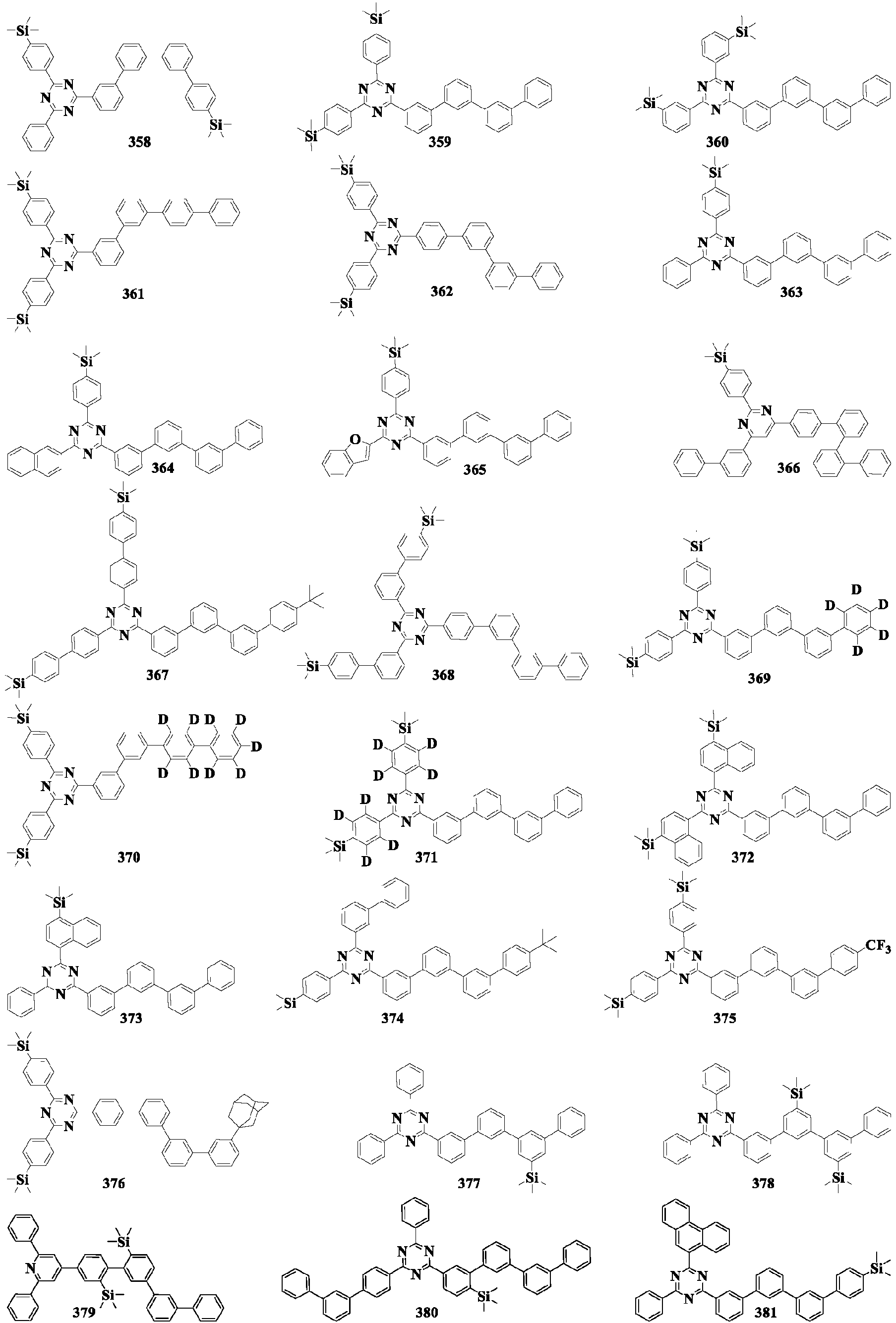

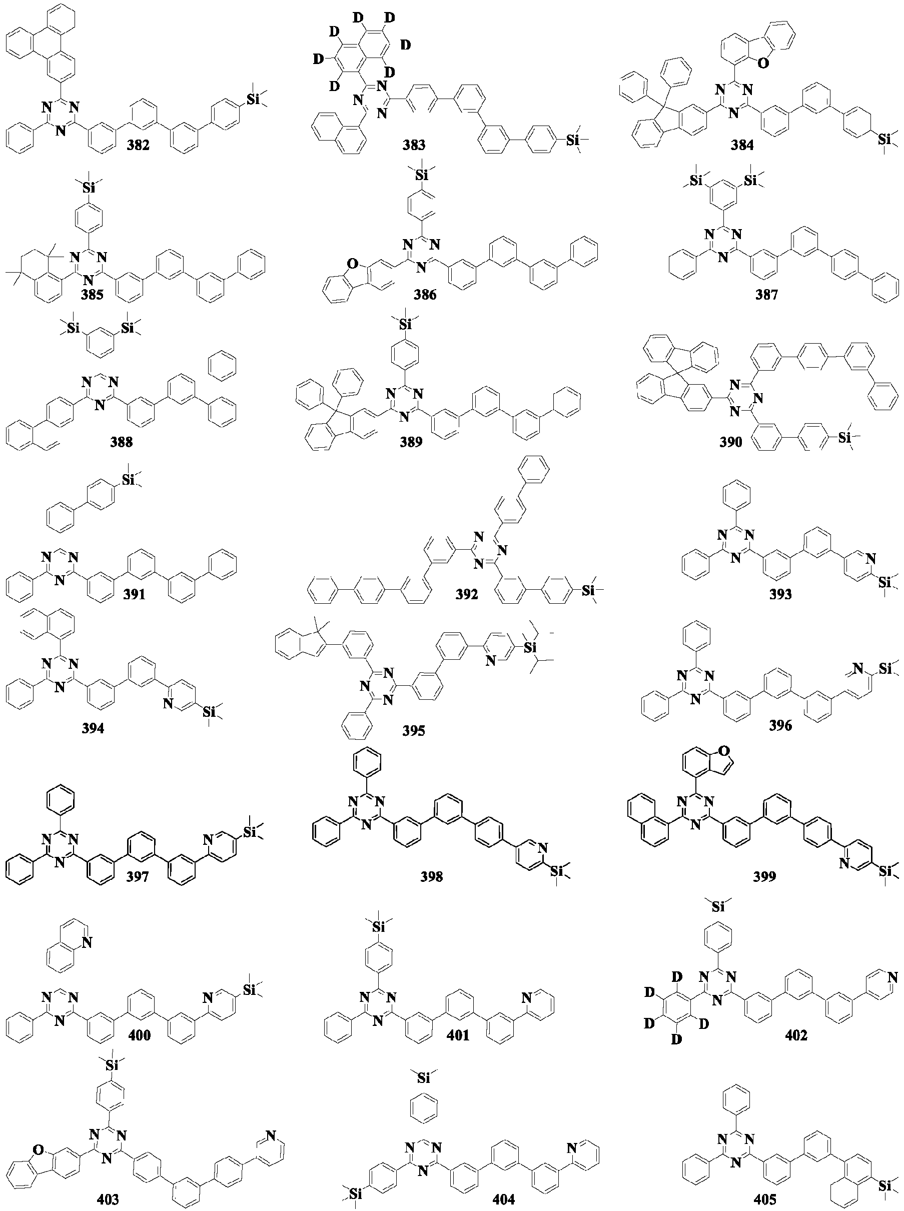

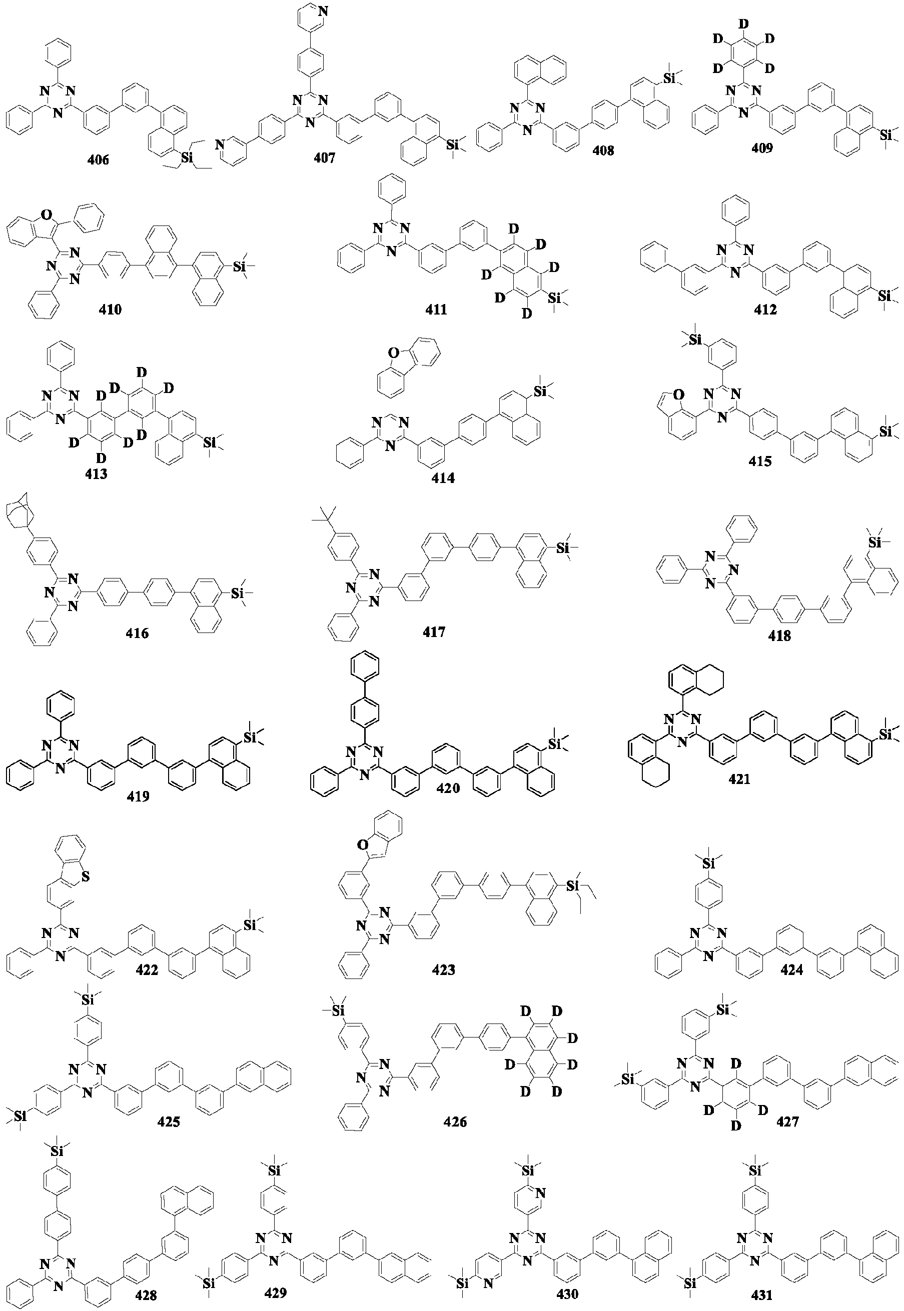

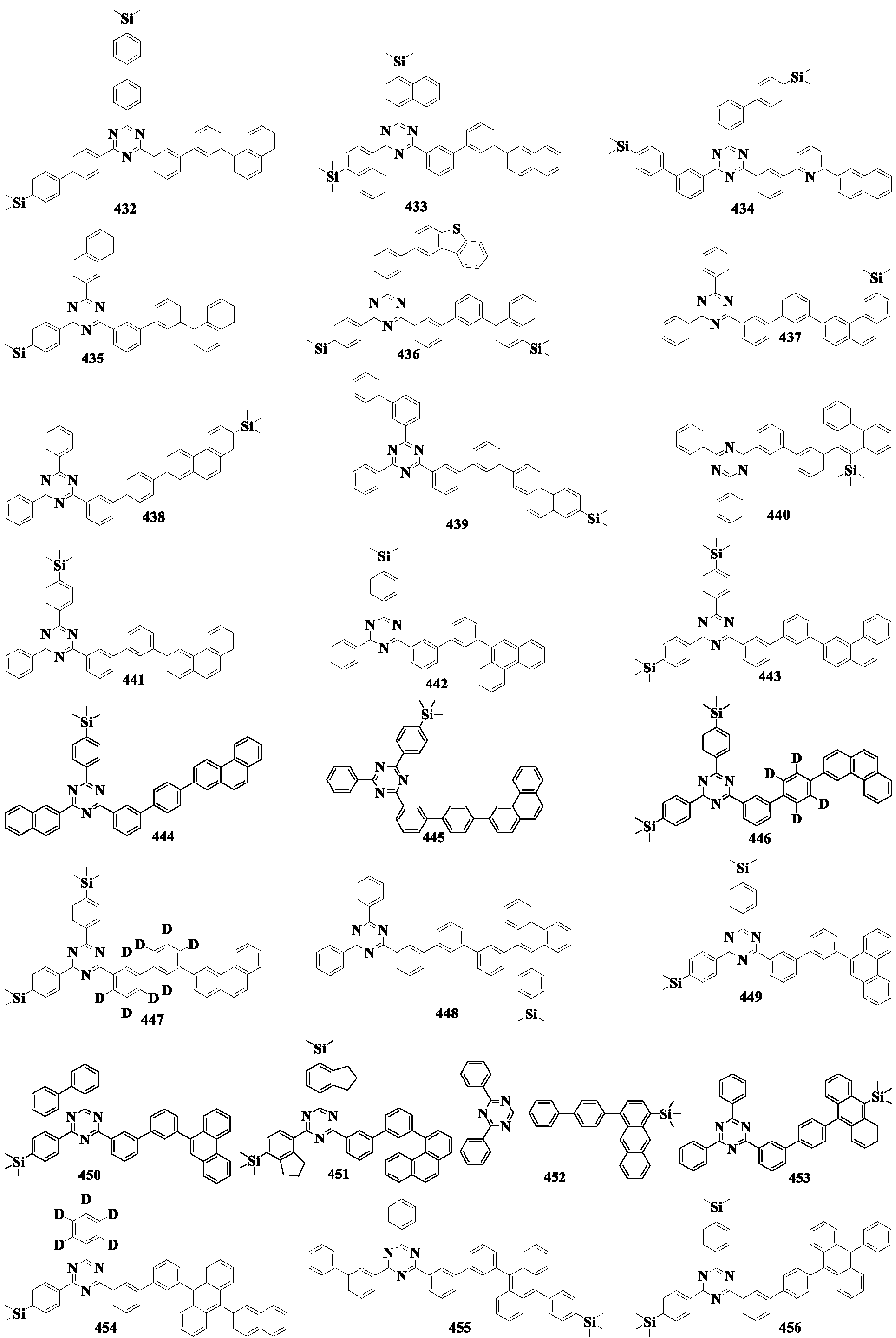

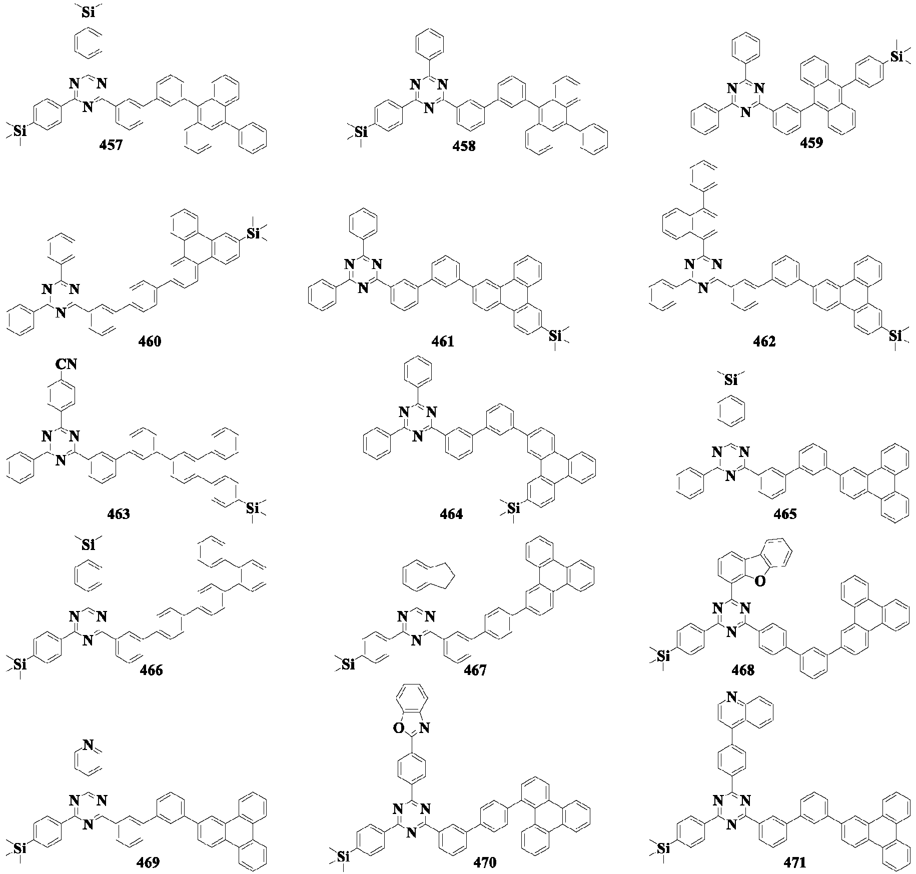

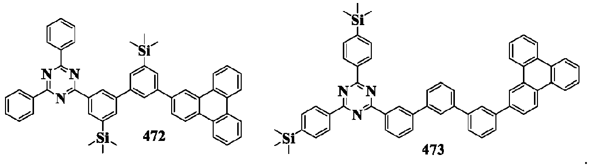

9. Dispositif électroluminescent organique, comprenant une anode, une cathode faisant face à l'anode et une couche organique située entre l'anode et la cathode ou à l'extérieur d'au moins une électrode de l'anode ou de la cathode, dans lequel la couche organique comprend un ou plusieurs composés hétérocycliques quelconques selon l'une quelconque des revendications 1 à 8.

10. Dispositif électroluminescent organique selon la revendication 9, dans lequel la couche organique est située entre l'anode et la cathode et comprend au moins un élément parmi une couche électroluminescente, une couche de transport d'électron ou une couche de blocage de trou, dans lequel la au moins une couche électroluminescente, couche de transport d'électron ou couche de blocage de trou comprend un ou plusieurs composés hétérocycliques quelconques selon l'une quelconque des revendications 1 à 8.
